(19) **Europäisches Patentamt / European Patent Office / Office européen des brevets**

(11) **EP 1 741 385 B1**

(12) **EUROPEAN PATENT SPECIFICATION**

(45) Date of publication and mention
of the grant of the patent:
**22.10.2008 Bulletin 2008/43**

(51) Int Cl.:
**A61B 5/053** (2006.01)

(21) Application number: **06013262.8**

(22) Date of filing: **27.06.2006**

(54) **Truncal visceral/subcutaneous fat measuring method and apparatus**

Viszerales/subkutanes Fettmessverfahren am Rumpf und Gerät dafür

Procédé et dispositif pour mesurer la graisse viscérale/ subcutanée du tronc

(84) Designated Contracting States:
**DE FR GB**

(30) Priority: **07.07.2005 JP 2005199169**
**11.07.2005 JP 2005201193**
**28.10.2005 JP 2005314098**

(43) Date of publication of application:
**10.01.2007 Bulletin 2007/02**

(73) Proprietor: **TANITA CORPORATION**
**Tokyo (JP)**

(72) Inventors:
• **Masuo, Yoshihisa**
**Otsu-shi**
**Shiga (JP)**

• **Kasahara, Yasuhiro**
**Tokyo (JP)**

(74) Representative: **Grünecker, Kinkeldey,**
**Stockmair & Schwanhäusser**
**Anwaltssozietät**
**Leopoldstrasse 4**
**80802 München (DE)**

(56) References cited:
**EP-A- 1 095 614       WO-A-02/24069**
**US-A1- 2004 059 242**

**Description**

The present invention relates to a truncal visceral/subcutaneous fat measuring method and an apparatus for performing said method.

A technique for estimating body fat tissues by use of a bioelectrical impedance has been spread as a technique for measuring body fat tissues and a body fat percentage. In reality, however, it does not measure fat tissues directly but electrically measures fat free tissues other than the fat tissues in which water is dominant. In particular, in whole body measurement, a conventional type (between-one-hand-and-one-foot lead system) models a body part between one hand and one foot in a supine position by one cylinder. As simple techniques, a between-palms lead system which makes a measurement in a standing position, a between-soles lead system integrated with a scale, and a technique for measuring an impedance by dividing a body into the upper limb and the lower limb, the upper limb, the lower limb and the trunk or five segments, e.g. the left and right upper extremities, the left and right lower extremities and the trunk, and applying a cylindrical model to each segment have been increasing popular. Further, a simplified impedance measurement technique comprising disposing current applying electrodes and voltage measuring electrodes around the navel of the trunk and measuring the impedance of the abdomen to estimate a visceral fat tissue amount has been applied for patent (refer to Patent Literatures 1 and 2).
Patent Literature 1
Japanese Patent Application No. 3,396,677
Patent Literature 2
Japanese Patent Application No. 3,396,674

However, the usefulness of information of body fat for screening lifestyle-related diseases such as diabetes, hypertension and hyperlipemia has been receiving particular attention, and the significance of measurement of visceral fat tissues and subcutaneous fat tissues adhered or accumulated around splanchnic organ tissues has been increasing day by day.

In particular, visceral fat tissues are fat tissues distributed around the abdomen of the trunk in a concentrated manner and have been determined by an image of a cross-sectional area of the fat tissues of the abdomen by X-ray CT or MRI. However, this requires a large-scale apparatus and has problems of X-ray exposure and high costs and is therefore not suited for measurement in the field and household. Consequently, the visceral fat tissues are generally estimated from correlation with the fat tissues of the whole body or correlation with the fat free tissues of the whole body and cannot secure adequate reliability for screening.

Recently, a method comprising disposing electrodes around the navel of the trunk and measuring the internal impedance of the trunk to estimate visceral fat tissue information has also been under development. However, this method is based on significant correlation existing among a skeletal muscle tissue layer, a subcutaneous fat tissue layer and visceral fat tissues and based on the premise that if information of any of these tissues can be acquired, the information can be roughly estimated. Therefore, while good results can be expected for highly independent, healthy subjects who can have highly significant correlation among the tissues, significant errors may be contained in measurement results for subjects having different correlations among the tissues, e.g. subjects having significantly enlarged visceral fat tissues and having significantly low correlation between the subcutaneous fat tissue layer or skeletal muscle tissue layer and the visceral fat tissues. That is, even this method under development has a significant problem in measurement on paralyzed patients and patients who need nursing care, particularly patients confined to bed, although the method can somehow make the measurement on healthy subjects capable of leading independent life regardless of where on the whole circumference of the navel the electrodes are disposed.

Further, this method under development is said to be a sophisticated technique in that it passes a current through a tissue part to be measured from the surface of the abdomen to acquire an impedance value associated with the internal tissue. However, it is an actual situation that measured impedance information itself has hardly useful sensitivity to visceral fat tissues due to the problem of the internal structure of the trunk which is a body part to be measured. That is, the trunk which is a body part to be measured is thick and short and has a multiple structure, i.e. a structure in which the visceral fat tissues to be measured together with splanchnic organ tissues and spinal tissues are covered with a skeletal muscle tissue layer showing very good electrical conductivity and the skeletal muscle tissue layer is covered with a subcutaneous fat tissue layer showing very poor electrical conductivity. In particular, around the visceral fat tissues to be measured, splanchnic organ tissues showing lower electrical conductivity than the skeletal muscle tissue layer and visceral fat tissues of poor electrical conductivity which are adhered and accumulated to the splanchnic organ tissues are dominant and constitute a complicated structure, resulting in very poor electrical conductivity of the tissues under the skeletal muscle tissue layer is very poor. For this reason, even if current applying electrodes are simply disposed around the abdomen, most of current passes through the skeletal muscle tissue layer, and current density distribution is observed from surface measuring electrodes as electrical potential distribution dominated by the skeletal muscle tissue layer. Further, applied current density distribution is determined from the surface area of the current applying electrode or the width of the electrode in the abdominal circumferential direction, and observation of information in a spreading resistance region showing high current density in the subcutaneous fat tissue layer right underneath the electrodes.

Further, since the trunk which is a body part to be measured is thick and short, sensitivity in the subcutaneous fat tissue layer in the current density concentrating (spreading resistance) region right underneath the current applying electrodes becomes high. Further, since the skeletal muscle tissues have very high electrical conductivity as compared with the fat tissues, most of current having passed through the subcutaneous fat tissue layer returns to the opposing current applying electrode via the skeletal muscle tissue layer and the subcutaneous fat tissue layer, and as a result, electrical potential distribution in the tissues under the skeletal muscle tissue layer is significantly distorted by the skeletal muscle tissue layer. Thus, in the conventional method, most of electrical potential measured is information of the subcutaneous fat tissue layer, energization of the visceral fat tissues to be measured, i.e. the splanchnic organ tissues and the visceral fat tissues adhered and accumulated therearound can be hardly expected, and only information with a very low measurement sensitivity of not higher than 10% of all impedance measurement section can be acquired.

To avoid these problems, a method comprising incorporating abdominal circumferential length having high correlation with a subcutaneous fat tissue layer area into an estimation expression to prevent an increase in estimation error is conceived. However, this method is merely indirect estimation by correlation between constituent tissues and is hardly called a measurement method having energization sensitivity required in the middle of the abdomen. That is, individual errors deviated from statistical correlation design cannot be assured, and particularly when the amount of the subcutaneous or visceral fat tissues is abnormally large or the intermediate skeletal muscle tissue layer is large or small, a significant error may occur. The subcutaneous fat tissue layer area has high correlation with the abdominal circumferential length, because the trunk of human being is concentric tissue arrangement design, the subcutaneous fat tissue layer is the outermost layer and its area is determined by outer circumferential length and subcutaneous fat tissue thickness.

To dispose electrodes on the trunk, a four-electrode technique is generally used. This method passes a current through the body of a subject and measures a potential difference which has occurred in a body part to be measured of the subject by the applied current so as to measure a bioelectrical impedance in the measured body part. When the four-electrode technique is applied to a thick and short body part to be measured such as the trunk, a current density concentrating region (or a spreading resistance region) when a current has just started to spread is, for example, right underneath current applying electrodes, so that a large potential difference occurs in the vicinity of the subcutaneous fat tissue layer and constitutes most of potential difference measured between voltage measuring electrodes. To reduce the influence by the spreading resistance, it is important to dispose the current applying electrodes and the voltage measuring electrodes with sufficient distance secured therebetween. Since general measurement is carried out under conditions which can secure a long measurement section and sufficient distance between the voltage measuring electrodes, so-called S/N sensitivity (N is the influence (noise) by the spreading resistance, and S is a signal measured between the voltage electrodes) should be secured sufficiently. However, in the case of a thick and short body part to be measured such as the trunk, when the voltage measuring electrodes are moved away to secure distance from the current applying electrodes so as to render N small, the distance between the voltage measuring electrodes becomes small. As a result, S becomes small, resulting in deterioration in S/N. Further, the spreading resistance portion showing high current density is a subcutaneous fat tissue layer portion and subjects liable to become obese with thick fat are common, so that N becomes quite large and S/N further deteriorates. Thus, when the four-electrode technique is applied to a thick and short body part to be measured such as the trunk, it is expected to be quite impossible to secure useful S/N sensitivity to visceral fat tissues merely by disposing the electrodes around the navel. S/N will be further described in detail in descriptions about Examples to be described later.

Thus, in the case of a lead system in which all of four electrodes are disposed on the abdomen to be measured, it is conceived that current applying electrodes are disposed in conductive layer window regions such as aponeuroses off the skeletal muscle tissue layer or a limitation is placed on electrode arrangement so as to use the skeletal muscle tissue layer as a conductive layer. However, in this case, an error in the electrode arrangement causes a severe influence on measurement accuracy.

Further, although the above limitation has been alleviated by shifting the positions of the electrodes to four extremities other than the abdomen to be measured as much as possible (lead system which conceives four extremities as substitutes for leads from the trunk by placing the electrodes on body parts protruding from the trunk such as the four extremities), a body part to be measured on the abdomen cannot be selected fully freely due to limitation on the arrangement structure on the four extremities protruding from the trunk.

WO 02/24069 A1 discloses an apparatus for determination of a body composition of a person, wherein said apparatus comprises several electrode pads with respective electrodes. Via leads, said electrode pads are connected to a switching unit which connects a current source between a pair of current-carrying electrodes and a detector between a pair of measuring electrodes. One electrode pad is applied to the trunk of the person, another electrode pad is applied to the wrist and a further electrode pad is applied to the ankle.

It is an objective of the present invention to provide a visceral/subcutaneous fat measuring method and an apparatus capable of measuring information of the subcutaneous fat tissue layer in the trunk with high accuracy.

According to the present invention, said objective is solved by a truncal visceral/subcutaneous fat measuring method according to claim 1 and a truncal visceral/subcutaneous fat measuring apparatus according to claim 5.

Preferred embodiments of the present invention are laid down in the subclaims.

Embodiment According to the First Characteristic of the Invention

According to one embodiment of the present invention, the body part protruding from the trunk may be an upper extremity, a lower extremity or the head. For example, the body part may be an ear of the head.

According to another embodiment of the present invention, the subcutaneous fat tissue layer information of the trunk may be acquired by measuring the impedance of the trunk with one of the voltage measuring electrodes placed in the vicinity of the one current applying electrode and the other voltage measuring electrode placed at a position remote from the one current applying electrode in the trunk longitudinal direction.

According to still another embodiment of the present invention, the measurement may be made with the one current applying electrode and the one voltage measuring electrode placed at any of the following regions, i.e. near the navel or at the lateral region or the dorsolateral region.

According to still another embodiment of the present invention, the subcutaneous fat tissue layer information may be acquired by applying a current to a body part where the subcutaneous fat tissue layer is thin or a body part where the skeletal muscle tissue layer has no or a thin muscle belly portion from the one current applying electrode, placing one of the voltage measuring electrodes at a position where the influence of spreading resistance right underneath the one current applying electrode is predominant, placing the other voltage measuring electrode at a remote position where the influence of the spreading resistance is weak, and measuring a potential difference between the voltage measuring electrodes.

According to still another embodiment of the present invention, the other voltage measuring electrode may measure the potential difference in a body part where the subcutaneous fat tissue layer is thin or a body part where the skeletal muscle tissue layer has no or a thin muscle belly portion.

According to still another embodiment of the present invention, subcutaneous fat tissue layer information and visceral fat tissue information may be acquired selectively by selecting between two pairs of voltage measuring electrodes. More specifically, at least one pair of current applying electrodes and at least two pairs of voltage measuring electrodes are provided; the subcutaneous fat tissue layer information is acquired by applying a current to a body part where the subcutaneous fat tissue layer is thin or a body part where the skeletal muscle tissue layer has no or a thin muscle belly portion from one of the current applying electrodes, placing one voltage measuring electrode in one voltage measuring electrode pair out of the at least two voltage measuring electrode pairs at a position where the influence of spreading resistance right underneath the one current applying electrode is predominant, placing the other voltage measuring electrode at a remote position where the influence of the spreading resistance is weak, and measuring a potential difference between the one voltage measuring electrode and the other voltage measuring electrode; and the visceral fat tissue information is acquired by placing the other voltage measuring electrode pair out of the at least two voltage measuring electrode pairs at a remote position where the influence of the spreading resistance is weak and measuring a potential difference.

According to still another embodiment of the present invention, the other voltage measuring electrode pair may measure the potential difference in a body part where the subcutaneous fat tissue layer is thin or a body part where the skeletal muscle tissue layer has no or a thin muscle belly portion.

According to still another embodiment of the present invention, the position where the influence of the spreading resistance is predominant may be any of the following regions, i.e. near the navel or at the lateral region or the dorsolateral region, and the remote position where the influence of the spreading resistance is weak may be between the navel and the upper border of the iliac crest.

According to still another embodiment of the present invention, the subcutaneous fat tissue layer information may be acquired by placing the one voltage measuring electrode at a position close to the one current applying electrode, placing the other voltage measuring electrode at a sufficiently remote position from the one voltage measuring electrode with respect to the one current applying electrode, and measuring a potential difference between the one voltage measuring electrode and the other voltage measuring electrode.

According to still another embodiment of the present invention, the subcutaneous fat tissue layer information may be acquired by placing the one voltage measuring electrode at a position close to the one current applying electrode, placing the other voltage measuring electrode at a sufficiently remote position from the one voltage measuring electrode placed in the vicinity of the one current applying electrode, and measuring a potential difference between the one voltage measuring electrode and the other voltage measuring electrode.

According to still another embodiment of the present invention, the sufficiently remote position may be at least three times the close position.

According to still another embodiment of the present invention, the visceral fat tissue volume of the trunk may be determined based on the impedance of the trunk which has been determined by use of the potential difference measured by the above at least two voltage measuring electrode pairs and body specifying information.

According to still another embodiment of the present invention, it is also possible that the subcutaneous fat tissue volume of the trunk is determined based on the impedance of the trunk which has been determined by use of the potential difference measured by the above one voltage measuring electrode pair and body specifying information, the impedance of the subcutaneous fat tissue layer of the trunk is determined based on the determined subcutaneous fat tissue volume of the trunk and the body specifying information, the skeletal muscle tissue volume of the trunk is determined based on the body specifying information, the impedance of the skeletal muscle tissue layer of the trunk is determined based on the determined skeletal muscle tissue volume of the trunk and the body specifying information, the splanchnic organ tissue volume of the trunk is determined based on the body specifying information, the impedance of the splanchnic organ tissue of the trunk is determined based on the determined splanchnic organ tissue volume of the trunk and the body specifying information, the impedance of the visceral fat tissue of the trunk is determined based on the impedance of the trunk which has been determined by use of the potential difference measured by the above other voltage measuring electrode pair, the determined impedance of the subcutaneous fat tissue layer of the trunk, the determined impedance of the skeletal muscle tissue layer of the trunk and the determined impedance of the splanchnic organ tissue of the trunk, and the visceral fat tissue volume of the trunk is determined based on the determined impedance of the visceral fat tissue of the trunk and the body specifying information.

According to still another embodiment of the present invention, the visceral fat tissue information may be acquired by applying a current to a body part where the subcutaneous fat tissue layer is thin or a body part where the skeletal muscle tissue layer has no or a thin muscle belly portion from the one current applying electrode.

According to still another embodiment of the present invention, the measurement may be made with the voltage measuring electrode pair placed on a body part protruding from the trunk.

According to still another embodiment of the present invention, the voltage measuring electrode pair may be placed on a body part protruding from the trunk on which the other current applying electrode is not placed.

According to still another embodiment of the present invention, the measurement may be made with the voltage measuring electrode pair placed at a position remote from the current applying electrode pair in the trunk longitudinal direction.

According to still another embodiment of the present invention, the voltage measuring electrode pair may measure the potential difference in a body part where the subcutaneous fat tissue layer is thin or a body part where the skeletal muscle tissue layer has no or a thin muscle belly portion.

According to still another embodiment of the present invention, it is also possible that the skeletal muscle tissue volume of the trunk is determined based on body specifying information, the impedance of the skeletal muscle tissue layer of the trunk is determined based on the determined skeletal muscle tissue volume of the trunk and the body specifying information, the splanchnic organ tissue volume of the trunk is determined based on the body specifying information, the impedance of the splanchnic organ tissue of the trunk is determined based on the determined splanchnic organ tissue volume of the trunk and the body specifying information, the impedance of the visceral fat tissue of the trunk is determined based on the determined impedance of the trunk, the determined impedance of the skeletal muscle tissue layer of the trunk and the determined impedance of the splanchnic organ tissue of the trunk, and the visceral fat tissue volume of the trunk is determined based on the determined impedance of the visceral fat tissue of the trunk and the body specifying information.

According to still another embodiment of the present invention, the body part may be a section between the navel and the upper border of the iliac crest or aponeurosis between the rectus abdominis muscle and the external abdominal oblique muscle.

According to still another embodiment of the present invention, the measurement of the impedance of the trunk may be carried out around the abdomen.

According to still another embodiment of the present invention, at least one electrode out of the voltage measuring electrode may be placed off the abdominal circumference.

According to still another embodiment of the present invention, one or both of the voltage measuring electrodes may be placed off the abdominal circumference.

According to still another embodiment of the present invention, the voltage measuring electrode pair may be placed in the above sections on the left and right sides when viewed with the navel as the center therebetween.

According to still another embodiment of the present invention, the voltage measuring electrode pair may be placed in the trunk longitudinal direction within an abdominal region off the abdominal circumference.

According to still another embodiment of the present invention, the amounts of currents passing through the splanchnic organ tissue and visceral fat tissue under the skeletal muscle tissue layer may be increased by use of skeletal muscles arranged long in the abdomen longitudinal direction as a virtual electrode layer by placing the above one current applying electrode on the skeletal muscles arranged long in the abdomen longitudinal direction above the navel.

According to still another embodiment of the present invention, it is also possible that another voltage measuring electrode pair is further placed on the skeletal muscles arranged long in the abdomen longitudinal direction so as to measure the impedance of the rectus abdominis muscle tissue layer and the visceral fat tissue volume of the trunk is determined by use of the measured impedance of the rectus abdominis muscle tissue layer.

According to still another embodiment of the present invention, it is also possible that the skeletal muscle tissue volume of the trunk is determined based on body specifying information; the impedance of the skeletal muscle tissue layer of the trunk is determined based on the determined skeletal muscle tissue volume of the trunk and the body specifying information, or the impedance of the skeletal muscle tissue layer of the trunk is determined based on the impedance of the rectus abdominis muscle tissue layer, or the impedance of the skeletal muscle tissue layer of the trunk is determined based on the impedance of the rectus abdominis muscle tissue layer and the body specifying information; the splanchnic organ tissue volume of the trunk is determined based on the body specifying information; the impedance of the splanchnic organ tissue of the trunk is determined based on the determined splanchnic organ tissue volume of the trunk and the body specifying information; the impedance of the visceral fat tissue of the trunk is determined based on the impedance of the trunk, the determined impedance of the skeletal muscle tissue layer of the trunk and the determined impedance of the splanchnic organ tissue of the trunk; and the visceral fat tissue volume of the trunk is determined based on the determined impedance of the visceral fat tissue of the trunk and the body specifying information.

According to still another embodiment of the present invention, it is also possible that a second voltage measuring electrode pair for measuring the impedance of the subcutaneous fat tissue layer of the trunk is further provided, one voltage measuring electrode out of the second voltage measuring electrode pair is placed at a position close to the above one current applying electrode where the influence of spreading resistance right underneath the one current applying electrode is predominant, the other voltage measuring electrode is placed at a remote position from the one current applying electrode where the influence of the spreading resistance is weak, and the visceral fat tissue volume of the trunk is determined by use of the impedance of the subcutaneous fat tissue layer of the trunk which has been measured by the second voltage measuring electrode pair.

According to still another embodiment of the present invention, the impedance of the rectus abdominis muscle tissue layer may be measured by an applied current of frequency f1 which is highly sensitive to the rectus abdominis muscle tissue layer, and the impedance of the trunk may be measured by an applied current of frequency f2 which is higher than f1 and is hardly influenced by muscle fibers.

According to still another embodiment of the present invention, it is also possible that a third voltage measuring electrode pair for measuring the impedance of the subcutaneous fat tissue layer of the trunk is further provided, one voltage measuring electrode out of the third voltage measuring electrode pair is placed at a position close to the above one current applying electrode where the influence of spreading resistance right underneath the one current applying electrode is predominant, the other voltage measuring electrode is placed at a remote position from the one current applying electrode where the influence of the spreading resistance is weak, and the visceral fat tissue volume of the trunk is determined by use of the impedance of the subcutaneous fat tissue layer of the trunk which has been measured by the voltage measuring electrode pair.

According to still another embodiment of the present invention, it is also possible that one current applying electrode out of a second current applying electrode pair is further placed at a position close to one voltage measuring electrode out of the third voltage measuring electrode pair, and the visceral fat tissue volume of the trunk is determined by use of the impedance of the subcutaneous fat tissue layer of the trunk which has been measured by the above voltage measuring electrode pair.

According to still another embodiment of the present invention, the step of determining the impedance of the visceral fat tissue of the trunk based on the impedance of the trunk, the determined impedance of the skeletal muscle tissue layer of the trunk and the determined impedance of the splanchnic organ tissue of the trunk may be characterized by an electrical equivalent circuit of the trunk in which the impedance of the skeletal muscle tissue layer of the trunk is connected in parallel to a series circuit of the impedance of the splanchnic organ tissue of the trunk and the impedance of the visceral fat tissue of the trunk.

According to still another embodiment of the present invention, the step of determining the impedance of the visceral fat tissue of the trunk based on the impedance of the trunk, the determined impedance of the skeletal muscle tissue layer of the trunk and the determined impedance of the splanchnic organ tissue of the trunk may be characterized by an electrical equivalent circuit of the trunk in which the impedance of the skeletal muscle tissue layer of the trunk and the impedance of the subcutaneous fat tissue layer of the trunk are connected in parallel to a series circuit of the impedance of the splanchnic organ tissue of the trunk and the impedance of the visceral fat tissue of the trunk.

According to still another embodiment of the present invention, the impedance of the skeletal muscle tissue layer of the trunk may be such that the impedance of the skeletal muscle tissue layer where the rectus abdominis muscle is predominant is connected in parallel to a series circuit of the impedance of the aponeurosis and the impedance of the skeletal muscle tissue layer of the back muscle and the abdominal oblique muscle.

According to still another embodiment of the present invention, the skeletal muscles arranged long in the abdomen longitudinal direction may be the rectus abdominis muscle tissue layer.

According to still another embodiment of the present invention, the above one current applying electrode pair may be placed between the upper portion of the rectus abdominis muscle tissue layer under the solar plexus on the front side of the trunk and the back waist lower portion.

According to still another embodiment of the present invention, the above one current applying electrode pair may be placed on the rectus abdominis muscle tissue layer on the front side of the trunk.

According to still another embodiment of the present invention, the above current applying electrodes and voltage measuring electrodes may be placed on both sides of white line symmetrically so that the electrodes equally contribute to energization and measurement on the left and right rectus abdominis muscle tissue layers.

According to still another embodiment of the present invention, the above current applying electrodes and voltage measuring electrodes may be placed on only either one of the left and right rectus abdominis muscle tissue layers.

According to still another embodiment of the present invention, the above voltage measuring electrodes may be placed with the tendinous intersections of the rectus abdominis muscle tissue layer as bases.

According to still another embodiment of the present invention, the above voltage measuring electrode may be placed in the hole of the navel.

According to one embodiment of the present invention, the body part protruding from the trunk may be four extremities or the head. For example, it may be an ear of the head.

According to still another embodiment of the present invention, the subcutaneous fat tissue layer information of the trunk may be acquired by measuring the impedance of the trunk with one voltage measuring electrode out of the voltage measuring electrode pair placed in the vicinity of the one current applying electrode and the other voltage measuring electrode placed at a position remote from the one current applying electrode in the trunk longitudinal direction.

According to still another embodiment of the present invention, the measurement may be made with the one current applying electrode and the one voltage measuring electrode placed at any of the following regions, i.e. near the navel or at the lateral region or the dorsolateral region.

According to still another embodiment of the present invention, the subcutaneous fat tissue layer information may be acquired by applying a current to a body part where the subcutaneous fat tissue layer is thin or a body part where the skeletal muscle tissue layer has no or a thin muscle belly portion from the one current applying electrode, placing one voltage measuring electrode out of the voltage measuring electrode pair at a position where the influence of spreading resistance right underneath the one current applying electrode is predominant, placing the other voltage measuring electrode at a remote position where the influence of the spreading resistance is weak, and measuring a potential difference between the one voltage measuring electrode and the other voltage measuring electrode.

According to still another embodiment of the present invention, the other voltage measuring electrode may measure the potential difference in a body part where the subcutaneous fat tissue layer is thin or a body part where the skeletal muscle tissue layer has no or a thin muscle belly portion.

According to still another embodiment of the present invention, the position where the influence of the spreading resistance is predominant may be any of the following regions, i.e. near the navel or at the lateral region or the dorsolateral region, and the remote position where the influence of the spreading resistance is weak may be between the navel and the upper border of the iliac crest.

According to still another embodiment of the present invention, the subcutaneous fat tissue layer information may be acquired by placing the one voltage measuring electrode at a position close to the one current applying electrode, placing the other voltage measuring electrode at a sufficiently remote position from the one voltage measuring electrode placed in the vicinity of the one current applying electrode, and measuring a potential difference between the one voltage measuring electrode and the other voltage measuring electrode.

According to still another embodiment of the present invention, the sufficiently remote position may be at least three times the close position.

According to still another embodiment of the present invention, it is also possible that at least one pair of current applying electrodes and at least two pairs of voltage measuring electrodes are provided; subcutaneous fat tissue layer information is acquired by applying a current to a body part where the subcutaneous fat tissue layer is thin or a body part where the skeletal muscle tissue layer has no or a thin muscle belly portion from one current applying electrode out of the current applying electrode pair, placing one voltage measuring electrode in one voltage measuring electrode pair out of the at least two voltage measuring electrode pairs at a position where the influence of spreading resistance right underneath the one current applying electrode is predominant, placing the other voltage measuring electrode at a remote position where the influence of the spreading resistance is weak, and measuring a potential difference between the one voltage measuring electrode and the other voltage measuring electrode; visceral fat tissue information is acquired by placing the other voltage measuring electrode pair out of the at least two voltage measuring electrode pairs at a remote position where the influence of the spreading resistance is weak and measuring a potential difference; and a switching device is provided which selectively acquires the subcutaneous fat tissue layer information and the visceral fat tissue information by selecting between the one voltage measuring electrode pair and the other voltage measuring electrode pair.

According to still another embodiment of the present invention, the other voltage measuring electrode pair may measure the potential difference in a body part where the subcutaneous fat tissue layer is thin or a body part where the skeletal muscle tissue layer has no or a thin muscle belly portion.

According to still another embodiment of the present invention, the position where the influence of the spreading resistance is predominant may be any of the following regions, i.e. near the navel or at the lateral region or the dorsolateral region, and the remote position where the influence of the spreading resistance is weak may be between the navel and the upper border of the iliac crest.

According to still another embodiment of the present invention, the subcutaneous fat tissue layer information may be acquired by placing the one voltage measuring electrode at a position close to the one current applying electrode, placing the other voltage measuring electrode at a sufficiently remote position from the one voltage measuring electrode placed in the vicinity of the one current applying electrode, and measuring a potential difference between the one voltage measuring electrode and the other voltage measuring electrode.

According to still another embodiment of the present invention, the sufficiently remote position may be at least three times the close position.

According to still another embodiment of the present invention, the apparatus of the present invention may further comprise trunk visceral fat tissue volume estimating means for estimating the visceral fat tissue volume of the trunk based on the impedance of the trunk which has been determined by use of the potential difference measured by the above at least two voltage measuring electrode pairs and body specifying information.

According to still another embodiment of the present invention, the apparatus of the present invention may further comprise trunk subcutaneous fat tissue volume estimating means for estimating the subcutaneous fat tissue volume of the trunk based on the impedance of the trunk which has been determined by use of the potential difference measured by the above one voltage measuring electrode pair and body specifying information, trunk subcutaneous fat tissue volume estimating means for estimating the subcutaneous fat tissue volume of the trunk based on the estimated subcutaneous fat tissue volume of the trunk and the body specifying information, trunk subcutaneous fat tissue layer impedance estimating means for estimating the impedance of the subcutaneous fat tissue layer of the trunk based on the estimated subcutaneous fat tissue volume of the trunk and the body specifying information, trunk splanchnic organ tissue impedance estimating means for estimating the splanchnic organ tissue volume of the trunk based on the body specifying information and estimating the impedance of the splanchnic organ tissue of the trunk based on the estimated splanchnic organ tissue volume of the trunk and the body specifying information, trunk visceral fat tissue impedance estimating means for estimating the impedance of the visceral fat tissue of the trunk based on the estimated impedance of the trunk, the estimated impedance of the skeletal muscle tissue layer of the trunk and the estimated impedance of the splanchnic organ tissue of the trunk, and trunk visceral fat tissue volume estimating means for estimating the visceral fat tissue volume of the trunk based on the estimated impedance of the visceral fat tissue of the trunk and the body specifying information.

According to still another embodiment of the present invention, the visceral fat tissue information may be acquired by applying a current to a body part where the subcutaneous fat tissue layer is thin or a body part where the skeletal muscle tissue layer has no or a thin muscle belly portion from the one current applying electrode.

According to still another embodiment of the present invention, the voltage measuring electrode pair may be placed at a position remote from the current applying electrode pair in the trunk longitudinal direction.

According to still another embodiment of the present invention, the voltage measuring electrode pair may measure the potential difference in a body part where the subcutaneous fat tissue layer is thin or a body part where the skeletal muscle tissue layer has no or a thin muscle belly portion.

According to still another embodiment of the present invention, the apparatus of the present invention may further comprise trunk skeletal muscle tissue volume estimating means for estimating the skeletal muscle tissue volume of the trunk based on body specifying information, trunk skeletal muscle tissue layer impedance estimating means for estimating the impedance of the skeletal muscle tissue layer of the trunk based on the estimated skeletal muscle tissue volume of the trunk and the body specifying information, trunk splanchnic organ tissue impedance estimating means for estimating the splanchnic organ tissue volume of the trunk based on the body specifying information and estimating the impedance of the splanchnic organ tissue of the trunk based on the estimated splanchnic organ tissue volume of the trunk and the body specifying information, trunk visceral fat tissue impedance estimating means for estimating the impedance of the visceral fat tissue of the trunk based on the estimated impedance of the trunk, the estimated impedance of the skeletal muscle tissue layer of the trunk and the estimated impedance of the splanchnic organ tissue of the trunk, and trunk visceral fat tissue volume estimating means for estimating the visceral fat tissue volume of the trunk based on the estimated impedance of the visceral fat tissue of the trunk and the body specifying information.

According to still another embodiment of the present invention, the above one current applying electrode may be placed on skeletal muscles arranged long in the abdomen longitudinal direction above the navel.

According to still another embodiment of the present invention, another voltage measuring electrode pair for measuring the impedance of the rectus abdominis muscle tissue layer may be further placed on the skeletal muscles arranged long in the abdomen longitudinal direction.

According to still another embodiment of the present invention, the apparatus of the present invention may further comprise trunk skeletal muscle tissue layer impedance estimating means for estimating the skeletal muscle tissue volume of the trunk based on body specifying information and estimating the impedance of the skeletal muscle tissue layer of the trunk based on the estimated skeletal muscle tissue volume of the trunk and the body specifying information, or, trunk skeletal muscle tissue layer impedance estimating means for estimating the impedance of the skeletal muscle tissue layer of the trunk based on the impedance of the rectus abdominis muscle tissue layer, trunk splanchnic organ tissue impedance estimating means for estimating the splanchnic organ tissue volume of the trunk based on the body specifying information and estimating the impedance of the splanchnic organ tissue of the trunk based on the estimated splanchnic organ tissue volume of the trunk and the body specifying information, trunk visceral fat tissue impedance estimating means for estimating the impedance of the visceral fat tissue of the trunk based on the estimated impedance of the trunk, the estimated impedance of the skeletal muscle tissue layer of the trunk and the estimated impedance of the splanchnic organ tissue of the trunk, and trunk visceral fat tissue volume estimating means for estimating the visceral fat tissue volume of the trunk based on the estimated impedance of the visceral fat tissue of the trunk and the body specifying information.

According to still another embodiment of the present invention, it is also possible that a second voltage measuring electrode pair for measuring the impedance of the subcutaneous fat tissue layer of the trunk is further provided, one voltage measuring electrode out of the second voltage measuring electrode pair is placed at a position close to the above one current applying electrode where the influence of spreading resistance right underneath the one current applying electrode is predominant, the other voltage measuring electrode is placed at a remote position from the one current applying electrode where the influence of the spreading resistance is weak, and the visceral fat tissue volume of the trunk is determined by use of the impedance of the subcutaneous fat tissue layer of the trunk which has been measured by the second voltage measuring electrode pair.

According to still another embodiment of the present invention, the apparatus of the present invention may further comprise trunk skeletal muscle tissue layer impedance estimating means for estimating the impedance of the skeletal muscle tissue layer of the trunk based on the impedance of the rectus abdominis muscle tissue layer and body specifying information, trunk splanchnic organ tissue impedance estimating means for estimating the splanchnic organ tissue volume of the trunk based on the body specifying information and estimating the impedance of the splanchnic organ tissue of the trunk based on the estimated splanchnic organ tissue volume of the trunk and the body specifying information, trunk visceral fat tissue impedance estimating means for estimating the impedance of the visceral fat tissue of the trunk based on the impedance of the trunk, the estimated impedance of the skeletal muscle tissue layer of the trunk and the estimated impedance of the splanchnic organ tissue of the trunk, and trunk visceral fat tissue volume estimating means for estimating the visceral fat tissue volume of the trunk based on the estimated impedance of the visceral fat tissue of the trunk and the body specifying information.

According to still another embodiment of the present invention, it is also possible that a third voltage measuring electrode pair for measuring the impedance of the subcutaneous fat tissue layer of the trunk is further provided, one voltage measuring electrode out of the third voltage measuring electrode pair is placed at a position close to the above one current applying electrode where the influence of spreading resistance right underneath the one current applying electrode is predominant, the other voltage measuring electrode is placed at a remote position from the one current applying electrode where the influence of the spreading resistance is weak, and the visceral fat tissue volume of the trunk is determined by use of the impedance of the subcutaneous fat tissue layer of the trunk which has been measured by the voltage measuring electrode pair.

According to still another embodiment of the present invention, it is also possible that one current applying electrode out of a second current applying electrode pair is further placed at a position close to one voltage measuring electrode out of the third voltage measuring electrode pair, and the visceral fat tissue volume of the trunk is determined by use of the impedance of the subcutaneous fat tissue layer which has been measured by the above voltage measuring electrode pair.

According to still another embodiment of the present invention, the trunk visceral fat tissue impedance estimating means may make the estimation, with an electrical equivalent circuit of the trunk in which the impedance of the skeletal muscle tissue layer of the trunk is connected in parallel to a series circuit of the impedance of the splanchnic organ tissue of the trunk and the impedance of the visceral fat tissue of the trunk.

According to still another embodiment of the present invention, the trunk visceral fat tissue impedance estimating means may make the estimation, with an electrical equivalent circuit of the trunk in which the impedance of the skeletal muscle tissue layer of the trunk and the impedance of the subcutaneous fat tissue layer of the trunk are connected in parallel to a series circuit of the impedance of the splanchnic organ tissue of the trunk and the impedance of the visceral fat tissue of the trunk.

According to still another embodiment of the present invention, the impedance of the skeletal muscle tissue layer of the trunk may be such that the impedance of the skeletal muscle tissue layer where the rectus abdominis muscle is predominant is connected in parallel to a series circuit of the impedance of the aponeurosis and the impedance of the skeletal muscle tissue layer of the back muscle and the abdominal oblique muscle.

According to still another embodiment of the present invention, the skeletal muscles arranged long in the abdomen longitudinal direction may be the rectus abdominis muscle tissue layer.

According to still another embodiment of the present invention, the above one current applying electrode pair may be placed between the upper portion of the rectus abdominis muscle tissue layer under the solar plexus on the front side of the trunk and the back waist lower portion.

According to still another embodiment of the present invention, the above one current applying electrode pair may be placed on the rectus abdominis muscle tissue layer on the front side of the trunk.

According to still another embodiment of the present invention, the apparatus of the present invention may further comprise breathing change influence removing means for removing the influence of change caused by breathing based on the impedance of the trunk which is measured in a sampling period shorter than breathing cycle time.

According to still another embodiment of the present invention, the apparatus of the present invention may further comprise abnormal value determination process means for performing an abnormal value determination process by comparing the measured impedance of the trunk with a general value of a group.

According to still another embodiment of the present invention, the apparatus of the present invention may further comprise display means for displaying advice information based on the result of determination made by the abnormal value determination process means.

According to still another embodiment of the present invention, the visceral fat tissue volume of the trunk may be represented by the visceral fat percentage of the trunk, the visceral fat tissue cross-sectional area of the trunk, the visceral fat tissue volume of the trunk or the visceral fat tissue weight of the trunk.

Embodiment According to the Second Characteristic of the Invention

According to one embodiment of the present invention, the body part which is highly useful in estimating the subcutaneous fat tissue volume is any or all of a region adjacent to the navel on the navel circumference, a region under the shoulder blade and the upper border of the iliac crest, or, any or all of the navel concave portion on the navel circumference, the backbone and the aponeurosis, or, a combination of a candidate body part with the greatest deposition of subcutaneous fat tissue and a body part with the smallest deposition of subcutaneous fat tissue.

According to another embodiment of the present invention, the body parts which are highly useful in estimating the subcutaneous fat tissue volume are the following three body parts, i.e. the region adjacent to the navel, the aponeurosis and the lateral region.

According to still another embodiment of the present invention, the body part protruding from the trunk is any of the four extremities, head and ears.

According to still another embodiment of the present invention, the position close to the one current applying electrode is a position where the influence of spreading resistance right underneath the one current applying electrode is predominant.

According to still another embodiment of the present invention, body specifying information is acquired, a potential difference between the above each voltage measuring electrode pair is measured to determine the impedance of the subcutaneous fat tissue layer of each of the above body parts of the abdomen, and the subcutaneous fat tissue volume of the trunk is determined based on the acquired body specifying information and the determined impedance of the subcutaneous fat tissue layer of each body part.

According to still another embodiment of the present invention, a potential difference between the above each voltage measuring electrode pair is measured to determine the impedance of the subcutaneous fat tissue layer of each of the above body parts of the abdomen, the impedance of the subcutaneous fat tissue layer of the trunk is determined based on the impedance of the subcutaneous fat tissue layer of each body part, and the subcutaneous fat tissue volume of the trunk is determined based on the determined impedance of the subcutaneous fat tissue layer of the trunk and body specifying information.

According to one embodiment of the present invention, in the above trunk subcutaneous fat measuring apparatus, the body part which is highly useful in estimating the subcutaneous fat tissue volume is any or all of a region adjacent to the navel on the navel circumference, a region under the shoulder blade and the upper border of the iliac crest, or, any or all of the navel concave portion on the navel circumference, the backbone and the aponeurosis, or, a combination of a candidate body part with the greatest deposition of subcutaneous fat tissue and a body part with the smallest deposition of subcutaneous fat tissue.

According to another embodiment of the present invention, in the above trunk subcutaneous fat measuring apparatus, the body parts which are highly useful in estimating the subcutaneous fat tissue volume are the following three body parts, i.e. the region adjacent to the navel, the aponeurosis and the lateral region.

According to still another embodiment of the present invention, in the above trunk subcutaneous fat measuring apparatus, the body part protruding from the trunk is any of the four extremities, head and ears.

According to still another embodiment of the present invention, in the above trunk subcutaneous fat measuring apparatus, the position close to the one current applying electrode is a position where the influence of spreading resistance right underneath the one current applying electrode is predominant.

According to still another embodiment of the present invention, the above trunk subcutaneous fat measuring apparatus comprises body specifying information acquiring means for acquiring body specifying information, trunk abdominal body part subcutaneous fat tissue layer impedance measuring means for measuring a potential difference between the above each voltage measuring electrode pair and measuring the impedance of the subcutaneous fat tissue layer of the above each body part of the abdomen, and trunk subcutaneous fat tissue volume estimating means for estimating the subcutaneous fat tissue volume of the trunk based on the acquired body specifying information and the measured impedance of the subcutaneous fat tissue layer of each body part.

According to still another embodiment of the present invention, the above trunk subcutaneous fat measuring apparatus comprises trunk abdominal body part subcutaneous fat tissue layer impedance measuring means for measuring a potential difference between the above each voltage measuring electrode pair and measuring the impedance of the subcutaneous fat tissue layer of the above each body part of the abdomen, trunk subcutaneous fat tissue layer impedance estimating means for estimating the impedance of the subcutaneous fat tissue layer of the trunk based on the measured impedance of the subcutaneous fat tissue layer of each body part, and trunk subcutaneous fat tissue volume estimating means for estimating the subcutaneous fat tissue volume of the trunk based on the estimated impedance of the subcutaneous fat tissue layer of the trunk and body specifying information.

According to still another embodiment of the present invention, there is provided a trunk visceral fat measuring method for measuring the visceral fat tissue of the trunk, which comprises the steps of:

measuring the bioelectrical impedance of a lower extremity, the bioelectrical impedance of an upper extremity, and the bioelectrical impedance of a trunk,
determining the skeletal muscle tissue volume of the trunk based on the measured bioelectrical impedances of the lower and upper extremities and body specifying information,
determining the impedance of the skeletal muscle tissue layer of the trunk based on the determined skeletal muscle tissue volume of the trunk and the body specifying information,
determining the impedance of the subcutaneous fat tissue layer of the trunk,
determining the impedance of the splanchnic organ tissue of the trunk based on the body specifying information,
determining the impedance of the visceral fat tissue of the trunk based on the determined bioelectrical impedance of the trunk, the determined impedance of the skeletal muscle tissue layer of the trunk, the determined impedance of the subcutaneous fat tissue layer of the trunk, and the determined impedance of the splanchnic organ tissue of the trunk, and
determining the visceral fat tissue volume of the trunk based on the determined impedance of the visceral fat tissue of the trunk and the body specifying information,

wherein
in the step of determining the impedance of the subcutaneous fat tissue layer of the trunk, one of current applying electrodes is placed on at least one body part which is highly useful in estimating a subcutaneous fat tissue volume on the truncal abdominal circumference, the other current applying electrode is placed on a body part protruding from the trunk, one voltage measuring electrode is placed in the vicinity of the one current applying electrode on the truncal abdominal circumference, and the other voltage measuring electrode is placed on a body part protruding from the trunk that is different from the body part on which the other current applying electrode is placed, to determine the impedance of the subcutaneous fat tissue layer of the trunk.

According to one embodiment of the present invention, in the above trunk visceral fat measuring method, the body part which is highly useful in estimating the subcutaneous fat tissue volume is any or all of a region adjacent to the navel on the navel circumference, a region under the shoulder blade and the upper border of the iliac crest, or, any or all of the navel concave portion on the navel circumference, the backbone and the aponeurosis, or, a combination of a candidate body part with the greatest deposition of subcutaneous fat tissue and a body part with the smallest deposition of subcutaneous fat tissue.

According to another embodiment of the present invention, in the above trunk visceral fat measuring method, the body parts which are highly useful in estimating the subcutaneous fat tissue volume are the following three body parts, i.e. the region adjacent to the navel, the aponeurosis and the lateral region.

According to still another embodiment of the present invention, in the above trunk visceral fat measuring method, the body part protruding from the trunk is any of the four extremities, head and ears.

According to still another embodiment of the present invention, in the above trunk visceral fat measuring method, the position close to the one current applying electrode is a position where the influence of spreading resistance right underneath the current applying electrode is predominant.

According to still another embodiment of the present invention, in the above trunk visceral fat measuring method, the step of determining the impedance of the visceral fat tissue of the trunk based on the determined bioelectrical impedance of the trunk, the determined impedance of the skeletal muscle tissue layer of the trunk, the determined impedance of the subcutaneous fat tissue layer of the trunk and the determined impedance of the splanchnic organ tissue of the trunk may be characterized by an electrical equivalent circuit of the trunk in which the impedance of the subcutaneous fat tissue layer of the trunk and the impedance of the skeletal muscle tissue layer of the trunk are connected in parallel to a series circuit of the impedance of the splanchnic organ tissue of the trunk and the impedance of the visceral fat tissue of the trunk.

According to still another embodiment of the present invention, in the above trunk visceral fat measuring method, the impedance of the splanchnic organ tissue is determined based on body specifying information and a splanchnic organ tissue volume determined from the body specifying information.

According to still another embodiment of the present invention, in the above trunk visceral fat measuring method, the body specifying information is information representing a physical feature.

According to still another embodiment of the present invention, in the above trunk visceral fat measuring method, the information representing a physical feature may be a body height, sex, a body weight, age, an extremity length (lower-extremity length, upper-extremity length), a trunk length (mid-trunk length), an abdominal circumference, an abdominal width or an abdominal thickness.

According to one embodiment of the present invention, in the above trunk visceral fat measuring apparatus, the body part which is highly useful in estimating the subcutaneous fat tissue volume is any or all of a region adjacent to the navel on the navel circumference, a region under the shoulder blade and the upper border of the iliac crest, or, any or all of the navel concave portion on the navel circumference, the backbone and the aponeurosis, or, a combination of a candidate body part with the greatest deposition of subcutaneous fat tissue and a body part with the smallest deposition of subcutaneous fat tissue.

According to another embodiment of the present invention, in the above trunk visceral fat measuring apparatus, the body parts which are highly useful in estimating the subcutaneous fat tissue volume are the following three body parts, i.e. the region adjacent to the navel, the aponeurosis and the lateral region.

According to still another embodiment of the present invention, in the above trunk visceral fat measuring apparatus, the body part protruding from the trunk is any of the four extremities, head and ears.

According to still another embodiment of the present invention, in the above trunk visceral fat measuring apparatus, the position close to the one current applying electrode is a position where the influence of spreading resistance right underneath the current applying electrode is predominant.

According to still another embodiment of the present invention, in the above trunk visceral fat measuring apparatus, the trunk visceral fat tissue impedance estimating means makes the estimation, with an electrical equivalent circuit of the trunk in which the impedance of the subcutaneous fat tissue layer of the trunk and the impedance of the skeletal muscle tissue layer of the trunk are connected in parallel to a series circuit of the impedance of the splanchnic organ tissue of the trunk and the impedance of the visceral fat tissue of the trunk.

According to still another embodiment of the present invention, in the above trunk visceral fat measuring apparatus, the trunk splanchnic organ tissue impedance estimating means estimates the splanchnic organ tissue volume of the trunk from body specifying information and estimates the impedance of the splanchnic organ tissue of the trunk based on the estimated splanchnic organ tissue volume of the trunk and the body specifying information.

According to still another embodiment of the present invention, in the above trunk visceral fat measuring apparatus, the body specifying information is information representing a physical feature.

According to still another embodiment of the present invention, in the above trunk visceral fat measuring apparatus, the information representing a physical feature may be a body height, sex, a body weight, age, an extremity length (lower-extremity length, upper-extremity length), a trunk length (mid-trunk length), an abdominal circumference, an abdominal width or an abdominal thickness.

According to still another embodiment of the present invention, the above trunk visceral fat measuring apparatus comprises trunk abdominal body part subcutaneous fat tissue layer impedance measuring means for measuring a potential difference between the above each voltage measuring electrode pair and measuring the impedance of the subcutaneous fat tissue layer of the above each body part of the abdomen, trunk subcutaneous fat tissue layer impedance estimating means for estimating the impedance of the subcutaneous fat tissue layer of the trunk based on the measured impedance of the subcutaneous fat tissue layer of each body part, and trunk subcutaneous fat tissue volume estimating means for estimating the subcutaneous fat tissue volume of the trunk based on the estimated impedance of the subcutaneous fat tissue layer of the trunk and body specifying information.

According to still another embodiment of the present invention, the above trunk visceral fat measuring apparatus further comprises trunk abdominal fat tissue volume estimating means for estimating the abdominal fat tissue volume of the trunk from the estimated visceral fat tissue volume of the trunk and the estimated subcutaneous fat tissue volume of the trunk.

According to still another embodiment of the present invention, the above trunk visceral fat measuring apparatus further comprises trunk visceral fat/subcutaneous fat ratio estimating means for estimating a trunk visceral fat/subcutaneous fat ratio from the estimated visceral fat tissue volume of the trunk and the estimated subcutaneous fat tissue volume of the trunk.

According to still another embodiment of the present invention, the above trunk visceral fat measuring apparatus further comprises breathing change influence removing means for removing the influence of change caused by breathing based on the bioelectrical impedance of the trunk which is measured in a sampling period shorter than breathing cycle time.

According to still another embodiment of the present invention, the above trunk visceral fat measuring apparatus further comprises abnormal value determination process means for performing an abnormal value determination process by comparing the measured bioelectrical impedance of the trunk with a general value of a group.

According to still another embodiment of the present invention, the above trunk visceral fat measuring apparatus further comprises display means for displaying advice information based on the result of determination made by the abnormal value determination process means.

According to still another embodiment of the present invention, in the above trunk visceral fat measuring apparatus, the above visceral fat tissue volume of the trunk is represented by the visceral fat percentage of the trunk.

According to still another embodiment of the present invention, in the above trunk visceral fat measuring apparatus, the above visceral fat tissue volume of the trunk is represented by the visceral fat tissue cross-sectional area of the trunk.

According to still another embodiment of the present invention, in the above trunk visceral fat measuring apparatus, the above visceral fat tissue volume of the trunk is represented by the visceral fat tissue volume of the trunk.

According to still another embodiment of the present invention, in the above trunk visceral fat measuring apparatus, the above visceral fat tissue volume of the trunk is represented by the visceral fat tissue weight of the trunk.

Embodiment According to the Third Characteristic of the Invention

According to one embodiment of the present invention, a potential difference between the shared electrode and the voltage measuring electrode when a current is applied between the shared electrode and the current applying electrode is measured, and the impedance of the trunk is measured based on the applied current and the measured potential difference.

According to another embodiment of the present invention, the truncal visceral/subcutaneous fat measuring method of the present invention further comprises:

determining the skeletal muscle tissue volume of the trunk based on body specifying information,
determining the impedance of the skeletal muscle tissue layer of the trunk based on the determined skeletal muscle tissue volume of the trunk and the body specifying information,
determining the splanchnic organ tissue volume of the trunk based on the body specifying information,
determining the impedance of the splanchnic organ tissue of the trunk based on the determined splanchnic organ tissue volume of the trunk and the body specifying information,
determining the impedance of the visceral fat tissue of the trunk based on the measured impedance of the trunk, the determined impedance of the skeletal muscle tissue layer of the trunk and the determined impedance of the splanchnic organ tissue of the trunk, and
determining the visceral fat tissue volume of the trunk based on the determined impedance of the visceral fat tissue of the trunk and the body
specifying information,

According to another embodiment of the present invention, the trunk visceral fat tissue impedance estimating means makes the estimation, with an electrical equivalent circuit of the trunk in which the impedance of the skeletal muscle tissue layer of the trunk is connected in parallel to a series circuit of the impedance of the splanchnic organ tissue of the trunk and the impedance of the visceral fat tissue of the trunk.

According to still another embodiment of the present invention, in the above truncal visceral/subcutaneous fat measuring method, the above body part which is useful for measurement may be a body part protruding from the trunk.

According to still another embodiment of the present invention, in the above truncal visceral/subcutaneous fat measuring method, the body part protruding from the trunk may be either one of an upper extremity and a lower extremity.

According to still another embodiment of the present invention, in the above truncal visceral/subcutaneous fat measuring method, the above shared electrode may be placed in a longitudinal shape in the navel circumferential direction of the abdomen.

According to still another embodiment of the present invention, in the above truncal visceral/subcutaneous fat measuring method, the body part which is useful for measurement may be the abdomen.

According to one embodiment of the present invention, the truncal visceral/subcutaneous fat measuring apparatus of the present invention comprises means for measuring the impedance of the trunk based on a potential difference between the shared electrode and the voltage measuring electrode when a current is applied between the shared electrode and the current applying electrode.

According to another embodiment of the present invention, the truncal visceral/subcutaneous fat measuring apparatus further comprises trunk skeletal muscle tissue layer impedance estimating means for estimating the skeletal muscle tissue volume of the trunk based on body specifying information and estimating the impedance of the skeletal muscle tissue layer of the trunk based on the estimated skeletal muscle tissue volume of the trunk and the body specifying information, trunk splanchnic organ tissue impedance estimating means for estimating the splanchnic organ tissue volume of the trunk based on the body specifying information and estimating the impedance of the splanchnic organ tissue of the trunk based on the estimated splanchnic organ tissue volume of the trunk and the body specifying information, trunk visceral fat tissue impedance estimating means for estimating the impedance of the visceral fat tissue of the trunk based on the impedance of the trunk which has been measured by the above measuring means, the estimated impedance of the skeletal muscle tissue layer of the trunk and the estimated impedance of the splanchnic organ tissue of the trunk, and trunk visceral fat tissue volume estimating means for estimating the visceral fat tissue volume of the trunk based on the estimated impedance of the visceral fat tissue of the trunk and the body specifying information.

According to still another embodiment of the present invention, the trunk visceral fat tissue impedance estimating means makes the estimation, with an electrical equivalent circuit of the trunk in which the impedance of the skeletal muscle tissue layer of the trunk is connected in parallel to a series circuit of the impedance of the splanchnic organ tissue of the trunk and the impedance of the visceral fat tissue of the trunk.

According to still another embodiment of the present invention, in the above truncal visceral/subcutaneous fat measuring apparatus, the above body part which is useful for measurement may be a body part protruding from the trunk.

According to still another embodiment of the present invention, in the above truncal visceral/subcutaneous fat measuring apparatus, the body part protruding from the trunk may be either one of an upper extremity and a lower extremity.

According to still another embodiment of the present invention, in the above truncal visceral/subcutaneous fat measuring apparatus, the above shared electrode may be placed in a longitudinal shape in the navel circumferential direction of the abdomen.

According to still another embodiment of the present invention, in the above truncal visceral/subcutaneous fat measuring apparatus, the body part which is useful for measurement may be the abdomen.

According to still another embodiment of the present invention, the above current applying electrodes and the above voltage measuring electrodes include a plurality of current applying electrodes and a plurality of voltage measuring electrodes which are placed on body parts which are useful for measurement, and the above measuring means preferably comprises a current applying electrode selecting section and a voltage measuring electrode selecting section which select electrodes required for measurement from these pluralities of electrodes.

According to still another embodiment of the present invention, the above plurality of current applying electrodes and the above plurality of voltage measuring electrodes preferably include electrodes placed on at least one of an upper extremity, a lower extremity, the abdomen and the head.

Preferably the visceral fat tissue and subcutaneous fat tissue layer of the trunk can be measured simultaneously with high accuracy by increasing the amount of a current applied to the splanchnic organ tissue and visceral fat tissue layer and the sensitivity thereof. As for the N component which is noise caused by disturbance of potential caused by the skeletal muscle tissue, the S/N property can be improved by disposing voltage measuring electrodes off the abdominal tissue dominant region.

Preferably the information of the subcutaneous fat tissue layer of the trunk can be measured with high accuracy, and the visceral fat tissue of the trunk can be measured with high accuracy by using the highly accurately measured subcutaneous fat tissue layer information for estimation of the information of the visceral fat tissue of the trunk.

Thus, since the shared electrode has a wide electrode area, the influence of displacement of the electrodes on the abdomen can be alleviated. Since a strict limitation on placement of the shared electrode is eliminated and a limitation exists only on placement of the other current applying electrode, the influence of displacement of the electrodes can be alleviated as a whole. Particularly, when the electrodes other than the shared electrode are placed on body parts protruding from the trunk such as four extremities, the influence of displacement of the electrodes on the trunk can be alleviated significantly because only the shared electrode is placed on the trunk. Further, more careful placement of the electrodes on body parts to be measured can be achieved. Accordingly, an improvement in accuracy by conscious constraint can also be expected. In addition, the influence of contact resistance with the skin and the influence of spreading resistance right underneath the current applying electrode can be alleviated and ignored by expanding the electrode area.

Further, even paralyzed patients and subjects confined to bed such as those who need nursing care can perform a measurement easily by using the front side of the abdomen as a measurement section. Further, since attachment of electrodes to the abdomen can help subjects be aware of a body part to be measured, it is advantageous to an improvement in measurement accuracy and securing of motivation by conscious constraint.

Further, while adhering to a combination with a conventional measurement method and ease of use, highly accurate screening information according to a required level can be exposed for the degree of accumulation of fat tissues adhering around splanchnic organ tissues.

Further, according to the present invention, since the visceral fat tissue and subcutaneous fat tissue layer of the trunk can be measured with high accuracy by a small and simple apparatus, it can be used as an optimum apparatus for domestic use. Further, the present invention can also perform checking of the condition of the abdomen prior to measurement, i.e. early checking of inflammation and abnormality in body fluid distribution in splanchnic organ tissues and the like and give appropriate health guideline advice according to the checking result. Therefore, users can acquire a variety of information useful for proper exercise of daily diet by food and exercise, maintenance of motivation therefor and sustainable self-management for maintenance and improvement of health in a simple manner, and the information is very useful.

In the following, the present invention is explained in greater detail by means of several embodiments thereof in conjunction with the accompanying drawings, wherein:

Fig. 1 is a perspective view of the appearance of a truncal visceral fat measuring apparatus as an example of the present invention.

Fig. 2 is a block diagram showing the constitution of the truncal visceral fat measuring apparatus of Fig. 1.

Fig. 3 is an enlarged perspective view of grips used in the apparatus of Fig. 1.

Fig. 4 is a perspective view of another example of the present apparatus shown in Figs. 1 to 3.

Fig. 5 is a schematic diagram illustrating a four-extremity lead system.

Fig. 6 is a schematic diagram of the structure of the abdomen.

Fig. 7 is a diagram showing the structure of the abdomen of Fig. 4 as an electrical equivalent circuit of the trunk without the subcutaneous fat tissue layer.

Fig. 8 is a diagram showing the structure of the abdomen of Fig. 4 as an electrical equivalent circuit of the trunk with the subcutaneous fat tissue layer.

Fig. 9 is a cross-sectional view of the abdomen at the navel height of the trunk shown in Fig. 6.

Fig. 10 is a diagram illustrating the model diagram of Fig. 9 as an electrical equivalent circuit.

Fig. 11 is a diagram illustrating the circuit of Fig. 10 in a simplified form.

Fig. 12 is a diagram illustrating the relationship between the distance between the electrodes and spreading resistance.

Fig. 13 is a diagram illustrating the relationship between the distance between the electrodes and spreading resistance.

Fig. 14 is a schematic diagram illustrating an example of an electrode arrangement that can be used in the present invention with the structure of the abdomen of the trunk.

Fig. 15 is a diagram illustrating an example of an electrode arrangement for measuring information of only the subcutaneous fat tissue layer.

Fig. 16 is a diagram illustrating an example of an electrode arrangement for measuring information of only the subcutaneous fat tissue layer.

Fig. 17 is a diagram illustrating an example of an electrode arrangement for measuring information of only the subcutaneous fat tissue layer.

Fig. 18 is a diagram illustrating an example of an electrode arrangement for measuring information of only the subcutaneous fat tissue layer.

Fig. 19 is a diagram illustrating an example of an electrode arrangement for measuring subcutaneous fat tissue layer information and visceral fat tissue information simultaneously as separate informations.

Fig. 20 is a diagram illustrating an example of an electrode arrangement for measuring subcutaneous fat tissue layer information and visceral fat tissue information simultaneously as separate informations.

Fig. 21 is a diagram illustrating an example of an electrode arrangement for measuring subcutaneous fat tissue layer information and visceral fat tissue information simultaneously as separate informations.

Fig. 22 is a diagram illustrating an example of an electrode arrangement for measuring subcutaneous fat tissue layer information and visceral fat tissue information simultaneously as separate informations.

Fig. 23 is a schematic diagram illustrating an example of an electrode arrangement that can be used in the present invention with the structure of the abdomen of the trunk.

Fig. 24 is a diagram illustrating an example of an electrode arrangement.

Fig. 25 is a diagram illustrating an example of an electrode arrangement.

Fig. 26 is a diagram illustrating an example of an electrode arrangement.

Fig. 27 is a diagram illustrating an example of an electrode arrangement that can be used in the present invention with the structure of the abdomen of the trunk.

Fig. 28 is a schematic diagram illustrating the structure of the abdomen of the trunk of Fig. 27.

Fig. 29 is a diagram illustrating the electrical equivalent circuit of Fig. 28.

Fig. 30 is a diagram illustrating an example of an electrode arrangement that can be used in the present invention with the structure of the abdomen of the trunk.

Fig. 31 is a diagram illustrating an example of an electrode arrangement that can be used in the present invention with the structure of the abdomen of the trunk.

Fig. 32 is a diagram illustrating an example of an electrode arrangement for carrying out measurement of the subcutaneous fat tissue layer that can be used in the present invention.

Fig. 33 is a diagram illustrating an example of an electrode arrangement for carrying out combined measurements of measurement of visceral fat tissue and measurement of the subcutaneous fat tissue layer that can be used in the present invention.

Fig. 34 is a diagram illustrating an electrode positioning method for an electrode arrangement in combined measurements that can be used in the present invention.

Fig. 35 is a diagram illustrating an example of an electrode arrangement that can be used in the present invention with the structure of the abdomen of the trunk.

Fig. 36 is a schematic diagram illustrating the structure of the abdomen of the trunk of Fig. 35.

Fig. 37 is a diagram illustrating the electrical equivalent circuit of Fig. 36.

Fig. 38 is a diagram illustrating an example of an electrode arrangement for carrying out combined measurements that can be used in the present invention.

Fig. 39 is a diagram illustrating an example of an electrode arrangement for carrying out combined measurements that can be used in the present invention.

Fig. 40 is a diagram illustrating an electrode positioning method for an electrode arrangement in combined measurements that can be used in the present invention.

Fig. 41 is a diagram illustrating an example of an electrode arrangement for carrying out combined measurements that can be used in the present invention.

Fig. 42 is a diagram illustrating the electrical equivalent circuit of the structure of the abdomen of the trunk with the impedances of the upper and lower extremities taken into consideration.

Fig. 43 is a diagram illustrating a lead system by energization between the upper extremity and the lower extremity, based on the electrical equivalent circuit shown in Fig. 42.

Fig. 44 is a diagram illustrating a lead system by energization between the upper extremity and the abdomen, based on the electrical equivalent circuit shown in Fig. 42.

Fig. 45 is a diagram illustrating a lead system by energization between the lower extremity and the abdomen, based on the electrical equivalent circuit shown in Fig. 42.

Fig. 46 is a diagram illustrating a lead system for measuring fat tissues, especially visceral fat tissues, by a combination of electrodes placed on the four extremities and the trunk.

Fig. 47 is a diagram illustrating a lead system for measuring fat tissues, especially visceral fat tissues, by a combination of electrodes placed on the four extremities and the trunk.

Fig. 48 is a diagram illustrating a lead system for measuring fat tissues, especially visceral fat tissues, by a combination of electrodes placed on the four extremities and the trunk.

Fig. 49 is a diagram illustrating a lead system for measuring fat tissues, especially visceral fat tissues, by a combination of electrodes placed on the four extremities and the trunk.

Fig. 50 is a diagram illustrating a lead system for measuring fat tissues, especially visceral fat tissues, by a combination of electrodes placed on the four extremities and the trunk.

Fig. 51 is a diagram illustrating a lead system for measuring fat tissues, especially visceral fat tissues, by a combination of electrodes placed on the four extremities and the trunk.

Fig. 52 is a diagram illustrating a lead system for measuring fat tissues, especially visceral fat tissues, by a combination of electrodes placed on the four extremities and the trunk.

Fig. 53 is a diagram illustrating a lead system for measuring fat tissues, especially visceral fat tissues, by a combination of electrodes placed on the four extremities and the trunk.

Fig. 54 is a diagram illustrating a lead system for measuring fat tissues, especially the subcutaneous fat tissue layer, by a combination of electrodes placed on the four extremities and the trunk.

Fig. 55 is a diagram illustrating a lead system for measuring fat tissues, especially the subcutaneous fat tissue layer, by a combination of electrodes placed on the four extremities and the trunk.

Fig. 56 is a diagram illustrating a lead system for measuring fat tissues, especially the subcutaneous fat tissue layer, by a combination of electrodes placed on the four extremities and the trunk.

Fig. 57 is a diagram illustrating a lead system for measuring fat tissues, especially the subcutaneous fat tissue layer, by a combination of electrodes placed on the four extremities and the trunk.

Fig. 58 is a diagram illustrating a lead system for measuring fat tissues, especially the subcutaneous fat tissue layer, by a combination of electrodes placed on the four extremities and the trunk.

Fig. 59 is a diagram illustrating a lead system for measuring fat tissues, especially the subcutaneous fat tissue layer, by a combination of electrodes placed on the four extremities and the trunk.

Fig. 60 is a diagram illustrating a specific measurement basic flow of visceral fat tissues by a trunk energization lead system as an example of the present invention.

Fig. 61 is a diagram illustrating a subcutaneous fat tissue volume and subcutaneous fat tissue layer impedance estimation process flow as a subroutine of the basic flow of Fig. 60.

Fig. 62 is a diagram illustrating a splanchnic organ tissue volume and splanchnic organ tissue impedance estimation process flow as a subroutine of the basic flow of Fig. 60.

Fig. 63 is a diagram illustrating a visceral fat tissue impedance and visceral fat tissue volume estimation process flow as a subroutine of the basic flow of Fig. 60.

Fig. 64 is a diagram illustrating an extremity and trunk impedance measurement process flow as a subroutine of the basic flow of Fig. 60.

Fig. 65 is a diagram illustrating a mid-trunk impedance measured data breathing change correction process flow as a subroutine of the extremity and trunk impedance measurement process flow of Fig. 64.

Fig. 66 is a diagram illustrating a flow of abnormal value determination process by drinking and eating and retention of urine in bladder as a subroutine of the extremity and trunk impedance measurement process flow of Fig. 64.

Fig. 67 is a diagram illustrating an extremity and trunk impedance measurement process flow different from the flow of Fig. 64 as a subroutine of the basic flow of Fig. 60.

Fig. 68 is a diagram illustrating a flow of process of determining abnormality in trunk abdominal splanchnic organ tissue and the like as a subroutine of the extremity and trunk impedance measurement process flow of Fig. 67.

Fig. 69 is a diagram illustrating a basic flowchart for measurements of the visceral and subcutaneous fat tissues of the trunk according to an example of the present invention.

Fig. 70 is a diagram illustrating a splanchnic organ tissue volume and splanchnic organ tissue impedance estimation process flow as a subroutine of the basic flow of Fig. 69.

Fig. 71 is a diagram illustrating a visceral fat tissue impedance and visceral fat tissue volume estimation process flow as a subroutine of the basic flow of Fig. 69.

Fig. 72 is a diagram illustrating a trunk impedance measurement process flow as a subroutine of the basic flow of Fig. 69.

Fig. 73 is a diagram illustrating a subcutaneous fat tissue volume estimation process flow as a subroutine of the basic flow of Fig. 69.

Fig. 74 is a schematic perspective view of the appearance of an example of a truncal visceral/subcutaneous fat measuring apparatus according to the present invention.

Fig. 75 is a schematic diagram illustrating a use form of the apparatus of Fig. 74 when the visceral and subcutaneous fats of the trunk are measured by use of the apparatus of Fig. 74.

Fig. 76 is a schematic diagram illustrating an abdomen contacting electrode section as another embodiment which can be substituted for an abdomen contacting electrode section in the apparatus of the example of Fig. 74.

Fig. 77 is a schematic diagram illustrating a use form of the abdomen contacting electrode section of Fig. 76.

Fig. 78 is a block diagram illustrating the constitution of the apparatus of Fig. 74.

Fig. 79 is a diagram illustrating a novel lead system for obtaining subcutaneous fat tissue layer information.

Fig. 80 is a diagram illustrating a novel lead system for obtaining subcutaneous fat tissue layer information.

Fig. 81 is a diagram illustrating a novel lead system for obtaining subcutaneous fat tissue layer information.

Fig. 82 is a diagram illustrating a novel lead system for obtaining subcutaneous fat tissue layer information.

Fig. 83 is a diagram illustrating a novel lead system for obtaining subcutaneous fat tissue layer information.

Fig. 84 is a diagram illustrating a novel lead system for obtaining subcutaneous fat tissue layer information.

Fig. 85 is a diagram illustrating an example of arrangement of electrodes on the optimum subcutaneous fat tissue measured body part in the present invention.

Fig. 86 is a diagram illustrating a basic flowchart for measurements of the visceral and subcutaneous fat tissues of the trunk according to an example of the present invention.

Fig. 87 is a diagram illustrating a trunk impedance measurement process flow as a subroutine of the basic flow of Fig. 86.

Fig. 88 is a schematic perspective view of the appearance of a first example of a truncal visceral fat measuring apparatus according to the present invention.

Fig. 89 is a schematic diagram illustrating a use form of the apparatus of Fig. 88.

Fig. 90 is a block diagram illustrating the basic constitution of the apparatus of Fig. 88.

Fig. 91 is a block diagram illustrating another constitution of the apparatus of Fig. 88.

Fig. 92 is a block diagram illustrating still another constitution of the apparatus of Fig. 88.

Fig. 93 is a schematic perspective view of the appearance of a second example of the truncal visceral fat measuring apparatus according to the present invention.

Fig. 94 is a schematic diagram illustrating a use form of the apparatus of Fig. 93.

Fig. 95 is a schematic diagram illustrating the appearance of a third example of the truncal visceral fat measuring apparatus according to the present invention and a use form thereof.

Fig. 96 is an enlarged view of a portion of the apparatus of Fig. 95.

Fig. 97 is a schematic perspective view of the appearance of a fourth example of the truncal visceral fat measuring apparatus according to the present invention.

Fig. 98 is a schematic diagram illustrating a use form of the apparatus of Fig. 97.

Fig. 99 is a schematic perspective view of a portion of a fifth example of the truncal visceral fat measuring apparatus according to the present invention.

Fig. 100 is a schematic perspective view of another portion of the apparatus of Fig. 99.

Fig. 101 is a diagram illustrating an electrode arrangement example involving the upper extremities.

Fig. 102 is a diagram illustrating an electrode arrangement example involving the upper extremities.

Fig. 103 is a diagram illustrating an electrode arrangement example involving the lower extremities.

Fig. 104 is a diagram illustrating an electrode arrangement example involving the lower extremities.

Fig. 105 is a diagram illustrating an electrode arrangement example of a shared electrode.

Fig. 106 is a diagram illustrating an electrode arrangement example of the shared electrode.

Fig. 107 is a diagram illustrating an electrode arrangement example of the shared electrode.

Fig. 108 is a diagram illustrating an electrode arrangement example involving electrodes disposed on the abdomen of the trunk.

Fig. 109 is a diagram illustrating an electrode arrangement example involving electrodes disposed on the abdomen of the trunk.

Fig. 110 is a diagram illustrating an electrode arrangement example involving electrodes disposed on the abdomen of the trunk.

Fig. 111 is a diagram illustrating an electrode arrangement example involving electrodes disposed on the abdomen of the trunk.

Fig. 112 is a diagram illustrating a trunk impedance measurement process flow as a subroutine of a basic flow of an example of the present invention.

DETAILED DESCRIPTION OF THE PREFERRED EMBODIMENTS

The principle of measurement of the visceral fat tissue volume of the trunk according to the present invention will be described before the embodiments and examples of the present invention are described in detail. The present invention basically makes it possible to estimate visceral fat tissue information (cross-sectional area, volume or weight) of the abdomen (middle portion) of the trunk, the ratio (V/S) of truncal visceral fat tissue volume to subcutaneous fat tissue volume and the total fat tissue volume (truncal abdominal fat tissue volume) of subcutaneous fat tissue volume and visceral fat tissue volume, by use of bioelectrical impedance information and body specifying information of each segment which are obtained by a four-extremity lead system such as the upper extremities (arms), lower extremities (legs) and trunk (middle portion of the trunk).

Further, the present invention relates to a method capable of measuring information about fat tissues accumulated in the trunk, particularly, information about visceral fat tissues adhered and accumulated around splanchnic organ tissues, with ease and high accuracy.

In particular, the present invention makes it possible to measure information about fat tissues accumulated in the trunk, particularly, information about subcutaneous fat tissues accumulated in the subcutaneous layer with high accuracy and ease. That is, calculation of the subcutaneous fat tissue volume, particularly, cross-sectional area (CSA), of the trunk, requires measured data of the abdominal circumferential length and the subcutaneous fat tissue thickness. While the abdominal circumferential length or abdominal width or thickness can be secured by actual measurements, the subcutaneous fat tissue thickness varies depending on spots the abdominal circumference and varies significantly between individuals. Accordingly, it is important to specify a body part which is highly useful in estimating a subcutaneous fat tissue volume in making a measurement. When a volume is estimated as the subcutaneous fat tissue volume of the trunk, it can be calculated by multiplying the above CSA by the length information of the abdomen of the trunk. The truncal abdominal length can be estimated from body specifying information since it has high correlation with a body height.

In other words, in particular, the present invention makes it possible to estimate a subcutaneous fat tissue volume (CSA) on the navel circumference with high accuracy by use of abdominal circumferential length information and specific, highly contributing subcutaneous fat tissue thickness information. In this case, as the abdominal circumferential length information, abdominal width or thickness information on the navel circumference may be useful estimation variable information in addition to abdominal circumferential length. By multiple regression based on body specifying information (such as body height H, body weight W, SEX or AGE), highly reliable abdominal circumferential length information can be secured.

The present invention uses the following techniques accordingly.

(1) Tissue information included in the bioelectrical impedance information of the trunk is assumed by a series-parallel equivalent circuit model based on the skeletal muscle tissue layer, splanchnic organ tissues and visceral fat tissues. The splanchnic organ tissues and the visceral fat tissues are considered to be in series (hence, a change in the amount of applied current can be expected according to the size of the visceral fat tissues.)

(2) When abdominal circumferential length can be secured as body specifying information, a subcutaneous fat tissue volume is also included, and the tissue information is assumed by a high-accuracy series-parallel equivalent circuit model based on the subcutaneous fat tissue layer, skeletal muscle tissue layer, splanchnic organ tissues and visceral fat tissues.

(3) Estimation of the subcutaneous fat tissue volume is constituted by a multiple regression formula using abdominal circumferential length out of body specifying information as a main explanatory variable. Further, square of the abdominal circumferential length is set as a main explanatory variable. When a trunk impedance which predominantly has the layer thickness information of the subcutaneous fat tissue layer is obtained by a specific technique of the present invention, a product of the trunk impedance and the abdominal circumferential length (first power) is set as a main explanatory variable.

(4) The skeletal muscle tissue layer information of the abdomen (middle portion) of the trunk is exposed by use of bioelectrical impedance information and body specifying information of each segment which are obtained by a four-extremity lead system based on the upper extremities (arms) and the lower extremities (legs) and is used for confirmation of unconfirmed information for estimation of visceral fat tissue information.

(5) Confirmation of splanchnic organ tissue information is constituted by a multiple regression formula using body height information out of body specifying information as a main explanatory variable. It is used for confirmation of unconfirmed information for estimation of visceral fat tissue information.

(6) Reference measurements of tissues used for multiple regression analysis (calibration curve preparation technique) for quantifying tissues can be achieved by a tissue cross-sectional area (CSA: Cross-Section Area) from an X-ray CT tomographic image of navel area, CSA by MRI, and the tissue volume and weight (conversion of the volume to the weight can be calculated from tissue density information by previous research) using DEXA or MRI (integration processing in the longitudinal direction for each slice) in the whole abdomen of the trunk. DEXA can achieve reference measurement of the total fat tissue information of abdominal visceral fat tissues and the subcutaneous fat tissue layer.

(7) To acquire visceral fat tissue information with high accuracy by use of the above techniques, a measure to replace a change in measured impedance information of the trunk by breathing with a given condition value is required. An impedance measurement sampling period is set within a half of a general breathing cycle, a change in breathing is monitored with time, and a breathing cycle and the maximum and minimum values in the breathing cycle are determined in each breathing cycle, thereby making possible acquisition of the median in quiet breathing.

(8) Further, advance checking of the adverse effects by drinking and eating before measurement and retention of urine in the bladder is enabled by measured impedance information. In general, information about the skeletal muscle tissue layer is predominantly reflected in the impedance value of the trunk, in a group of ordinary healthy subjects. Further, the information about the skeletal muscle tissue layer of the trunk is very small as a measurement value and is not significantly different among individuals. The reason is that since it has design having a high correlation with antigravity muscles that develop by supporting the weights thereof under the gravity of the earth, it is determined mostly by a body size except for groups of special subjects such as subjects who are confined to bed and free from the influence of gravity and athletes of sports which impose stress whish is several times larger than the weights of the athletes on the athletes. Accordingly, in estimation of the skeletal muscle tissue volume of the abdomen of the trunk, a result with higher measurement sensitivity can be expected from the skeletal muscle tissues of the four extremities. In addition to the skeletal muscle tissues and the change in breathing, the adverse effects by drinking and eating and retention of urine in the bladder significantly influence the impedance of the abdomen of the trunk. Therefore, when the impedance values of the middle portions of the trunks are collected as group data and the mean value [mean] and deviation [SD] are checked, it is understood that the effects by drinking and eating and retention of urine in the bladder exceed 2SD. However, when quasi-general groups such as athletes of certain level are taken into account, the effects can be screened by setting 3SD as a criterion.

(9) Further, a changed body part by local inflammation in the skeletal muscle tissue layer and splanchnic organ tissues of the abdomen of the trunk can be specified to a certain extent by comparison of truncal abdominal impedance values obtained by four-extremity lead systems (four types) differing in energization route. Thus, it is considered to be possible to check the conditions of the splanchnic organ tissues and skeletal muscle tissue layer from the viewpoint of a change in body fluid distribution of the abdomen of the trunk which can be considered pathological such as inflammation.

Next, the measurement principle of the present invention based on the above techniques will be further described step by step.

1. Correlations between Sections of Trunk and Development of Skeletal Muscle Tissues

(1) When the trunk is divided into an upper section, a middle section and a lower section, the relationships between the sections of the trunk and development of skeletal muscle tissues are as follows.

(a) The upper section of the trunk has a high correlation with the upper extremities, particularly, skeletal muscle tissues in the proximal brachial regions.
(b) The lower section of the trunk has a high correlation with the lower extremities, particularly, skeletal muscle tissues of the upper arms in the proximal femoral regions.
(c) The middle section of the trunk has a high correlation with skeletal muscle tissues of the upper arms in the femoral regions of the lower extremities (since the proportions of psoas major muscles, iliopsoas muscles and the like which control the femoral regions of the lower extremities are large as occupied skeletal muscle tissues).

(2) Skeletal muscle tissues in the middle section of the trunk are constituted by abdominal muscles and back muscles and have functional development as joints between the upper and lower extremities (the joints deal with rotational movement in a clockwise or counterclockwise direction or bending and stretching movement in an anterior or posterior direction).

2. Estimation of Mid-Trunk Skeletal Muscle Tissues from Extremities

(3) Therefore, development of skeletal muscle tissues (tissue volume) of the middle portion of the trunk has a close relationship with development of skeletal muscle tissues in the upper and lower extremities. That is, the skeletal muscle tissue volume of the middle portion of the trunk can be estimated from the skeletal muscle tissue volumes of the upper and lower extremities. The skeletal muscle tissue volume of the middle portion of the trunk can be estimated by preparing a multiple regression formula by setting the skeletal muscle tissue volume of the middle portion of the trunk as an induced variable and the skeletal muscle tissue volumes of the upper and lower extremities as independent explanatory variables.

```
Mid-Trunk  Skeletal  Muscle  Tissue  Volume  [MMtm]  =  a0  x

Lower-Extremity  Skeletal  Muscle  Tissue  Volume  [MMl]  +  b0  x

Upper-Extremity  Skeletal  Muscle  Tissue  Volume  [MMu]  +  c0 ...

expression 1
```

wherein a0, b0 and c0 are regression coefficients and constants.
(4) Further, it is obvious from previous studies that the skeletal muscle tissue volumes of the extremities can be estimated from the measured impedance value of measurement section and the length information of the section.

$$\text{Lower-Extremity Skeletal Muscle Tissue Volume } [MMl] = a1 \times Ll^2/Zl$$
$$+ b1 \text{ ... expression 2}$$

$$\text{Upper-Extremity Skeletal Muscle Tissue Volume [MMu]} = a2 \times Lu^2/Zu + b2 \ldots \text{expression 3}$$

wherein a1, a2, b1 and b2 are regression coefficients and constants, Ll represents the length of the lower extremity, Lu represents the length of the upper extremity, Zl represents the impedance value of the lower extremity, and Zu represents the impedance value of the upper extremity.

(5) The lengths of the extremities may be estimated from body (individual) specifying information such as a body height, a body weight, sex and age in the case of an ordinary subject (Particularly, body height information for each sex is highly useful.)

(6) Similarly, by adding body specifying information such as sex, age and a body height as explanatory variables to the expressions for estimating the skeletal muscle tissue volumes of the upper and lower extremities and the skeletal muscle tissue volume of the middle portion of the trunk, qualitative changes in the nervous system and tissues due to growth and aging can be slightly corrected statistically.

(7) As standards in preparing the multiple regression estimation formula, skeletal muscle tissue volumes measured by an MRI method or a DEXA method are used.

(8) Further, a simpler technique which can be expected to achieve an accuracy improvement is a technique of incorporating the impedance information of the extremities directly into the expression for estimating the skeletal muscle tissue volume of the middle portion of the trunk, when trunk and extremity length information is not obtained by measurement but estimated from body specifying information such as a body height.

$$\text{Mid-Trunk Skeletal Muscle Tissue Volume [MMtm]} = a3 \times H^2/Zl + b3 \times H^2/Zu + c3 \ldots \text{expression 4}$$

wherein a3, b3 and c3 are regression coefficients and constants, H represents a body height, Zl represents the impedance value of the lower extremity, and Zu represents the impedance value of the upper extremity.

(9) As a method which can be expected to achieve a greater accuracy improvement, the following variations of the estimation expressions are conceivable, when trunk length information is further obtained by measurement. The expressions 1 to 3 can be modified as follows.

$$\text{Mid-Trunk Skeletal Muscle Tissue Volume [MMtm]} = a0' \times \text{Lower-Extremity Skeletal Muscle Tissue Volume [MMl]} \times Ltm^2/Ltm'^2 + b0' \times \text{Upper-Extremity Skeletal Muscle Tissue Volume [MMu]} \times Ltm^2/Ltm'^2 + c0' \ldots \text{expression 5}$$

wherein a0', b0' and c0' are regression coefficients and constants, Ltm represents a trunk length (or mid-trunk length), and Ltm' represents the estimated value of the trunk length (or mid-trunk length) from an extremity length or a body height.

$$Ltm' = aa \times Ll + bb \times Lu + cc \ldots \text{expression 5-1}$$

or

$$Ltm' = aa1 \times H + bb1 \ldots \text{expression 5-2}$$

wherein aa, aa1, bb, bb1 and cc are regression coefficients and constants.

(10) When a further accuracy improvement is expected, a method using information of portions proximal to the extremities may be used, although there is a disadvantage of increasing restrictions on measurement (degrading ease of measurement). By attaching or sticking voltage measuring electrodes to the knees and elbows, measurements can be carried out by the same extremity lead system as that used for measurements of the extremities. The reason is that information of proximal portions excluding remote portions is more useful than information of the lower or upper extremities from remote portions as information associated with the skeletal muscle tissues of the middle portion of the trunk. That is, the upper arms (upper-extremity proximal portions) have a high correlation with the upper portion of the trunk, the thighs (lower-extremity proximal portions) have a high correlation with the lower portion of the trunk, and the upper, middle and lower portions of the trunk also have a useful relationship therebetween. Accordingly, even in a method of measuring the skeletal muscle tissue volume of the thigh or upper arm with respect to the skeletal muscle tissue volumes of the upper and lower extremities and using the information for estimating the skeletal muscle tissue volume of the middle portion of the trunk, estimation expressions can be prepared in the same steps as those described above.

(11) The skeletal muscle tissues of the trunk may be regarded as follows.

(a) Since the upper extremities and the lower extremities have a high correlation, the upper portion of the trunk is regarded as the upper extremities, and the lower portion of the trunk is regarded as the lower extremities.
(b) The upper, middle and lower portions of the trunk as a whole are regarded as a trunk.

Since correlations between the above body parts are high, the above concepts do not differ significantly by choosing an ordinary healthy person or a nearly ordinary healthy person who can lead an independent life as a subject.

3. Formation of Electrical Equivalent Circuit Model of Trunk Constituting Tissues

(12) The impedance of the trunk which is determined by an extremity lead system is information of the middle portion of the trunk. This impedance will be described in detail in descriptions of examples to be described later.
(13) It can be considered that the trunk is primarily constituted by a subcutaneous fat tissue layer, a skeletal muscle tissue layer (abdominal muscles, back muscles), splanchnic organ tissues and visceral fat tissues adhered therebetween. Bone tissues are not named as constituting tissues because the bone tissues have a very high quantitative correlation with the skeletal muscle tissue layer and can be considered as an integrated tissue structure with the skeletal muscle tissue layer. It is assumed that as to volume-resistivity, conductivity becomes very high by including myeloid tissues in a living body and it has properties close to those of the skeletal muscle tissue layer and the splanchnic organ tissues. Therefore, when these four tissues are represented by an electrical equivalent circuit model, the splanchnic organ tissues and the visceral fat tissues are constituted in series, and the subcutaneous fat tissue layer and the skeletal muscle tissue layer are constituted in parallel with the serially combined tissue layer. This equivalent circuit model will be described in detail in descriptions of Examples to be described later. According to this model, a current passes predominantly through the skeletal muscle tissue layer when the current is passed in the longitudinal direction of the trunk. Because the visceral fat tissues adhere to gaps around the splanchnic organ tissues and because the splanchnic organ tissues shows electrical conductivity close to that of the skeletal muscle tissue layer when the visceral fat tissues do not exist or exist in small quantity, a current also passes through the splanchnic organ tissue side. Further, the larger the quantity of the visceral fat tissues becomes, the smaller the amount of a current passing through a combined tissue layer as a complex of the splanchnic organ tissues and the visceral fat tissues becomes. A model expression when the measured impedance of the mid-trunk and the four tissues constituting the mid-trunk are represented by an equivalent circuit model can be expressed as follows.

```
Ztm = ZFS // ZMM // (ZVM + ZFV) ... expression 6
```

impedance of entire mid-trunk: Ztm
impedance of subcutaneous fat tissue layer: ZFS ... volume-resistivity is high.
impedance of skeletal muscle tissue layer: ZMM ... volume-resistivity is low.
impedance of splanchnic organ tissues: ZVM ... volume-resistivity is considered to be close to that of skeletal muscle tissue layer.
impedance of visceral fat tissues: ZFV ... volume-resistivity is considered to be equal to or slightly lower than that of subcutaneous fat tissue layer. It is conceived that the quantity of blood vessels in the tissues and the amount of

EP 1 741 385 B1

blood are large due to faster synthesis and decomposition of fat tissues than subcutaneous fat tissues.
The electrical characteristics between tissues are determined by volume-resistivity ρ [Ωm] rather than impedance. According to the above relationships, the electrical characteristic values of the tissues are generally described by the following relationships.

$$\rho MM \ll \rho(VM + FV) < \rho FS$$

$$\rho VM \ll \rho FV$$

$$\rho MM = \rho VM, \ or, \ \rho MM < \rho VM$$

$$\rho FV = \rho FS, \ or, \ \rho FV < FS$$

volume-resistivity of subcutaneous fat tissue layer: ρFS
volume-resistivity of combined tissue layer of splanchnic organ tissues and visceral fat tissues inside skeletal muscle tissue layer: ρ(VM + FV)
volume-resistivity of skeletal muscle tissue layer: pMM
Therefore, the comparative relationship of the electrical characteristics between the tissues is conceived as follows.

$$ZFS \gg (ZVM + ZFV) \gg ZMM \ ... \ expression \ 7$$

The following two approaches are conceivable from the relational expressions 6 and 7 as techniques capable of estimating visceral fat tissue information.
(14) Approach 1
The subcutaneous fat tissue layer is omitted from the viewpoint of the equivalent circuit of the mid-trunk since it has high volume-resistivity as compared with other constituting tissues. That is, it can be considered that an impedance value measured in the mid-trunk includes measured information about fat free tissues including visceral fat tissues excluding the subcutaneous fat tissue layer of the mid-trunk. Therefore, this relational expression can be expressed as follows.

$$Ztm \fallingdotseq ZMM \ // \ (ZVM + ZFV) \ ... \ expression \ 8$$

The expression 8 can be converted into the following expression.

$$1/Ztm \fallingdotseq 1/ZMM + 1/(ZVM + ZFV) \ ... \ expression \ 9$$

The impedance ZFV of the visceral fat tissues can be calculated by exposing the impedance ZMM of the skeletal muscle tissue layer and the impedance ZVM of the splanchnic organ tissues by the following means. Then, a visceral fat tissue volume can be estimated from the impedance information of the visceral fat tissues. The expression 9 is turned into the following expression 10 by deriving ZFV from the expression 9, and impedance information having visceral fat tissue information can be determined.

$$ZFV = 1/[1/Ztm - 1/ZMM] - ZVM \ ... \ expression \ 10$$

(15) Approach 2

Although the subcutaneous fat tissue layer is not taken into consideration in the above approach 1, this can cause an error for subjects having a large quantity of the subcutaneous fat tissue layer. Hence, the approach 2 is a method following the expression 6 as it is.

The impedance ZMM of the skeletal muscle tissue layer and the impedance ZVM of the splanchnic organ tissues are the same as those in the above technique, and the impedance ZFS of the subcutaneous fat tissue layer has an useful relationship with a subcutaneous fat tissue volume as in the case of the other tissues. It is generally reported that the subcutaneous fat tissue volume has a very high correlation with circumferential length on the tissue surface, i.e. abdominal circumferential length (particularly for subjects having a large quantity of subcutaneous fat tissues or when the subcutaneous fat tissues are large as compared with fat free tissues excluding the subcutaneous fat tissues). Therefore, the subcutaneous fat tissue layer can be estimated from abdominal circumferential length information. Consequently, the impedance of the subcutaneous fat tissue layer can be estimated from the abdominal circumferential length information. Then, the impedance ZFV of the visceral fat tissues can be calculated in the same manner as in the above approach. Then, a visceral fat tissue volume can be estimated from the impedance information of the visceral fat tissues.

The expression 6 can be converted into the following expressions.

```
1/Ztm = 1/ZFS + 1/ZMM + 1/(ZVM + ZFV) ... expression 11
```

```
ZFV = 1/[1/Ztm - 1/ZMM - 1/ZFS] - ZVM ... expression 12
```

4 . Estimation of Impedance [ZVM] from Splanchnic Organ Tissue Volume [VM]

(16) The splanchnic organ tissue volume [VM] of the mid-trunk can be estimated from body (individual) specifying information such as a body height, a body weight, sex and age. Of explanatory variables, the influence of body height term is significant.

```
For Male: Splanchnic Organ Tissue Volume [VM] = a4 x Body Height
[H] + b4 x Body Weight [W] + c4 x age [Age] + d4 ... expression
13-1
```

```
For Female: Splanchnic Organ Tissue Volume [VM] = a5 x Body Height
[H] + b5 x Body Weight [W] + c5 x age [Age] + d5 ... expression
13-2
```

wherein a4, a5, b4, b5, c4, c5, d4 and d5 are regression coefficients and constants.

Measurement of the reference volume for the splanchnic organ tissue volume VM used in the calibration curve (regression formula) is a tissue volume obtained by integrating CSA (tissue cross-sectional area) for each slice obtained by MRI or X-ray CT in the longitudinal direction or CSA from one slice of the navel site or the like. The tissue volume can be converted into a tissue amount by converting tissue density information known by previous research papers and the like into weight.

(17) Next, the impedance ZVM of the splanchnic organ tissues is estimated.

For each tissue, a cylindrical model is used so that the relationship between an impedance and a tissue volume can be expressed by an expression. The expression used can be expressed as follows.

$$VM \propto LVM^2/ZVM \ldots \text{expression 14-1}$$

This expression can be converted into the following expression.

$$ZVM \propto LVM^2/VM \ldots \text{expression 14-2}$$

wherein LVM is the virtual cylinder length of the cylindrical model. Since LVM has a high correlation with a trunk length [Lt], a mid-trunk length [Ltm] and a body height [H], the following expression holds.

$$LVM \propto Lt \propto Ltm \propto H \ldots \text{expression 15}$$

Thus, when the body height H (Lt or Ltm may be used in the expression as long as measured information of the trunk is obtained) is used in place of LVM, the following expression holds.

$$ZVM = a6 \times H^2/VM + b6 \ldots \text{expression 16}$$

Thereby, the impedance ZVM of the splanchnic organ tissues can be estimated.
In the above expression, a6 and b6 are regression coefficients and constants.
Although the above expression 16 is a single regression formula, an improvement in estimation accuracy can be expected by converting the expression 16 into a multiple regression formula incorporating body specifying information as explanatory variables.

$$\text{For Male: } ZVM = a7 \times H^2/VM + b7 \times H + c7 \times W + d7 \times Age + e7 \ldots$$

$$\text{expression 17-1}$$

$$\text{For Female: } ZVM = a8 \times H^2/VM + b8 \times H + c8 \times W + d8 \times Age + e8 \ldots$$

$$\text{expression 17-2}$$

wherein a7, a8, b7, b8, c7, c8, d7, d8, e7 and e8 are regression coefficients and constants.

5. Estimation of Impedance [ZMM] from Skeletal Muscle Tissue Volume [MM]

(18) As the skeletal muscle tissue volume [MM] of the mid-trunk, the mid-trunk skeletal muscle volume [MMtm] from the extremity skeletal muscle tissue volume (extremity impedance information) obtained by the above expressions 1, 4 and 5 is used.

$$MM = MMtm \ldots \text{expression 18}$$

(19) Next, the impedance ZMM of the skeletal muscle tissue layer is estimated.
For each tissue, a cylindrical model is used so that the relationship between an impedance and a tissue volume can be expressed by an expression. The expression used can be expressed as follows.

$$MM \propto Ltm^2/ZMM \ldots \text{expression 19-1}$$

This expression can be converted into the following expression.

$$ZMM \propto Ltm^2/MM \quad ... \text{ expression 19-2}$$

wherein Ltm is a mid-trunk length when the cylindrical model is used.
Since Ltm has a high correlation with a trunk length [Lt] and a body height [H], the following expression holds.

$$Ltm \propto Lt \propto H \quad ... \text{ expression 20}$$

Thus, when the body height H (when the measured information Ltm or Lt of the trunk is not obtained) is used in place of Ltm, the following expression holds.

$$ZMM = a9 \times H^2/MM + b9 \quad ... \text{ expression 21}$$

Thereby, the impedance ZMM of the skeletal muscle tissue layer can be estimated.
In the above expression, a9 and b9 are regression coefficients and constants.
Although the above expression 21 is a single regression formula, an improvement in estimation accuracy can be expected by converting the expression 21 into a multiple regression formula incorporating body specifying information as explanatory variables as described above.

6. Estimation of Impedance [ZFS] from Subcutaneous Fat Tissue Volume [FS]

(20) The subcutaneous fat tissue volume [FS] of the mid-trunk can be estimated from abdominal circumferential length [Lw][2]. Further, an improvement in accuracy can be expected by adding other body specifying information as explanatory variables to form multiple regression formulae.

For Male: Subcutaneous Fat Tissue Volume [FS] = a10 × Abdominal Circumferential Length [Lw]$^2$ + b10 × Body Height [H] + c10 × Body Weight [W] + d10 × age [Age] + e10 ... expression 22-1

For Female: Subcutaneous Fat Tissue Volume [FS] = a11 × Abdominal Circumferential Length [Lw]$^2$ + b11 × Body Height [H] + c11 × Body Weight [W] + d11 × age [Age] + e11 ... expression 22-2

wherein a10, a11, b10, b11, c10, c11, d10, d11, e10 and e11 are regression coefficients and constants.
Measurement of the reference volume for the subcutaneous fat tissue volume FS used in the calibration curve (regression formula) is a tissue volume obtained by integrating CSA (tissue cross-sectional area) for each slice obtained by MRI or X-ray CT in the longitudinal direction or CSA from one slice of the navel site or the like. The tissue volume can be converted into a tissue amount by converting tissue density information known by previous research papers and the like into weight.
(21) Next, the impedance ZFS of the subcutaneous fat tissue layer is estimated.
For each tissue, a cylindrical model is used so that the relationship between an impedance and a tissue volume can be expressed by an expression. The expression used can be expressed as follows.

$$FS \propto Ltm^2/ZFS \ldots \text{expression 23-1}$$

This expression can be converted into the following expression.

$$ZFS \propto Ltm^2/FS \ldots \text{expression 23-2}$$

wherein Ltm is a mid-trunk length when the cylindrical model is used.
Since Ltm has a high correlation with a trunk length [Lt] and a body height [H], the following expression holds.

$$Ltm \propto Lt \propto H \ldots \text{expression 20}$$

Thus, when the body height H (when the measured information Ltm or Lt of the trunk is not obtained) is used in place of Ltm, the following expression holds.

$$ZFS = a12 \times H^2/FS + b12 \ldots \text{expression 24}$$

Thereby, the impedance ZFS of the subcutaneous fat tissue layer can be estimated.
In the above expression, a12 and b12 are regression coefficients and constants.
Although the above expression 24 is a single regression formula, an improvement in estimation accuracy can be expected by converting the expression 24 into a multiple regression formula incorporating body specifying information as explanatory variables as described above.

7. Estimation of Visceral Fat Tissue Volume [FV]

(22) The impedance [ZFV] of visceral fat tissues can be determined by substituting the measured impedance [Ztm] of the mid-trunk, the impedance [ZVM] of splanchnic organ tissues which has been obtained by the expressions 16 and 17, and the impedance [ZMM] of the skeletal muscle tissue layer which has been obtained by the expression 21 or the impedance [ZFS] of the subcutaneous fat tissue layer which has been obtained by the expression 24 into the expression 10 or 12.
(23) A visceral fat tissue volume [FV] is estimated from the impedance [ZFV] information of the visceral fat tissues. For each tissue, a cylindrical model is used so that the relationship between an impedance and a tissue volume can be expressed by an expression. The expression used can be expressed as follows.

$$FV \propto LFV^2/ZFV \ldots \text{expression 25}$$

wherein LFV is the virtual cylinder length of the cylindrical model. Since LFV has a high correlation with a trunk length [Lt], a mid-trunk length [Ltm] and a body height [H], the following expression holds.

$$LFV \propto Lt \propto Ltm \propto H \ldots \text{expression 26}$$

Thus, when the body height H (Lt or Ltm may be used in the expression as long as measured information of the trunk is obtained) is used in place of LFV, the following expression holds.

$$FV = a13 \times H^2/ZFV + b13 \ldots \text{expression 27}$$

Thereby, the visceral fat tissue volume FV can be estimated.

In the above expression, a13 and b13 are regression coefficients and constants.

Although the above expression 27 is a single regression formula, an improvement in estimation accuracy can be expected by converting the expression 27 into a multiple regression formula incorporating body specifying information as explanatory variables.

```
For Male: FV = a14 x H²/ZFV + b14 x H + c14 x W + d14 x Age + e14 ...

expression 28-1
```

```
For Female: FV = a15 x H²/ZFV + b15 x H + c15 x W + d15 x Age + e15 ...

expression 28-2
```

wherein a14, a15, b14, b15, c14, c15, d14, d15, e14 and e15 are regression coefficients and constants.

9. Estimation of Trunk Abdominal Fat Tissue Volume [FM]

(24) An abdominal fat tissue volume [FM] can be estimated from the subcutaneous fat tissue volume [FS] obtained by the expression 22 and the visceral fat tissue volume [FV] obtained by the expression 27 or 28.

```
FM = FS + FV ... expression 29
```

(25) As another method of estimating the abdominal fat tissue volume [FM], an abdominal fat tissue volume is measured by use of a DEXA method as standard measured information, and the abdominal fat tissue volume [FM] can be estimated by using principal parameters of the subcutaneous fat tissue volume [FS] obtained by the expression 22 and the visceral fat tissue volume [FV] obtained by the expression 27 or 28 as explanatory variables. That is, a multiple regression formula is prepared from abdominal circumferential length $[Lw]^2$, $H^2/ZFV$ and body specifying information.

9. Estimation of Trunk Abdominal Visceral Fat/Subcutaneous Fat Ratio [V/S]

(26) A visceral fat/subcutaneous fat ratio [V/S] can be determined from the subcutaneous fat tissue volume [FS] from the expression 22 and the visceral fat tissue volume [FV] from the expression 27 or 28.

```
V/S = FV/FS ... expression 30
```

10. Determination of Abnormality in Splanchnic Organ Tissues by Impedance of Abdomen (Middle Portion) of Trunk

(27) The impedance Ztm of the abdomen (middle portion) of the trunk which is required to estimate the visceral fat tissue volume requires measurement of information of high stability and reliability because it is a body part which changes significantly by breathing and drinking and eating. Thus, highly reliable impedance information of the abdomen of the trunk can be secured by performing the following processes. Further, determination of tissue abnormality in the abdomen of the trunk is also possible from the viewpoint as information associated with disturbance in body fluid distribution in the trunk.

(28) Process of Removing Influence of Change by Breathing

(a) The impedance of the abdomen of the trunk is measured in a sampling period which is shorter than 1/2 of general breathing cycle time.

(b) Measured data in each sampling is subjected to a smoothing process by moving average or the like.

(c) The periodicity of breathing and the maximum and minimum values in each period are detected from the processed time-series data.

(d) The maximum value and the minimum value in each period are averaged, respectively.

(e) The averaged maximum value and the averaged minimum value are averaged to calculate the mean value of breathing.

(f) It is determined that the mean value of breathing has been confirmed at the point when the mean value of breathing in each breathing cycle enters a stable range within a predetermined number of times, and the impedance value of the confirmed median value is registered as the impedance value of the abdomen of the trunk, thereby ending the measurement.

(29) Process of Determining Abnormal Value by Drinking and Eating and Retention of Water (e.g. Urine) in Bladder or the like

(a) An impedance of $26.7 \pm 4.8\Omega$ (mean$\pm$SD) of the abdomen of the trunk is a general value for a group.

(b) On the contrary, a value at the time of constipation or when urine is retained in the bladder or the stomach is filled with food and drink exceeds mean$\pm$3SD.

(c) Thus, when a measured value exceeding 3SD is obtained, a subject is informed of a possibility of the influence of drinking and eating, urine in the bladder and the like and is urged to make the measurement in the optimum environment. However, a subject whose skeletal muscle tissues have actually developed without their influence and whose splanchnic organ tissues are different from standard size is urged to continue the measurement.

(d) Further, as a method of increasing determination sensitivity, the specified value is subdivided according to sex, body weights and body heights. Alternatively, the specified value is specified as a value per unit by dividing it by a body weight or a body height.

(30) Process of Determining Abnormality in Abdominal Splanchnic Organ Tissues and the like

(a) In measurement of the impedance of the abdomen of the trunk, abnormality in the condition of splanchnic organ tissues or skeletal muscle tissues by illness or inflammation and specification of the corresponding body part can be detected based on small differences between measured impedances differing in routes of energization through the abdomen of the trunk by a difference in routes of energization from the extremities and a combination of voltage measuring electrode arrangements.

(b) A difference in trunk abdomen measured values from the following four lead systems is used as information for determination.

(c) The impedance value of the abdomen of the trunk by energization from the upper-left extremity is observed to be lower than that by energization from the upper-right extremity because of the influence of the heart situated to the left (the left lung is smaller than the right lung accordingly).

(d) It is considered a normal condition that measured values on the left and right sides are nearly symmetrical.

$$Ztmlr \fallingdotseq Ztmll < Ztmrr \fallingdotseq Ztmrl$$

In the above expression,

Ztmrr represents a between-upper-right-extremity-and-lower-right-extremity energization route trunk abdominal bioelectrical impedance,

Ztmlr represents a between-upper-left-extremity-and-lower-right-extremity energization route trunk abdominal bioelectrical impedance,

Ztmrl represents a between-upper-right-extremity-and-lower-left-extremity energization route trunk abdominal bioelectrical impedance, and

Ztmll represents a between-upper-left-extremity-and-lower-left-extremity energization route trunk abdominal bioelectrical impedance.

(e) When this relationship is not satisfied, there is a possibility that splanchnic organ tissues or skeletal muscle tissues and skeleton (bone and joint) tissues in the abdomen of the trunk are in a pathological condition or have inflammation.

(f) For example, when edema caused by inflammation is observed in the joint section at the base of the left thigh, Ztmlr > Ztmll and Ztmrr > Ztmrl hold.

(g) Further, when there is abnormality in the left intestine region due to constipation or the like, such a difference in balance as described above also occurs.

(h) Further, when water enters the right lung, Ztmlr ≒ Ztmll >= Ztmrr ≒ Ztmrl holds.

(i) In such a case, it is determined that there is abnormality in splanchnic organ tissues, skeletal muscle tissues, joint tissues or the like, and a subject is informed of the abnormality.

<Measurement of Subcutaneous Fat Tissue Volume or Selective Measurement of Subcutaneous Fat Tissue Volume and Visceral Fat Tissue Volume>

In measurement of subcutaneous fat tissue volume or selective measurement of subcutaneous fat tissue volume and visceral fat tissue volume, the following 11 to 16 are considered, in particular.

11. Formation of Electrical Equivalent Circuit Model of Trunk Constituting Tissues (Supplement 1)

Electrical characteristics between tissues are determined by volume-resistivity $\rho$ [$\Omega$m] rather than impedance. From the relationships described in "3. Formation of Electrical Equivalent Circuit Model of Trunk Constituting Tissues", the electrical characteristic value of each tissue is generally described by the following relationships.

$$\rho MM \; << \; \rho(VM+FV) \; < \; \rho FS$$

$$\rho VM \; << \; \rho FV$$

$$\rho MM \; = \; \rho VM, \; or, \; \rho MM \; < \; \rho VM$$

$$\rho FV \; = \; \rho FS, \; or, \; \rho FV \; < \; FS$$

volume-resistivity of subcutaneous fat tissue layer: $\rho FS$

volume-resistivity of composite tissue layer of splanchnic organ tissues and visceral fat tissues under skeletal muscle tissue layer:

$$\rho(VM+FV)$$

volume-resistivity of skeletal muscle tissue layer: $\rho MM$

Therefore, in association with the expression 6, the comparative relationship of the electrical characteristics between the tissues is conceived as follows.

$$ZFS \; >> \; (ZVM \; + \; ZFV) \; >> \; ZMM \; ... \; expression \; 31$$

12. Estimations of Trunk Skeletal Muscle Tissue Cross-Sectional Area (AMM) and Trunk Skeletal Muscle Tissue Layer Impedance (ZMM)

(32) A visceral fat tissue volume can be expressed by a cross-sectional area or a volume. In the case of the cross-sectional area, in measurement around the navel, a cross-sectional area by CT (X ray-CT, MRI) is considered to be a general measurement standard. Meanwhile, in the case of the volume, it can be determined by integrating a cross-sectional area by slicing by CT with a plurality of slice information in the longitudinal direction. A skeletal muscle tissue volume (skeletal muscle tissue volume) is considered to have a high correlation with both of the cross-sectional area and the volume. In this case, the cross-sectional area is considered. The cross-sectional area (AMM) of skeletal muscle tissues can be roughly estimated by body specifying information, because the development design of the skeletal muscle tissue layer of a body is mostly determined by development and adaptation for supporting its own weight under the gravity of the earth. Therefore, the cross-sectional area (AMM) can be estimated by body specifying information, except for those unadapted to gravity such as athletes, paralyzed patients and those who need nursing care. This estimation is made by substituting a body height H, a body weight W and age Age into the following expression:

```
AMM = a x H + b x W + c x Age + d ... expression 32
```

wherein a, b, c and d are constants.

(33) A trunk skeletal muscle tissue layer impedance (ZMM) can also be estimated by body specifying information. For the sake of convenience, the above estimated cross-sectional area (AMM) is used in this case. This estimation can be made by use of the following expression:

```
ZMM = a0 x H/AMM + b0 ... expression 33
```

wherein a0 and b0 are constants.

## 13. Estimations of Visceral Fat Tissue Impedance (ZFV) and Visceral Fat Tissue Volume (AFV)

The following two approaches are conceivable from the relational expressions 6 and 31 as techniques capable of estimating visceral fat tissue information.

(34) Approach 1

The subcutaneous fat tissue layer is omitted from the viewpoint of the equivalent circuit of the trunk since it has high volume resistivity as compared with other constituting tissues. That is, it can be considered that an impedance value measured in the trunk includes measured information about fat free tissues including visceral fat tissues excluding the subcutaneous fat tissue layer of the trunk. Therefore, this relational expression can be expressed as follows.

```
Ztm ≒ ZMM // (ZVM + ZFV) ... expression 34
```

The expression 34 can be converted into the following expression.

```
1/Ztm ≒ 1/ZMM + 1/(ZVM + ZFV) ... expression 35
```

The impedance ZFV of the visceral fat tissues can be calculated by exposing the impedance ZMM of the skeletal muscle tissue layer and the impedance ZVM of the splanchnic organ tissues by the following means. Then, a visceral fat tissue volume can be estimated from the impedance information of the visceral fat tissues. The expression 35 is turned into the following expression 36 by deriving ZFV from the expression 35, and impedance information having visceral fat tissue information can be determined.

```
ZFV = 1/[1/Ztm - 1/ZMM] - ZVM ... expression 36
```

(35) Approach 2

Although the subcutaneous fat tissue layer is not taken into consideration in the above approach 1, this can cause an error for subjects having a large quantity of subcutaneous fat tissues. Hence, the approach 2 is a method following the expression 6 as it is.

The impedance ZMM of the skeletal muscle tissue layer and the impedance ZVM of the splanchnic organ tissues are the same as those in the above technique, and the impedance ZFS of the subcutaneous fat tissue layer has an useful relationship with a subcutaneous fat tissue volume as in the case of the other tissues. It is generally reported that the subcutaneous fat tissue volume has a very high correlation with circumferential length on the tissue surface, i.e. abdominal circumferential length (particularly for subjects having a large quantity of subcutaneous fat tissues or when the subcutaneous fat tissues are large as compared with fat free tissues excluding the subcutaneous fat tissues). Therefore, the subcutaneous fat tissue layer can be estimated from abdominal circumferential length information. Consequently, the impedance of the subcutaneous fat tissue layer can be estimated from the abdominal circumferential length information. Then, the impedance ZFV of the visceral fat tissues can be calculated in the same manner as in the above approach. Then, a visceral fat tissue volume can be estimated from the impedance information of the visceral fat tissues.

The expression 6 can be converted into the following expressions.

```
1/Ztm = 1/ZFS + 1/ZMM + 1/(ZVM + ZFV) ... expression 37
```

```
ZFV = 1/[1/Ztm - 1/ZMM - 1/ZFS] - ZVM ... expression 38
```

(36) The visceral fat tissue volume (AFV) is treated as a visceral fat tissue cross-sectional area in this case. The visceral fat tissue volume (AFV) can be calculated from the above impedance information and body height information in the following expression 39.

```
AFV = aa x H/ZFV + bb ... expression 39
```

wherein aa and bb are constants.

14. Estimations of Splanchnic Organ Tissue Volume [AVM] and Splanchnic Organ Tissue Impedance [ZVM]

(37) The splanchnic organ tissue volume [VM] of the trunk can be estimated from body (individual) specifying information including a body height, a body weight, gender and age. Of explanatory variables, the influence of body height term is significant.

```
Splanchnic Organ Tissue Volume [AVM] = a1 x Body Height [H] + b1

x Body Weight [W] + c1 x age [Age] + d1 ... expression 40
```

wherein a1, b1, c1 and d1 are constants showing different values for a male and a female.

Measurement of the reference volume for the splanchnic organ tissue volume AVM used in the calibration curve (regression formula) is a tissue volume obtained by integrating CSA (tissue cross-sectional area) for each slice obtained by MRI or X-ray CT in the longitudinal direction or CSA from one slice of the navel site or the like. The tissue volume can be converted into a tissue amount by converting tissue density information known by previous research papers and the like into weight.

(38) Next, the impedance ZVM of the splanchnic organ tissues is estimated.

The impedance [ZVM] of the splanchnic organ tissues can be estimated from body (individual) specifying information including a body height, a body weight, gender and age. Of explanatory variables, the influence of body height term is significant. For the sake of convenience, the above estimated splanchnic organ tissue volume [AVM] is used in this case. This estimation can be made by use of the following expression.

```
ZVM = a2 x H/AVM + b2 ... expression 41
```

wherein a2 and b2 are constants.

### 15. Estimation of Subcutaneous Fat Tissue Volume [AFS]

(39) A method of measuring the subcutaneous fat tissue volume [AFS] of the trunk will be described later. Measurement of the reference volume for the subcutaneous fat tissue volume FS used in the calibration curve (regression formula) is a tissue volume obtained by integrating CSA (tissue cross-sectional area) for each slice obtained by MRI or X-ray CT in the longitudinal direction or CSA from one slice of the navel site or the like. The tissue volume can be converted into a tissue amount by converting tissue density information known by previous research papers and the like into weight.

### 16. Estimation of Trunk Visceral Fat/Subcutaneous Fat Ratio [V/S]

(40) A visceral fat/subcutaneous fat ratio [V/S] can be determined from a subcutaneous fat tissue volume [AFS] from the expression 22-1 or 22-2 or a subcutaneous fat tissue volume [AFS] from an expression 45 to be described later and a visceral fat tissue volume [AFV] from the expression 39.

```
V/S = AFV/AFS ... expression 42
```

<Measurement of Visceral Fat Tissue Volume and the like Using Rectus Abdominis Muscle Tissue Volume>

A visceral fat tissue volume can be measured by using rectus abdominis muscle tissues as a virtual electrode. When visceral fat is measured by using the rectus abdominis muscle tissues as a virtual electrode, the following 17 and 18 are considered, in particular.

### 17. Formation of Electrical Equivalent Circuit Model of Trunk Constituting Tissues (Supplement 2)

With respect to formation of an electrical equivalent circuit model of trunk constituting tissues, the following points are also considered, in addition to the above sections "3. Formation of Electrical Equivalent Circuit Model of Trunk Constituting Tissues" and "11. Formation of Electrical Equivalent Circuit Model of Trunk Constituting Tissues (Supplement 1)".

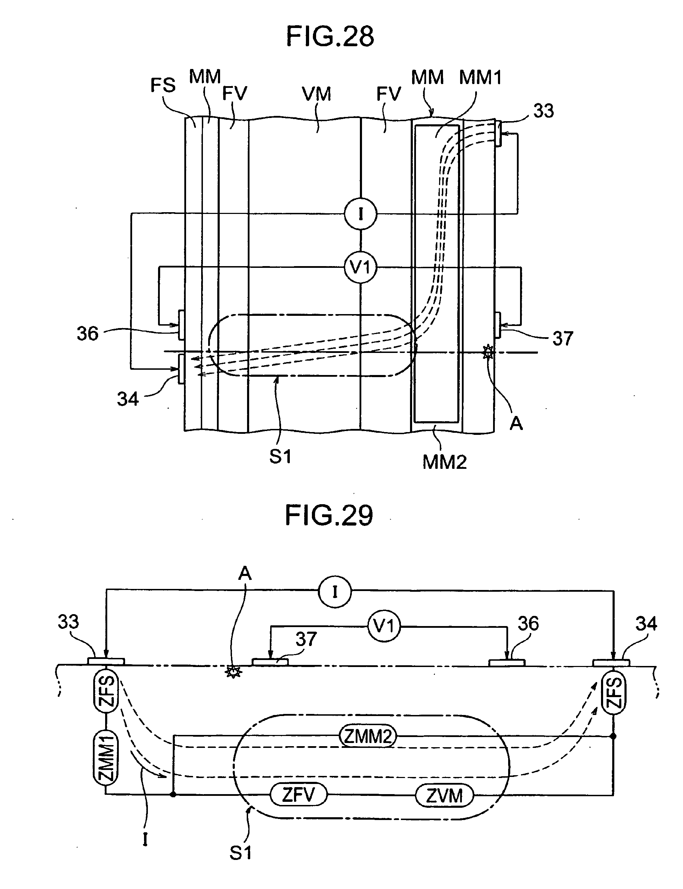

(41) When electrodes are merely disposed on the navel circumference in accordance with the conventional method, the impedance of the subcutaneous fat tissue layer is measured predominantly due to the influence of spreading resistance right underneath current applying electrodes, and separation and removal of the subcutaneous fat tissue layer by an ideal four-electrode technique has not been achieved. According to the present invention, the ideal four-electrode technique can be implemented and the impedance of the subcutaneous fat tissue layer can be deleted by applying novel electrode arrangement methods.
As will be described later, according to one electrode arrangement method of the present invention, the rectus abdominis muscle tissue layer is used as a virtual electrode. Accordingly, an electrical equivalent circuit model can be expressed as shown in Fig. 29 to be described later, i.e. as follows.

```
Ztm = 2 x ZFS + ZMM2 // (ZVM + ZFV) ... expression 43
```

(42) The skeletal muscle tissue layer (MM) is divided into the rectus abdominis muscle tissue layer (MM1) and the skeletal muscle tissue layer (MM2) in which obliquely running muscle fibers are predominant other than the rectus abdominis muscle tissue layer. ZMM1 and ZMM2 are impedances of MM1 and MM2, respectively. According to one electrode arrangement method of the present invention, since electrodes are placed at remote sites from current applying electrodes where the influence of spreading resistance around the current applying electrodes can be ignored, the subcutaneous fat tissue layer can be separated and removed, and since the rectus abdominis muscle tissue layer is used as a virtual electrode, the series impedance component of the rectus abdominis muscle tissue layer can be removed by securing a distance between the electrodes which corresponds to a drop in voltage in the rectus abdominis muscle tissue layer. Thus, when ZMM = ZMM2, the expression 43 can be expressed as follows.

```
Ztm = ZMM // (ZVM + ZFV) ... expression 44
```

18. Estimation of Subcutaneous Fat Tissue Volume [AFS]

(43) As described in the above section "6. Estimation of Impedance [ZFS] from Subcutaneous Fat Tissue Volume [FS]", the subcutaneous fat tissue volume AFS of the trunk can be estimated from abdominal circumferential length [Lw][2]. However, as shown below, it can also be estimated from the impedance information ZFS of the subcutaneous fat tissue layer and the abdominal circumferential length Lw.

```
Subcutaneous Fat Tissue Volume [AFS] = aa0 x ZFS x Lw + bb0 ...

expression 45
```

wherein aa0 and bb0 are constants.
A method of deriving the above expression 45 will be described later.

<Measurement of Visceral Fat Tissues and the Like Using Impedance of Rectus Abdominis Muscle Tissue Layer>

Visceral fat tissues and the like can be measured more accurately by use of the impedance of the rectus abdominis muscle tissue layer. In this case, the following 19 and 20 are also considered, in particular.

19. Estimations of Trunk Skeletal Muscle Tissue Cross-Sectional Area [AMM] and Trunk Skeletal Muscle Tissue Layer Impedance [ZMM]

(44) For estimations of trunk skeletal muscle tissue cross-sectional area (AMM) and trunk skeletal muscle tissue layer impedance (ZMM), the following concept is adopted, in addition to the above 12. The trunk skeletal muscle tissue layer impedance (ZMM) is estimated from the measured information of the impedance (ZMM1) of the rectus abdominis muscle tissue layer. The skeletal muscle tissue layer of the abdomen of the trunk is divided into muscles of the back and muscles of the abdomen. Since the muscles of the back are dominated by erector muscles, they are determined while one stands and lives under the gravity of the earth and have a high correlation with body specifying information accordingly. The information of muscle development associated with the activity of individual is apt to be seen in the muscles of the abdomen, and variations among individuals are also seen prominently in the muscles of the abdomen. The muscles of the abdomen are constituted by the rectus abdominis muscle tissue layer, the abdominal oblique muscle tissue layer and the like. The rectus abdominis muscle tissue layer is closely related with development of muscles. Further, in the present technique, the rectus abdominis muscle tissue layer can be acquired as impedance information. Accordingly, it can be estimated from the impedance information of the rectus abdominis muscle tissue layer and body specifying information, except for those unadapted to gravity such as athletes, paralyzed patients and those who need nursing care. This estimation is made by substituting the impedance ZMM1 of the rectus abdominis muscle tissue layer, a body height H, a body weight W and age Age into the following expression:

$$ZMM = a0 \times ZMM1 + b0 \times H + c0 \times W + d0 \times Age + e0 \ldots \text{expression } 46$$

wherein a0, b0, c0, d0 and e0 are constants showing different values for a male and a female.

20. Estimations of Visceral Fat Tissue Impedance [ZFV] and Visceral Fat Tissue Volume [AFV]

(45) The impedance ZFV of visceral fat tissues can also be estimated in the following manner, in addition to the method described in the above section "13. Estimations of Visceral Fat Tissue Impedance (ZFV) and Visceral Fat Tissue Volume (AFV)".
First, the expression 6 is converted into the following expression.

$$1/Ztm = 1/ZMM + 1/(ZVM + ZFV) \ldots \text{expression } 47$$

When ZFV is derived from the expression 47, the following expression is obtained, and impedance information having visceral fat tissue information can be obtained.

$$ZFV = 1/[1/Ztm - 1/ZMM] - ZVM \ldots \text{expression } 48$$

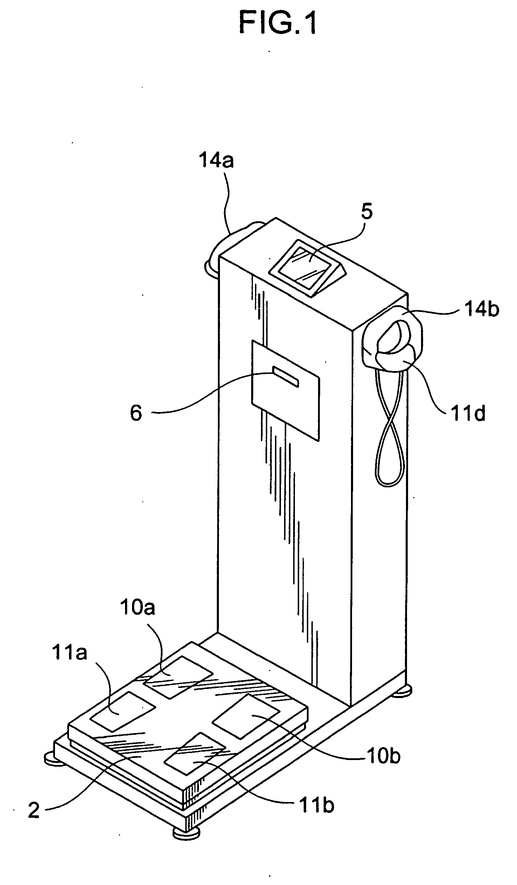

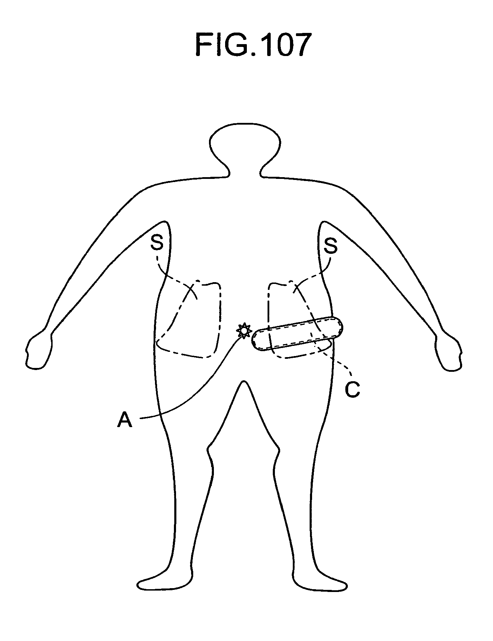

In the above expressions, Ztm is an actual measurement value. For the impedance ZMM of the skeletal muscle tissue layer, a two-frequency measured value can be used. Further, since the impedance ZMM of the skeletal muscle tissue layer of the trunk and the impedance ZVM of splanchnic organ tissues can be estimated as described above, ZFV can be extracted by substituting their estimated values. That is, it can be calculated by substituting the expressions 46 and 41 into the expression 48.
Next, examples of the truncal visceral/subcutaneous fat measuring method and apparatus of the present invention for measuring the visceral fat tissue volume and/or subcutaneous fat tissue volume of the trunk based on the above measurement principles of the present invention will be described. In particular, the examples of the present invention are divided into examples according to the first, second and third characteristics, and these examples will be described with reference to Figs. 1 to 73, Figs. 74 to 91 and Figs. 92 to 123, respectively.

Example According to the First Characteristic of the Invention

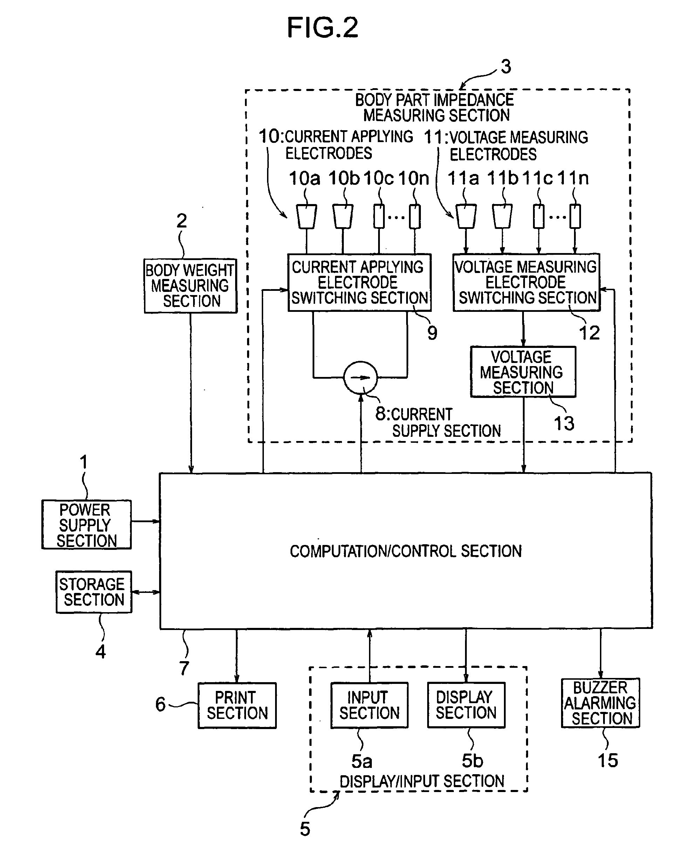

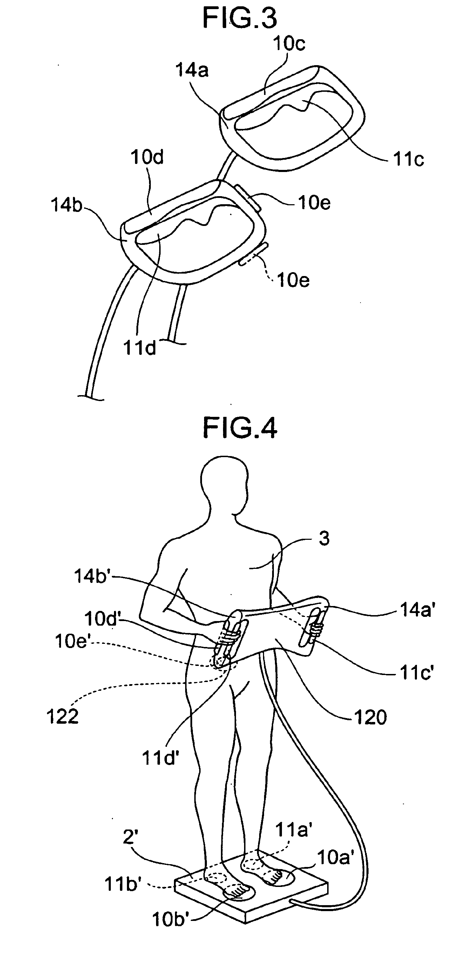

Fig. 1 is a schematic perspective view of the appearance of one example of a truncal visceral/subcutaneous fat measuring apparatus according to the present invention. Fig. 2 is a block diagram illustrating the constitution of the apparatus of Fig. 1. Fig. 3 is an enlarged perspective view of grip sections used in this apparatus. As shown in these drawings, the truncal visceral/subcutaneous fat measuring apparatus of the present example primarily comprises a power supply section 1, a body weight measuring section 2, a body part impedance measuring section 3, a storage section 4, a display/input section 5, a print section 6, and a computation/control section 7.
The power supply section 1 supplies electric power to the sections in the electrical system of the present apparatus.
The body weight measuring section 2 comprises a weight detecting section, an amplifying section and an AD converting section as in the case of a known scale and measures a voltage based on body weight specifying information (body weight).
The body part impedance measuring section 3 comprises a current supply section (single-frequency or two-frequency driven, for example) 8, a current applying electrode switching section 9, current applying electrodes 10 (10a to 10n) (n is an integer of larger than 5), voltage measuring electrodes 11 (11a to 11n) (n is an integer of larger than 4), a voltage measuring electrode switching section 12 and a voltage measuring section 13 as in the case of a known bioelectrical impedance measuring apparatus (such as a body fat meter or a body composition meter) and measures potential differences based on bioelectrical impedances between various body parts (various body part impedances).

The storage section 4 stores body specifying information such as a body height, extremity lengths, a trunk length and a mid-trunk length and the above expressions 1 to 48. Further, the storage section 4 stores appropriate messages for health guidance advice which will be described later.

The display/input section 5 comprises a touch-panel type liquid crystal display obtained by integration of an input section 5a and a display section 5b. A user inputs body specifying information including a body height through the display/input section 5, and the display/input section 5 also displays various results, advice information and other data. The print section 6 prints various results, advice information and other data displayed in the display section 5b.

The computation/control section 7 performs various inputs and outputs, measurements, computations and the like, such as computations of body weight specifying information (body weight), impedances of various body parts (such as an upper-extremity impedance, a lower-extremity impedance and a trunk impedance), mid-trunk skeletal muscle tissue volume, lower-extremity skeletal muscle tissue volume, upper-extremity skeletal muscle tissue volume, splanchnic organ tissue volume, subcutaneous fat tissue volume, visceral fat tissue volume, trunk abdominal fat tissue volume, abdominal fat tissue volume and trunk abdominal visceral fat/subcutaneous fat ratio and the like based on the above expressions 1 to 48 and the like, a process of removing the influence of change by breathing, a process of determining abnormality in splanchnic organ tissues, and the like.

As clearly shown in Fig. 1, the present apparatus has a nearly L-shaped appearance. The apparatus has the body weight measuring section 2 at its bottom, the display/input section 5 at its top, the print section 6 on it front side, and grip sections 14a and 14b on the left and right sides of its upper portion.

The body weight measuring section 2 has a left-foot current applying electrode 10a, a left-foot voltage measuring electrode 11a, a right-foot current applying electrode 10b and a right-foot voltage measuring electrode 11b. The grip section 14a has a left-hand current applying electrode 10c and a left-hand voltage measuring electrode 11c, and the grip section 14b has a right-hand current applying electrode 10d and a right-hand voltage measuring electrode 11d. Further, for example, the grip section 14b may have a current applying electrode 10e for the abdomen of the trunk as shown in Fig. 3 and may have a given number of additional current applying electrodes 10f to 10n and voltage measuring electrodes 11e to 11n as required in addition to these electrodes 10a to 10e and 11a to 11d as shown in Fig. 2. These electrodes are switched appropriately by the current applying electrode switching section 9 and the voltage measuring electrode switching section 12 as required and used.

When a user grips the grip sections 14a and 14b, the left and right hands of the user can contact the left-hand current applying electrode 10c and the left-hand voltage measuring electrode 11c and the right-hand current applying electrode 10d and the right-hand voltage measuring electrode 11d, respectively. Further, when the user stands on the body weight measuring section 2, the bottoms of the left and right feet of the user can contact the left-foot current applying electrode 10a and the left-foot voltage measuring electrode 11a and the right-foot current applying electrode 10b and the right-foot voltage measuring electrode 11b, respectively. Further, when the user moves the current applying electrode 10e of the grip section 14b while gripping the grip section 14b and presses the electrode 10e against the abdomen of the trunk and a current is applied from the electrode 10e, information of trunk abdominal tissues can be measured. Thus, while the present apparatus adheres to the grip electrodes 14a and 14b which symbolize easy measurement of body composition and the foot electrodes 10a, 10b, 11a and 11b provided on the platform of the scale, it has the additional electrode to be attached to the abdomen of the trunk which makes it possible to measure information of visceral fat tissues and subcutaneous fat tissues in the abdomen of the trunk as additional measured information, thereby making it possible to facilitate measurements on various body parts.

These current applying electrodes 10 and voltage measuring electrodes 11 may be implemented by metal-plating the surfaces of an SUS material and a resin material, for example. In the electrodes of this type, the surfaces of the metal electrodes are coated with a water-retentive polymer film, so that the electrodes are used in measurement after sprayed or wetted with water. By being wetted with water, the electrodes can secure stability in electrical contact with the skin. Further, although not particularly shown, electrodes of stickable type can also be used. These secure stability in contact with the skin by attaching a replaceable adhesive pad to the base electrode surfaces of the electrodes. The electrodes of this type are commonly used in low-frequency therapy equipment or as electrodes for electrocardiograms, for example. They are classified into a disposable form which is removed and disposed after measurement and a form which is disposed and replaced only when the pad surface has become dirty and lost adhesiveness or water has evaporated and protected by a cover sheet until disposed.

Further, a position at which the current applying electrode 10n or the voltage measuring electrode 11n is placed is not particularly limited. For instance, the current applying electrode 10e may be placed at any position at which the electrode 10e is conveniently pressed against the abdomen of a user and used. As shown in Fig. 3, the current applying electrode 10e may be positioned at a lateral side of a hand gripping the grip section. Alternatively, as shown by dotted lines in Fig. 3, the electrode 10e may be positioned at the back side of a hand gripping the grip section. With such a configuration, a simple apparatus which is easy to use can be implemented; that is, a compact and simple apparatus which can be pressed against the abdomen by a hand to make a measurement can be provided. However, it may take a form of being pressed against the abdomen by both hands. This form is advantageous in that the apparatus can be pressed stably.

Fig. 4 illustrates another example of the present apparatus shown in Figs. 1 to 3 in a simplified form. The basic configuration of this apparatus is the same as that shown in the block diagram of Fig. 2. The same members as those in the example shown in Figs. 1 to 3 are given the same numbers. In this example, an operation panel 120 is placed in front of a user 3. On the left and right sides of this operation panel 120, grip sections 14a' and 14b' are provided. As in the above example, the grip sections 14a' and 14b' are provided with a left-hand current applying electrode 10c' (not shown) and a left-hand voltage measuring electrode 11c' and a right-hand current applying electrode 10d' and a right-hand voltage measuring electrode 11d', respectively. Further, in this example, a current applying electrode 10e' for the abdomen is provided on a side face 122 on the right side of the abdomen of the user 3. With such a configuration, when the user 3 presses his abdomen 122 against a side face of the operation panel 120 while standing on a body weight measuring section 2' and gripping the grip sections 14a' and 14b', a current from the current applying electrode 10e' can also be used for measurement freehand.

Next, embodiments of electrode switching in measurement of only the impedance of the trunk out of measurements of bioelectrical impedances between various body parts by an extremity lead system used in the present apparatus will be particularly described.

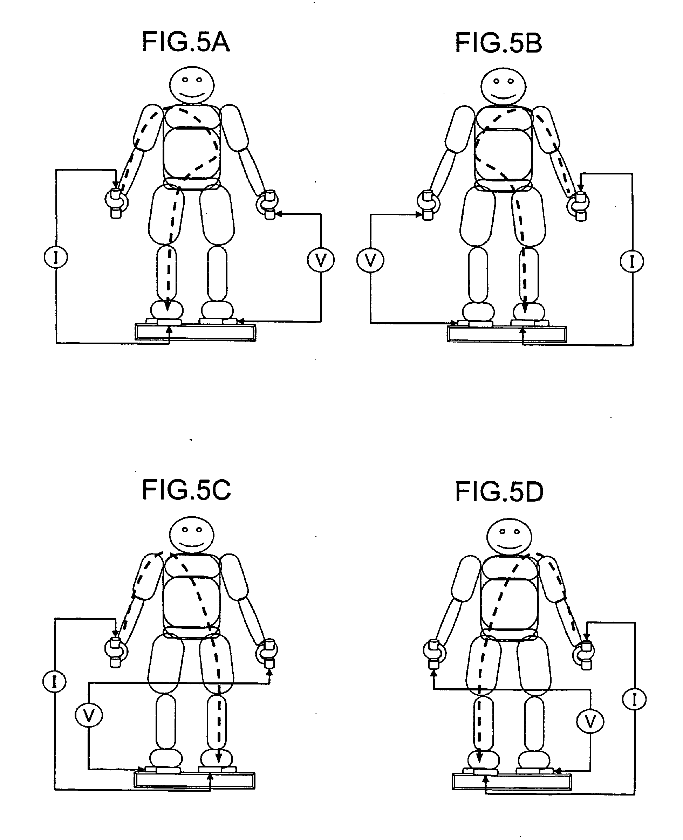

Fig. 5A illustrates a case where the impedance of the trunk is measured by passing a current between the right hand and the right foot and measuring a potential difference between the left hand and the left foot (illustrates a between-upper-right-extremity-and-lower-right-extremity energization route trunk abdominal bioelectrical impedance Ztmrr). In this case, the right-hand current applying electrode 10d and the right-foot current applying electrode 10b are used as current applying electrodes, and the left-hand voltage measuring electrode 11c and the left-foot voltage measuring electrode 11a are used as voltage measuring electrodes.

Fig. 5B illustrates a case where the impedance of the trunk is measured by passing a current between the left hand and the left foot and measuring a potential difference between the right hand and the right foot (illustrates a between-upper-left-extremity-and-lower-left-extremity energization route trunk abdominal bioelectrical impedance Ztmll). In this case, the left-hand current applying electrode 10c and the left-foot current applying electrode 10a are used as current applying electrodes, and the right-hand voltage measuring electrode 11d and the right-foot voltage measuring electrode 11b are used as voltage measuring electrodes.

Fig. 5C illustrates a case where the impedance of the trunk is measured by passing a current between the right hand and the left foot and measuring a potential difference between the left hand and the right foot (illustrates a between-upper-right-extremity-and-lower-left-extremity energization route trunk abdominal bioelectrical impedance Ztmrl) . In this case, the right-hand current applying electrode 10d and the left-foot current applying electrode 10a are used as current applying electrodes, and the left-hand voltage measuring electrode 11c and the right-foot voltage measuring electrode 11b are used as voltage measuring electrodes.

Fig. 5D illustrates a case where the impedance of the trunk is measured by passing a current between the left hand and the right foot and measuring a potential difference between the right hand and the left foot (illustrates a between-upper-left-extremity-and-lower-right-extremity energization route trunk abdominal bioelectrical impedance Ztmlr). In this case, the left-hand current applying electrode 10c and the right-foot current applying electrode 10b are used as current applying electrodes, and the right-hand voltage measuring electrode 11d and the left-foot voltage measuring electrode 11a are used as voltage measuring electrodes. Such electrode switchings in measurements of bioelectrical impedances between various body parts according to the extremity lead system are performed by the current applying electrode switching section 9 and the voltage measuring electrode switching section 12 under control of the computation/control section 7 with a subject (user) touching each electrode.

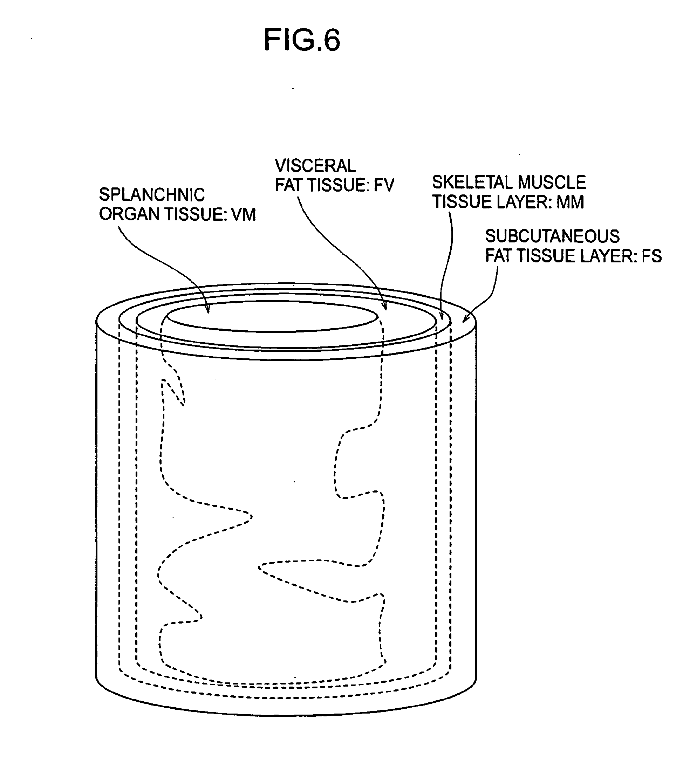

Fig. 6 is a diagram which schematically shows the structure of the abdomen (middle portion) of the trunk. Tissues constituting the abdomen of the trunk are considered to be a subcutaneous fat tissue layer (FS), a skeletal muscle tissue layer (MM), splanchnic organ tissues (VM) and visceral fat tissues (FV) which adhere therebetween. It is considered that when a current is passed through the trunk, most of the current passes through the skeletal muscle tissue layer. The reason is that the electrical conductivity of the skeletal muscle tissue layer is higher than those of other tissues. The splanchnic organ tissues are considered to be in series with the visceral fat tissues, and it is realized that a change in the amount of applied current can be expected according to the size of the visceral fat tissues.

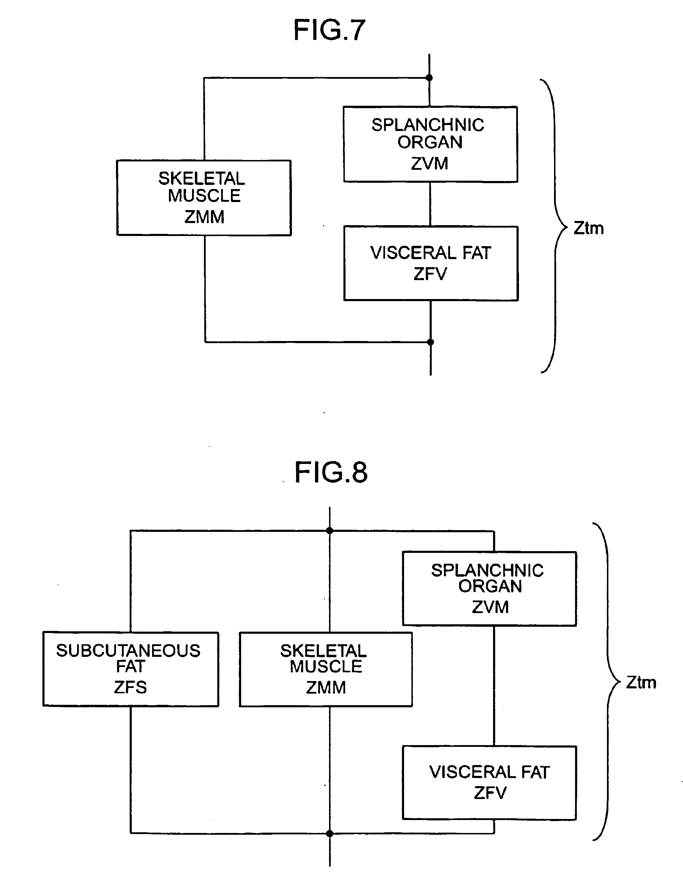

Fig. 7 is a diagram showing the structure of the abdomen of Fig. 6 as an electrical equivalent circuit. That is, Fig. 7 shows a simplified trunk abdomen equivalent circuit without the subcutaneous fat tissue layer. It is the trunk abdomen equivalent circuit considered in the technique of Approach 1 in (14) of the above "3. Formation of Electrical Equivalent Circuit Model of Trunk Constituting Tissues". Further, similarly, Fig. 8 is a diagram showing the structure of the abdomen of Fig. 6 as an electrical equivalent circuit. That is, Fig. 8 shows a trunk abdomen equivalent circuit without the subcutaneous fat tissue layer. It is the trunk abdomen equivalent circuit considered in the technique of Approach 2 in (15) of the above "3. Formation of Electrical Equivalent Circuit Model of Trunk Constituting Tissues". As for symbols used in these drawings, Ztm represents the impedance of the whole middle portion of the trunk, ZFS represents the impedance of the subcutaneous fat tissue layer, ZMM represents the impedance of the skeletal muscle tissue layer, ZVM represents the impedance of the splanchnic organ tissues, and ZFV represents the impedance of the visceral fat tissues, as described above. Further, as described above, with respect to the equivalent circuit of Fig. 7, the following relational expression holds.

$$\mathrm{Ztm} \fallingdotseq \mathrm{ZMM} \mathbin{/\mkern-5mu/} (\mathrm{ZVM} + \mathrm{ZFV})$$

With respect to the equivalent circuit of Fig. 8, the following relational expression holds.

$$\mathrm{Ztm} = \mathrm{ZFS} \mathbin{/\mkern-5mu/} \mathrm{ZMM} \mathbin{/\mkern-5mu/} (\mathrm{ZVM} + \mathrm{ZFV})$$

<Measurement of Subcutaneous Fat or Selective Measurement of Subcutaneous Fat and Visceral Fat>

Techniques used in measurement of subcutaneous fat or selective measurement of subcutaneous fat and visceral fat will be particularly described.

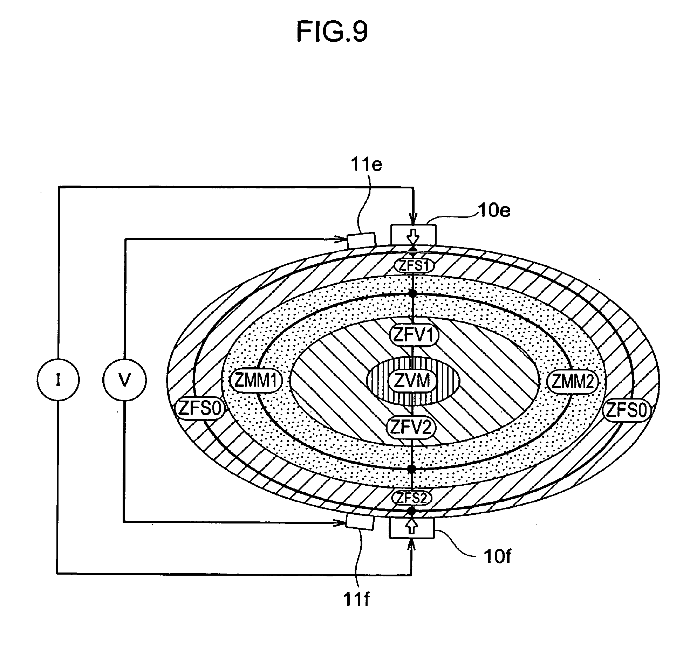

Fig. 9 is a cross-sectional view at the navel height of the trunk shown in Fig. 6. As shown in this drawing, the cross section of the trunk includes the outermost subcutaneous fat tissue layer (FS), the skeletal muscle tissue layer (MM) which is situated immediately medial to the subcutaneous fat tissue layer, the innermost splanchnic organ tissues (VM) and visceral fat tissues (FV) which surround the splanchnic organ tissues.

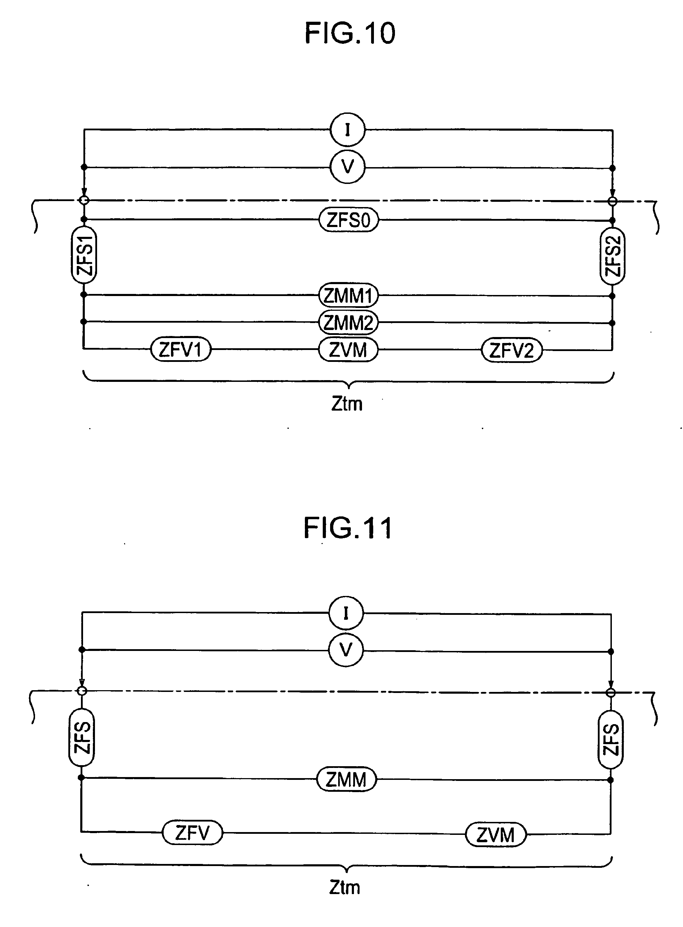

Fig. 10 is a diagram showing the schematic diagram shown in Fig. 9 as an electrical equivalent circuit. For example, when a current (I) is applied from the current applying electrodes 10e and 10f and a potential difference (V) is measured by the voltage measuring electrodes 11e and 11f, electrical resistances in this equivalent circuit appear primarily as the impedances (ZFS1, ZFS2) of the subcutaneous fat tissue layer on the front and back sides of the navel, the impedance (ZFS0) of the subcutaneous fat tissue layer around the abdomen, the impedances (ZMM1, ZMM2) of the skeletal muscle tissue layer on the left and right sides of the navel, the impedances (ZFV1, ZFV2) of the visceral fat tissues on the front and back sides of the navel, and the impedance (ZVM) of the splanchnic organ tissues in the middle of the trunk.

Fig. 11 is a diagram showing the circuit of Fig. 10 in a more simplified form. Since ZFS1 and ZFS2 are thought to have nearly the same size, they are indicated as the same value ZFS in this case, and ZMM1 and ZMM2 or ZFV1 and ZFV2 are indicated as ZMM and ZFV, respectively. Further, ZFS0 is omitted since its electrical conductivity is thought to be significantly lower than those of other regions. It should be clear from the description in the above (13) in "3. Formation of Electrical Equivalent Circuit Model of Trunk Constituting Tissues" that ZFS0 can be omitted.

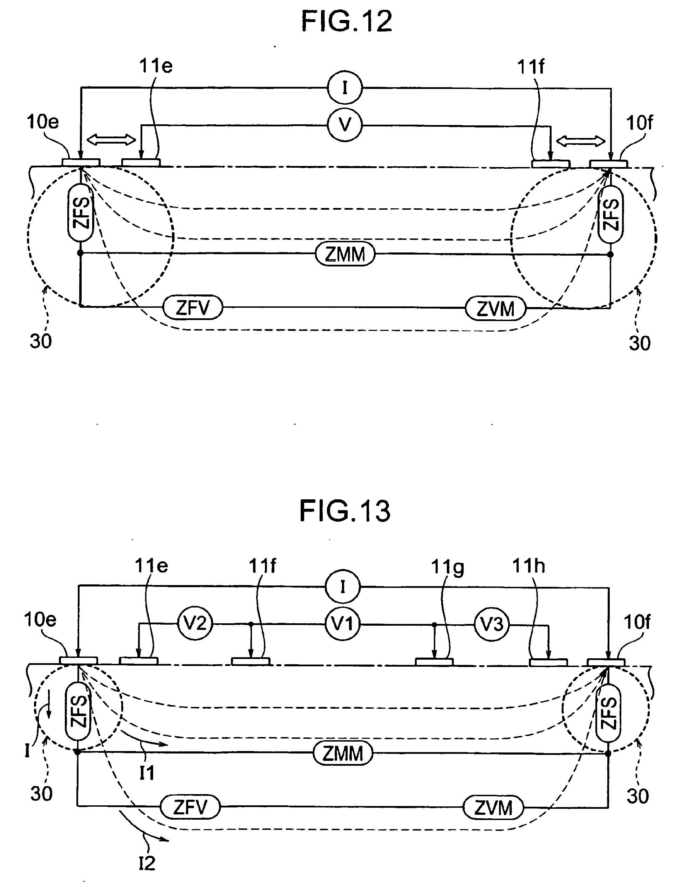

Next, the relationship between the distance between the electrodes and spreading resistance in a four-electrode technique will be described with reference to Fig. 12. Fig. 12 is a diagram showing the relationship between the distance between the electrodes and spreading resistance. In Fig. 12, regions 30 circled in a dotted line indicate spreading resistance regions. Although a current from the current applying electrodes gradually spreads through the body of a subject after applied, the current spreads not so widely in the regions immediately after application, i.e. the spreading resistance regions. Therefore, current density in these regions becomes very high as compared with other regions. Accordingly, when the current applying electrodes 10e and 10f and the voltage measuring electrodes 11e and 11f are disposed very close to each other, a potential difference measured by the voltage measuring electrodes 11e and 11f is influenced greatly by the current in the spreading resistance regions.

For example, as is obvious from the above expression 31, the impedance (ZFS) of the subcutaneous fat tissue layer around the navel, the impedance (ZFS0) of the subcutaneous fat tissue layer around the abdomen, the impedance (ZMM) of the skeletal muscle tissue layer, the impedance (ZFV) of the visceral fat tissues and the impedance (ZVM) of the splanchnic organ tissues in the middle of the trunk have the following relationship.

$$\mathrm{ZFS} \gg (\mathrm{ZVM} + \mathrm{ZFV}) \gg \mathrm{ZMM}$$

Therefore, a potential difference measuring impedance $\Sigma Z1$ when the current applying electrodes and the voltage measuring electrodes are disposed very close to each other with almost no distance therebetween is expressed as follows.

38

$$\Sigma Z1 = 2 \times ZFS + ZMM//(ZVM + ZFV) \fallingdotseq 2 \times ZFS$$

As is clear from this, since ZFS is amplified several times by the influence of spreading resistance, information by ZFS is dominant in this case.

To make the influence of spreading resistance small, the distance between the current applying electrodes and the voltage measuring electrodes must be large. For example, a potential difference measuring impedance $\Sigma Z2$ when the current applying electrodes and the voltage measuring electrodes are disposed with a distance of about 10 cm therebetween is expressed as follows.

$$\Sigma Z2 \fallingdotseq 2 \times ZFS + ZMM//(ZVM + ZFV)$$

As is obvious, although the influence of spreading resistance has been somewhat reduced by increasing the distance between the electrodes, the information by ZFS is still dominant.

To examine the influence of spreading resistance closely, a case where the electrodes 11e and 11f, the electrodes 11f and 11g, and the electrodes 11g and 11h are disposed with a distance of about 10 cm, i.e. about 1/3 of the distance between the electrodes 13L and 13R, therebetween, respectively, as shown in Fig. 13 is considered. However, the electrodes 10e and 11e and the electrodes 10f and 11h are disposed very close to each other with almost no distance therebetween. A potential difference measuring impedance $\Sigma Z3$ in this case is expressed as follows.

$$\Sigma Z3 \fallingdotseq 2 \times ZFS + ZMM//(ZVM + ZFV)$$

The relationship between voltage drops (potential differences) measured between the electrodes is roughly expressed as follows.

$$V1 = I \times ZMM//(ZVM + ZFV)$$

$$V2 = V3 = I \times 2 \times ZFS$$

$$V1:(V2+V3) \fallingdotseq 1\sim2:10\sim20 = S:N$$

Variations such as 1-2 in S and 10-20 in N in the above expression are ascribable to an individual difference in the thickness of the subcutaneous fat tissue layer and the degree of development of the skeletal muscle tissue layer. As is understood from these results, it is not ensured that satisfactory S/N can be secured even if the distance between the electrodes is adjusted.

Further, since most of current passes predominantly through the skeletal muscle tissue layer, sufficient sensitivity of energization through a composite tissue layer comprising splanchnic organ tissues and visceral fat tissues cannot be secured. That is, when a current passing through the skeletal muscle tissue layer is I1 and a current passing through the splanchnic organ tissues and visceral fat tissues to be measured is I2, the following expressions hold.

$$V1 = I \times ZMM//(ZVM + ZFV) = I1 \times ZMM = I2 \times (ZVM + ZFV)$$

$$I = I1 + I2$$

Therefore, the following expression holds.

$$ZMM:(ZVM + ZFV) = I2:I1 \fallingdotseq 1:2 \text{ to } 5$$

As is clear from this, even if the influence of spreading resistance can be eliminated, the current passing through the skeletal muscle tissue layer is two to five times larger than the current passing through the splanchnic organ tissues and the visceral fat tissues, so that the S/N property further deteriorates. Thus, in a thick and short body part to be measured such as the trunk, an improvement in the S/N property is limited, because the upper limit is determined by the distance between the current applying electrodes even if the distance between the electrodes is adjusted.

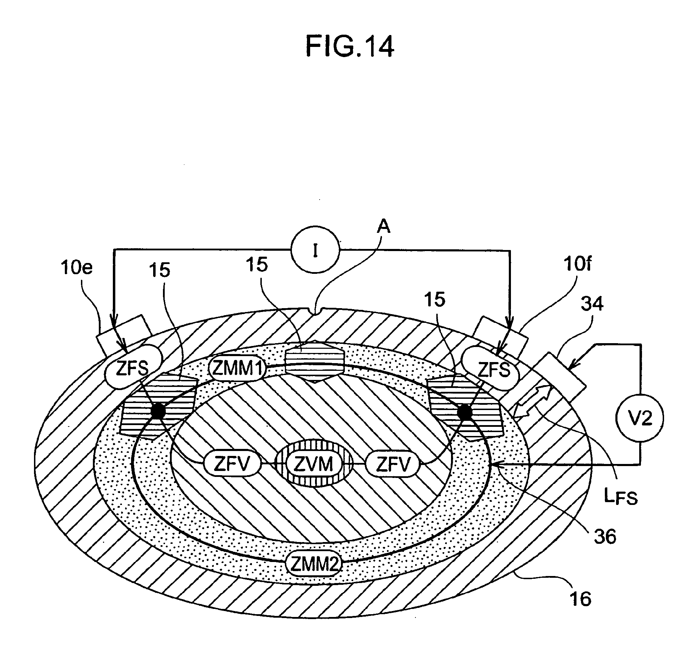

Fig. 14 shows an example of an electrode disposition method for acquiring subcutaneous fat tissue layer information in the same manner as in Fig. 6. Based on this electrode disposition method, subcutaneous fat tissue layer information and visceral fat tissue information can be measured simultaneously as independent informations. The apparatus of the present invention has voltage measuring electrodes for measuring visceral fat tissues and voltage measuring electrodes for measuring a subcutaneous fat tissue layer and can measure visceral fat tissue information and subcutaneous fat tissue layer information by switching an arrangement of these electrodes selectively by switching means. An object of measuring the informations simultaneously is to make it possible to relatively remove change error factors during measurement caused by breathing or the like by making both measurements in the same environment simultaneously, e.g. measuring the error factors at sampling timing faster than a change in breathing. Thus, the influences of heartbeat and other body motions other than breathing are also conceivable. The same obj ect can be achieved by a smoothing process in the same measurement environment, in addition to the increase in speed.

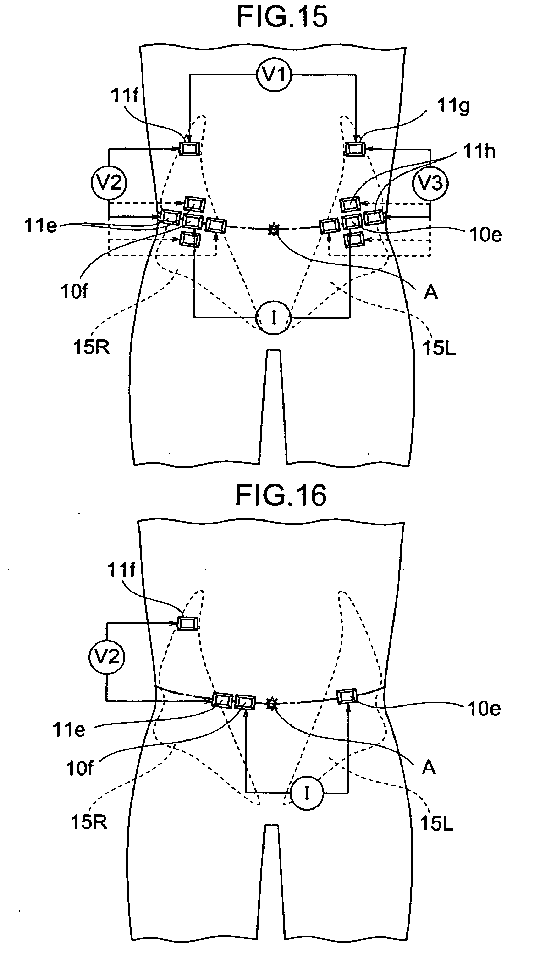

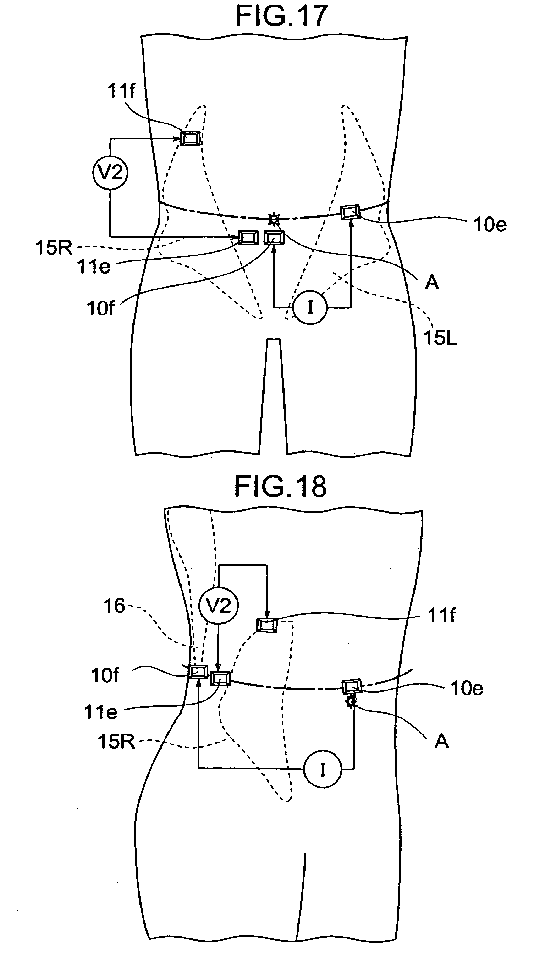

Figs. 15 to 18 show specific examples of electrode arrangements for acquiring subcutaneous fat tissue layer information (not visceral fat tissue information). Fig. 15 is a diagram illustrating an electrode arrangement example of measuring the impedance of the subcutaneous fat tissue layer right underneath current applying electrodes 10e and 10f disposed in the left and right aponeurosis sections 15, wherein V2 indicates a right-front-side subcutaneous fat tissue layer measured potential difference and V3 indicates a left-front-side subcutaneous fat tissue layer measured potential difference. Fig. 16 is a diagram illustrating an electrode arrangement example of measuring the impedance of the subcutaneous fat tissue layer right underneath the current applying electrode 10f disposed in the vicinity of the navel A, wherein V2 indicates an around-navel subcutaneous fat tissue layer measured potential difference. Fig. 17 is a diagram illustrating an electrode arrangement example of measuring the impedance of the subcutaneous fat tissue layer right underneath the current applying electrode 10f disposed under the navel A, wherein V2 indicates an under-navel subcutaneous fat tissue layer measured potential difference. Fig. 18 is a diagram illustrating an electrode arrangement example of measuring the impedance of the subcutaneous fat tissue layer right underneath the current applying electrode 10f disposed in a lateral region 16, wherein V2 indicates the subcutaneous fat tissue layer measured potential difference of the lateral region 16.

To acquire subcutaneous fat tissue layer information (more specifically, a potential difference value or an impedance value), spreading resistance is used in this case. The spreading resistance has been generally considered unfavorable. However, since it can be said that spreading resistance right underneath the current applying electrode in particular represents information about the subcutaneous fat tissue layer, useful subcutaneous fat tissue layer information can be acquired by measuring a potential difference in this region.

To measure spreading resistance, at least one pair of current applying electrodes and at least one pair of voltage measuring electrodes capable of measuring a potential difference which occurs in a subject by a current applied from the current applying electrodes are provided. One of the current applying electrodes, for example, a current applying electrode, is used to apply a current to a body part where the subcutaneous fat tissue layer is thin or a body part having no or a thin abdominal muscle portion of the skeletal muscle tissue layer, and the other current applying electrode, e.g. the current applying electrode 10a, is used to apply a current to a body part where the subcutaneous fat tissue layer is thick (or a subcutaneous fat tissue layer measured body part) .

Meanwhile, voltage measuring electrodes 11e included in the voltage measuring electrode pairs are disposed at a site where the influence of spreading resistance right underneath the current applying electrode is dominant, i.e., in the vicinity of the current applying electrodes. Meanwhile, the other voltage measuring electrodes 11f are disposed at a remote site where the influence of the spreading resistance right underneath the current applying electrode is weak (site remote from the current applying electrodes by at least three times the distance between the current applying electrode and the voltage measuring electrodes 11e), i.e., at a body part where the electrodes are not or hardly influenced by the subcutaneous fat tissue layer right underneath the current applying electrode. The former voltage measuring electrodes 11e may be disposed at a body part where the subcutaneous fat tissue layer is accumulated very thickly to the extent that the subcutaneous fat tissue layer reflects individual differences, such as around the navel, a lateral abdominal region (upper border of the iliac crest) or a lateral back region in the trunk, and the latter voltage measuring electrodes 11f may be disposed at a body part where the fat tissue layer is hardly accumulated to the extent that the subcutaneous fat tissue layer reflects individual differences, e.g. between the navel and the upper border of the iliac crest (near the aponeurosis between the external abdominal oblique muscle and the rectus abdominis muscle). The same information can be acquired regardless of where around the current applying electrode the voltage measuring electrodes are disposed. For instance, as shown in Fig. 15, the subcutaneous fat tissue layers on the left and right sides can be measured by disposing the voltage measuring electrodes in the vicinity of the left and right current applying electrodes which oppose each other with the navel and the backbone as the central axis.

The measurement values of potential differences V2 and V3 which occur between the voltage measuring electrodes 11e and 11f and between the voltage measuring electrodes 11g and 11h by a current applied from the current applying electrodes are considered impedance information which is proportional to the impedance (ZFS) value of the subcutaneous fat tissue layer and to the thickness ($L_{FS}$) information of the subcutaneous fat tissue layer. When the impedance of the spreading resistance is represented by $\Delta Z$ and a constant corresponding to the area of the current applying electrode is represented by A0, the following expression holds.

$$\Delta Z \propto ZFS \propto L_{FS}/A0 \propto L_{FS}$$

Thus, the cross-sectional area AFS of the subcutaneous fat tissue layer can be determined by the following expression.

$$AFS = Lw \times L_{FS} = aa0 \times ZFS \times Lw + bb0 \quad ... \quad expression\ 45$$

In the above expression, Lw represents an abdominal circumferential length, i.e. the length of the abdominal circumference, and aa0 and bb0 are constants showing different values for a male and a female.

To acquire visceral fat tissue information (e.g. a potential difference value or an impedance value) together with the subcutaneous fat tissue layer information, at least one more pair of voltage measuring electrodes which are disposed in a different arrangement from the voltage electrode arrangement for measuring the subcutaneous fat tissue layer information (thus, at least two pairs of voltage measuring electrodes are required to measure subcutaneous fat tissue information and visceral fat tissue information simultaneously.)

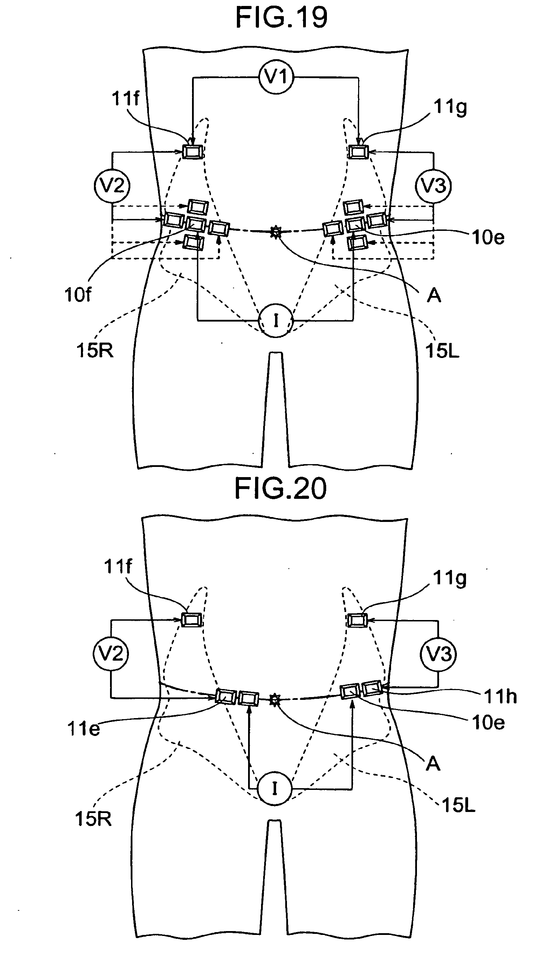

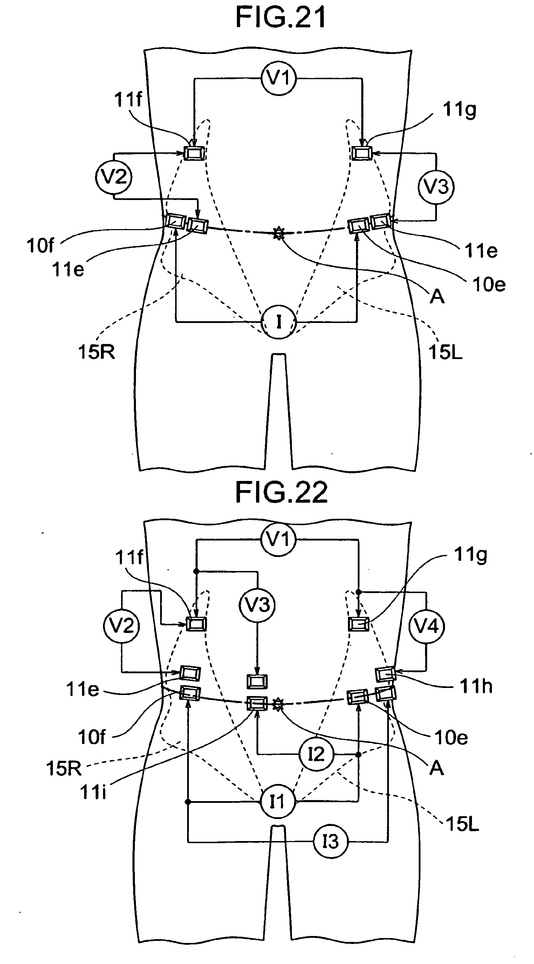

Figs. 19 to 22 show specific examples of electrode arrangements for measuring subcutaneous fat tissue layer information or both subcutaneous fat tissue layer information and visceral fat tissue information simultaneously (selectively). Fig. 19 is a diagram illustrating an electrode arrangement example of measuring visceral fat tissues and the impedance of the subcutaneous fat tissue layer right underneath current applying electrodes 13R and 13L disposed in the left and right aponeurosis sections 15R and 15L, wherein V1 indicates a visceral fat tissue measured potential difference, V2 indicates a right-front-side subcutaneous fat tissue layer measured potential difference and V3 indicates a left-front-side subcutaneous fat tissue layer measured potential difference. Since the tissue layer balance between the left and right sides of a living body are considered to be nearly symmetrical, V2 ≒ V3 holds, and the same measurement results are obtained by using any of voltage measuring electrodes disposed in four directions in the vicinity of the current applying electrodes. Fig. 20 is a diagram illustrating an electrode arrangement example of measuring the visceral fat tissue layer and the impedance of the subcutaneous fat tissue layer right underneath the current applying electrodes disposed in the vicinity of the navel A and the left aponeurosis section 15L, wherein V1 indicates a visceral fat tissue measured potential difference, V2 indicates an around-navel subcutaneous fat tissue layer measured potential, and V3 indicates a left-front-side subcutaneous fat tissue layer measured potential difference. Fig. 21 is a diagram illustrating an electrode arrangement example of measuring visceral fat tissues and the impedance of the subcutaneous fat tissue layer right underneath the current applying electrodes 10e and 10f disposed in the right and left aponeurosis sections 15L and 15R, wherein V1 indicates a visceral fat tissue measured potential difference, V2 indicates a right-abdominal-side subcutaneous fat tissue layer measured potential difference and V3 indicates a left-front-side subcutaneous fat tissue layer measured potential difference. Fig. 22 is a diagram illustrating an electrode arrangement example of measuring visceral fat tissues and the impedance of the subcutaneous fat tissue layer right underneath the current applying electrodes disposed at multiple sites, wherein V1 in combination with I1 indicates a visceral fat tissue measured potential difference, V2 in combination with I1 indicates a right-front-side subcutaneous fat tissue layer measured potential difference, V3 in combination with I2 indicates an around-navel subcutaneous fat tissue layer measured potential difference, and V4 in combination with I3 indicates a left-abdominal-side subcutaneous fat tissue layer measured potential difference.

Measurement of the visceral fat tissues is not direct measurement of the visceral fat tissues. The model of Fig. 9 or Fig. 10 is assumed, and a complex of the splanchnic organ tissues and the visceral fat tissues is measured. In the measurement of the visceral fat tissues, to secure an optimum S/N condition, the amounts of currents passing from the current applying electrodes through the splanchnic organ tissues and visceral fat tissues under the skeletal muscle tissue layer are increased to secure the measurement sensitivity for the tissues to be measured. Further, a current is applied from a body part where the subcutaneous fat tissue layer is thin or a body part where the skeletal muscle tissue layer has no or a thin muscle belly portion by the current applying electrodes to minimize the influence of spreading resistance and improve the sensitivity of energization through the splanchnic organ tissues and the visceral fat tissues. Further, when an abdominal circumferential cross-sectional area is a measurement reference, a body part to which a current is applied from the current applying electrodes 10e and 10f is a body part where the subcutaneous fat tissue layer is deposited the most thinly or a muscle joining area with good electrical conductivity where the skeletal muscle tissue layer has no or a little muscle belly portion. An example thereof is a tendinous portion (such as tendinous intersection or aponeurosis) 15. More specifically, the body part is a section between the navel and the upper border of the iliac crest or a tendinous portion (aponeurosis) between the rectus abdominis muscle and the external abdominal oblique muscle.

To acquire visceral fat tissue information as well as subcutaneous fat tissue layer information, additional voltage measuring electrode pairs, i.e. V1 in Figs. 19 to 21 and V1 and V3 in Fig. 22, are provided in the examples of Figs. 19 to 22. These additional voltage measuring electrode pairs are disposed at a site with good electrical conductivity where the subcutaneous fat tissue layer is thin or where the skeletal muscle tissue layer has no or a thin muscle belly portion. As is obvious from the drawings, the voltage measuring electrodes 11f and 11g included in the voltage measuring electrode pairs for acquiring subcutaneous fat tissue layer information can also be used as voltage measuring electrodes included in these additional voltage measuring electrode pairs for acquiring visceral fat tissue information. For example, in Fig. 19, the voltage measuring electrodes 11f and 11g included in the voltage measuring electrode pair V1 for acquiring visceral fat tissue information can also be used as voltage measuring electrodes included in the voltage measuring electrode pair V2 for acquiring subcutaneous fat tissue layer information or voltage measuring electrodes included in the voltage measuring electrode pair V3 for acquiring subcutaneous fat tissue layer information.

Thus, in the constitutions of Figs. 19 to 22, at least two voltage measuring electrode pairs, i.e. the voltage measuring electrode pair for measuring the subcutaneous fat tissue layer and the voltage measuring electrode pair for measuring visceral fat tissues are provided. A potential difference to be measured (any of V1 to V3), in other words, a body part be measured, can be selected easily by the voltage measuring electrode switching section 9 shown in Fig. 2. Therefore, visceral fat tissue information and subcutaneous fat tissue layer information can be easily measured separately by switching the electrode arrangement configuration having both an arrangement for measuring visceral fat tissues and an arrangement for measuring the subcutaneous fat tissue layer by the switching means.

As is obvious from the above description, in one aspect of the present invention, the best distance condition is secured by adopting the electrode arrangement off the navel circumference, and the impedance (ZFS) of the subcutaneous fat tissue layer is separated and removed as proper measurement of the four-electrode technique. Further, in another aspect of the present invention, not all of the four electrodes are disposed on the abdominal circumference, but at least one of them is disposed off the abdominal circumference so as to secure a more optimum S/N condition. As such a disposition method, for example, it is conceivable to dispose the current applying electrodes on the navel (abdominal) circumference and dispose one or both of the voltage measuring electrodes off the circumference, as shown in Figs. 19 to 22. Further, it is also possible to dispose one of the current applying electrodes on the navel (abdominal) circumference and dispose the other current applying electrode off the circumference. Further, the current applying electrodes or the voltage measuring electrodes may be disposed in the above sections, i.e. body parts where the subcutaneous fat tissue layer is thin, on the left and right sides when viewed with the navel of a subject as the center therebetween. However, the voltage measuring electrodes are disposed in the trunk longitudinal direction within an abdominal region off the navel (abdominal) circumference.

<Measurement of Visceral Fat and the like Using Aponeurosis>

Next, measurement of visceral fat and the like by use of the aponeurosis will be described.

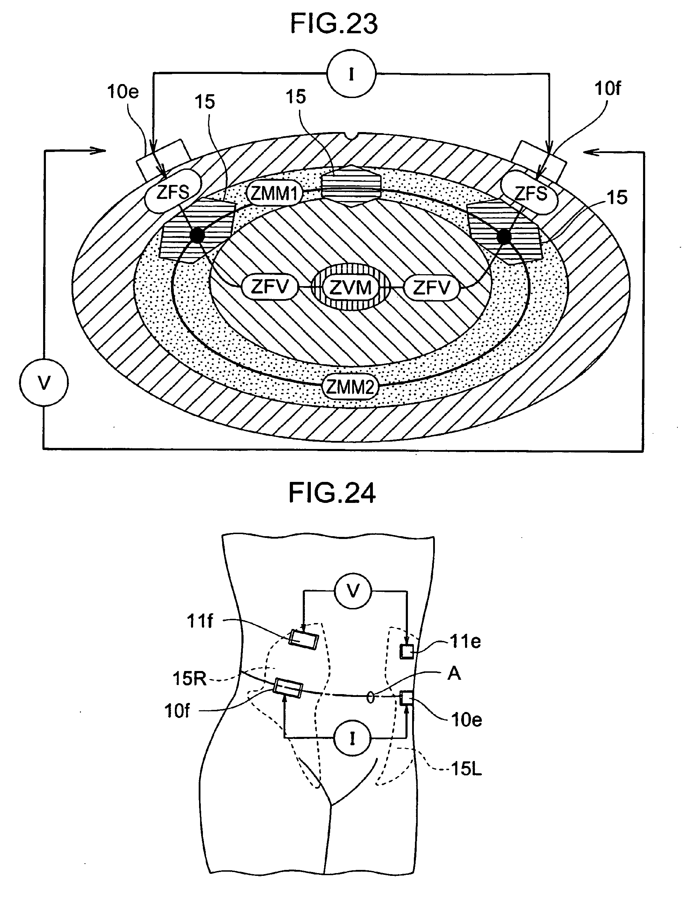

In an electrical equivalent circuit of Fig. 23, for example, when a current (I) is applied from the current applying electrodes 10e and 10f and a potential difference (V) is measured by the voltage measuring electrodes 11f and 11e, electrical resistances in this equivalent circuit appear primarily as the impedances (ZFS1, ZFS2) of the subcutaneous fat tissue layer on the front and back sides of the navel, the impedance (ZFS0) of the subcutaneous fat tissue layer around the abdomen, the impedances (ZMM1, ZMM2) of the skeletal muscle tissue layer on the left and right sides of the navel, the impedances (ZFV1, ZFV2) of the visceral fat tissues on the front and back sides of the navel, and the impedance (ZVM) of the splanchnic organ tissues in the middle of the trunk.

Fig. 23 shows an example of the electrode disposition method which can be used in the present invention, in the same manner as in Fig. 9 or Fig. 14. To secure an optimum S/N condition, the amounts of currents passing from the current applying electrodes 10e and 10f through the splanchnic organ tissues and visceral fat tissues under the skeletal muscle tissue layer are increased to secure the measurement sensitivity for the tissues to be measured. Further, a current is applied from a body part where the subcutaneous fat tissue layer is thin, in other words, a body part where the impedance (ZFS) of the subcutaneous fat tissue layer is small to minimize the influence of spreading resistance and improve the sensitivity of energization through the splanchnic organ tissues and the visceral fat tissues. To reduce the influence of the spreading resistance, measurement of potential difference by the voltage measuring electrodes 11e and 11f is preferably carried out in a body part where the influence of the subcutaneous fat tissue layer is small or the subcutaneous fat tissue layer is thin, in other words, a body part where the impedance (ZFS) of the subcutaneous fat tissue layer is small. Further, when an abdominal circumferential cross-sectional area is a measurement reference, a body part to which a current is applied from the current applying electrodes 10e and 10f is a body part where the subcutaneous fat tissue layer is deposited the most thinly or a skeletal muscle joining area where the skeletal muscle tissue layer has no or a thin muscle belly portion. An example thereof is a tendinous portion (such as tendinous intersection or aponeurosis) 15. More specifically, the body part is a section between the navel and the upper border of the iliac crest or a tendinous portion (aponeurosis) between the rectus abdominis muscle and the external abdominal oblique muscle.

Further, to secure the optimum S/N condition, not all of the four electrodes are preferably disposed on the abdominal circumference, but at least one of them is disposed off the abdominal circumference. By disposing the electrode off the navel circumference, the best distance condition can be secured, and the impedance (ZFS) of the subcutaneous fat tissue layer can be separated and removed as proper measurement of the four-electrode technique.

As such a disposition method, for example, it is conceivable to dispose the current applying electrodes on the abdominal (navel) circumference and dispose one or both of the voltage measuring electrodes off the abdominal (navel) circumference. Further, it is also possible to dispose one of the current applying electrodes on the abdominal (navel) circumference and dispose the other current applying electrode off the abdominal (navel) circumference. Further, the current applying electrodes or the voltage measuring electrodes may be disposed in the above sections, i.e. body parts where the subcutaneous fat tissue layer is thin, on the left and right sides when viewed with the navel A of a subject as the center therebetween. However, the voltage measuring electrodes are disposed in the trunk longitudinal direction within an abdominal region off the abdominal (navel) circumference.

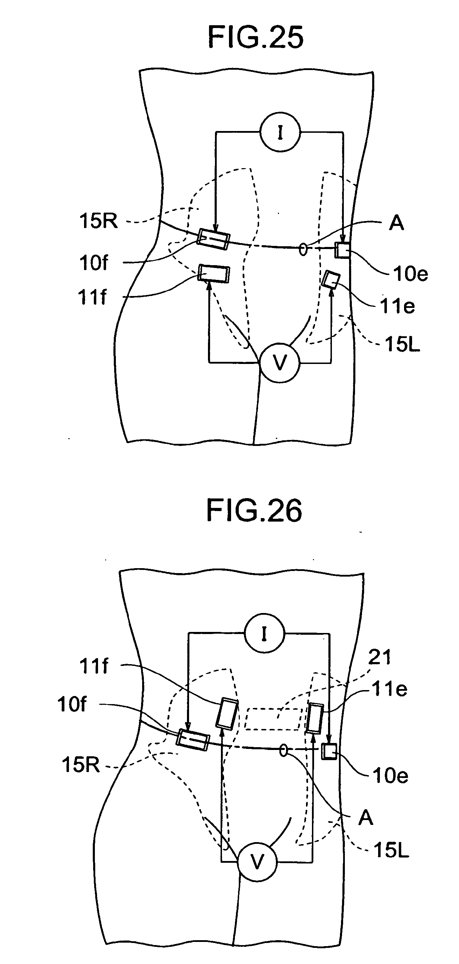

Figs. 24 to 26 show examples of actual electrode arrangements. Fig. 24 is a diagram showing the voltage measuring electrodes disposed above the navel circumference. Fig. 25 is a diagram showing the voltage measuring electrodes disposed below the navel circumference. Fig. 26 is a diagram showing the voltage measuring electrodes disposed above the navel circumference as in Fig. 24 and at the aponeurosis positions of tendinous intersections 21 slightly above the navel A of the rectus abdominis muscle.

<Measurement of Visceral Fat and the like Using Rectus Abdominis Muscle>

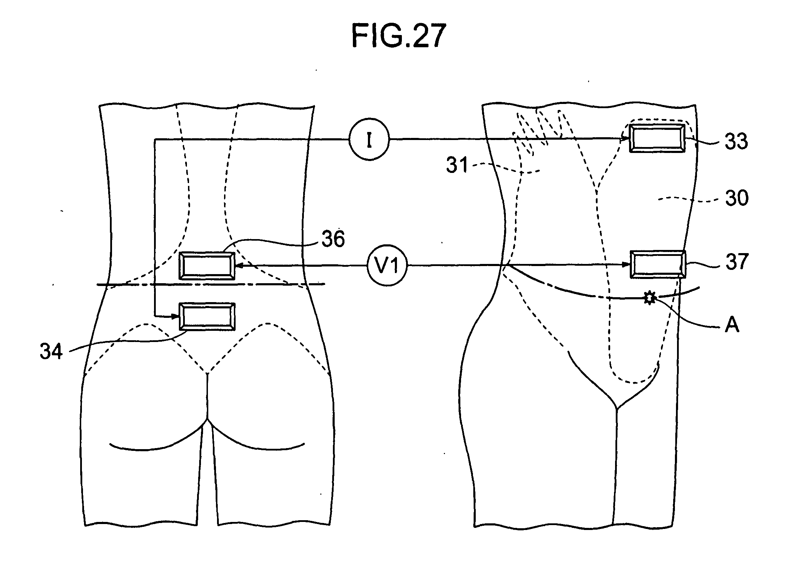

Next, measurement of visceral fat and the like by use of the rectus abdominis muscle will be described. By using the skeletal muscle (rectus abdominis muscle) tissue layer as a virtual electrode layer, the amounts of currents passing through the splanchnic organ tissues and visceral fat tissues under the skeletal muscle tissue layer can be increased so as to secure measurement sensitivity for visceral fat tissues. Figs. 27 to 29 show methods of disposing rectus abdominis muscle tissue layer virtual electrodes which can be used in the present invention. Fig. 27 is a diagram showing the actual structure of the abdomen of the trunk from the front side and back side thereof. Fig. 28 is a diagram which schematically shows the structure of Fig. 27. Fig. 29 is a diagram showing Fig. 27 as an equivalent circuit.

As shown in Fig. 27, muscle fibers in a rectus abdominis muscle tissue layer 50 are arranged regularly along the longitudinal direction of the trunk. When a current with a frequency of about 50 kHz which is highly sensitive to muscle fibers is passed along the length of the trunk, they become an electroconductive guide which leads the current predominantly to the rectus abdominis muscle tissue layer. Since muscle fibers in an external abdominal oblique muscle tissue layer 20 run obliquely, it has lower electrical conductivity than the rectus abdominis muscle tissue layer 50.

In particular, in the trunk longitudinal direction, in a section where a current passes through the trunk obliquely between under the epigastric fossa and the lower back, the muscle fibers in the external abdominal oblique muscle tissue layer 20 are arranged perpendicularly to the current passing direction, resulting in the highest volume-resistivity. Thus, their function as an electroconductive guide is low.

As shown in Fig. 28, the structure of the abdomen of the trunk is represented as a tissue layer structure comprising, from the outside toward the inside, a subcutaneous fat tissue layer (FS), a skeletal muscle tissue layer (MM), visceral fat tissues (FV) and splanchnic organ tissues (VM). This is as shown in Fig. 6. Further, the skeletal muscle tissue layer (MM) is divided into a rectus abdominis muscle tissue layer (MM1) and a skeletal muscle tissue layer (MM2) in which muscle fibers run obliquely.

The electrical characteristics between tissues are determined by volume-resistivity $\rho$ [$\Omega$m]. In consideration of the electrical characteristic values of the tissues of the rectus abdominis muscle tissue layer (MM1) and the skeletal muscle tissue layer (MM2), the electrical characteristic values of the tissues described in the above "3. Formation of Electrical Equivalent Circuit Model of Trunk Constituting Tissues", "11. Formation of Electrical Equivalent Circuit Model of Trunk Constituting Tissues (Supplement 1)" and "17. Formation of Electrical Equivalent Circuit Model of Trunk Constituting Tissues (Supplement 2)" are generally described by the following relationships.

$$\rho MM1 \ll \rho(VM+FV) < \rho FS$$

$$\rho VM \ll \rho FV$$

$$\rho MM1 < \rho MM2$$

$$\rho MM2 = \rho VM, \text{ or, } \rho MM2 < \rho VM$$

$$\rho FV = \rho FS, \text{ or, } \rho FV < \rho FS$$

volume-resistivity of subcutaneous fat tissue layer: $\rho FS$
volume-resistivity of composite tissue layer of splanchnic organ tissues and visceral fat tissues: $\rho(VM+FV)$
volume-resistivity of rectus abdominis muscle tissue layer in skeletal muscle tissue layer: $\rho MM1$
volume-resistivity of skeletal muscle tissue layer in which muscle fibers run obliquely other than rectus abdominis muscle tissue layer: $\rho MM2$

Therefore, by substituting the impedance information into the above expression 31, the expression can be expressed as follows.

$$ZFS \gg Z(VM+FV) > ZMM2 > ZMM1$$

impedance of subcutaneous fat tissue layer: ZFS
impedance of rectus abdominis muscle tissue layer in skeletal muscle tissue layer: ZMM1
impedance of skeletal muscle tissue layer in which muscle fibers run obliquely other than rectus abdominis muscle tissue layer: ZMM2 impedance of composite tissue layer of splanchnic organ tissues and visceral fat tissues: Z(VM+FV) = ZVM+ZFV

In the example shown in Figs. 27 to 29, a current applying electrode 33 is placed in the vicinity of the upper edge of the upper portion of the rectus abdominis muscle tissue layer under the epigastric fossa on the front side of the trunk, and the other current applying electrode 34 is placed on the lower back. An electrode 36 included in a voltage measuring electrode pair for measuring visceral fat tissues is placed at a remote site where the influence of spreading resistance around the current applying electrode can be ignored. The other electrode 37 is placed in the vicinity of the navel circumference on the rectus abdominis muscle tissue layer so as to exclude the series impedance component of the rectus abdominis muscle tissue layer.

When a current I is applied between the voltage 33 and the voltage 34, the current passes along the route indicated by dotted lines in Figs. 28 and 29. Therefore, a potential difference V1 which is measured by the electrodes 36 and 37 is a visceral fat tissue measured potential difference, and the measurement range of V1 is indicated by S1. At the selected positions of the current applying electrodes, i.e. under the epigastric fossa on the front side and on the lower back, the subcutaneous fat tissue layer is very thin. Thus, it is easy to secure the distance between the current applying electrode and the voltage measuring electrode for avoiding the influence of spreading resistance, and the distance may be small. Consequently, the following impedance is obtained from I and V1.

$$Ztm = V1/I = ZMM2 // (ZVM+ZFV)$$

In the above expression, Ztm represents a combined impedance of the abdomen of the trunk which is complemented by the voltage measuring electrodes 36 and 37. When ZMM = ZMM2 holds, the above expression is turned into the following expression.

$$Ztm = ZMM // (ZVM+ZFV)$$

This is the same as the above expression 44. Thus, a visceral fat tissue volume can be measured by the above technique.

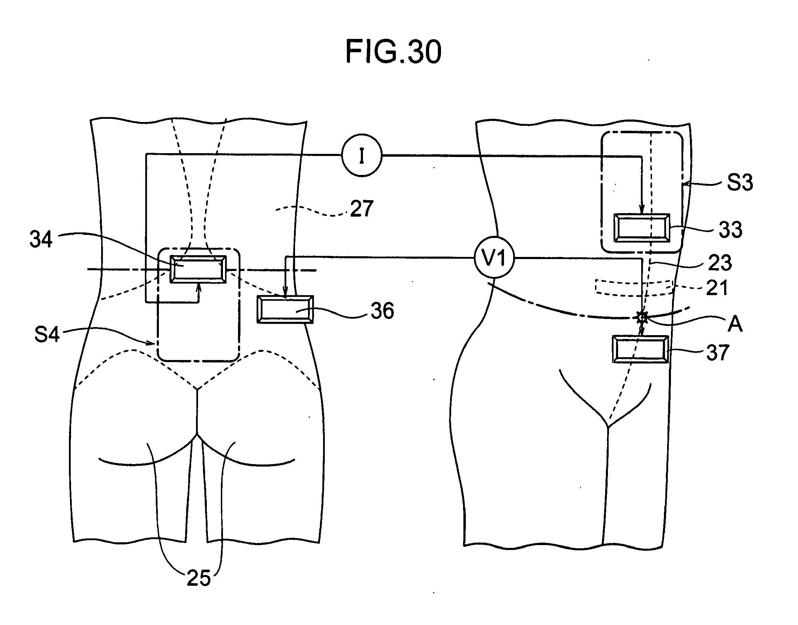

The position of the current applying electrode 33 on the front side may be as high as around the upper portion of the second section of the upper portion of the rectus abdominis muscle tissue layer 50 when the upper portion of the rectus abdominis muscle tissue layer is divided into six sections by the tendinous intersection 21, as shown in Fig. 30. In this case, the electrode width over the left and right rectus abdominis muscle tissue layers with a white line 23 therebetween is secured. The possible position of the current applying electrode 34 on the back side ranges from the position (height) of the navel A to the joined aponeurosis between the left and right gluteus maximus 25. Further, in Fig. 30, S3 and S4 indicate allowable ranges of the positions of the current applying electrodes 33 and 34.

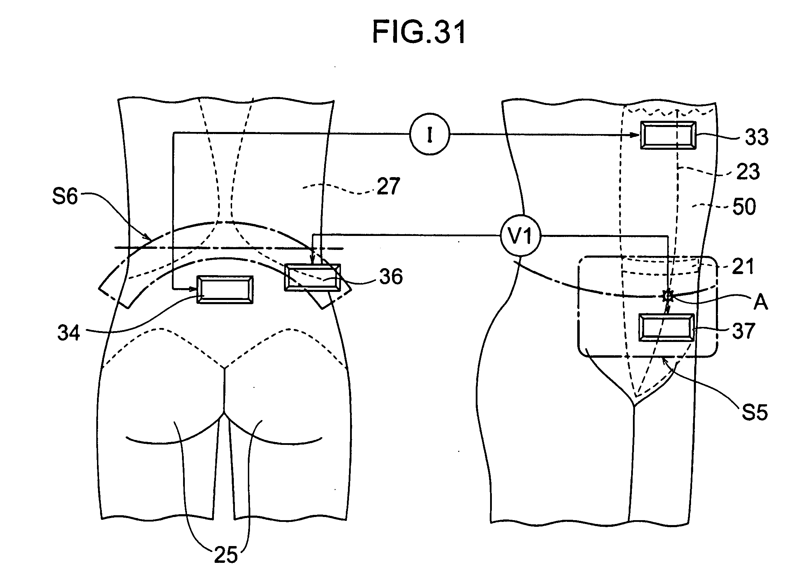

Further, the possible position of the voltage measuring electrode 37 on the front side ranges from slightly above the position of the navel to lower than the position of the navel on the rectus abdominis muscle tissue layer, as shown in Fig. 31. A portion of the joined aponeurosis between the external abdominal oblique muscle tissue layer and the rectus abdominis muscle tissue layer which is close to the rectus abdominis muscle tissue layer is also included in the allowable range of the position of the electrode 37. The possible position of the voltage measuring electrode 36 on the back side ranges over the joined aponeurosis between the back muscle 27 and the gluteus maximus 25 to the sides of the abdomen while securing the distance to avoid the influence of spreading resistance from the current applying electrodes. Further, in Fig. 31, S5 and S6 indicate allowable ranges of the positions of the voltage measuring electrodes 37 and 36.

The above visceral fat tissue measuring technique may be combined with a subcutaneous fat tissue layer measuring technique. A combination of the measuring techniques will be described hereinafter.

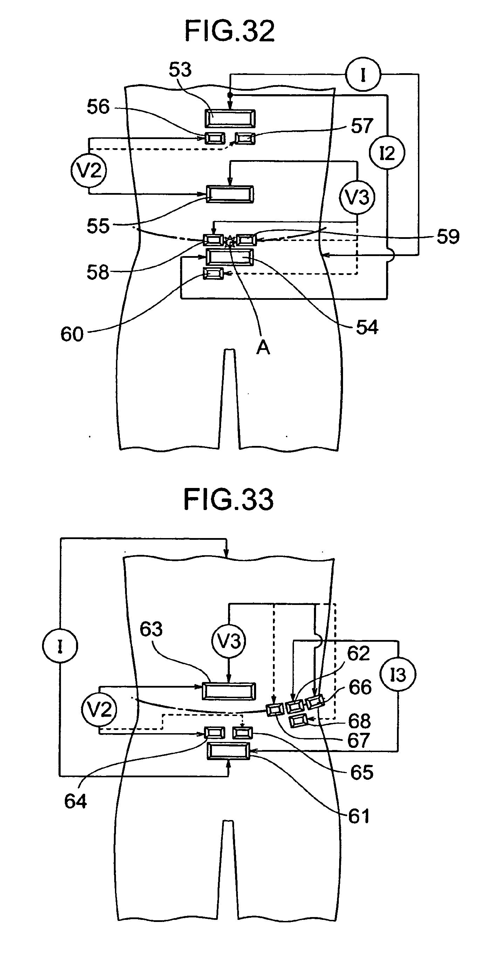

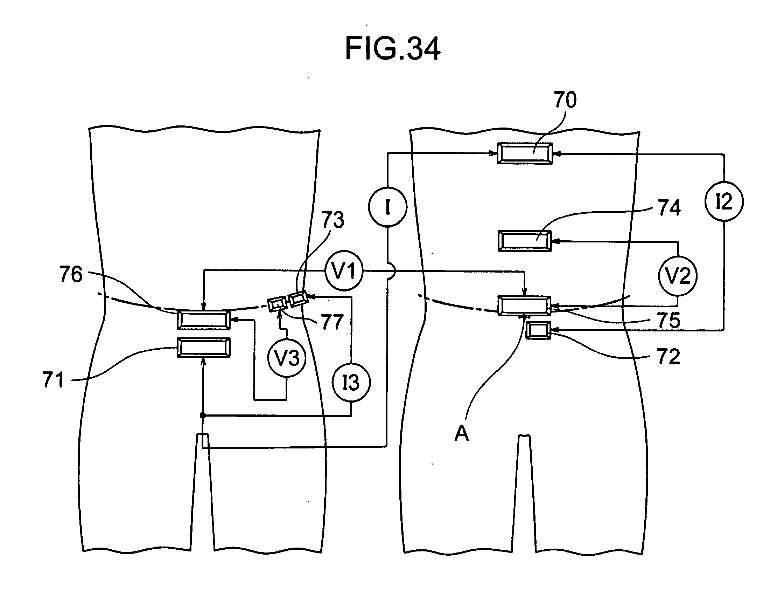

Figs. 32 to 34 show electrode arrangement examples for measuring the impedance of the subcutaneous fat tissue layer and combined electrode arrangement examples for measuring visceral fat tissues and the subcutaneous fat tissue layer. Fig. 32 is a diagram illustrating the front side of the abdomen of a subject when viewed with the navel A at the center. Fig. 33 is a diagram illustrating the back side of the subject. Fig. 34 is a diagram illustrating the back side of the subject on the left side and the front side of the subject on the right side.

(i) Electrode Arrangement Example for Measuring Impedance of Subcutaneous Fat Tissue Layer Right Under Current Applying Electrode Placed on Rectus Abdominis Muscle Tissue Layer

In Fig. 32, 53 and 54 represent current applying electrodes, and 55 to 60 represent voltage measuring electrodes. The electrode 53 corresponds to 33 in Fig. 27, and its position is the same as that in Fig. 27. The electrodes 53 and 54 constitute a current applying electrode pair for measuring the subcutaneous fat tissue layer. The electrodes 55 and 56 constitute a voltage measuring electrode pair for measuring the subcutaneous fat tissue layer in the upper portion of the abdomen (under the epigastric fossa). Further, the electrodes 55 and 58 constitute a voltage measuring electrode pair for measuring the subcutaneous fat tissue layer around the navel. The electrodes 56 and 58 are placed in the vicinity of the current applying electrodes 53 and 54 and placed at positions where the influence of spreading resistance right underneath the current applying electrodes is predominant, and the electrode 55 is placed at a remote site from the current applying electrodes 53 and 54 where the influence of the spreading resistance right underneath the current applying electrodes is weak. V2 represents a potential difference measured in the subcutaneous fat tissue layer in the upper portion of the abdomen (under the epigastric fossa) by an applied current I2, and V3 represents a potential difference measured in the subcutaneous fat tissue layer around the navel by the applied current I2.

The voltage measuring electrodes 56 and 58 situated in the vicinity of the current applying electrodes may be placed at positions in any of the four directions from the current applying electrodes. For example, as indicated by dotted arrows, the electrode 57 may be placed in place of the electrode 56, and the electrode 59 or 60 may be placed in place of the electrode 58. In this case, nearly the same measurement results are obtained by using any of the voltage measuring electrodes placed at positions in the vicinity of and in any of the four directions from the current applying electrodes.

(ii) Electrode Arrangement Example for Measuring Impedance of Subcutaneous Fat Tissue Layer Right Under Current Applying Electrode Placed on Lower Back

In Fig. 33, 61 and 62 represent current applying electrodes, and 63 to 68 represent voltage measuring electrodes. The electrode 61 corresponds to 34 in Fig. 27, and its position is the same as that in Fig. 27. The electrodes 61 and 62 constitute a current applying electrode pair for measuring the subcutaneous fat tissue layer. The electrodes 63 and 64 constitute a voltage measuring electrode pair for measuring the subcutaneous fat tissue layer around the navel of the waist. Further, the electrodes 63 and 66 constitute a voltage measuring electrode pair for measuring the subcutaneous fat tissue layer in a lateral region of the waist at the height of the navel. The electrodes 64 and 66 are placed in the vicinity of the current applying electrodes 61 and 62 and placed at positions where the influence of spreading resistance right underneath the current applying electrodes is predominant, and the electrode 63 is placed at a remote site from the current applying electrodes 61 and 62 where the influence of the spreading resistance right underneath the current applying electrodes is weak. V2 represents a potential difference measured in the subcutaneous fat tissue layer around the navel of the waist by an applied current I2, and V3 represents a potential difference measured in the subcutaneous fat tissue layer in a lateral region of the waist at the height of the navel by the applied current I2.

The voltage measuring electrodes 64 and 66 situated in the vicinity of the current applying electrodes may be placed at positions in any of the four directions from the current applying electrodes. For example, as indicated by dotted arrows, the electrode 65 may be placed in place of the electrode 64, and the electrode 67 or 68 may be placed in place of the electrode 66. In this case, nearly the same measurement results are obtained by using any of the voltage measuring electrodes placed at positions in the vicinity of and in any of the four directions from the current applying electrodes.

(iii) Combined Electrode Arrangement Example for Measuring Visceral Fat Tissues and Subcutaneous Fat Tissue Layer

In Fig. 34, 70 to 73 represent current applying electrodes, and 74 to 77 represent voltage measuring electrodes. The electrodes 61 and 62 constitute a current applying electrode pair for measuring visceral fat tissues. Further, the electrodes 70 and 72 constitute a current applying electrode pair for measuring the subcutaneous fat tissue layer around the navel, and the electrodes 71 and 73 constitute a current applying electrode pair for measuring the subcutaneous fat tissue layer in the lower back. The electrodes 70 and 71 correspond to 33 and 34 in Fig. 27, and their positions are the same as those in Fig. 27.

The electrodes 74 and 75 constitute a voltage measuring electrode pair for measuring the subcutaneous fat tissue layer around the navel. Further, the electrodes 76 and 77 constitute a voltage measuring electrode pair for measuring the subcutaneous fat tissue layer in the lower back. The electrodes 75 and 77 are placed in the vicinity of the current applying electrodes 72 and 73 and placed at positions where the influence of spreading resistance right underneath the current applying electrodes is predominant, and the electrodes 74 and 76 are placed at remote sites from the current applying electrodes 70 and 72 and the current applying electrodes 71 and 73 where the influence of the spreading resistance right underneath the current applying electrodes is weak. V1 represents a potential difference measured in visceral fat tissues by an applied current I1, V2 represents a potential difference measured in the subcutaneous fat tissue layer around the navel by an applied current I2, and V3 represents a potential difference measured in the subcutaneous fat tissue layer in the lower back by an applied current I3.

As shown in Fig. 34, the information of the subcutaneous fat tissue layer and the information of visceral fat tissues can be measured simultaneously as separate informations by carrying out measurement of impedance by at least two electrode pairs comprising the voltage measuring electrodes that are not or hardly influenced by the subcutaneous fat tissue layer right underneath the current applying electrodes and the voltage measuring electrodes that are significantly influenced by the subcutaneous fat tissue layer right underneath the current applying electrodes (measurement in spreading resistance regions).

For example, the information of the subcutaneous fat tissue layer and the information of visceral fat tissues can be measured as separate informations by having both the electrode arrangement for measuring the subcutaneous fat tissue layer and the electrode arrangement for measuring visceral fat tissues (primary information obtained by energization is tissue layer information by energization through a composite tissue layer of splanchnic organ tissues and visceral fat tissues) and selecting an appropriate current applying electrode pair and an appropriate voltage measuring electrode pair by the current applying electrode switching section 9 and the voltage measuring electrode switching section 12 of Fig. 2.

Since both the tissue informations can be measured simultaneously in the same environment by measuring change error factors during measurement by breathing or the like at sampling timing faster than a change in breathing, the error factors can be relatively removed. Accordingly, the influences of heartbeat and the like other than breathing can also be removed. The same effect is obtained by a smoothing process in the same measurement environment in addition to the increase in speed.

<Measurement of Visceral Fat and the Like Using Impedance of Rectus Abdominis Muscle>

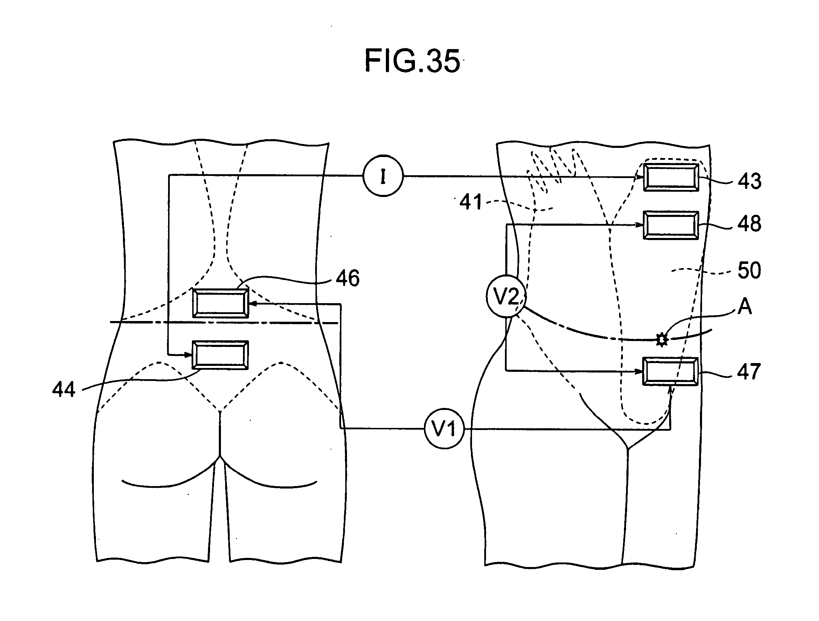

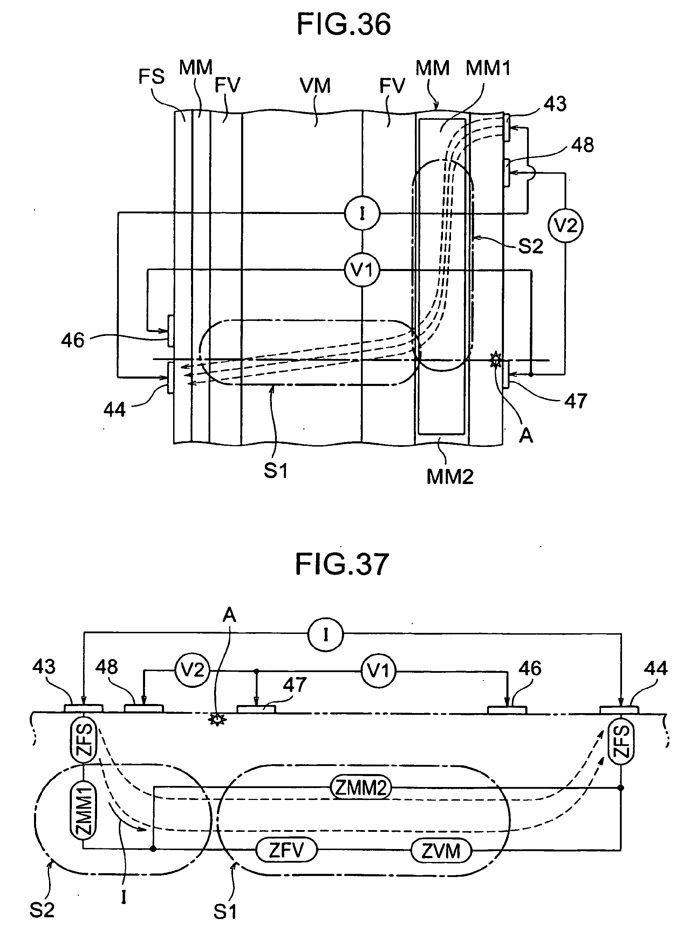

Next, measurement of visceral fat and the like by use of the impedance of the rectus abdominis muscle will be described. By using the skeletal muscle (rectus abdominis muscle) tissue layer as a virtual electrode layer, the amounts of currents passing through the splanchnic organ tissues and visceral fat tissues under the skeletal muscle tissue layer are increased to secure measurement sensitivity for visceral fat tissues, and the impedance of the rectus abdominis muscle tissue layer is measured. Figs. 35 to 37 show methods of disposing rectus abdominis muscle tissue layer virtual electrodes in measurement of the rectus abdominis muscle tissue layer which can be used in the present invention. Fig. 35 is a diagram showing the actual structure of the abdomen of the trunk from the front side and back side thereof. Fig. 36 is a diagram which schematically shows the structure of Fig. 35. Fig. 37 is a diagram showing Fig. 35 as an equivalent circuit.

In the example shown in Figs. 35 to 37, one current applying electrode 43 is placed in the vicinity of the upper edge of the upper portion of the rectus abdominis muscle tissue layer under the epigastric fossa on the front side of the trunk, and the other current applying electrode 44 is placed on the lower back. An electrode 46 included in a voltage measuring electrode pair for measuring visceral fat tissues is placed at a remote site where the influence of spreading resistance around the current applying electrode can be ignored. The other electrode 47 is placed in the vicinity of the navel circumference on the rectus abdominis muscle tissue layer so as to exclude the series impedance component of the rectus abdominis muscle tissue layer. Further, an electrode 48 included in a voltage measuring electrode pair for measuring the rectus abdominis muscle tissue layer is placed at a remote site on the rectus abdominis muscle tissue layer from the current applying electrode 43 where the influence of spreading resistance around the current applying electrode can be ignored. The other electrode 47 is implemented by the above voltage measuring electrode for measuring visceral fat tissues. The voltage measuring electrode 47 for measuring the rectus abdominis muscle tissue layer may be placed above, below or on the navel A over the middle to lower portions of the rectus abdominis muscle tissue layer.

When a current I is applied between the voltage 43 and the voltage 44, the current passes along the route indicated by dotted lines in Figs. 11 and 12. Therefore, a potential difference V1 which is measured by the electrodes 46 and 47 is a visceral fat tissue measured potential difference, and the measurement range of V1 is indicated by S1. Further, a potential difference V2 which is measured by the electrodes 47 and 48 is a rectus abdominis muscle tissue layer measured potential difference, and the measurement range of V2 is indicated by S2. At the selected positions of the current applying electrodes, i.e. under the epigastric fossa on the front side and on the lower back, the subcutaneous fat tissue layer is very thin. Thus, it is easy to secure the distance between the current applying electrode and the voltage measuring electrode for avoiding the influence of spreading resistance, and the distance may be small. Consequently, the following impedance is obtained from I, V1 and V2.

$$Ztm = V1/I = ZMM2 // (ZVM+ZFV)$$

$$ZMM1 = V2/I$$

As described above, in one aspect of the present invention, a two-frequency measured value can be used for the impedance ZMM of the skeletal muscle tissue layer. More specifically, the impedance of the rectus abdominis muscle tissue layer is measured by an applied current of frequency f1 which is highly sensitive to the rectus abdominis muscle tissue layer, and the bioelectrical impedance of the trunk is measured by an applied current of frequency f2 which is higher than f1 and is hardly influenced by muscle fibers.

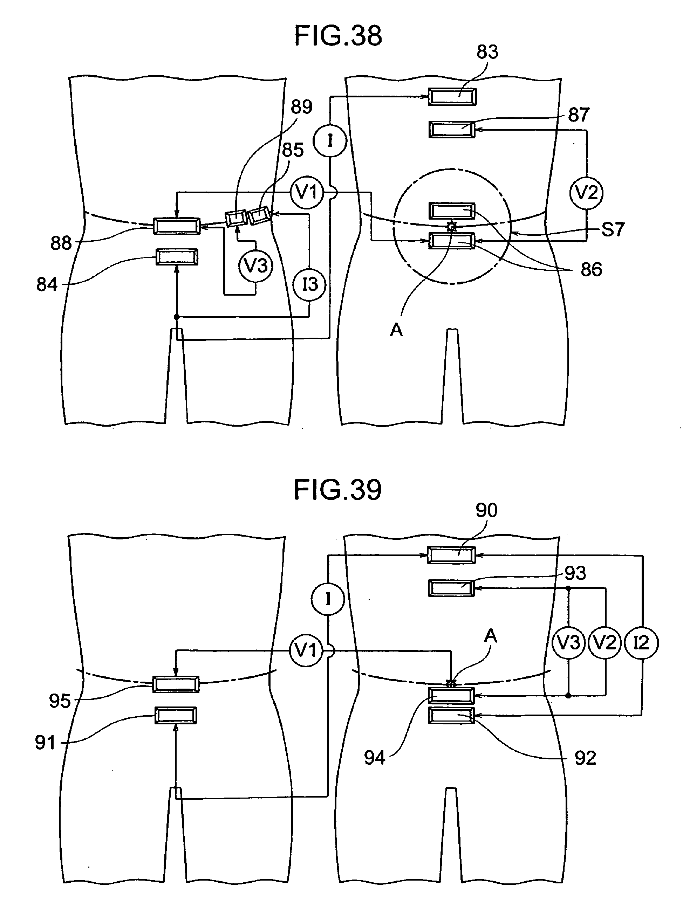

Figs. 38 to 41 illustrate combined electrode arrangement examples for measurements of visceral fat tissues, the rectus abdominis muscle tissue layer and the subcutaneous fat tissue layer and electrode positioning methods which can be used in the present invention. In these drawings, the right side is a front view of the abdomen of a subject when viewed with the navel A of the subject at the center, and the left side is a rear view of the subject.

(i) Measuring Electrode Arrangement for Measuring Visceral Fat Tissues, Rectus Abdominis Muscle Tissue Layer and Subcutaneous Fat Tissue Layer

In Fig. 38, 83 to 85 represent current applying electrodes, and 86 to 89 represent voltage measuring electrodes. The electrodes 83, 84, 86, 87 and 88 correspond to 43, 44, 47, 48 and 46 in Fig. 35, and their positions are the same as those in Fig. 35. The electrode 86 may be placed above, below or on the navel A over the middle to lower portions of the rectus abdominis muscle tissue layer. S7 indicates the allowable range of the position of the electrode 86.

The electrodes 84 and 85 constitute a current applying electrode pair for measuring the subcutaneous fat tissue layer. The electrodes 88 and 89 constitute a voltage measuring electrode pair for measuring the subcutaneous fat tissue layer. The electrode 89 is placed in the vicinity of the current applying electrode 85 and placed at a position where the influence of spreading resistance right underneath the current applying electrode is predominant, and the electrode 88 is placed at a remote site from the current applying electrode 84 where the influence of spreading resistance right underneath the current applying electrode is weak. V1 represents a potential difference measured in visceral fat tissues by an applied current I, V2 represents a potential difference measured in the rectus abdominis muscle tissue layer by the applied current I, and V3 represents a potential difference measured in the subcutaneous fat tissue layer (particularly, a potential difference measured in the subcutaneous fat tissue layer in the lower back, in consideration of the positions of the electrodes 85 and 89) by an applied current I3.

(ii) Measuring Electrode Arrangement with Shared Voltage Electrodes

In Fig. 39, 90 to 92 represent current applying electrodes, and 93 to 95 represent voltage measuring electrodes. Fig. 39 is the same as Fig. 38 with respect to the positions of electrodes for measuring visceral fat tissues and electrodes for measuring the rectus abdominis muscle tissue layer and is different from Fig. 38 with respect only to the positions of electrodes for measuring the subcutaneous fat tissue layer. The electrodes 90 and 92 constitute a current applying electrode pair for measuring the subcutaneous fat tissue layer. The electrodes 93 and 94 constitute a voltage measuring electrode pair for measuring the subcutaneous fat tissue layer. Further, the electrodes 93 and 94 also serve as electrodes for measuring the rectus abdominis muscle tissue layer. These voltage measuring electrodes are placed according to the above relationship with the current applying electrodes in consideration of the influence of spreading resistance right underneath the current applying electrodes. V1 represents a potential difference measured in visceral fat tissues by an applied current I, V2 represents a potential difference measured in the rectus abdominis muscle tissue layer by the applied current I, and V3 represents a potential difference measured in the subcutaneous fat tissue layer (particularly, a potential difference measured in the subcutaneous fat tissue layer around the navel, in consideration of the positions of the electrodes 93 and 94) by an applied current I2.

(iii) Measuring Electrode Arrangement Using Tendinous Intersection or Navel Position for Positioning of Electrodes

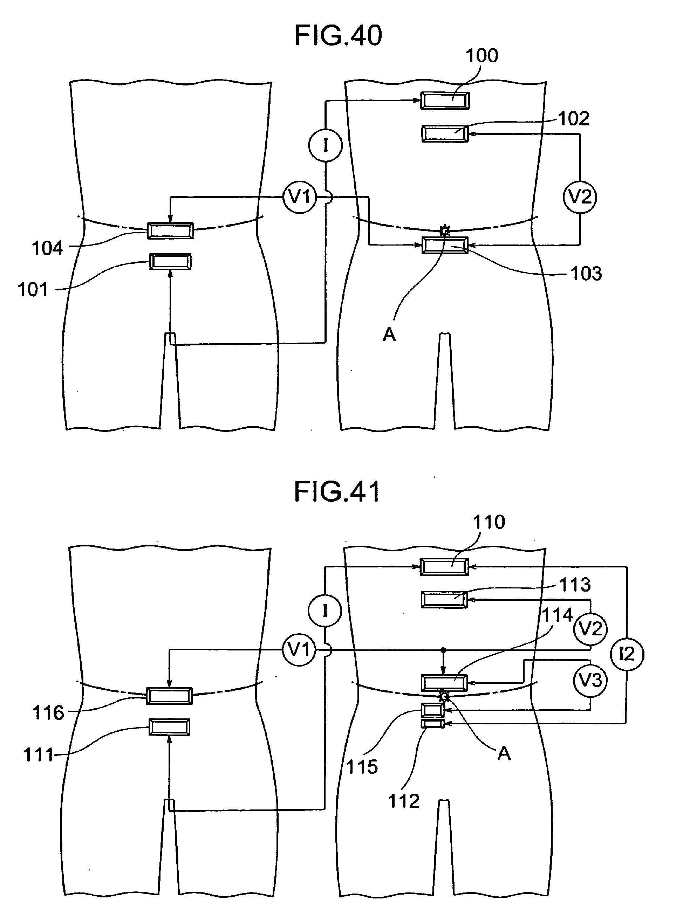

In Fig. 40, 100 and 101 represent current applying electrodes, and 102 to 104 represent voltage measuring electrodes. Fig. 40 illustrates a method of positioning voltage measuring electrodes for measuring the rectus abdominis muscle tissue layer. When the electrode 102 for measuring the rectus abdominis muscle tissue layer is to be placed, the tendinous intersection is used as a mark for attaching the electrode. The rectus abdominis muscle tissue layer is situated in the longitudinal direction of the trunk and is divided into the upper portion, the middle portion and the lower portion (umbilical region exists) by the tendinous intersections (tendons) . This tendinous intersection is used as a mark for attaching the voltage measuring electrode. Further, when the electrode 104 for measuring the rectus abdominis muscle tissue layer is to be placed, the position of the navel is used as a mark for attaching the electrode. That is, the electrode 104 is placed above, below or on the navel A over the middle to lower portions of the rectus abdominis muscle tissue layer. The concave portion of the navel is considered to be within the allowable range as a measurement area if it has such a length that a part of the concave portion can contact and secure the voltage measuring electrode. V1 represents a potential difference measured in visceral fat tissues by an applied current I, and V2 represents a potential difference measured in the rectus abdominis muscle tissue layer by the applied current I.

(iv) Measuring Electrode Arrangement Using Left or Right Half of Rectus Abdominis Muscle Tissue Layer for Measurement

In Fig. 41, 110 to 112 represent current applying electrodes, and 113 to 116 represent voltage measuring electrodes. Fig. 18 is the same as Fig. 38 with respect to the positions of electrodes for measuring visceral fat tissues and electrodes for measuring the rectus abdominis muscle tissue layer and is different from Fig. 38 with respect only to the positions of electrodes for measuring the subcutaneous fat tissue layer. The electrodes 110 and 112 constitute a current applying electrode pair for measuring the subcutaneous fat tissue layer. The electrodes 114 and 115 constitute a voltage measuring electrode pair for measuring the subcutaneous fat tissue layer. These voltage measuring electrodes are placed according to the above relationship with the current applying electrodes in consideration of the influence of spreading resistance right underneath the current applying electrodes. The current applying electrode 112 and the voltage measuring electrode 115 are placed on the left half of the rectus abdominis muscle tissue layer. Since the rectus abdominis muscle tissue layer is separated into the left and right portions by a white line (refer to Fig. 30), only the left or right portion separated by the white line can be used for measurement and an energization guide. Accordingly, the lengths of the electrodes are smaller than those of other electrodes. Although the electrodes 112 and 115 are placed on the left half in Fig. 41, they may be placed on the right half. Meanwhile, the current applying electrode 80 and the voltage measuring electrodes 113 and 114 which are disposed on the rectus abdominis muscle tissue layer have such a length over the white line that enables the electrodes to perform equal energization and measurement on the left and right rectus abdominis muscle tissue layers.

In the examples shown in Figs. 35 to 41, the current applying electrode pair is placed between the upper portion of the rectus abdominis muscle tissue layer under the epigastric fossa on the front side of the trunk and the lower back. However, the current applying electrode pair may be placed on the rectus abdominis muscle tissue layer on the front side of the trunk. In that case, one electrode out of the current applying electrode pair is placed in the vicinity of the upper edge of the upper portion of the rectus abdominis muscle tissue layer under the epigastric fossa on the front side of the trunk, and the other electrode is placed under the navel on the rectus abdominis muscle tissue layer. Further, one electrode out of the voltage measuring electrode pair is placed at a distance from the above one current applying electrode. In the region in the vicinity of the upper edge of the upper portion of the rectus abdominis muscle tissue layer under the epigastric fossa on the front side of the trunk, the subcutaneous fat tissue layer is distributed thinly. Therefore, the influence of spreading resistance is small, and the voltage measuring electrode may be placed at a position which is close to the current applying electrode to a certain extent. Further, the other voltage measuring electrode is placed on the rectus abdominis muscle tissue layer at such a distance from the other current applying electrode that enables the voltage measuring electrode to avoid the influence of spreading resistance. Since the subcutaneous fat tissue layer is distributed thickly in the vicinity of the navel, a long distance for avoiding the influence of spreading resistance must be secured.

<Measurements of Visceral/Subcutaneous Fats of Trunk by Combined Electrode Arrangement for Extremities and Trunk>

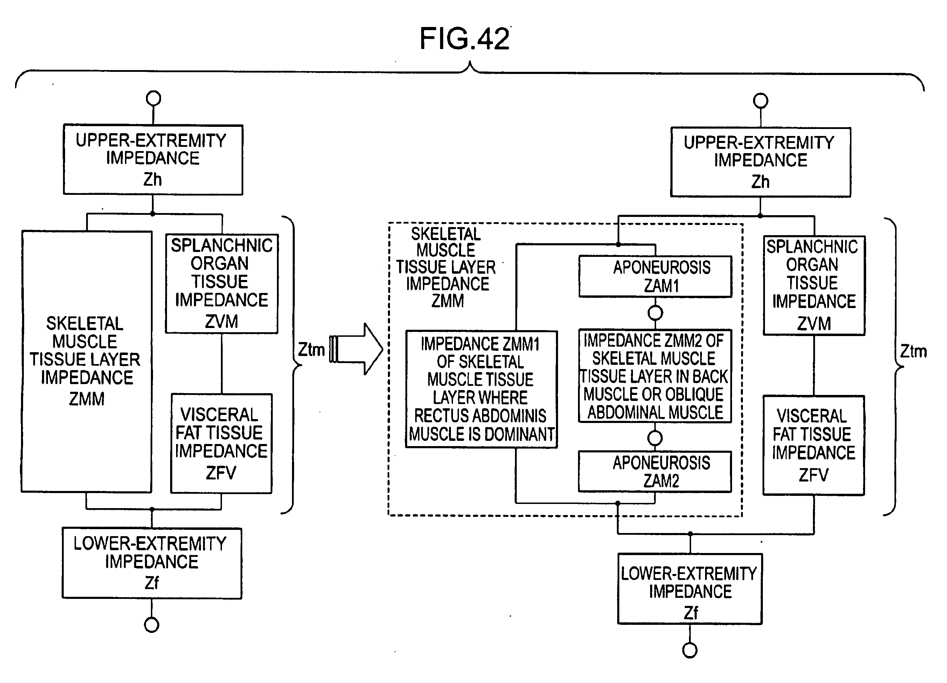

Next, the visceral/subcutaneous fats of the trunk according to the present invention using the above techniques comprehensively will be described. Figs. 42 to 45 illustrate electrical equivalent circuits of the abdomen (trunk) of the trunk including the upper and lower extremities which are premises in the technique of the present invention in the same manner as in Figs. 7 and 8.

Fig. 42 is a diagram illustrating electrical equivalent circuits of the structure of the abdomen of the trunk with the impedance $Zh$ of the upper extremity and the impedance $Zf$ of the lower extremity taken into account. In particular, Fig. 42 illustrates, on its right side, an electrical equivalent circuit showing a more detailed structure of the impedance of the skeletal muscle tissue layer to obtain the impedance $ZMM$ of the skeletal muscle tissue layer. As shown in Fig. 42, the impedance $Zh$ of the upper extremity and the impedance $Zf$ of the lower extremity are connected in series with the impedance $Ztm$ of the middle portion of the trunk. Further, of the impedances $Ztm$ of the middle portion of the trunk, the impedance $ZMM$ of the skeletal muscle tissue layer in particular can be regarded such that "skeletal muscle $ZMM1$ where rectus abdominis muscle is predominant" is connected in parallel to a series circuit of "aponeurosis $ZAM1$", "skeletal muscle $ZMM2$ in back muscle or oblique abdominal muscle" and "aponeurosis $ZAM2$". The following relational expression roughly holds among these impedances.

$$ZMM = ZMM1 \mathbin{//} (ZAM1+ZMM2+ZAM2)$$

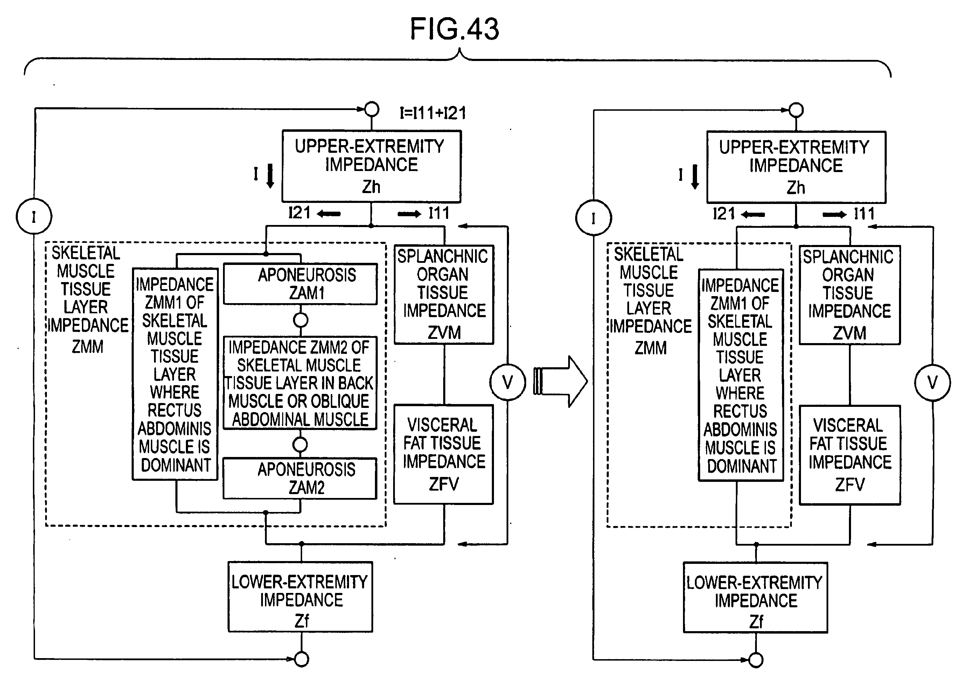

Figs. 43 to 45 illustrate a lead system by energization between the upper extremity and the lower extremity, a lead system by energization between the upper extremity and the abdomen of the trunk and a lead system by energization between the lower extremity and the abdomen of the trunk, based on the electrical equivalent circuits shown in Fig. 42, respectively. In each of the above diagrams, the circuit shown on the left side is a simplified version of the circuit shown on the right side.

As shown in Fig. 43, in the case of energization between the upper extremity and the lower extremity, the impedance $ZMM$ of the skeletal muscle tissue layer in particular can be regarded as "skeletal muscle $ZMM1$ where rectus abdominis muscle is predominant". Consequently, it can be regarded as a simplified electrical equivalent circuit as shown on the right side of Fig. 43. More specifically, the electrical equivalent circuit on the right side premises that the following relational expression holds.

$$ZAM1 \fallingdotseq ZAM2 \gg ZMM2 > ZMM1$$

$$\therefore ZMM \fallingdotseq ZMM1$$

In this circuit, a current $I$ from the current applying electrode is branched and primarily flows $I21$ on the skeletal muscle $ZMM$ side and $I11$ on the visceral $ZMV$ and $ZFV$ side.

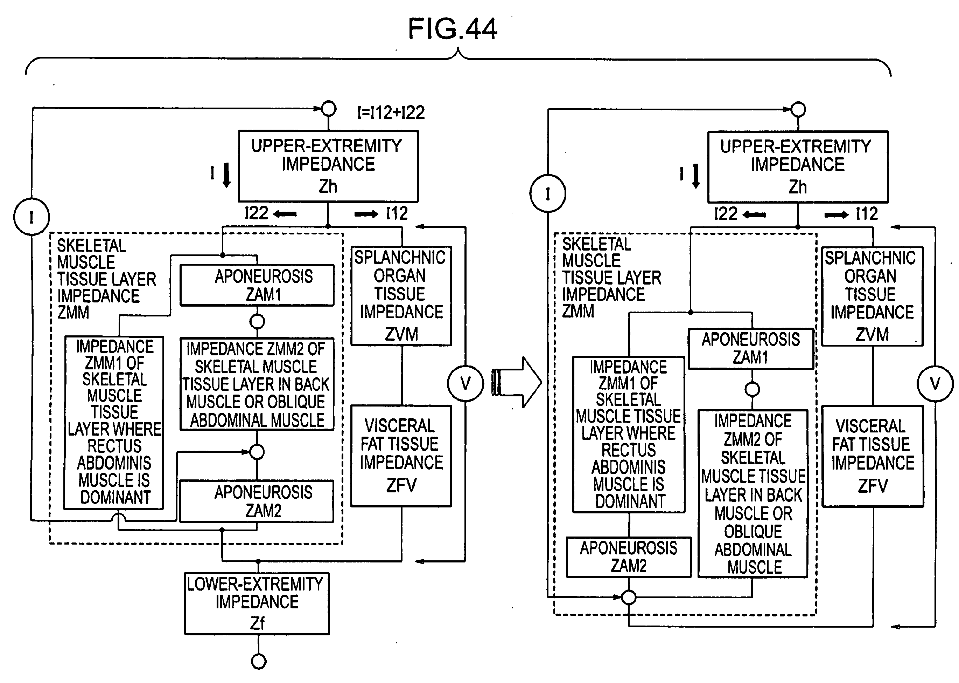

As shown in Fig. 44, in the case of energization between the upper extremity and the abdomen of the trunk, a current is assumed to pass through the aponeurosis $ZAM2$ since the resistance of the aponeurosis $ZAM2$ is low. Consequently, it can be regarded as a simplified electrical equivalent circuit as shown on the right side of Fig. 44. Further, in this circuit diagram, a current $I$ from the current applying electrode is branched and flows $I22$ on the skeletal muscle $ZMM$ side and $I12$ on the visceral $ZMV$ and $ZFV$ side. In this case, the following relationship holds between $I22$ and $I12$.

$$I22 < I12$$

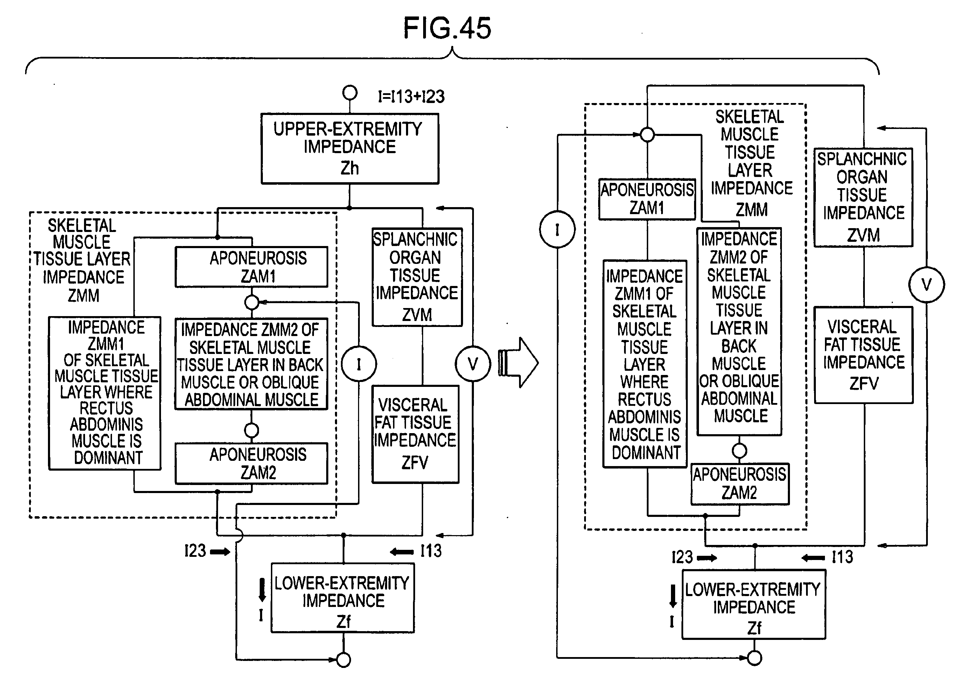

Further, as shown in Fig. 45, in the case of energization between the lower extremity and the abdomen of the trunk, it can be seen that a current is applied from between the "aponeurosis ZAM1" and the "skeletal muscle ZMM2 in back muscle or oblique abdominal muscle" which are constituents of the skeletal muscle ZMM. Consequently, it can be regarded as a simplified electrical equivalent circuit as shown on the right side of Fig. 45. Further, in this circuit diagram, a current I from the current applying electrode is branched and flows I23 on the skeletal muscle ZMM side and I13 on the visceral ZMV and ZFV side. In this case, the following relationship holds between I23 and I13.

$$I23 < I13$$

Next, the basic concept for carrying out the measurements of the visceral/subcutaneous fats of the trunk according to the present invention will be described briefly.

The present invention uses the concept of four-electrode technique. Of fundamental four electrodes used in the four-electrode technique, one of current applying electrodes required for energization is placed on any of the extremities or head protruding from the trunk (for example, it is attached to an ear of the head as a clip electrode by clipping the ear), and the other current applying electrode is placed on the abdomen of the trunk. According to this constitution, for example, since the current applying electrode is attached to an ear as a clip electrode and measurement can be carried out between the electrode and the trunk, one confined to bed and having disabled arms can become a subject to be measured (in this case, the ear clip electrode may take a form in which it is pulled out of the main unit of the apparatus by a lead). Further, according to the present invention, only one of the current applying electrodes may be placed on the trunk, and other electrodes, i.e. two voltage measuring electrodes, may be placed on the remaining extremities or head other than that on which the other current applying electrode is placed. In this case, a novel lead system to which the lead system of Organ et al. has been applied can be introduced. According to this method, by placing current applying electrodes only on one side of the abdomen of the trunk, restrictions on voltage measuring electrodes to be placed on the abdomen of the trunk can be improved significantly.

In the present invention, energization through the trunk is simplified to measure the information of the visceral fat tissues and subcutaneous fat tissue layer of the abdomen of the trunk easily. For example, to measure the subcutaneous fat tissue layer, the other current applying electrode is placed on the abdomen of the trunk which is a target to be measured, and one of the voltage measuring electrodes is placed in the vicinity of the current applying electrode as a voltage measuring electrode arrangement for measuring a spreading resistance component right underneath the current applying electrode. The other voltage measuring electrode secures the information of the subcutaneous fat tissue layer (thickness) from a potential difference obtained between the other voltage measuring electrode and one voltage measuring electrode placed at such a distance from the current applying electrode that it is not influenced by the spreading resistance from the current applying electrode. The other voltage measuring electrode may be placed on the abdomen of the trunk or may be placed on the extremities according to the novel lead system to which the lead system of Organ et al. has been applied.

In the voltage measuring electrode arrangement for measuring the subcutaneous fat tissue layer, one voltage measuring electrode is placed at a position close to the current applying electrode placed on the trunk where the voltage measuring electrode is predominantly influenced by the spreading resistance right underneath the current applying electrode, and the other voltage measuring electrode is placed at such a distance from the current applying electrode that the influence of the spreading resistance can be almost ignored. Both of the electrodes may be placed on or near the navel circumference, or one of them may be placed in the trunk longitudinal direction. When it is placed in the longitudinal direction, it does not have to be within the energized region as long as a potential comparable to the potential level of a measuring target is obtained.

Subcutaneous fat tissue layer thickness distribution on the navel circumference varies among individuals depending on body parts. Thus, it is advantageous for improving accuracy in estimation of the subcutaneous fat tissue volume that measurement information of multiple body parts which characterize individuals can be secured. A plurality of current applying electrodes to be disposed on the trunk which correspond to one current applying electrode disposed on the extremities and head are placed on or near the navel circumference, and a required number of voltage measuring electrodes which make it possible to measure the information of the subcutaneous fat tissue layer right underneath the current applying electrodes are placed.

In the present invention, in measuring visceral fat tissues, the other current applying electrode to be disposed on the abdomen of the trunk which is a target to be measured is placed on the low-electroconductivity aponeurosis (connective tissues with skeletal muscle tissues) excluding the skeletal muscle tissue layer which constitutes the abdomen of the trunk. Thereby, it is prevented that a current passing from the extremities and head dominates the skeletal muscle tissue layer on the surface of the trunk, the ratio of current passing through a deep-seated composite tissue layer of splanchnic organ tissues and visceral fat tissues is improved, and an improvement in the sensitivity of estimation of visceral fat tissues becomes possible.

To be more specific, in this case,

(1) A current applying electrode is placed on a body part protruding from the trunk (e.g. the extremities or head), and the other current applying electrode is placed on the abdomen of the trunk.

(2) The other current applying electrode is placed on the aponeurosis excluding skeletal muscle tissues of the abdomen of the trunk.

(3) The optimum aponeuroses are joined regions between the left and right rectus abdominis muscles and the external abdominal oblique muscle.

(4) When a current is applied from the upper extremity and the head, the other current applying electrode is placed near the navel circumference in the above aponeurosis.

(5) When a current is applied from the lower extremity, the other current applying electrode is placed in the vicinity of the macroseptum and above the navel circumference in the above aponeurosis.

(6) Only one of the current applying electrodes is placed on the trunk, and other electrodes, i.e., two voltage measuring electrodes, are placed on the remaining extremities or head other than that on which the other current applying electrode is placed. In this case as well, the novel lead system to which the lead system of Organ et al. has been applied can be introduced.

Further, in measuring visceral fat tissues, the other current applying electrode to be disposed on the abdomen of the trunk which is a target to be measured is placed on the low-electroconductivity aponeurosis (connective tissues with the skeletal muscle tissue layer) excluding the skeletal muscle tissue layer which constitutes the abdomen of the trunk. Thereby, it is prevented that a current passing from the extremities and head dominates the skeletal muscle tissue layer on the surface of the trunk, the ratio of current passing through a deep-seated composite tissue layer of splanchnic organ tissues and visceral fat tissues is improved, and the sensitivity of estimation of visceral fat tissues can be improved. To be more specific, in this case,

(1) As a lead system for securing measured information of the subcutaneous fat tissue layer and measured information of visceral fat tissues, voltage measuring electrodes may be placed on the trunk such that they can measure both the subcutaneous fat tissue layer and visceral fat tissues.

(2) The two voltage measuring electrodes may be placed on the abdomen of the trunk.

(3) When a current is applied from the upper extremity and the head, one of the voltage measuring electrodes, together with the above current applying electrode, is placed on or near the navel circumference, and the other voltage measuring electrode is placed on or near the navel circumference or within the energized region in the trunk longitudinal direction.

(4) When a current is applied from the lower extremity, one of the voltage measuring electrodes is placed in the vicinity of the above current applying electrode, and the other voltage measuring electrode is placed on or near the navel circumference or within the energized region in the trunk longitudinal direction.

(5) As for the positions of the voltage measuring electrodes in measuring visceral fat tissues, the voltage measuring electrodes are placed at such a distance from the current applying electrode placed on the trunk that the influence of spreading resistance right underneath the electrode can be almost ignored. When a current is applied from the upper extremity and the head, one of the voltage measuring electrodes is placed on or near the navel circumference, and the other voltage measuring electrode is placed within the energized region in the trunk longitudinal direction. When a current is applied from the lower extremity, one of the voltage measuring electrodes is placed above the navel circumference, and the other voltage measuring electrode is placed within the energized region in the trunk longitudinal direction.

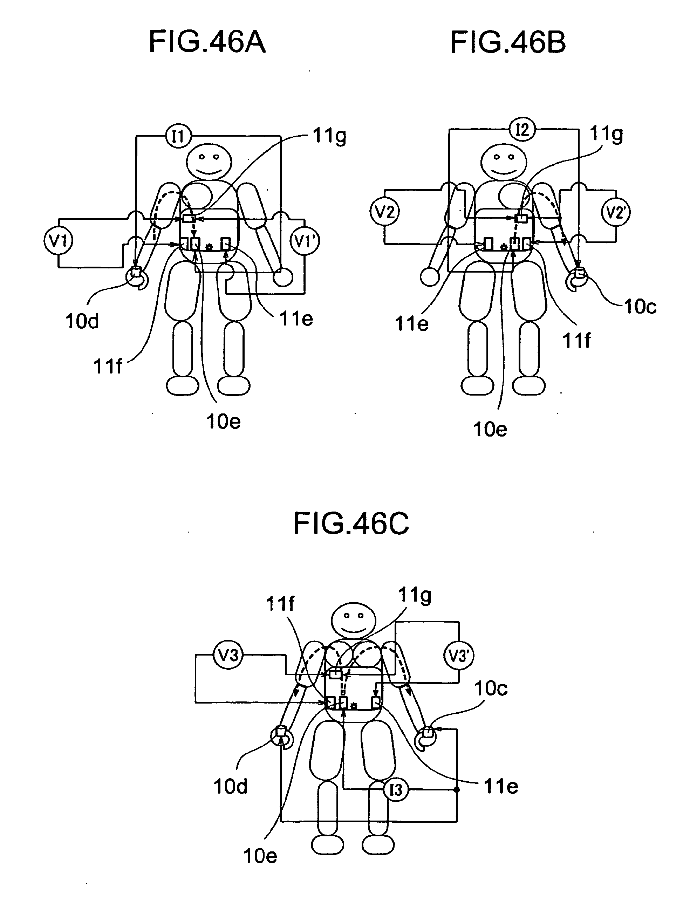

Next, with reference to Figs. 46 to 53, lead systems according to the present invention for measuring fat tissues, especially visceral fat tissues in this case, by combined electrode arrangements on the extremities and the trunk will be described.

Figs. 46A to 46C illustrate a lead system in which a current applying electrode is placed on the palm (as a grip electrode), the other current applying electrode is placed in the abdomen of the trunk (on the aponeurosis) and two voltage measuring electrode pairs are placed on the abdomen of the trunk.

Fig. 46A relates to right-arm/trunk-abdomen energization measurement, in particular. Fig. 46A illustrates an electrode arrangement in which a current applying electrode 10d is placed on the palm of the right hand (as a grip electrode 14b) and the other current applying electrode 10e is placed in the abdomen of the trunk (on the aponeurosis) so as to apply a current 11 and a pair of voltage measuring electrodes 11e and 11g and a pair of voltage measuring electrodes 11f and 11g are placed on the abdomen of the trunk so as to measure potential differences V1 and V1', thereby measuring visceral fat tissues. In particular, the voltage measuring electrode 11f may be placed at any position which is so distant from the current applying electrode 10e on the navel circumference that the electrode 11f can avoid the influence of spreading resistance and at which an equivalent potential can be measured. Nearly the same measurement result can be obtained from the potential differences V1 and V1'.

Fig. 46B relates to left-arm/trunk-abdomen energization measurement for measuring potential differences V2 and V2' by applying a current I2. The positions of the electrodes in Fig. 46A and those in Fig. 46B are symmetrical.

Fig. 46C relates to both-arms/trunk-abdomen energization measurement resulting from combining Fig. 46A with Fig. 46B. Fig. 46C illustrates an electrode arrangement in which a current applying electrode 10d is placed on the palm of the right hand (as a grip electrode 14b) and the other current applying electrode 10e is placed in the abdomen of the trunk (on the aponeurosis) or a current applying electrode 10c is placed on the palm of the left hand (as a grip electrode 14a) and the other current applying electrode 10e is placed in the abdomen of the trunk (on the aponeurosis) so as to apply a current I3 and a pair of voltage measuring electrodes 11e and 11g and a pair of voltage measuring electrodes 11f and 11g are placed on the abdomen of the trunk so as to measure potential differences V3 and V3', thereby measuring visceral fat tissues.

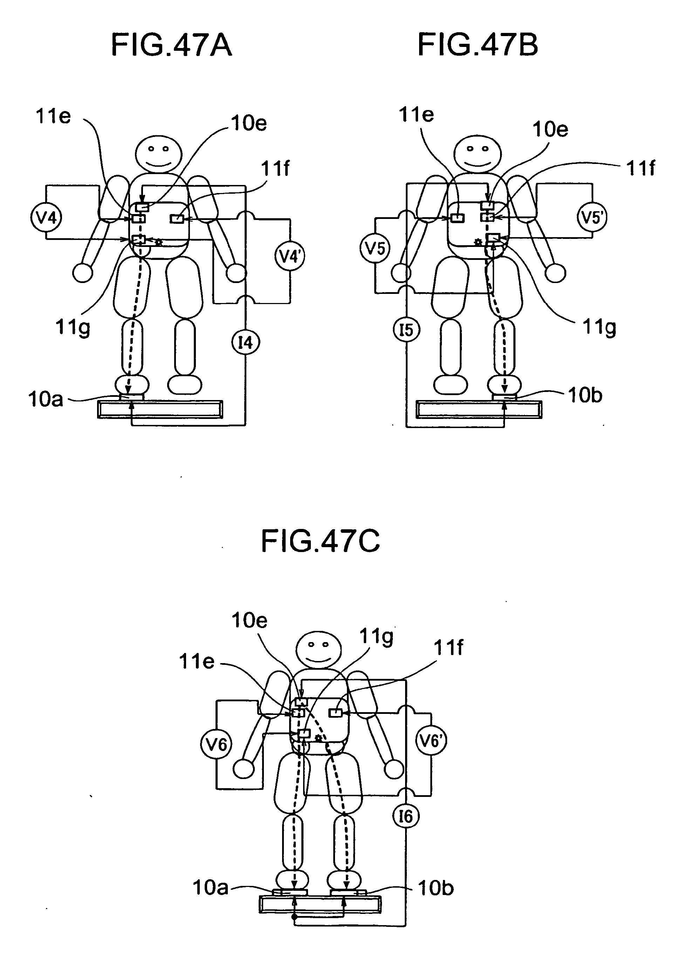

Figs. 47A to 47C illustrate a lead system in which a current applying electrode is placed at the bottom of foot (as a foot electrode), the other current applying electrode is placed in the abdomen of the trunk (on the aponeurosis) and two voltage measuring electrode pairs are placed on the abdomen of the trunk.

Fig. 47A relates to right-leg/trunk-abdomen energization measurement, in particular. Fig. 47A illustrates an electrode arrangement in which a current applying electrode 10a is placed at the bottom of the right leg (as a foot electrode) and the other current applying electrode 10e is placed in the abdomen of the trunk (on the aponeurosis) so as to apply a current I4 and a pair of voltage measuring electrodes 11f and 11g and a pair of voltage measuring electrodes 11e and 11g are placed on the abdomen of the trunk so as to measure potential differences V4 and V4', thereby measuring visceral fat tissues.

Fig. 47B relates to left-leg/trunk-abdomen energization measurement for measuring potential differences V5 and V5' by applying a current I5. The positions of the electrodes in Fig. 47A and those in Fig. 47B are symmetrical.

Fig. 47C relates to both-legs/trunk-abdomen energization measurement resulting from combining Fig. 47A with Fig. 47B. Fig. 47C illustrates an electrode arrangement in which a current applying electrode 10a is placed at the bottom of the right leg (as a foot electrode) and the other current applying electrode 10e is placed in the abdomen of the trunk (on the aponeurosis) or a current applying electrode 10b is placed at the bottom of the left leg (as a foot electrode) and the other current applying electrode 10e is placed in the abdomen of the trunk (on the aponeurosis) so as to apply a current 16 and a pair of voltage measuring electrodes 11e and 11g and a pair of voltage measuring electrodes 11f and 11g are placed on the abdomen of the trunk so as to measure potential differences V6 and V6', thereby measuring visceral fat tissues.

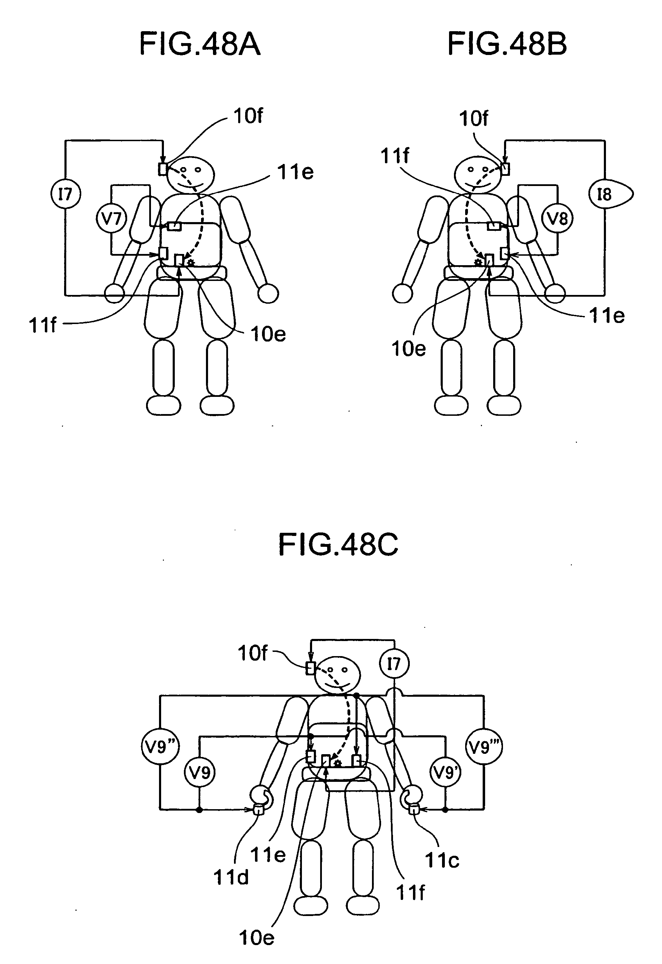

Figs. 48A to 48C illustrate a lead system in which a current applying electrode is placed on an ear of the head (as a clip electrode that clips an earlobe or the like), the other current applying electrode is placed in the abdomen of the trunk (on the aponeurosis), and one or more voltage measuring electrode pairs are placed on the abdomen of the trunk.

Fig. 48A relates to right-ear/trunk-abdomen energization measurement, in particular. Fig. 48A illustrates an electrode arrangement in which a current applying electrode 10f is placed on the right ear of the head (as a clip electrode that clips an earlobe or the like) and the other current applying electrode 10e is placed in the abdomen of the trunk (on the aponeurosis) so as to apply a current I7 and a pair of voltage measuring electrodes 11e and 11f are placed on the abdomen of the trunk so as to measure a potential difference V7, thereby measuring visceral fat tissues.

Fig. 48B relates to left-ear/trunk-abdomen energization measurement for measuring a potential difference V8 by applying a current I8. The positions of the electrodes in Fig. 48A and those in Fig. 48B are symmetrical.

Fig. 48C relates to right-ear/trunk-abdomen energization measurement resulting from combining Fig. 48A with Fig. 48B according to the lead system of Organ et al. Fig. 48C illustrates an electrode arrangement in which a current applying electrode 10f is placed on the right ear of the head (as a clip electrode that clips an earlobe or the like) and the other current applying electrode 10e is placed in the abdomen of the trunk (on the aponeurosis) so as to apply a current I7 and a pair of voltage measuring electrodes 11e, 11d and 11c and a pair of voltage measuring electrodes 11f, 11d and 11c are placed on the abdomen of the trunk so as to measure potential differences V9, V9', V9" and V9''', thereby measuring visceral fat tissues.

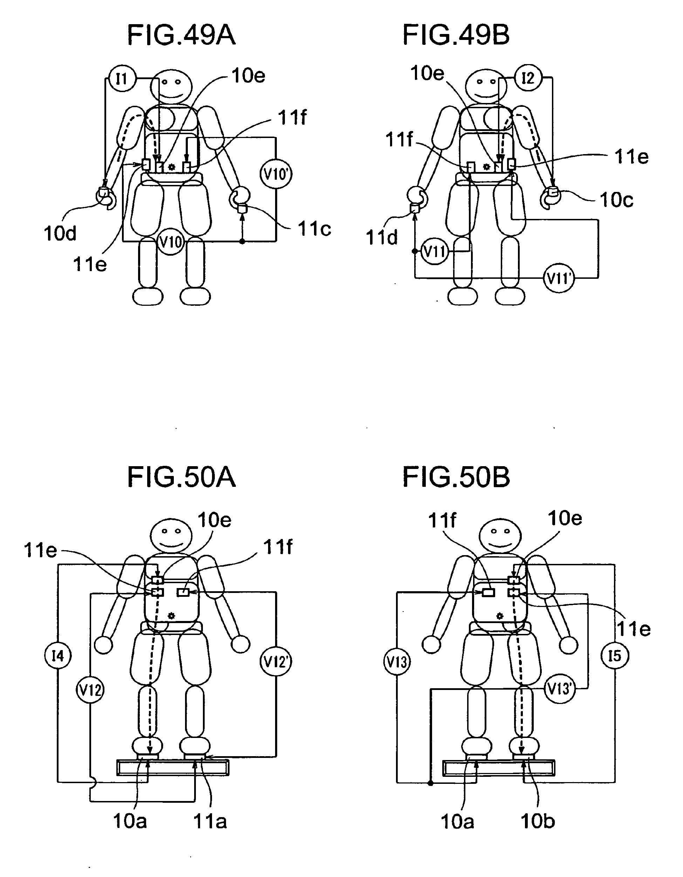

Figs. 49A and 49B illustrate a lead system in which a current applying electrode is placed on a palm (as a grip electrode), the other current applying electrode is placed in the abdomen of the trunk (on the aponeurosis), one voltage measuring electrode out of two voltage measuring electrode pairs is placed on the other palm (as a grip electrode), and the other voltage measuring electrodes are placed on the abdomen of the trunk.

Fig. 49A relates to right-arm/trunk-abdomen energization measurement according to the lead system of Organ et al., in particular. Fig. 49A illustrates an electrode arrangement in which a current applying electrode 10d is placed on the palm of the right hand (as a grip electrode) and the other current applying electrode 10e is placed in the abdomen of the trunk (on the aponeurosis) so as to apply a current I1 and a voltage measuring electrode 11c which is common to the voltage measuring electrode pairs is placed on the palm of the left hand (as a grip electrode) and the other voltage measuring electrodes 11e and 11f are placed on the abdomen of the trunk so as to measure potential differences V10 and V10', thereby measuring visceral fat tissues.

Fig. 49B relates to left-arm/trunk-abdomen energization measurement according to the lead system of Organ et al. for measuring potential differences V11 and V11' by applying a current I2. The positions of the electrodes in Fig. 49A and those in Fig. 49B are symmetrical.

Figs. 50A and 50B illustrate a lead system in which a current applying electrode is placed at the bottom of foot (as a foot electrode), the other current applying electrode is placed in the abdomen of the trunk (on the aponeurosis), one voltage measuring electrode out of two voltage measuring electrode pairs is placed at the bottom of the other foot (as a foot electrode), and the other voltage measuring electrodes are placed on the abdomen of the trunk.

Fig. 50A relates to right-leg/trunk-abdomen energization measurement according to the lead system of Organ et al., in particular. Fig. 50A illustrates an electrode arrangement in which a current applying electrode 10a is placed at the bottom of the right leg (as a foot electrode) and the other current applying electrode 10e is placed in the abdomen of the trunk (on the aponeurosis) so as to apply a current I4 and a voltage measuring electrode 11a which is common to the voltage measuring electrode pairs is placed at the bottom of the left leg (as a foot electrode) and the other voltage measuring electrodes 11e and 11f are placed on the abdomen of the trunk so as to measure potential differences V12 and V12', thereby measuring visceral fat tissues.

Fig. 50B relates to left-leg/trunk-abdomen energization measurement according to the lead system of Organ et al. for measuring potential differences V13 and V13' by applying a current I5. The positions of the electrodes in Fig. 50A and those in Fig. 50B are symmetrical.

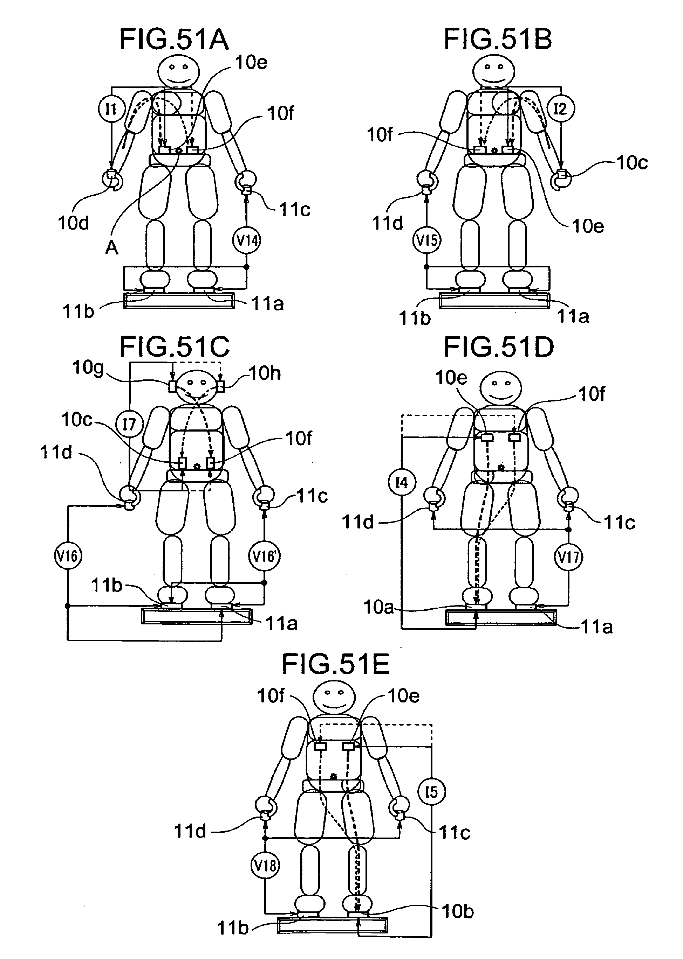

Figs. 51A to 51E illustrate a lead system in which a current applying electrode is placed on one of the four extremities (and the head), the other current applying electrode is placed in the abdomen of the trunk (on the aponeurosis), one of two voltage measuring electrode pairs is placed on the other extremity of the same kind as that on which the above extremity current applying electrode is placed, and the other voltage measuring electrodes are placed on the left and right extremities of the upper or lower extremity on which the current applying electrodes are not placed.

Fig. 51A relates to right-arm/trunk-abdomen energization measurement according to the lead system of Organ et al., in particular. Fig. 51A illustrates an electrode arrangement in which a current applying electrode 10d is placed on the palm of the right hand (as a grip electrode) and the other current applying electrodes 10e (left side) and 10f (right side) are placed on the left and right sides of the navel A so as to apply a current I1 and a voltage measuring electrode 11c which is common to the voltage measuring electrode pairs is placed on the palm of the left hand (as a grip electrode) and the other voltage measuring electrodes 11a and 11b are placed at the bottoms of the left and right legs (as foot electrodes) so as to measure a potential difference V14, thereby measuring visceral fat tissues.

Fig. 51B relates to left-arm/trunk-abdomen energization measurement according to the lead system of Organ et al. for measuring potential differences V15 and V15' by applying a current I2. The positions of the electrodes in Fig. 51A and those in Fig. 51B are symmetrical.

Fig. 51C relates to right-ear/trunk-abdomen energization measurement according to the lead system of Organ et al. Fig. 51C illustrates an electrode arrangement in which a current applying electrode 10h (left side) or 10g (right side) is placed on the left and right ears of the head (as a clip electrode which clips an earlobe or the like) and the other current applying electrodes 10f (left side) or 10c (right side) is placed in the abdomen of the trunk (on the aponeurosis) so as to apply a current I7 and a voltage measuring electrode 11d out of one voltage measuring electrode pair is placed on the palm of the right hand, the other voltage measuring electrode 11b is placed at the bottom of the left or right leg, a voltage measuring electrode 11c out of the other voltage measuring electrode pair is placed on the palm of the left hand and the other voltage measuring electrode 11a is placed at the bottom of the left or right leg so as to measure potential differences V16 and V16', thereby measuring visceral fat tissues.

Fig. 51D relates to right-leg/trunk-abdomen energization measurement according to the lead system of Organ et al. Fig. 51D illustrates an electrode arrangement in which a current applying electrode 10a is placed at the bottom of the right leg (as a foot electrode) and the other current applying electrodes 10e and 10f are placed in the abdomen of the trunk (on the aponeurosis) so as to apply a current 14 and a voltage measuring electrode 11a which is common to the voltage measuring electrode pairs is placed at the bottom of the left leg (as a foot electrode) and the other voltage measuring electrodes 11c and 11d are placed on the palms of the left and right hands so as to measure V17, thereby measuring visceral fat tissues.

Fig. 51E relates to left-leg/trunk-abdomen energization measurement according to the lead system of Organ et al. for measuring potential differences V18 and V18' by applying a current I5. The positions of the electrodes in Fig. 51A and those in Fig. 51E are symmetrical.

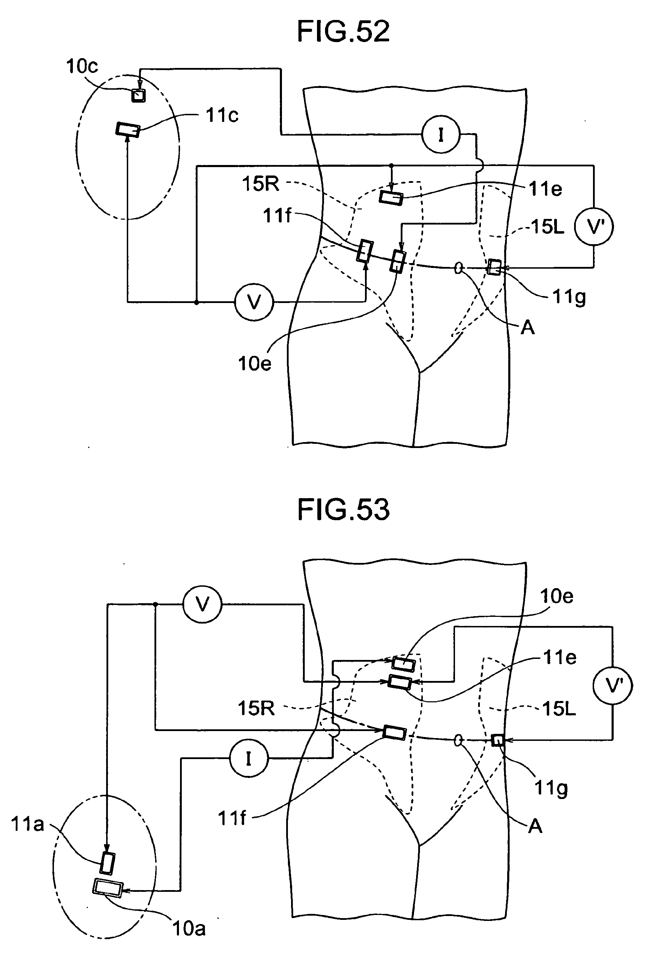

Figs. 52 and 53 are diagrams illustrating specific electrode arrangement examples of the above described arrangement methods for measuring visceral fat tissues in the same manner as in Fig. 15 and other drawings.

Fig. 52 illustrates an electrode arrangement in which a current applying electrode 10e is placed on the right aponeurosis 15R around the navel A of the trunk and the other current applying electrode 10c is placed at a position on the upper-right extremity which is sufficiently distant from the trunk, e.g. the palm of the right hand, so as to apply a current I, and a voltage measuring electrode 11c which is common to voltage measuring electrode pairs is placed at a position on the upper-left extremity, e.g. the palm of the left hand, or, a voltage measuring electrode 11e may be placed at a position on the right aponeurosis 15R which is sufficiently distant from the current applying electrode 10e, the other voltage measuring electrode 11f is placed in the vicinity of the current applying electrode 10e, and the other voltage measuring electrode 11g is placed on the left aponeurosis 15L around the navel A of the trunk so as to measure potential differences V and V', thereby measuring visceral fat tissues.

Fig. 53 illustrates an electrode arrangement in which a current applying electrode 10e is placed on the right aponeurosis 15R of the trunk and the other current applying electrode 10a is placed at a position on the lower-right extremity which is sufficiently distant from the trunk, e.g. the bottom of the right leg, so as to apply a current I; a voltage measuring electrode 11a which is common to voltage measuring electrode pairs is placed at a position on the lower-left extremity, e.g. the bottom of the left leg, or, a voltage measuring electrode 11f may be placed at a position on the right aponeurosis 15R around the navel A which is sufficiently distant from the current applying electrode 10e, or, the other voltage measuring electrode 11e may be placed in the vicinity of the current applying electrode 10e so as to measure a potential difference V; and a voltage measuring electrode 11e is placed in the vicinity of the current applying electrode 10e and the other voltage measuring electrode 11g is placed on the left aponeurosis 15L around the navel A of the trunk so as to measure a potential difference V', thereby measuring visceral fat tissues.

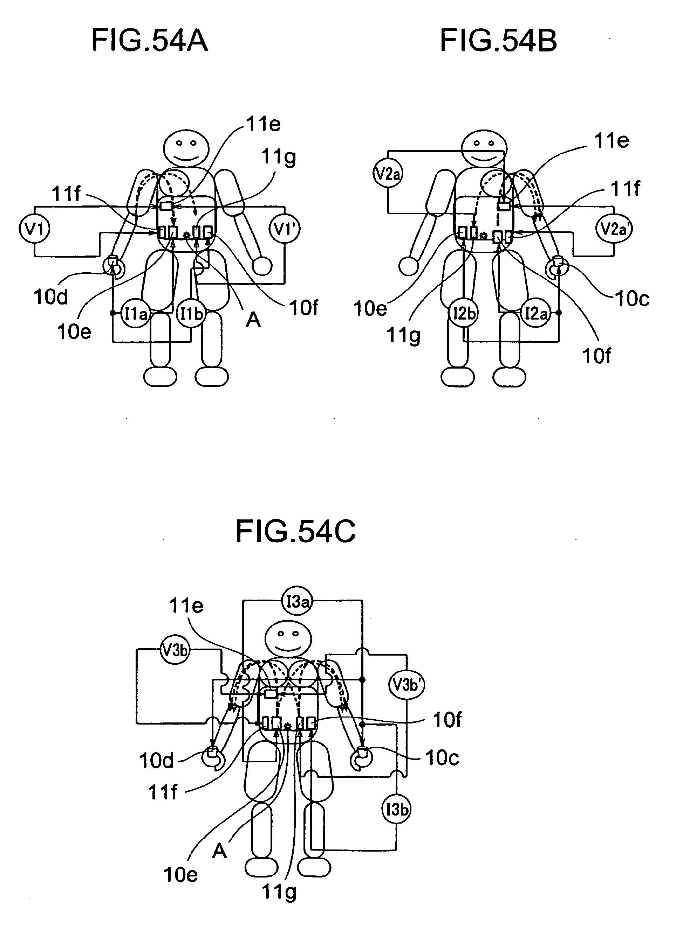

Further, with reference to Figs. 54 to 59, lead systems for measuring fat tissues, especially the subcutaneous fat tissue layer in this case, by combined electrode arrangements on the extremities and the trunk will be described.

Figs. 54A to 54U illustrate a lead system in which a current applying electrode (grip electrode) is placed on the palm, the other current applying electrode is placed in the abdomen of the trunk (on the aponeurosis) and two voltage measuring electrode pairs are placed on the abdomen of the trunk.

Fig. 54A relates to right-arm/trunk-abdomen energization measurement, in particular. Fig. 54A illustrates an electrode arrangement in which a current applying electrode 10d is placed on the palm of the right hand (as a grip electrode 14b) and the other current applying electrodes 10e (right side) and 10f (left side) are placed on the left and right sides of the navel A in the abdomen of the trunk (on the aponeurosis) so as to apply currents I1a and I1b and a voltage measuring electrode 11e which is common to the voltage measuring electrode pairs is placed on the abdomen of the trunk at a sufficient distance from the current applying electrodes 10e and 10f and the other voltage measuring electrodes 11f (right side) and 11g (left side) are placed in the vicinity of the current applying electrodes 10e and 10f so as to measure potential differences V1 and V1', thereby measuring the subcutaneous fat tissue layer or measuring the subcutaneous fat tissue layer and visceral fat tissues selectively.

Fig. 54B relates to left-arm/trunk-abdomen energization measurement for measuring potential differences V2a and V2a' by applying currents I2a and I2b. The positions of the electrodes in Fig. 54A and those in Fig. 54B are symmetrical.

Fig. 54C relates to both-arms/trunk-abdomen energization measurement. Fig. 54C illustrates an electrode arrangement in which current applying electrodes 10d and 10c are short-circuited, the 10d is placed on the palm of the right hand (as a grip electrode 14b), the 10c is placed on the palm of the left hand (as a grip electrode 14a), the other current applying electrode 10f is placed on the left side of the navel A in the abdomen of the trunk (on the aponeurosis) and the other current applying electrode 10e is placed on the right side of the navel A in the abdomen of the trunk (on the aponeurosis) so as to apply currents 13a and 13b and a voltage measuring electrode 11e which is common to the voltage measuring electrode pairs is placed on the abdomen of the trunk at a sufficient distance from the current applying electrodes 10e and 10f and the other voltage measuring electrodes 11f (right side) and 11g (left side) are placed in the vicinity of the current applying electrodes 10e and 10f so as to measure V3b and V3b', thereby measuring the subcutaneous fat tissue layer or measuring the subcutaneous fat tissue layer and visceral fat tissues selectively.

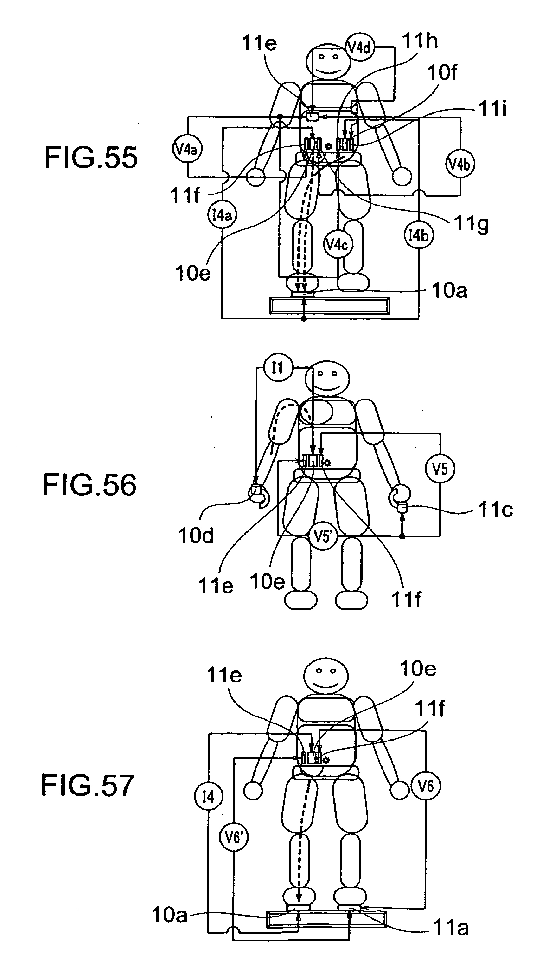

Fig. 55 illustrates a lead system in which a current applying electrode is placed at the bottom of foot (as a foot electrode), the other current applying electrode is placed in the abdomen of the trunk (on the aponeurosis) and two voltage measuring electrode pairs are placed on the abdomen of the trunk.

Fig. 55 relates to right-leg/trunk-abdomen energization measurement, in particular. Fig. 55 illustrates an electrode arrangement in which a current applying electrode 10a which is common to current applying electrode pairs is placed at the bottom of the right leg (as a foot electrode) and the other current applying electrodes 10e (right side) and 10f (left side) are placed on the left and right sides of the navel A in the abdomen of the trunk (on the aponeurosis) so as to apply currents I4a and I4b; a voltage measuring electrode 11e is placed on the abdomen of the trunk at a sufficient distance from the current applying electrodes 10e and 10f and the other voltage measuring electrode 11f is placed in the vicinity of and on the right side of the current applying electrode 10e so as to measure V4a; a voltage measuring electrode 11e is placed on the abdomen of the trunk at a sufficient distance from the current applying electrodes 10e and 10f and the other voltage measuring electrode 11g is placed in the vicinity of and on the left side of the current applying electrode 10e so as to measure V4b; a voltage measuring electrode 11e is placed on the abdomen of the trunk at a sufficient distance from the current applying electrodes 10e and 10f and the other voltage measuring electrode 11h is placed in the vicinity of and on the right side of the current applying electrode 10f so as to measure V4c; and a voltage measuring electrode 11e is placed on the abdomen of the trunk at a sufficient distance from the current applying electrodes 10e and 10f and the other voltage measuring electrode 11i is placed in the vicinity of and on the left side of the current applying electrode 10f so as to measure V4d, thereby measuring the subcutaneous fat tissue layer or measuring the subcutaneous fat tissue layer and visceral fat tissues selectively.

Fig. 56 illustrates a lead system in which a current applying electrode is placed on a palm (as a grip electrode), the other current applying electrode is placed in the abdomen of the trunk (on the aponeurosis), a voltage measuring electrode is placed on the other palm (as a grip electrode), and the other voltage measuring electrodes are placed on the abdomen of the trunk.

Fig. 56 relates to right-arm/trunk-abdomen energization measurement according to the lead system of Organ et al., in particular. Fig. 56 illustrates an electrode arrangement in which a current applying electrode 10d is placed on the palm of the right hand (as a grip electrode) and the other current applying electrode 10e is placed in the abdomen of the trunk (on the aponeurosis) so as to apply a current I1 and a voltage measuring electrode 11c which is common to two voltage measuring electrode pairs is placed on the palm of the left hand (as a grip electrode) and the other voltage measuring electrodes are placed in the vicinity of and on the left and right sides of the current applying electrode 10e on the abdomen of the trunk so as to measure potential differences V5 and V5', thereby measuring the subcutaneous fat tissue layer or measuring the subcutaneous fat tissue layer and visceral fat tissues selectively.

Fig. 57 illustrates a lead system in which a current applying electrode is placed at the bottom of foot (as a foot electrode), the other current applying electrode is placed in the abdomen of the trunk (on the aponeurosis), a voltage measuring electrode is placed at the bottom of the other foot (as a foot electrode), and the other voltage measuring electrodes are placed on the abdomen of the trunk.

Fig. 57 relates to right-leg/trunk-abdomen energization measurement according to the lead system of Organ et al., in particular. Fig. 57 illustrates an electrode arrangement in which a current applying electrode 10a is placed at the bottom of the right leg (as a foot electrode) and the other current applying electrode 10e is placed in the abdomen of the trunk (on the aponeurosis) so as to apply a current I4 and a voltage measuring electrode 11a which is common to two voltage measuring electrode pairs is placed at the bottom of the left leg (as a foot electrode) and the other voltage measuring electrodes 11f and 11e are placed in the vicinity of and on the left and right sides of the current applying electrode 10e on the abdomen of the trunk so as to measure potential differences V6 and V6', thereby measuring the subcutaneous fat tissue layer or measuring the subcutaneous fat tissue layer and visceral fat tissues selectively.

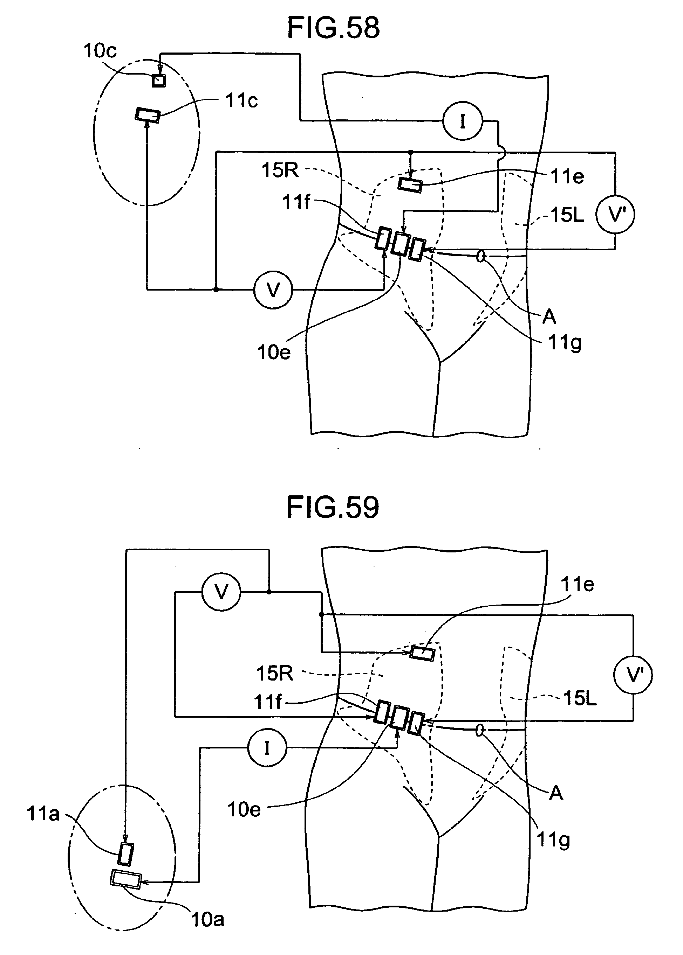

Figs. 58 and 59 are diagrams illustrating specific electrode arrangement examples of the above described arrangement methods for measuring the subcutaneous fat tissue layer or measuring the subcutaneous fat tissue layer and visceral fat tissues selectively, in the same manner as in Fig. 15 and other drawings.

Fig. 58 illustrates an electrode arrangement in which a current applying electrode 10e is placed on the right aponeurosis 15R around the navel A of the trunk and the other current applying electrode 10c is placed at a position on the upper-right extremity which is sufficiently distant from the trunk, e.g. the palm of the right hand, so as to apply a current I, and a voltage measuring electrode 11c which is common to voltage measuring electrode pairs is placed at a position on the upper-left extremity, e.g. the palm of the left hand, or, a voltage measuring electrode 11e may be placed at a position on the right aponeurosis 15R which is sufficiently distant from the current applying electrode 10e, the other voltage measuring electrode 11f is placed in the vicinity of and on the right side of the current applying electrode 10e and the other voltage measuring electrode 11g is placed in the vicinity of and on the left side of the current applying electrode 10e so as to measure potential differences V and V', thereby measuring the subcutaneous fat tissue layer or measuring the subcutaneous fat tissue layer and visceral fat tissues selectively.

Fig. 59 illustrates an electrode arrangement in which a current applying electrode 10e is placed on the right aponeurosis 15R of the trunk and the other current applying electrode 10a is placed at a position on the lower-right extremity which is sufficiently distant from the trunk, e.g. the bottom of the right leg, so as to apply a current I; a voltage measuring electrode 11a which is common to voltage measuring electrode pairs is placed at a position on the lower-left extremity, e.g. the bottom of the left leg, or, a voltage measuring electrode 11e may be placed at a position on the right aponeurosis 15R which is sufficiently distant from the current applying electrode 10e, the other voltage measuring electrode 11f is placed on the right aponeurosis 15R around the navel A of the trunk and in the vicinity of the current applying electrode 10e, and the other voltage measuring electrode 11g is placed on the right aponeurosis 15R around the navel A of the trunk and in the vicinity of the current applying electrode 10e so as to measure potential differences V and V', thereby measuring the subcutaneous fat tissue layer or measuring the subcutaneous fat tissue layer and visceral fat tissues selectively.

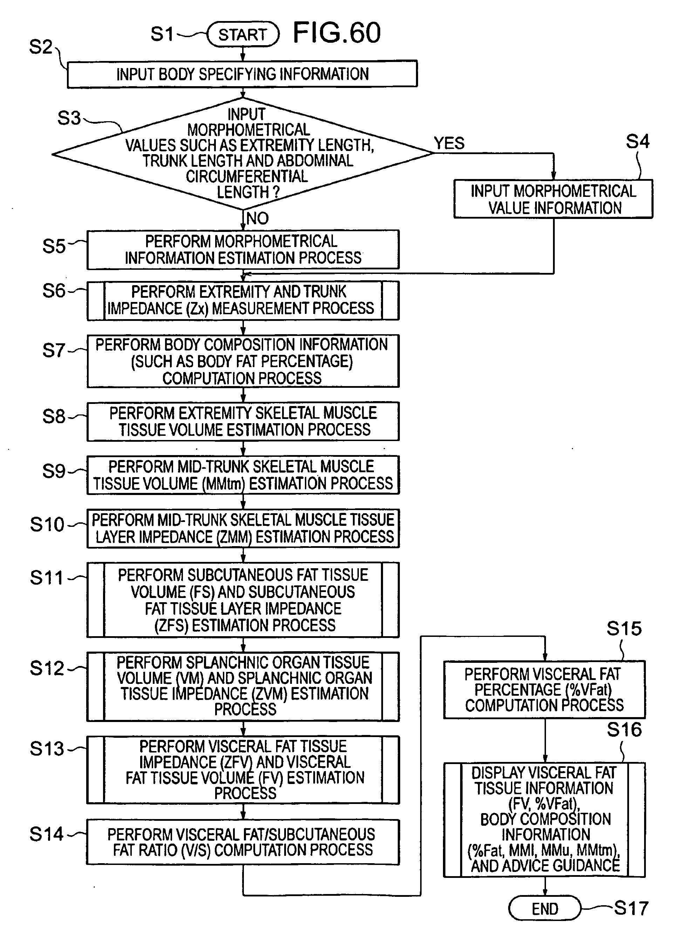

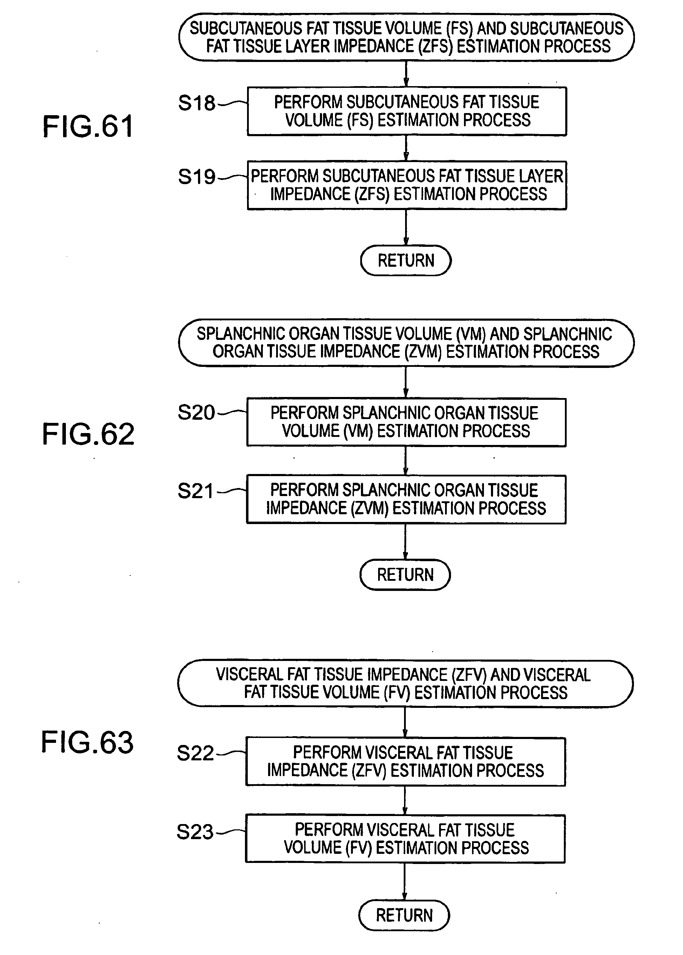

Finally, an example of the operations of the truncal visceral fat measuring apparatus in examples of the present invention shown in Figs. 1 and 2 will be described, with reference to a main flowchart shown in Fig. 60 and subroutine flowcharts shown in Figs. 61 to 68.

In the main flowchart shown in Fig. 60, firstly, when the power switch (not shown) in the input section 5a is pressed, electric power is supplied from the power supply section 1 to the sections in the electrical system, and the display section 5b displays a screen for inputting body specifying information (such as a body height, a body weight, gender and age) including a body height (STEP S1).

Then, a user inputs a body height, a body weight, sex, age and the like from the input section 5a in accordance with the above screen (STEP S2). In this case, the body weight may also be determined such that a voltage based on body weight specifying information (body weight) is measured and body weight specifying information (body weight) is calculated by the computation/control section 7. These input values are stored in the storage section 4.

Then, in STEP S3, it is determined whether morphometric measured values such as four-extremity lengths, trunk length and abdominal circumferential length are to be input. When these morphometric measured values are to be input, morphometry is carried out and measured values of the four-extremity lengths (upper-extremity length, lower-extremity length), trunk length (mid-trunk length), abdominal circumferential length and the like are input from the input section 5a in STEP S4, and the computation/control section 7 then proceeds to STEP S6. When it is determined in STEP S3 that the morphometric measured values are not input, the computation/control section 7 proceeds to STEP S5. These input values are also stored in the storage section 4. Similarly, numerical value data and other data obtained in the following processes are also stored in the storage section 4.

In STEP S5, the computation/control section 7 performs a morphometrical information estimation process (for example, using a calibration curve prepared from human body information database) for estimating upper-extremity length, lower-extremity length, mid-trunk length, abdominal circumferential length or the like from the body specifying information such as the body height, body weight, sex and age stored in the storage section 4.

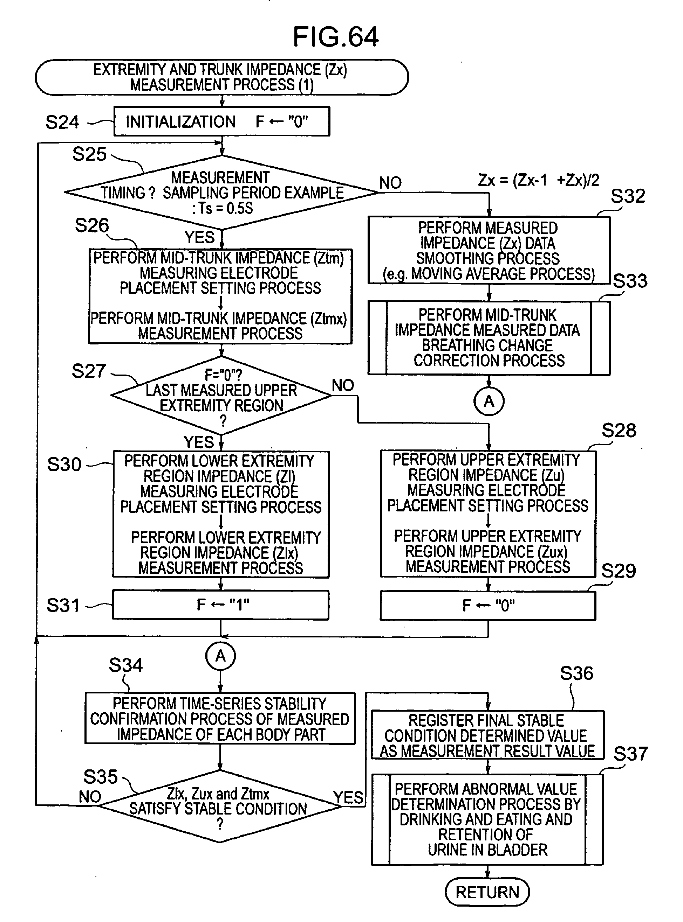

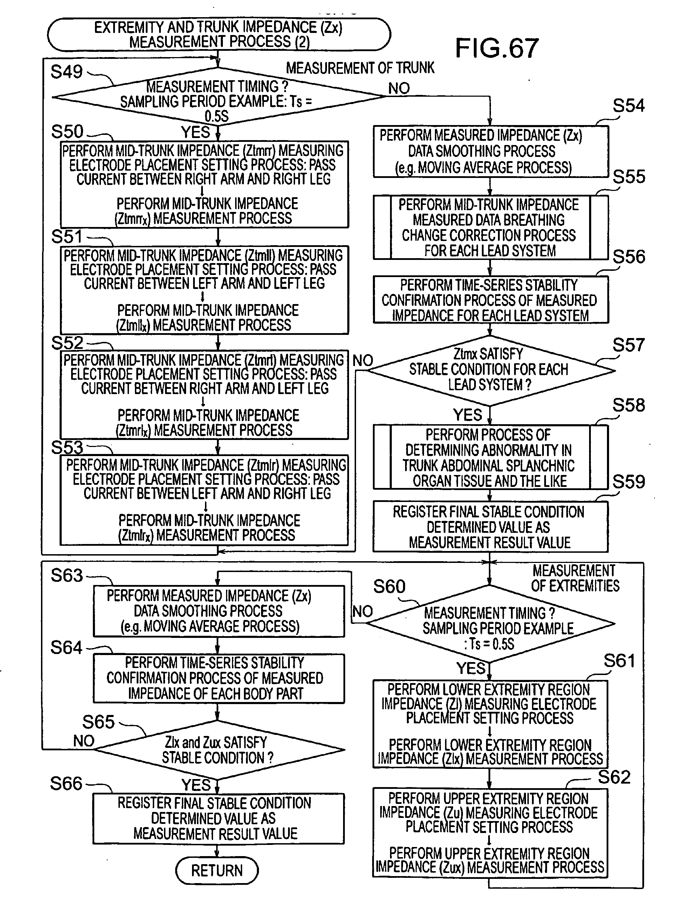

Then, in STEP S6, an extremity and trunk impedance measurement process is carried out in the body part impedance measuring section 3. This extremity and trunk impedance measurement process will be described later with reference to the subroutine flowcharts shown in Figs. 64 and 67.

Then, in STEP S7, the computation/control section 7 performs a body composition information (such as a body fat percentage) computation process. According to this computation process, for example, a body fat percentage %Fat is obtained from the following arithmetic expression by use of the length Lu of the upper extremity, the impedance value Zu of the upper extremity, the length L1 of the lower extremity, the impedance value Zl of the lower extremity, the length Ltm of the trunk and the impedance Ztm of the whole mid-trunk that are stored in the storage section 4.

$$\%Fat = a \times Lu^2/Zu + b \times Ll^2/Zl + c \times Ltm^2/Ztm + d$$

wherein a, b, c and d are constants.

Then, in STEP S8, the computation/control section 7 performs an extremity skeletal muscle tissue volume estimation process. In this extremity skeletal muscle tissue volume estimation process, the skeletal muscle tissue volume of the lower extremity is calculated by use of the numerical values stored in the storage section 4 and in accordance with the above expression 2, and the skeletal muscle tissue volume of the upper extremity is calculated by use of the numerical values stored in the storage section 4 and in accordance with the above expression 3.

Then, in STEP S9, the computation/control section 7 performs a mid-trunk skeletal muscle tissue volume estimation process. In this mid-trunk skeletal muscle tissue volume estimation process, the skeletal muscle tissue volume of the mid-trunk is calculated based on the numerical values stored in the storage section 4, the skeletal muscle tissue volumes of the lower and upper extremities which have been obtained in STEP S8 and the above expression 1 or expression 4 or expression 5, expression 5, expression 5-1, expression 5-2 which are stored in the storage section 4.

Then, in STEP S10, the computation/control section 7 performs a mid-trunk skeletal muscle tissue layer impedance estimation process. In this estimation process, the impedance of the skeletal muscle tissue layer of the mid-trunk is calculated by use of the numerical values stored in the storage section 4, the skeletal muscle tissue volume of the mid-trunk which has been obtained in STEP S9 and the above expressions 19-1, 19-2, expression 20, expression 21.

In STEP S11, the computation/control section 7 performs a subcutaneous fat tissue volume and subcutaneous fat tissue layer impedance estimation process. This STEP S11 will be further described later with reference to the subroutine flowchart shown in Fig. 61.

In STEP S12, the computation/control section 7 performs a splanchnic organ tissue volume and splanchnic organ tissue impedance estimation process. This STEP S12 will be further described later with reference to the subroutine flowchart shown in Fig. 62.

In STEP S13, the computation/control section 7 performs a visceral fat tissue impedance and visceral fat tissue volume estimation process. This STEP S13 will be further described later with reference to the subroutine flowchart shown in Fig. 63.

Then, in STEP S14, the computation/control section 7 performs a visceral fat/subcutaneous fat ratio computation process. In this computation process, the fat tissue volume of the abdomen of the trunk is calculated by use of the subcutaneous fat tissue volume and visceral fat tissue volume stored in the storage section 4 and in accordance with the above expression 29 stored in the storage section 4, and the visceral fat/subcutaneous fat ratio is calculated in accordance with the above expression 30 stored in the storage section 4.

Then, in STEP S15, the computation/control section 7 performs a visceral fat percentage computation process. In this computation process, a truncal visceral fat percentage %VFat is calculated by use of the body fat percentage %Fat, visceral fat tissue V and subcutaneous fat tissue S calculated by the above computation processes and stored in the storage section 4 and in accordance with the following expression.

```
%VFat = %Fat x (V/S)/[(V/S) + 1]
```

Then, in STEP S16, the computation/control section 7 displays the visceral fat tissue information and body composition information calculated by the above computation processes and advice guidance obtained by a process to be described later. Thereby, a series of processes are ended (STEP S17).

Next, the above subcutaneous fat tissue volume and subcutaneous fat tissue layer impedance estimation process in STEP S11 will be described in detail with reference to the subroutine flowchart shown in Fig. 61. In this estimation process, in STEP S18, a subcutaneous fat tissue volume is calculated by use of the numerical values stored in the storage section 4 and the above expression 22-1 or 22-2, and in STEP S19, a subcutaneous fat tissue layer impedance is calculated by use of the numerical values stored in the storage section 4, the subcutaneous fat tissue volume and the above expressions 23-1, 23-2 and 24.

Next, the above splanchnic organ tissue volume and splanchnic organ tissue impedance estimation process in STEP S12 will be described in detail with reference to the subroutine flowchart shown in Fig. 62. In this estimation process, in STEP S20, a splanchnic organ tissue volume is calculated by use of the numerical values stored in the storage section 4 and the above expressions 13-1 and 13-2, and in STEP S21, a splanchnic organ tissue impedance is calculated by use of the numerical values stored in the storage section 4 and the above expressions 14-1, 14-2, 15, 16, 17-1 and 17-2.

Next, the above visceral fat tissue impedance and visceral fat tissue volume estimation process in STEP S13 will be described in detail with reference to the subroutine flowchart shown in Fig. 63. In this estimation process, in STEP S22, a visceral fat tissue impedance is calculated by use of the numerical values stored in the storage section 4 and the above expressions 6 to 12, 16, 17, 21 and 24, and in STEP S23, a visceral fat tissue volume is calculated by use of the numerical values stored in the storage section 4, the calculated visceral fat tissue impedance and the above expressions 25, 26, 27, 28-1 and 28-2.

Next, the extremity and trunk impedance measurement process in STEP S6 will be described in detail with reference to the subroutine flowchart of Fig. 64 showing a first embodiment. In this first embodiment, "process of removing influence of change by breathing" and "process of determining abnormal value by drinking and eating and retention of water (e.g. urine) in bladder or the like" as described in (28) and (29) in the above 10. are conducted. Firstly, in STEP S24, the computation/control section 7 initializes various memory counter numbers for measurement data of the impedance Ztm of the abdomen (middle portion) of the trunk and a flag F.

Then, in STEP S25, the computation/control section 7 determines whether it is measurement timing. When it has been determined that it is measurement timing, the computation/control section 7 performs a mid-trunk impedance (Ztm) measuring electrode placement setting process and a mid-trunk impedance (Ztmx) measurement process in STEP S26. In this case, the computation/control section 7 selects any of the electrode placements as described with reference to Fig. 3.

Then, in STEP S27, the computation/control section 7 determines whether F is "0" and it is the last measured upper extremity region. If not, the computation/control section 7 proceeds to STEP S28 and performs an upper extremity region impedance (Zu) measuring electrode placement setting process and an upper extremity region impedance (Zux) measurement process. Further, in STEP S29, F is set at "0".

In STEP S27, if it is determined that F is "0" and it is the last measured upper extremity, the computation/control section 7 performs a lower extremity region impedance (Zl) measuring electrode placement setting process and a lower extremity region impedance (Zlx) measurement process in STEP 30 and sets F at "1". The computation/control section 7 repeats the operations of STEPS S25 to S31.

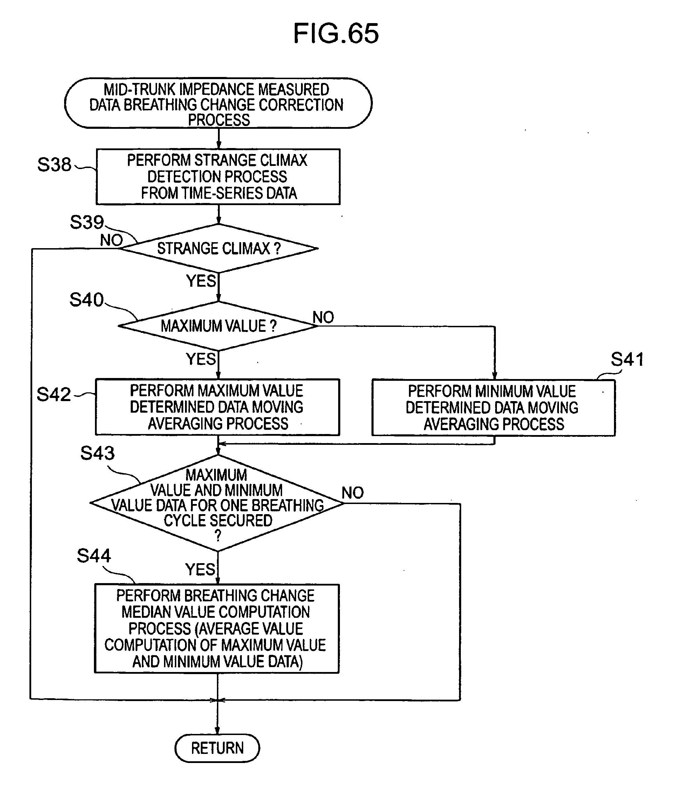

When it has been determined in STEP S25 that it is not measurement timing, the computation/control section 7 proceeds to STEP S32 and performs a measured impedance (Zx) data smoothing process (e.g. a moving average process). Then, in STEP S33, the computation/control section 7 performs a mid-trunk impedance measured data breathing change correction process. This correction process will be further described later with reference to the subroutine flowchart of Fig. 65.

Then, in STEP S34, the computation/control section 7 performs a time-series stability confirmation process of measured impedance of each body part. This is carried out by determining whether each value after the mid-trunk impedance measured data breathing change correction process in STEP S33 has converged to a value within a predetermined change in a predetermined number of times. In STEP S35, the computation/control section 7 determines whether the measured Zlx, Zux and Ztmx satisfy a stable condition. This determination is made such that a median breathing value is determined at the point when a median breathing value in each breathing cycle enters a stable range within a predetermined number of times. When it is determined in this STEP S35 that the stable condition is satisfied, the computation/control section 7 proceeds to STEP S36 and registers the impedance value of the determined median value as the impedance value of the abdomen of the trunk and a final stable condition determined value as a measurement result value in the storage section 4. When it is determined in STEP S35 that the stable condition is not satisfied, the computation/control section 7 returns to STEP S25 and repeats the above processes.

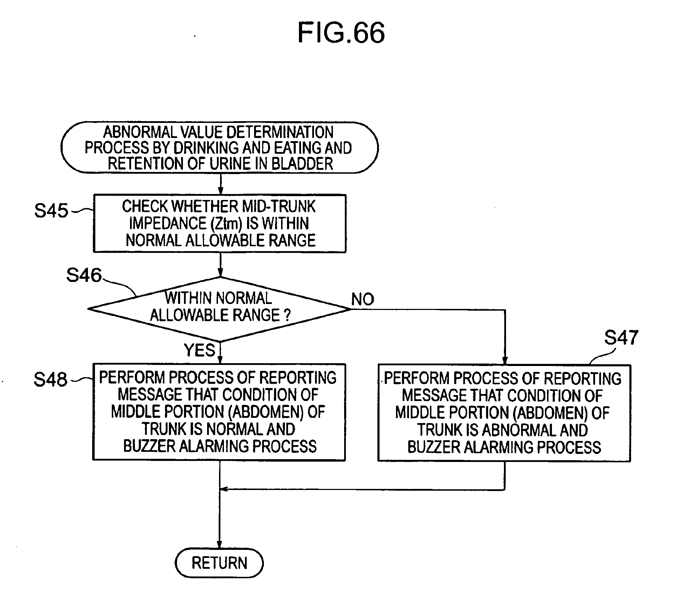

Subsequent to STEP S36, the computation/control section 7 performs an abnormal value determination process by drinking and eating and retention of urine in bladder in STEP S37. This abnormal value determination process will be further described later with reference to the subroutine flowchart of Fig. 66.

Next, the mid-trunk impedance measured data breathing change correction process in STEP S33 will be described in detail with reference to the subroutine flowchart of Fig. 65. Firstly, in STEP S38, the computation/control section 7 performs a strange climax detection process from the time-series data processed in STEP S33. In STEP S39, the computation/control section 7 determines whether it is a strange climax. This is carried out by detecting data of the point of polarity change of differential coefficients or differential values before and after the climax. When it is determined in STEP S39 that it is a strange climax, the computation/control section 7 proceeds to STEP S40 and determines whether it is the maximum value. This is a step of classifying the maximum value and the minimum value. When it is not the maximum value, a minimum value determined data moving averaging process is performed in STEP S41 in accordance with the following expression stored in the storage section 4.

$$[Ztm]min_x \leftarrow ([Ztm]min_{x-1} + [Ztm]min_x)/2 \ ... \ \text{expression 50}$$

When it is determined in STEP S40 that it is the maximum value, a maximum value determined data moving averaging process is performed in STEP S42 in accordance with the following expression stored in the storage section 4.

$$[Ztm]max_x \leftarrow ([Ztm]max_{x-1} + [Ztm]max_x)/2 \ ... \ \text{expression 51}$$

Then, in STEP S43, it is determined whether data of the maximum and minimum values for one breathing cycle has been secured. When it has been determined in STEP S43 that the data has been secured, a breathing change median value computation process (average value computation of maximum value and minimum value data) is performed in STEP S44 in accordance with the following expression stored in the storage section 4.

$$[Ztm]_x \leftarrow ([Ztm]max_x + [Ztm]min_x)/2 \ ... \ \text{expression 52}$$

When the computation/control section 7 has determined that it is not a strange climax in STEP S39, the computation/control section 7 proceeds to "return". Further, when the computation/control section 7 has determined that the data of the maximum and minimum values for one breathing cycle has not been secured in STEP S43, the computation/control section 7 proceeds to "return".

Next, the abnormal value determination process by drinking and eating and retention of urine in bladder in STEP S37 will be described in detail with reference to the subroutine flowchart of Fig. 66. Firstly, in STEP S45, the computation/control section 7 checks whether the mid-trunk impedance (Ztm) is within a normal acceptable range, in accordance with the following expression stored in the storage section 4.

$$\text{Mean} - 3SD \leq Ztm \leq \text{Mean} + 3SD$$

In this case, $26.7 \pm 14.4$ (Mean$\pm$3SD) is conceivable as an example of acceptable value, for $26.7 \pm 4.8$ (Mean$\pm$SD).

In STEP S46, it is determined whether the mid-trunk impedance is within the acceptable range. When it is determined that the mid-trunk impedance is not within the acceptable range, the computation/control section 7 proceeds to STEP S47 and performs a process of reporting a message that the condition of the middle portion (abdomen) of the trunk is abnormal and a buzzer alarming process. That is, appropriate advice is displayed in the display section 5b, and a buzzer sound is generated in the buzzer alarming section 15. As this advice, for example, "Please prepare for defection or urination due to abnormal trunk condition." is displayed with buzzer sounds. Further, when the same determination result is obtained after the preparation, it is possible to complete the measurement by use of an abnormal value so as not to abort the measurement.

When it is determined in STEP S46 that the mid-trunk impedance is within the acceptable range, the computation/control section 7 performs a process of reporting a message that the condition of the middle portion (abdomen) of the trunk is normal and a buzzer alarming process. That is, appropriate advice is displayed in the display section 5b, and a buzzer sound is generated in the buzzer alarming section 15. As this advice, for example, "The condition of the trunk is normal." is displayed with a buzzer sound.

Next, the extremity and trunk impedance measurement process in STEP S6 will be described in detail with reference to the subroutine flowchart of Fig. 67 showing a second embodiment. In this second embodiment, "process of removing influence of change by breathing" and "process of determining abnormality in splanchnic organ tissues of abdomen" as described in (28) and (30) in the above 10. are conducted. Firstly, in STEP S49, the computation/control section 7 determines whether it is measurement timing. When it has been determined that it is measurement timing, the computation/control section 7 performs a mid-trunk impedance (Ztmrr) measuring electrode placement setting process (energization between the right arm and the right leg) (refer to Fig. 3A) and a mid-trunk impedance (Ztmrr$_x$) measurement process in STEP S50 as part of trunk measurements. Then, in STEP S51, the computation/control section 7 performs a mid-trunk impedance (Ztmll) measuring electrode placement setting process (energization between the left arm and the left leg) (refer to Fig. 3B) and a mid-trunk impedance (Ztmll$_x$) measurement process. Then, in STEP S52, the computation/control section 7 performs a mid-trunk impedance (Ztmrl) measuring electrode placement setting process (energization between the right arm and the left leg) (refer to Fig. 3C) and a mid-trunk impedance (Ztmrl$_x$) measurement process. Then, in STEP S53, the computation/control section 7 performs a mid-trunk impedance (Ztmlr) measuring electrode placement setting process (energization between the left arm and the right leg) (refer to Fig. 3D) and a mid-trunk impedance (Ztmlr$_x$) measurement process. The computation/control section 7 carries out the above measurement operations until a predetermined number of samples are obtained.

When it has been determined in STEP S49 that it is not measurement timing, the computation/control section 7 proceeds to STEP S54 and performs a measured impedance (Zx) data smoothing process (e.g. a moving average process) (mid-trunk: Ztmrr$_x$, Ztmll$_x$, Ztmrl$_x$, Ztmlr$_x$). Then, in STEP S55, the computation/control section 7 performs a mid-trunk impedance measured data breathing change correction process for each lead system. Descriptions of this correction process for each lead system will be omitted since it is the same as the correction process described above with reference to the subroutine flowchart of Fig. 65.

Then, in STEP S56, the computation/control section 7 performs a time-series stability confirmation process of measured impedance for each lead system. This is carried out by determining whether each value after the mid-trunk impedance measured data breathing change correction process for each lead system in STEP S55 has converged to a value within a predetermined change in a predetermined number of times. In STEP S57, the computation/control section 7 determines whether each measured Ztmx satisfies a stable condition. This determination is made such that a median breathing value is determined at the point when a median breathing value in each breathing cycle enters a stable range within a predetermined number of times. When it is determined in this STEP S57 that the stable condition is not satisfied, the computation/control section 7 returns to STEP S49 and repeats the above measurement operations and processes.

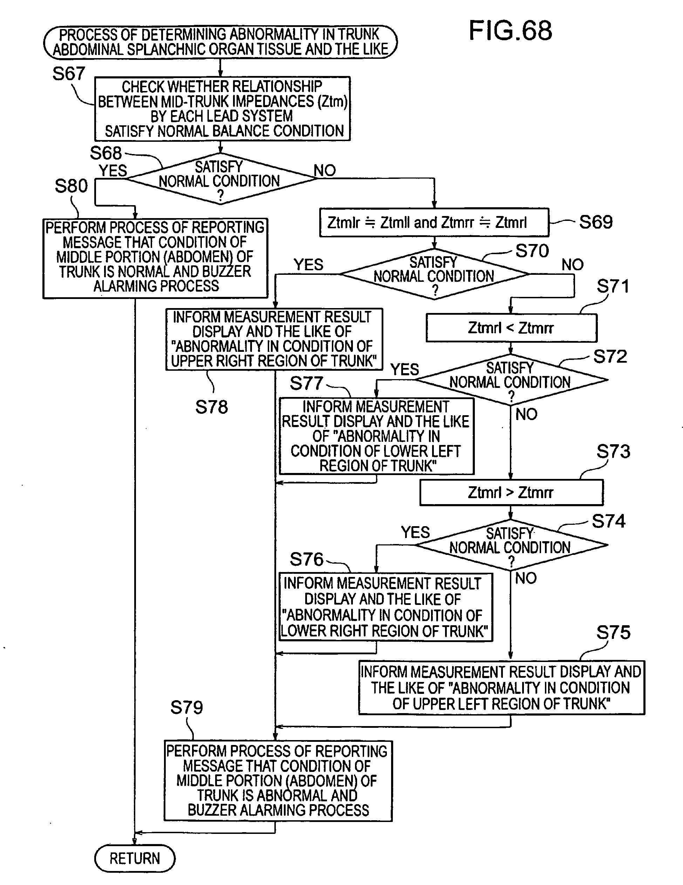

When it has been determined in STEP S57 that the stable condition is satisfied, the computation/control section 7 proceeds to STEP S58 and performs a process of determining abnormality in the splanchnic organ tissue of the abdomen of the trunk and the like. This determination process will be further described later with reference to the subroutine flowchart of Fig. 68.

Then, in STEP S59, the computation/control section 7 stores and registers a final stable condition determined value in the measurement of the mid-trunk impedance as a measurement result value in the storage section 4 (Ztm ← Ztmlr$_x$). In this case, Ztmlr (impedance upon energization between the left arm and the right leg) is adopted out of the four trunk impedances.

Then, the computation/control section 7 proceeds to STEP S60 for measurements of extremities and determines whether it is measurement timing. If it has been determined that it is measurement timing, the computation/control section 7 performs a lower extremity region impedance (Zl) measuring electrode placement setting process and a lower extremity region impedance (Zlx) measurement process in STEP S61 and performs an upper extremity region impedance (Zu) measuring electrode placement setting process and an upper extremity region impedance (Zux) measurement process in STEP S62. The computation/control section 7 performs the above measurement operations repeatedly.

When it has been determined in STEP S60 that it is not measurement timing, the computation/control section 7 performs a measured impedance (Zx) data smoothing process (e.g. a moving average process) (upper extremity region: $Zu_x$, lower extremity region: $Zl_x$) in STEP S63. Then, in STEP S64, the computation/control section 7 performs a time-series stability confirmation process of measured impedance of each body part. This is carried out by determining whether each value after the process in STEP S64 has converged to a value within a predetermined change in a predetermined number of times. In STEP S65, the computation/control section 7 determines whether the measured $Zl_x$ and $Zu_x$ satisfy a stable condition. This determination is made such that a median breathing value is determined at the point when a median breathing value in each breathing cycle enters a stable range within a predetermined number of times. When it has been determined in this STEP S65 that the stable condition is not satisfied, the computation/control section 7 returns to STEP S60 and repeats the above measurement operations and processes.

When it has been determined in STEP S65 that the stable condition is satisfied, the computation/control section 7 stores and registers a final stable condition determined value in the measurement of the extremity impedances as a measurement result value in the storage section 4 ($Zl \leftarrow Zl_x$, $Zu \leftarrow Zu_x$).

Next, the process of determining abnormality in the splanchnic organ tissue of the abdomen of the trunk and the like in STEP S58 will be described in detail with reference to the subroutine flowchart of Fig. 68. Firstly, in STEP S67, the computation/control section 7 checks whether the relationship among the mid-trunk impedances (Ztm) by each lead system satisfies the normal balance condition. As described in (d) in (30) in the above 10., the normal condition is the relationship of Ztmlr ≒ Ztmll < Ztmrr ≒ Ztmrl.

When it has been determined in STEP S68 that the relationship does not satisfy the normal condition, the computation/control section 7 proceeds to STEP S69 and determines whether the following expressions, Ztmlr ≒ Ztmll and Ztmrr ≒ Ztmrl, are satisfied in STEP S70. When it has been determined that the condition is not satisfied, the computation/control section 7 proceeds to STEP S71 and determines whether the following expression, Ztmrl < Ztmrr, is satisfied in STEP S72. When this condition is not satisfied, the computation/control section 7 proceeds to STEP S73 and determines whether the following expression, Ztmrl > Ztmrl, is satisfied in STEP S74. When this condition is not satisfied, the computation/control section 7 determines that there is an abnormal balance in the tissue of the upper left region of the middle portion (abdomen) of the trunk, and in STEP S75, the computation/control section 7 performs a process of reporting a message that the condition of the middle portion (abdomen) of the trunk is abnormal and displays appropriate advice in the display section 5b. As this advice, for instance, "The condition of the upper left region of the trunk is abnormal." may be displayed.

When it has been determined in STEP S74 that the condition is satisfied, the computation/control section 7 determines that there is an abnormal balance in the tissue of the lower right region of the middle portion (abdomen) of the trunk, and in STEP S76, the computation/control section 7 performs a process of reporting a message that the condition of the middle portion (abdomen) of the trunk is abnormal and displays appropriate advice in the display section 5b. As this advice, for example, "The condition of the lower right region of the trunk is abnormal." may be displayed.

When it has been determined in STEP S72 that the condition is satisfied, the computation/control section 7 determines that there is an abnormal balance in the tissue of the lower left region of the middle portion (abdomen) of the trunk, and in STEP S77, the computation/control section 7 performs a process of reporting a message that the condition of the middle portion (abdomen) of the trunk is abnormal and displays appropriate advice in the display section 5b. As this advice, for example, "The condition of the lower left region of the trunk is abnormal." may be displayed.

When it has been determined in STEP S70 that the condition is satisfied, the computation/control section 7 determines that there is an abnormal balance in the tissue of the upper right region of the middle portion (abdomen) of the trunk, and in STEP S78, the computation/control section 7 performs a process of reporting a message that the condition of the middle portion (abdomen) of the trunk is abnormal and displays appropriate advice in the display section 5b. As this advice, for example, "The condition of the upper right region of the trunk is abnormal." may be displayed.

Thus, the computation/control section 7 performs a process of reporting a message that the condition of the middle portion (abdomen) of the trunk is abnormal and a buzzer alarming process in STEP S79. For example, a message such as "Please prepare for defection or urination due to abnormal trunk condition." is displayed in the display section 5b and buzzer sounds are generated in the buzzer alarming section 15. Further, when the same determination result is obtained after the preparation, it is possible to complete the measurement by use of an abnormal value so as not to abort the measurement.

When it has been determined in STEP S68 that the condition is satisfied, the computation/control section 7 performs a process of reporting a message that the condition of the middle portion (abdomen) of the trunk is normal and a buzzer alarming process in STEP S80. That is, appropriate advice is displayed in the display section 5b, and a buzzer sound is generated in the buzzer alarming section 15. As this advice, for example, "The condition of the trunk is normal." is displayed with a buzzer sound.

<Measurement of Subcutaneous Fat>

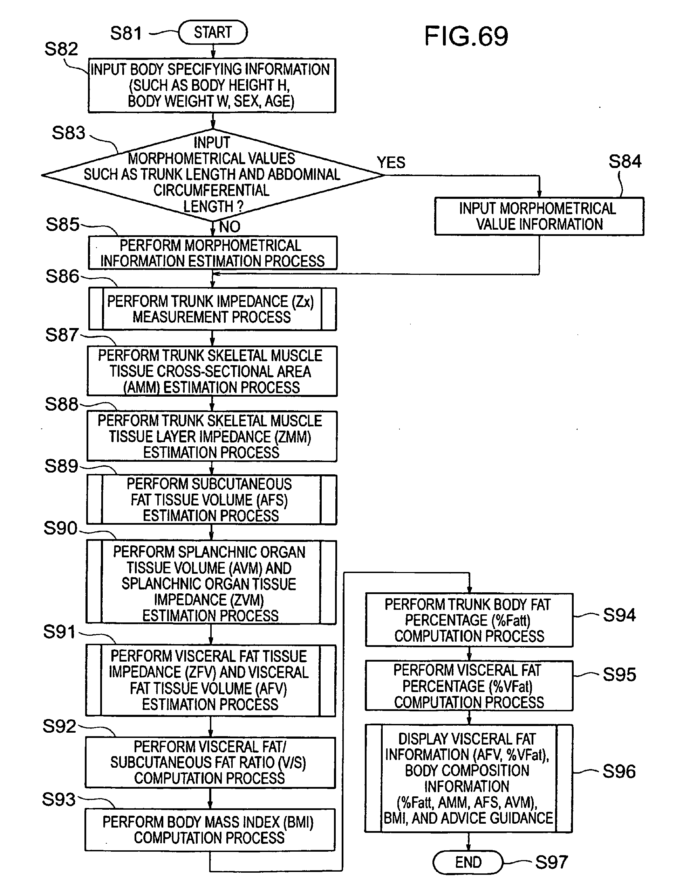

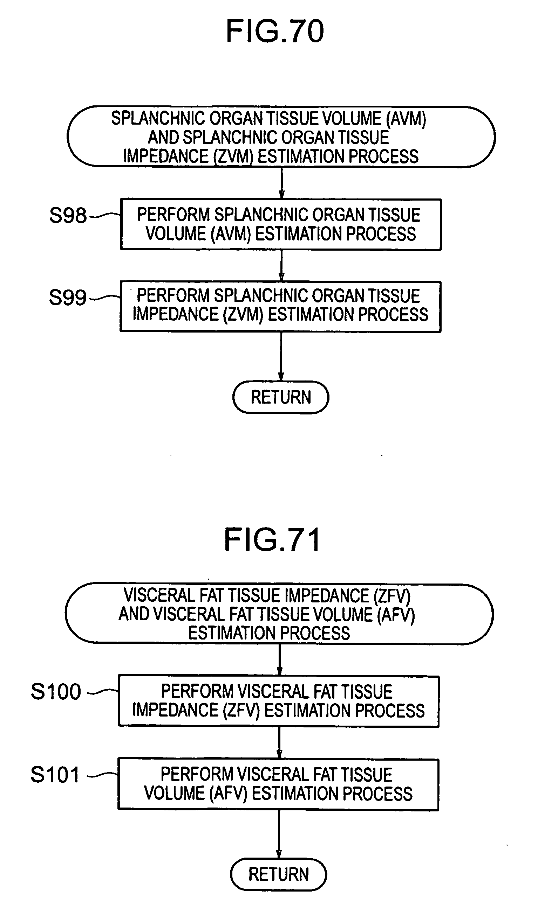

Next, operations for measuring the subcutaneous fat tissue layer will be primarily described with reference to a basic flowchart shown in Fig. 69 and subroutine flowcharts shown in Figs. 70 to 72, 64 and 65.

In the basic flowchart shown in Fig. 69, firstly, when the power switch (not shown) in the display/input section 5 is pressed, electric power is supplied from the power supply section 1 to the sections in the electrical system, and the display section 5b displays a screen for inputting body specifying information (such as a body height, a body weight, sex and age) including a body height (STEP S81).

Then, a user inputs a body height, a body weight, sex, age and the like from the display/input section 5 in accordance with the above screen (STEP S82). In this case, although the body weight may be input from the display/input section 5, it is also possible that data measured by the body weight measuring section 2 is automatically input and body weight specifying information (body weight) is calculated by the computation/control section 7. These input values are stored in the storage section 4.

Then, in STEP S83, it is determined whether morphometric measured values such as trunk length and abdominal circumferential length are to be input. When these morphometric measured values are to be input, morphometry is carried out and measured values of the trunk length, abdominal circumferential length and the like are input from the display/input section 5 in STEP S84, and the computation/control section 7 then proceeds to STEP S86. When it is determined in STEP S83 that the morphometric measured values are not input, the computation/control section 7 proceeds to STEP S85. These input values are also stored in the storage section 4. Similarly, numerical value data and other data obtained in the following processes are also stored in the storage section 4.

In STEP S85, the computation/control section 7 performs a morphometrical information estimation process (for example, using a calibration curve prepared from human body information database) for estimating trunk length, abdominal circumferential length or the like from the body specifying information such as the body height, body weight, sex and age stored in the storage section 4.

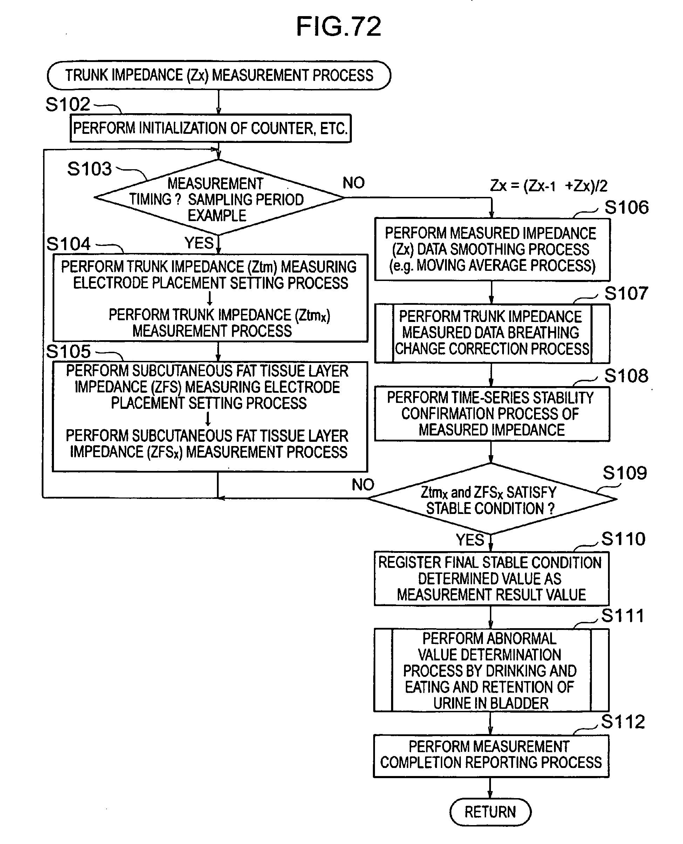

Then, in STEP S86, a trunk impedance measurement process is carried out in the impedance measuring section. This trunk impedance measurement process will be further described later with reference to the subroutine flowchart shown in Fig. 72.

Then, in STEP S87, the computation/control section 7 performs a trunk skeletal muscle tissue cross-sectional area (AMM) estimation process. This computation is carried out based on the above expression 32 by using, for example, the body height H, body weight W and age Age stored in the storage section 4.

Then, in STEP S88, the computation/control section 7 performs a trunk skeletal muscle tissue layer impedance (ZMM) estimation process. This ZMM is estimated based on the above expression 33 by use of the body height H stored in the storage section 4 and the AMM estimated in STEP S87.

Then, in STEP S89, the computation/control section 7 performs a subcutaneous fat tissue volume (AFS) estimation process. This estimation process can be carried out based on the above expression 45.

In STEP S90, the computation/control section 7 performs a splanchnic organ tissue volume (AVM) and splanchnic organ tissue impedance (ZVM) estimation process. This STEP S90 will be further described later with reference to the subroutine flowchart shown in Fig. 70.

In STEP S91, the computation/control section 7 performs a visceral fat tissue impedance (ZFV) and visceral fat tissue volume (AFV) estimation process. This STEP S91 will be further described later with reference to the subroutine flowchart shown in Fig. 71.

Then, in STEP S92, the computation/control section 7 computes a visceral fat/subcutaneous fat ratio (V/S). This computation is carried out in accordance with the above expression 42 stored in the storage section 4.

Then, in STEP S93, the computation/control section 7 computes a body mass index (BMI). The body mass index can be calculated from the body weight W and body height H stored in the storage section 4 in accordance with the following expression.

$$BMI = W/H^2 \quad \text{... expression 53}$$

Further, in STEP S94, the computation/control section 7 computes a trunk body fat percentage (%Fatt). The trunk body fat percentage can be calculated from the subcutaneous fat tissue volume (AFS), visceral fat tissue volume (AFV), trunk skeletal muscle cross-sectional area (AMM) and splanchnic organ tissue volume (AVM) stored in the storage section 4 in accordance with the following expression.

$$\%Fatt = (AFS + AFV)/[(AFS + AFV) + AMM + AVM] \times 100 \quad \text{... expression } 54$$

Then, in STEP S95, the computation/control section 7 computes a visceral fat percentage (%VFat) . The visceral fat percentage is calculated from the trunk body fat percentage (%Fatt) and visceral fat/subcutaneous fat ratio (V/S) calculated by the above computations and stored in the storage section 4 in accordance with the following expression.

```
%VFat = %Fatt x (V/S)/[(V/S) + 1]
```

Finally, in STEP S96, the computation/control section 7 displays the visceral fat tissue information (AFV, %VFat), body composition information (%Fatt, AMM, AFS, AVM) and body mass index (BMI) obtained by the above computations and advice guidelines obtained by processes to be described later in the display section 5b. Thereby, a series of processes are ended (STEP S97).

Next, the above splanchnic organ tissue volume (AVM) and splanchnic organ tissue impedance (ZVM) estimation process in STEP S90 will be described in detail with reference to the subroutine flowchart of Fig. 70. This estimation process is carried out by calculating a splanchnic organ tissue volume (AVM) in STEP S98 by use of the numerical values stored in the storage section 4 and the above expression 40 and calculating a splanchnic organ tissue impedance (ZVM) in STEP S19 by use of the numerical values stored in the storage section 4 and the above expression 41.

Next, the above visceral fat tissue impedance (ZFV) and visceral fat tissue volume (AFV) estimation process in STEP S91 will be described in detail with reference to the subroutine flowchart of Fig. 71. This estimation process is carried out by calculating a visceral fat tissue impedance (ZFV) in STEP S100 by use of the numerical values stored in the storage section 4 and the above expression 36 and calculating a visceral fat tissue volume (AFV) in STEP S101 by use of the body height H stored in the storage section 4, the calculated visceral fat tissue impedance (ZFV) and the above expression 39.

Next, the trunk impedance measurement process in STEP S86 will be described in detail with reference to the subroutine flowchart of Fig. 72 showing a first embodiment. In this first embodiment, "process of removing influence of change by breathing" and "process of determining abnormal value by drinking and eating and retention of water (e.g. urine) in bladder or the like" as described in (28) and (29) in the above 10. are conducted. Firstly, in STEP S102, the computation/control section 7 initializes a counter and the number of samples for measurement data of the impedance $Ztm$ of the trunk, based on an instruction from the display/input section 5 or the like.

Then, in STEP S103, the computation/control section 7 determines whether it is measurement timing. When it has been determined that it is measurement timing, the computation/control section 7 performs a trunk impedance (Ztm) measuring electrode placement setting process and a trunk impedance ($Ztm_x$) measurement process in STEP S104. Further, the computation/control section 7 performs a subcutaneous fat tissue layer impedance (ZFS) measuring electrode placement setting process and a subcutaneous fat tissue layer impedance ($ZFS_x$) measurement process in STEP S105 and then returns to STEP S103.

Meanwhile, when it has been determined in STEP S103 that it is not measurement timing, the computation/control section 7 proceeds to STEP S106 and performs a measured impedance (Zx) data smoothing process (e.g. a moving average process), i.e. $Z_x = (Z_{x-1} + Z_x)/2$, on the trunk impedance ($Ztm_x$) and the subcutaneous fat tissue layer impedance ($ZFS_x$). Then, in STEP S107, the computation/control section 7 performs a trunk impedance measured data breathing change correction process. This correction process is the same as the correction process described with reference to Fig. 65. Since the subcutaneous fat tissue layer impedance ($ZFS_x$) is hardly influenced by a change in breathing, it is not subjected to the correction process, unlike the trunk impedance.

Then, in STEP S108, the computation/control section 7 performs a time-series stability confirmation process of measured impedance of each body part. This is carried out by determining whether each value after the trunk impedance measured data breathing change correction process in STEP S107 has converged to a value within a predetermined change in a predetermined number of times. In STEP S109, the computation/control section 7 determines whether the measured $Ztm_x$ and $ZFS_x$ satisfy a stable condition. This determination is made such that a median breathing value is determined at the point when a median breathing value in each breathing cycle enters a stable range within a predetermined number of times. When it is determined in this STEP S109 that the stable condition is satisfied, the computation/control section 7 proceeds to STEP S110 and registers the impedance value of the determined median value as the impedance value of the trunk or the subcutaneous fat tissue layer impedance value and a final stable condition determined value as a measurement result value in the storage section 4. Meanwhile, when it is determined in STEP S109 that the stable condition is not satisfied, the computation/control section 7 returns to STEP S103 and repeats the above processes.

Subsequent to STEP S110, the computation/control section 7 performs an abnormal value determination process by drinking and eating and retention of urine in bladder in STEP S111 and informs completion of measurements by means of an alarming buzzer 15 (refer to Fig. 2) in STEP S112, thereby completing measurements. The abnormal value determination process in STEP S111 is as shown in the subroutine flowchart of Fig. 68.

<Measurements of Visceral Fat Tissues and the like Using Aponeurosis>

Finally, operations for measuring visceral fat tissues and the like by use of aponeurosis will be described. The operations are nearly the same as those described in Figs. 69 to 72. Therefore, only differences therebetween will be described hereinafter.

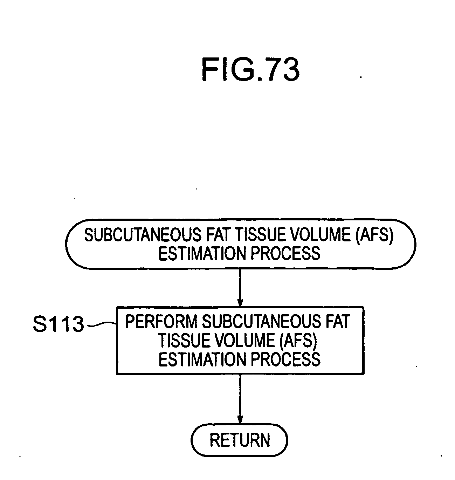

Firstly, in this example, the subcutaneous fat tissue volume (AFS) estimation process in STEP S89 is carried out in accordance with the subroutine flowchart shown in Fig. 73. As shown in Fig. 73, this estimation process is carried out by use of the numerical values stored in the storage section 4 and the above expression 22-1 or 22-2.

Further, in this example, the measurement process in STEP S105, i.e. the process associated with the subcutaneous fat tissue layer impedance, is not carried out, and in relation to this, the processes associated with the subcutaneous fat tissue layer impedance in STEPS S103, S107, S109 and S110 are not carried out.

Otherwise, this example may be considered to be the same as that described in Figs. 69 and 72.

Example According to the Second Characteristic of the Invention

Next, an example according to the second characteristic of the truncal visceral/subcutaneous fat measuring method and apparatus of the present invention for measuring the visceral fat tissues and/or subcutaneous fat tissues of the trunk based on the above measurement principle of the present invention will be described.

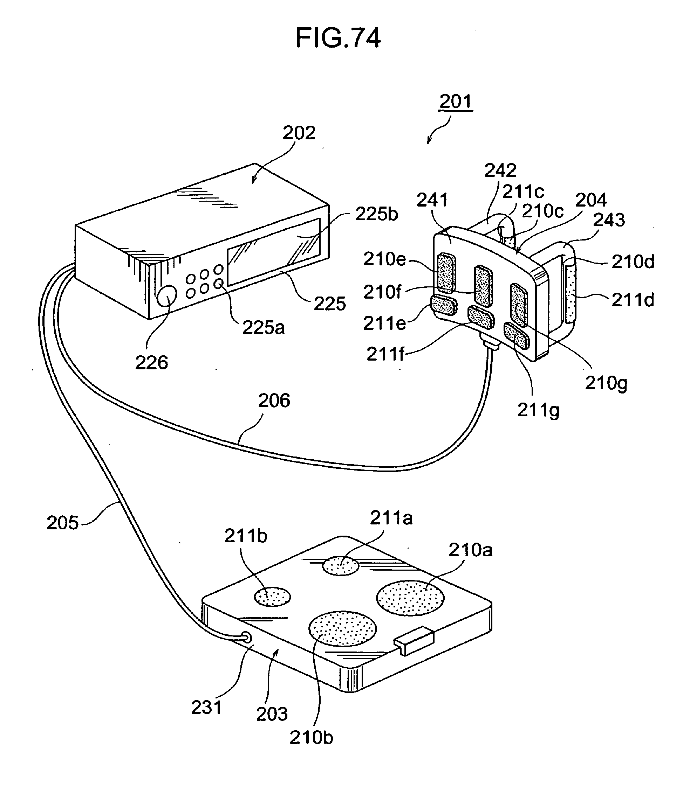

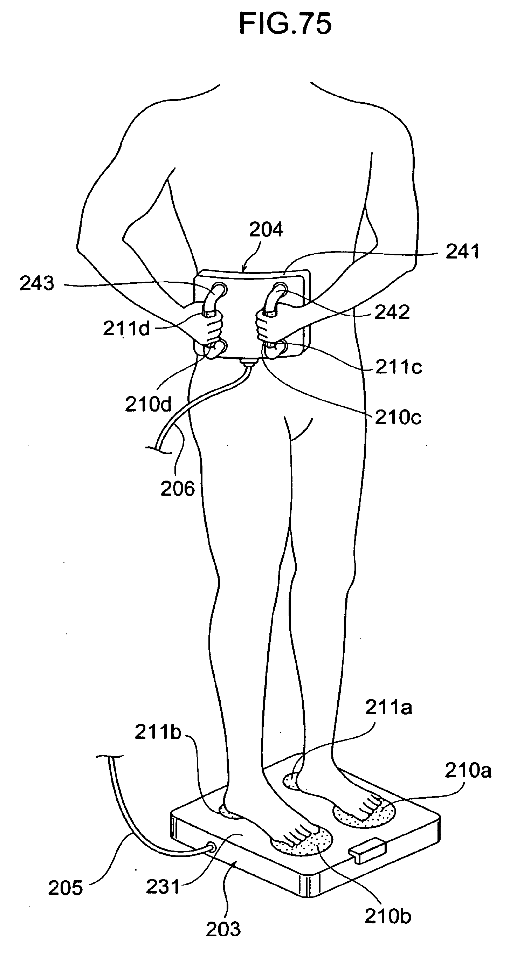

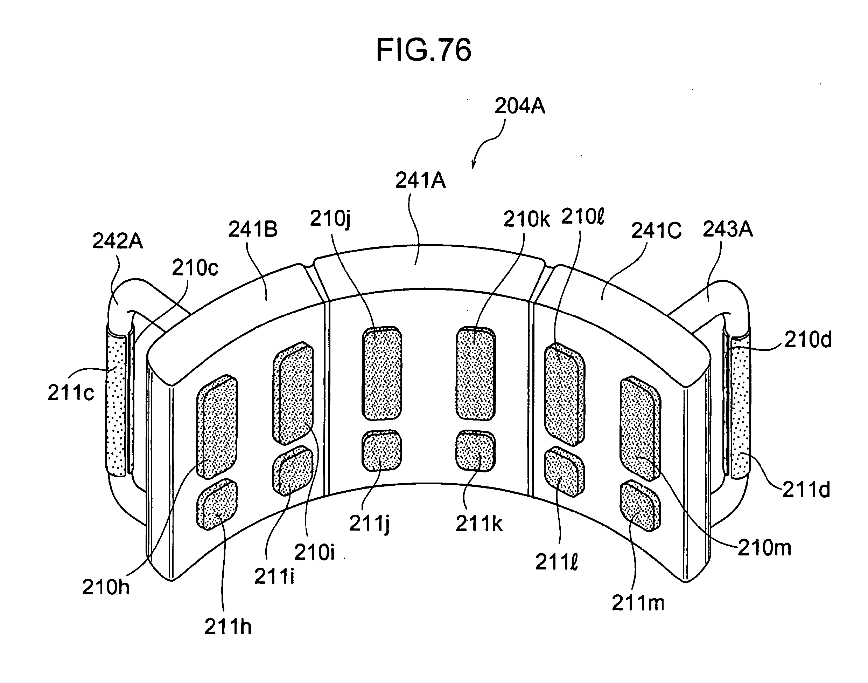

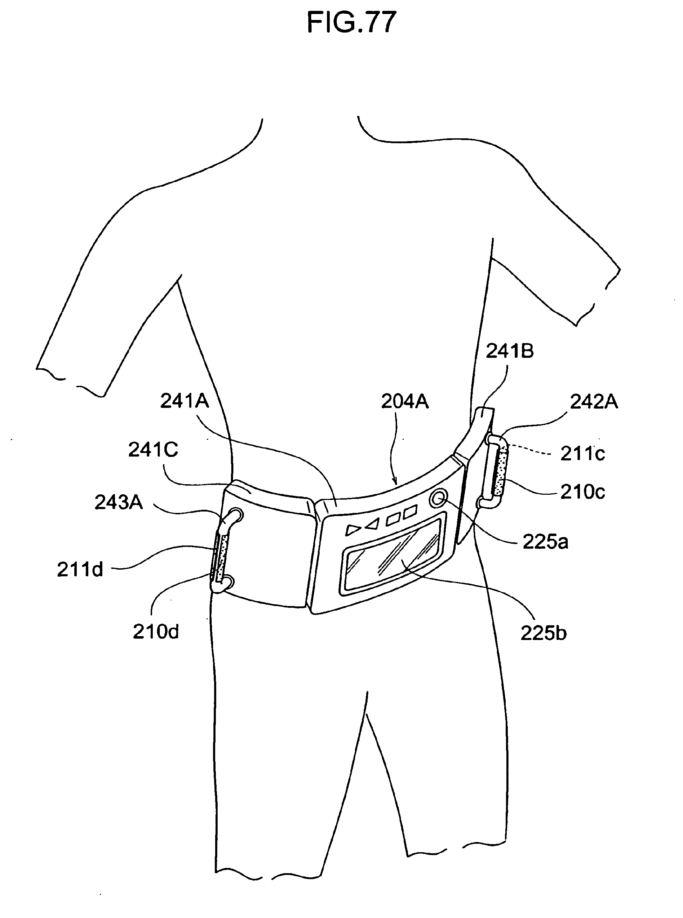

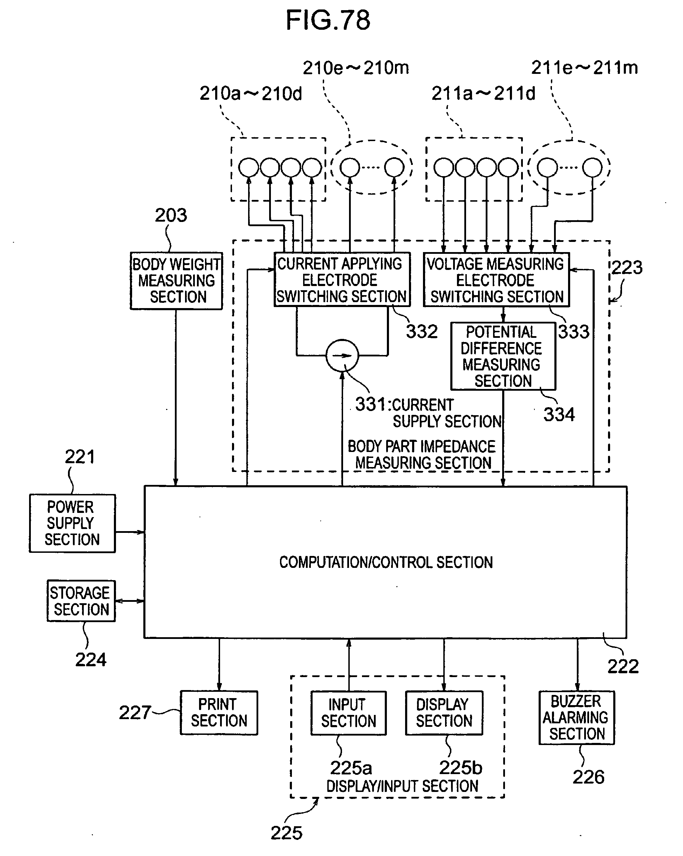

Fig. 74 is a schematic perspective view of the appearance of one example of the truncal visceral/subcutaneous fat measuring apparatus according to the present invention. Fig. 75 is a schematic diagram for illustrating a use form of the apparatus of Fig. 74 when the visceral fat tissues and subcutaneous fat tissue layer of the trunk are measured by use of the apparatus. Fig. 76 is a schematic diagram showing an abdomen contacting electrode section as another embodiment which can substitute an abdomen contacting electrode section in the apparatus of the example of Fig. 74. Fig. 77 is a schematic diagram for illustrating a use form of the abdomen contacting electrode section of Fig. 76. Fig. 78 is a block diagram illustrating the constitution of the apparatus (including a case where the abdomen contacting electrode section of Fig. 76 is used) of Fig. 74.

As shown in these Figs. 74 to 78, a truncal visceral/subcutaneous fat measuring apparatus 201 of the present example primarily comprises a main unit 202, a body weight measuring section 203, and an abdomen contacting electrode section 204 or 204A. The body weight measuring section 203 is connected to the main unit 202 via an electric cable 205, and the abdomen contacting electrode section 204 or 204A is connected to the main unit 202 via an electric cable 206. As clearly shown in Fig. 78, the main unit 202 primarily comprises a power supply section 221, a computation/control section 222, a body part impedance measuring section 223, a storage section 224, a display/input section 225, a buzzer alarming section 226 and a print section 227.

The body part impedance measuring section 223 primarily comprises a current supply section 331, a current applying electrode switching section 332, a voltage measuring electrode switching section 333 and a voltage measuring section 334. As clearly shown in Fig. 74, the display/input section 225 provides an input section 225a comprising various operation keys and a display section 225b comprising a liquid crystal panel on the front surface of the main unit 202. Further, the buzzer alarming section 226 is also provided on the front side of the main unit 202. The power supply section 221 supplies electric power to the sections in the electrical system of the present apparatus.

As shown in Fig. 74, the body weight measuring section 203 comprises a platform 213 which incorporates a weight detecting section, an amplifying section and an AD converting section as in the case of a known scale and measures a voltage based on body weight specifying information (body weight). Further, on the top surface of the platform 231, a left-foot current applying electrode 210a, a right-foot current applying electrode 210b, a left-foot voltage measuring electrode 211a and a right-foot voltage measuring electrode 211b are provided.

As clearly shown in Fig. 74, the abdomen contacting electrode section 204 comprises an abdomen contacting plate 241 and a left-hand grip 242 and a right-hand grip 243 which are provided on the left and right sides on the front surface of the plate 241. On the rear surface of the abdomen contacting plate 241, there are provided three current applying electrodes 210e, 210f and 210g and three voltage measuring electrodes 211e, 211f and 211g in the vicinity of the above current applying electrodes. Further, the left-hand grip 242 has a left-hand current applying electrode 210c and a left-hand voltage measuring electrode 211c, and the right-hand grip 243 has a right-hand current applying electrode 210d and a right-hand voltage measuring electrode 211d. Each of the left-hand grip 242 and the right-hand grip 243 may have a single electrode which serves as a current applying electrode and a voltage measuring electrode.

As clearly shown in Fig. 76, the abdomen contacting electrode section 204A comprises an abdomen contacting center plate 241A, an abdomen contacting left plate 241B, and an abdomen contacting right plate 241C. The abdomen contacting left plate 241B and the abdomen contacting right plate 241C are hinge-connected to the side edges of the abdomen contacting center plate 241A via appropriate flexible joints. Thereby, the abdomen contacting center plate 241A, the abdomen contacting left plate 241B and the abdomen contacting right plate 241C are closely attached to the abdomen, whereby electrodes provided on the plates can make close contact with the surface of the abdomen. In the vicinity of the left-side edge on the front surface of the abdomen contacting left plate 241B is provided a left-hand grip 242A. In the vicinity of the right-side edge on the front surface of the abdomen contacting right plate 241C is provided a right-hand grip 243A.

On the rear surface of the abdomen contacting left plate 241B, there are provided two current applying electrodes 210h and 201i and two voltage measuring electrodes 211h and 211i in the vicinity of the above current applying electrodes, for example. Further, on the rear surface of the abdomen contacting center plate 241A, there are provided two current applying electrodes 210j and 201k and two voltage measuring electrodes 211j and 211k in the vicinity of the above current applying electrodes, for example. In addition, on the rear surface of the abdomen contacting right plate 241C, there are provided two current applying electrodes 210l and 201m and two voltage measuring electrodes 2111 and 211m in the vicinity of the above current applying electrodes, for example. The left-hand grip 242A has a left-hand current applying electrode 210c and a left-hand voltage measuring electrode 211c, and the right-hand grip 243A has a right-hand current applying electrode 210d and a right-hand voltage measuring electrode 211d. Each of the left-hand grip 242A and the right-hand grip 243A may have a single electrode which serves as a current applying electrode and a voltage measuring electrode.

As clearly shown in Fig. 77, on the front surface of the abdomen contacting center plate 241A, an input section 225a comprising various operation keys and a display section 225b are provided. In this case, it is also possible to use the abdomen contacting center plate 241A as a main unit in place of the main unit 202 which is provided in the example of Fig. 74. Further, the above input section and display section may be provided on the rear surface in place of the front surface. In addition, the present apparatus may be constituted by the constituents shown in Fig. 77 without the platform 231.

As described above, the numbers, positional relationships and shapes of the current applying electrodes and voltage measuring electrodes disposed on the rear surface of each plate are not limited to those of the examples shown in the above drawings, and it is important to select optimum numbers, positional relationships and shapes to obtain the following information easily in the following measurement of subcutaneous fat tissue thickness.

(a) A subcutaneous fat tissue thickness measurement body part uses information of any or all of a region adjacent to the navel on the navel circumference, a region under the shoulder blade and the upper border of the iliac crest (lateral region) as a body part with the greatest deposition of subcutaneous fat tissues.

(b) The subcutaneous fat tissue thickness measurement body part uses information of any or all of the navel concave portion on the navel circumference, the backbone and the aponeurosis (joined tendon between the rectus abdominis muscle and the external abdominal oblique muscle) as a body part with the smallest deposition of subcutaneous fat tissues and, particularly, uses information of the aponeurosis (joined tendon between the rectus abdominis muscle and the external abdominal oblique muscle) which varies significantly between individuals.

(c) The subcutaneous fat tissue thickness measurement body part uses information of a combination of the candidate body part with the greatest deposition of subcutaneous fat tissues and the body part with the smallest deposition of subcutaneous fat tissue.

(d) Subcutaneous fat tissue distribution is axisymmetrical at the straight light connecting the navel with the backbone in a cross-sectional area on the navel circumference. Thus, it is sufficient to measure subcutaneous fat tissue distribution information on either the left or right side.

(e) Association of distribution between body parts of subcutaneous fat tissue thickness matches nicely between the region adjacent to the navel and the region under the shoulder blade and can therefore be substituted by either information.

(f) Therefore, highly reliable estimation can be made at three points of the front abdominal region formed by the region adjacent to the navel, the aponeurosis and the upper border of the iliac crest (lateral region).

(g) This subcutaneous fat tissue thickness measurement uses a potential difference right underneath current applying electrodes placed on the abdomen of the trunk or impedance information.

The computation/control section 222 performs various inputs and outputs, measurements, computations and the like, such as computations of body weight specifying information (body weight), impedances of various body parts (such as an upper-extremity impedance, a lower-extremity impedance and a trunk impedance), mid-trunk skeletal muscle tissue volume, lower-extremity skeletal muscle tissue volume, upper-extremity skeletal muscle tissue volume, splanchnic organ tissue volume, subcutaneous fat tissue volume, visceral fat tissue volume, trunk abdominal fat tissue volume, abdominal fat tissue volume and trunk abdominal visceral fat/subcutaneous fat ratio and the like based on the expressions 1 to 54 and the like, a process of removing the influence of change by breathing, a process of determining abnormality in splanchnic organ tissues, and the like.

The body part impedance measuring section 223 comprises the current supply section 331, the current applying electrode switching section 332, the voltage measuring electrode switching section 333 and the voltage measuring section 334, as in the case of a known bioelectrical impedance measuring apparatus (such as a body fat meter or body composition meter). The computation/control section 222 switches and uses the above current applying electrodes 210a to 210m and voltage measuring electrodes 211a to 211m appropriately through the current applying electrode switching section 332 and the voltage measuring electrode switching section 333, whereby potential differences based on bioelectrical impedances between various body parts (various body part impedances) can be measured in the voltage measuring section 334.

The storage section 224 stores body specifying information such as a body height, extremity lengths, a trunk length and a mid-trunk length and the expressions 1 to 54. Further, the storage section 224 stores appropriate messages for health guidance advice which will be described later.

The display/input section 225 comprises a touch-panel type liquid crystal display resulting from integration of the input section 225a and the display section 225b. A user inputs body specifying information including a body height through the display/input section 225, and the display/input section 225 also displays various results, advice information and other data. The buzzer alarming section 226 generates buzzer sounds according to measurement results. The print section 227 prints various results, advice information and other data displayed in the display section 225b.

The apparatus according to the present invention is not limited to the exemplary constitution shown in Fig. 74 or 76 and may take various constitutions and shapes. For example, in the example of Fig. 74, although the main unit 202 and the body weight measuring section 203 are constituted as separate units, the main unit 202 and the body weight measuring section 203 may be constituted as one unit.

Next, use forms of the examples shown in Figs. 74 and 76 will be described with reference to Figs. 75 and 77 in particular. First, the use form of the example shown in Fig. 74 will be described. As shown in Fig. 75, a user who intends to measure body fat tissues holds the left-hand grip 242 and right-hand grip 243 of the abdomen contacting electrode section 204 in the left and right hands, stands on the platform 231 of the body weight measuring section 203, and presses the rear surface of the abdomen contacting plate 241 of the abdomen contacting electrode section 204 against a predetermined position on the abdomen. Before or after standing on the platform 231, the user can input predetermined body specifying information or perform operations according to various instructions displayed in the display section 225b by operating the input section 225a of the main unit 202.

In this state, the left and right hands of the user can contact the left-hand current applying electrode 210c and the left-hand voltage measuring electrode 211c and the right-hand current applying electrode 210d and the right-hand voltage measuring electrode 211d, respectively. Further, the bottoms of the left and right feet of the user can contact the left-foot current applying electrode 210a and the left-foot voltage measuring electrode 211a and the right-foot current applying electrode 210b and the right-foot voltage measuring electrode 211b, respectively. In addition, a predetermined portion on the abdomen of the user can contact predetermined current applying electrodes out of the current applying electrodes 210e to 210g provided in the abdomen contacting electrode section 204 and predetermined voltage measuring electrodes out of the voltage measuring electrodes 211e to 211g provided in the abdomen contacting electrode section 204.

Similarly, when the abdomen contacting electrode section 204A of the example of Fig. 76 is used, a user who intends to measure body fat tissues holds the left-hand grip 242A and right-hand grip 243A of the abdomen contacting electrode section 204A in the left and right hands, stands on the platform 231 of the body weight measuring section 203, and presses the rear surfaces of the abdomen contacting center plate 241A, abdomen contacting left plate 241B and abdomen contacting right plate 241C of the abdomen contacting electrode section 204A against a predetermined position on the abdomen. In this state, the left and right hands of the user can contact the left-hand current applying electrode 210c and the left-hand voltage measuring electrode 211c and the right-hand current applying electrode 210d and the right-hand voltage measuring electrode 211d, respectively. Further, the bottoms of the left and right feet of the user can contact the left-foot current applying electrode 210a and the left-foot voltage measuring electrode 211a and the right-foot current applying electrode 210b and the right-foot voltage measuring electrode 211b, respectively. In addition, a predetermined portion on the abdomen of the user can contact predetermined current applying electrodes out of the current applying electrodes 210h to 210m provided in the abdomen contacting electrode section 204A and predetermined voltage measuring electrodes out of the voltage measuring electrodes 211h to 211m provided in the abdomen contacting electrode section 204A.

These current applying electrodes 210a to 210m and voltage measuring electrodes 211a to 211m may be implemented by metal-plating the surfaces of an SUS material and a resin material, for example. In the electrodes of this type, the surfaces of the metal electrodes are coated with a water-retentive polymer film, so that the electrodes are used in measurement after sprayed or wetted with water. By being wetted with water, the electrodes can secure stability in electrical contact with the skin. Further, although not particularly shown, electrodes of stickable type can also be used. These secure stability in contact with the skin by attaching a replaceable adhesive pad to the base electrode surfaces of the electrodes. The electrodes of this type are commonly used in low-frequency therapy equipment or as electrodes for electrocardiograms, for example. They are classified into a disposable form which is removed and disposed after measurement and a form which is disposed and replaced only when the pad surface has become dirty and lost adhesiveness or water has evaporated and protected by a cover sheet until disposed.

The same descriptions as those given above with respect to Figs. 5 to 14 are also applied to the example according to the second characteristic of the present invention.

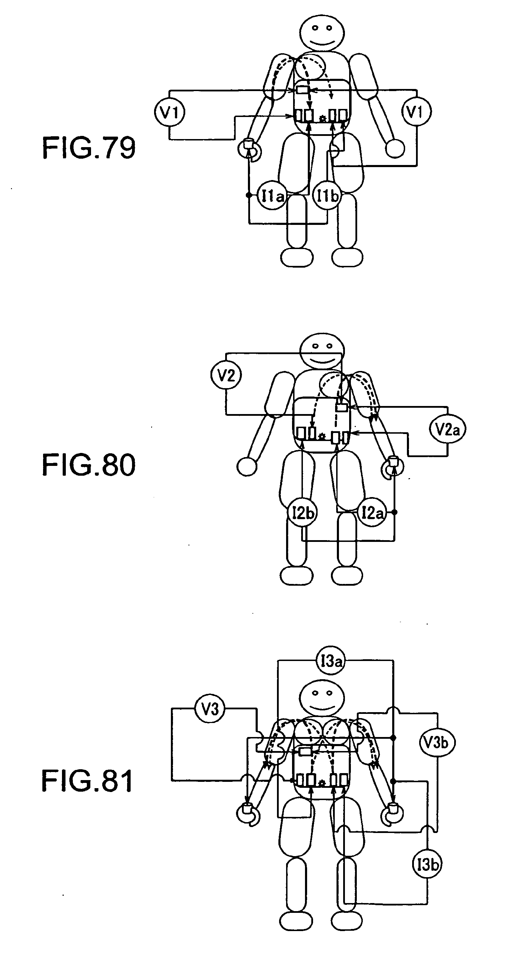

Further, with reference to Figs. 79 to 84, a lead system for measuring fat tissues, particularly, the subcutaneous fat tissue layer, by an electrode arrangement for a combination of the extremities and the trunk will be described. Figs. 79 to 84 illustrate a lead system in which a current applying electrode (grip electrode) is placed on the palm, the other current applying electrode is placed in the abdomen of the trunk (on the aponeurosis) and two pairs of voltage measuring electrodes are placed on the abdomen of the trunk.

Figs. 79 to 81 illustrate a novel lead system in which a current applying electrode is placed on the palm as a grip electrode, the other current applying electrode is placed in the abdomen of the trunk (on the aponeurosis) and two voltage measuring electrodes are placed on the abdomen of the trunk. Fig. 79 relates to right-arm/trunk-abdomen energization measurement, Fig. 80 relates to left-arm/trunk-abdomen energization measurement, and Fig. 81 relates to both-arms/trunk-abdomen energization measurement. In the right-arm/trunk-abdomen energization measurement of Fig. 79, one of the voltage measuring electrodes is placed in the vicinity of the current applying electrode placed in the vicinity of the navel circumference of the abdomen of the trunk (advantageous when placed on the skeletal muscle tissue layer), and the other voltage measuring electrode may be placed at any position at which an equivalent potential can be measured as long as it can secure such a distance that the influence of spreading resistance can be avoided from the current applying electrode on the navel circumference. This concept can also be applied to electrode arrangements of novel lead systems illustrated in Fig. 80 and subsequent drawings.

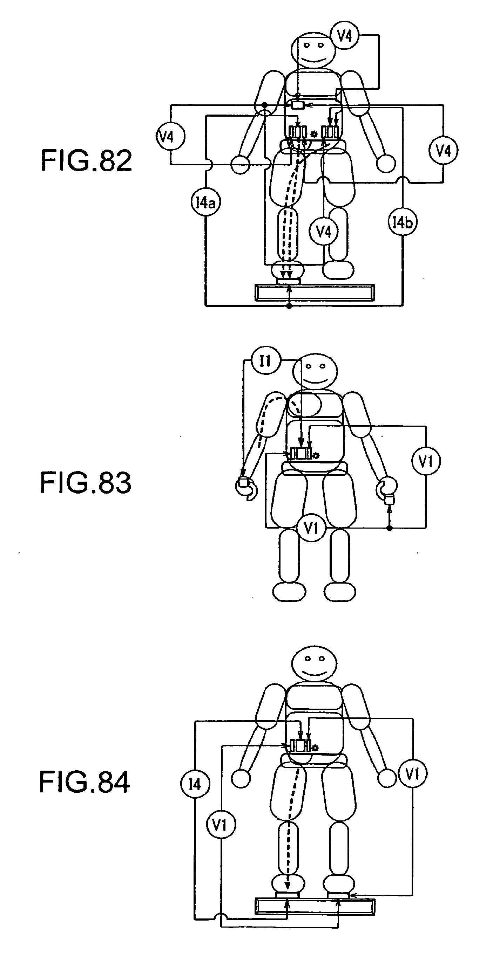

Fig. 82 illustrates an electrode arrangement for right-leg/trunk-abdomen energization measurement in a novel lead system in which one current applying electrode is placed on the bottom of a foot as a foot electrode, the other current applying electrode is placed in the abdomen of the trunk (on the aponeurosis) and two voltage measuring electrodes are placed on the abdomen of the trunk. Further, although not shown, there are also electrode arrangements for left-leg/trunk-abdomen energization measurement and both-legs/trunk-abdomen energization measurement to which the same concept as that of the electrode arrangement illustrated in Fig. 82 is applied. Further, although not shown, there is also a novel lead system according to the same concept in which one current applying electrode is placed on an ear of the head (as a clip electrode which clips an earlobe or the like) and the other current applying electrode is placed in the abdomen of the trunk (on the aponeurosis). In this novel lead system, right-ear/trunk-abdomen energization measurement, left-ear/trunk-abdomen energization measurement, and right-ear/trunk-abdomen energization measurement by a lead system of Organ et al. are conceivable.

Fig. 83 illustrates an electrode arrangement for right-arm/trunk-abdomen energization measurement by the lead system of Organ et al. out of novel lead systems in which one current applying electrode is placed on a palm, the other current applying electrode is placed in the abdomen of the trunk (on the aponeurosis), one voltage measuring electrode is placed on the other palm as a grip electrode and the other voltage measuring electrode is placed on the abdomen of the trunk. In this novel lead system as well, although not shown, left-arm/trunk-abdomen energization measurement and the like according to the lead system of Organ et al. according to the same concept are conceivable.

Fig. 84 illustrates an electrode arrangement for right-leg/trunk-abdomen energization measurement by the lead system of Organ et al. out of novel lead systems in which one current applying electrode is placed on the bottom of a foot as a foot electrode, the other current applying electrode is placed in the abdomen of the trunk (on the aponeurosis), one voltage measuring electrode is placed on the bottom of the other foot as a foot electrode and the other voltage measuring electrode is placed on the abdomen of the trunk. In this novel lead system as well, although not shown, left-leg/trunk-abdomen energization measurement and the like according to the lead system of Organ et al. according to the same concept are conceivable.

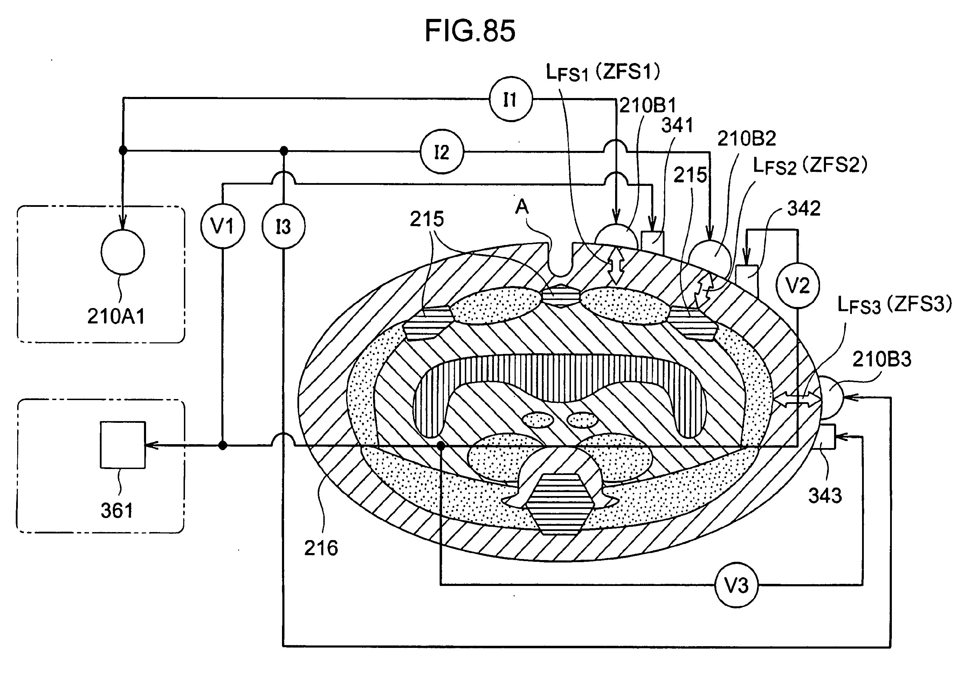

Fig. 85 is a schematic diagram showing an electrode arrangement example for an optimum subcutaneous fat tissue measuring body part for obtaining subcutaneous fat tissue layer information by the present invention by use of such a novel lead system as described with reference to Figs. 79 to 84. The electrode arrangement example of Fig. 85 is an electrode arrangement example for measuring subcutaneous fat tissue layer thickness in the optimum three-point body part described in (f) out of the above (a) to (g), i.e., "three points of the front abdominal region formed by the region adjacent to the navel, the aponeurosis and the upper border of the iliac crest (lateral region)" as a subcutaneous fat tissue thickness measuring body part in the present invention. In the electrode arrangement shown in Fig. 85, a current applying electrode 210B1 is placed adjacent to the navel A of the abdomen 216, another current applying electrode 210B2 is placed on an aponeurosis 215, another current applying electrode 210B3 is placed in a lateral region, and the other current applying electrode 210A1 is placed on any of the four extremities. Meanwhile, voltage measuring electrodes 341, 342 and 343 are placed at positions where the influence of spreading resistance right underneath the current applying electrode is predominant, i.e. in the vicinity of the current applying electrodes 210B1, 210B2 and 210B3, as described above with reference to Fig. 15. On the other hand, the other voltage measuring electrode 361 is placed on any of the four extremities (position different from the position whether the extremity current applying electrode is placed). The measured value of potential difference V1 which occurs between the voltage measuring electrodes 361 and 341 by a current I1 applied by the current applying electrodes 210A1 and 210B1 is proportional to the impedance ZFS1 value of the subcutaneous fat tissue layer adjacent to the navel and to the thickness LFS1 of the subcutaneous fat tissue layer adjacent to the navel. Similarly, the measured value of potential difference V2 which occurs between the voltage measuring electrodes 361 and 342 by a current I2 applied by the current applying electrodes 210A1 and 210B2 is proportional to the impedance ZFS2 of the subcutaneous fat tissue layer in the aponeurosis and to the thickness LFS2 of the subcutaneous fat tissue layer adjacent in the aponeurosis. Similarly, the measured value of potential difference V3 which occurs between the voltage measuring electrodes 361 and 343 by a current I3 applied by the current applying electrodes 210A1 and 210B3 is proportional to the impedance ZFS3 of the subcutaneous fat tissue layer in the lateral region and to the thickness LFS3 of the subcutaneous fat tissue layer adjacent in the aponeurosis.

In the apparatus of the above example of the present invention, as the current applying electrode 210A1 in the electrode arrangement example of Fig. 85, one of the current applying electrodes 210a and 210b provided on the top surface of the platform 231 of the body weight measuring section 203 and the current applying electrodes 210c and 210d provided in the left-hand grip 242 and right-hand grip 243 of the abdomen contacting electrode section 204 is switched and selected by the current applying electrode switching section 332 under control of the computation/control section 222 and used. Similarly, as the current applying electrode 210B1, 210B2 and 210B3 in the electrode arrangement example of Fig. 85, ones out of the current applying electrodes 210e to 210m provided in the abdomen contacting electrode sections 204 and 204A are switched and selected by the current applying electrode switching section 332 under control of the computation/control section 222 and used. In this case, depending on a specific form of the apparatus of the present invention, a user must change a position on the abdomen against which the abdomen contacting electrode section 204 or 204A is pressed in accordance with an instruction displayed in the display section 225b of the main unit 202 so that predetermined current applying electrodes are pressed against and contacted with the optimum position on the abdomen. As the voltage measuring electrode 361 in the electrode arrangement example of Fig. 85, one of the voltage measuring electrodes 211a and 211b provided on the top surface of the platform 231 of the body weight measuring section 203 and the voltage measuring electrodes 211c and 211d provided in the left-hand grip 242 and right-hand grip 243 of the abdomen contacting electrode section 204 is switched and selected by the voltage measuring electrode switching section 333 under control of the computation/control section 222 and used. Further, as the voltage measuring electrode 341, 342 and 343 in the electrode arrangement example of Fig. 85, ones out of the voltage measuring electrodes 211e to 211m provided in the abdomen contacting electrode sections 204 and 204A are switched and selected by the voltage measuring electrode switching section 333 under control of the computation/control section 222 and used. In this case, depending on a specific form of the apparatus of the present invention, a user must change a position on the abdomen against which the abdomen contacting electrode section 204 or 204A is pressed in accordance with an instruction displayed in the display section 225b of the main unit 202 so that predetermined voltage measuring electrodes are pressed against and contacted with the optimum position on the abdomen.

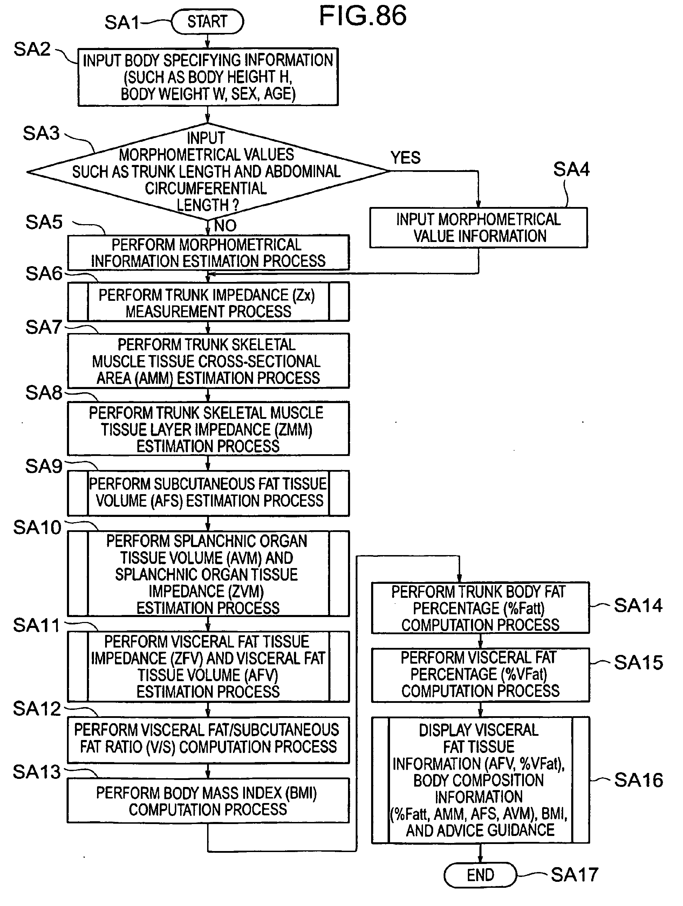

Next, the operations of the truncal visceral/subcutaneous fat measuring apparatus (truncal subcutaneous fat measuring apparatus) of the example of the present invention shown in Figs. 74 to 78 will be described with reference to subroutine flowcharts associated with a basic flowchart shown in Fig. 86.

The basic flowchart shown in Fig. 86 is substantially the same as the basic flowchart shown in Fig. 69 except for step numbers and reference numbers. Therefore, in this basic flowchart as well, the same processes as those described with respect to the basic flowchart of Fig. 69 are conducted.

Further, in STEP SA9, firstly, a subcutaneous fat tissue layer impedance ZFS is calculated based on the following expression in place of the above expression 24.

$$\text{ZFS} = aa1 \times \text{ZFS1} + bb1 \times \text{ZFS2} + cc1 \times \text{ZFS3} + dd1 \quad \text{... expression 49}$$

wherein aa1, bb1, cc1 and dd1 are constants giving different values for a male and a female.

Then, a subcutaneous fat tissue volume (AFS) is calculated based on the above expression 45.

A splanchnic organ tissue volume (AVM) and splanchnic organ tissue impedance (ZVM) estimation process in STEP SA10 is the same as that described with respect to the subroutine flowchart of Fig. 70.

A visceral fat tissue impedance (ZFV) and visceral fat tissue volume (AFV) estimation process in STEP SA11 is the same as that described with respect to the subroutine flowchart of Fig. 71.

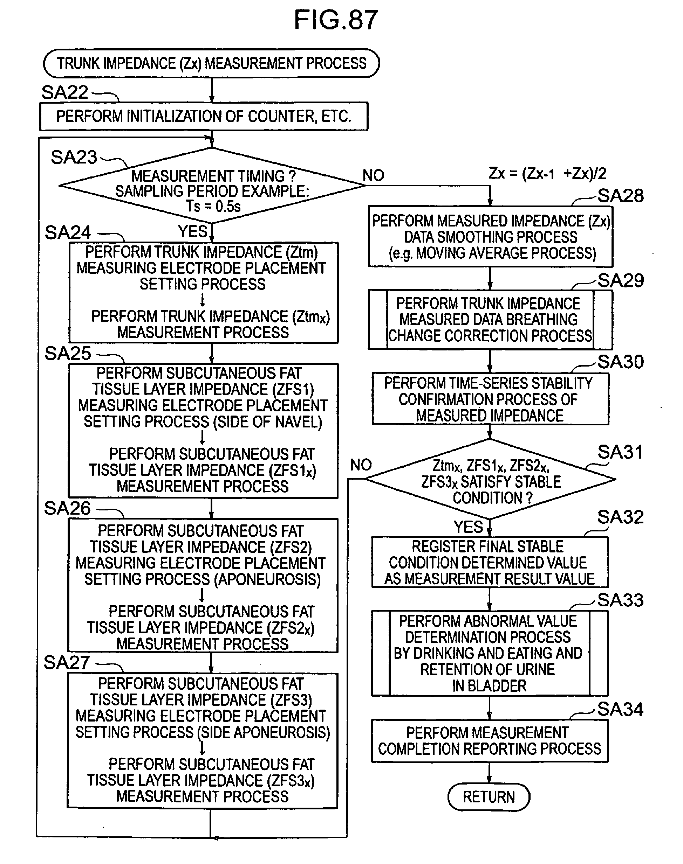

Next, a trunk impedance measurement process in STEP SA6 will be described in detail with reference to a subroutine flowchart of Fig. 87 showing a first embodiment. In this first embodiment, "process of removing influence of change by breathing" and "process of determining abnormal value by drinking and eating and retention of water (e.g. urine) in bladder or the like" as described in (28) and (29) in the above 10. are conducted. Firstly, in STEP SA23, the computation/control section 222 initializes a counter and the number of samples for measurement data of the impedance Ztm of the trunk based on an instruction from the input section 225a.

Then, in STEP SA23, the computation/control section 222 determines whether it is measurement timing. When it has been determined that it is measurement timing, the computation/control section 222 performs a trunk impedance (Ztm) measuring electrode placement setting process and a trunk impedance (Ztmx) measurement process in STEP SA24. Further, in STEP SA25, the computation/control section 222 performs a subcutaneous fat tissue layer impedance (ZFS1) measuring electrode placement setting process and a subcutaneous fat tissue layer impedance (ZFS1x) measurement process (side of the navel). Further, in STEP SA26, the computation/control section 222 performs a subcutaneous fat tissue layer impedance (ZFS2) measuring electrode placement setting process and a subcutaneous fat tissue layer impedance (ZFS2x) measurement process (aponeurosis). Further, in STEP SA27, the computation/control section 222 performs a subcutaneous fat tissue layer impedance (ZFS3) measuring electrode placement setting process and a subcutaneous fat tissue layer impedance ($ZFS3_x$) measurement process (side aponeurosis). Then, the computation/control section 222 returns to STEP SA23.

Meanwhile, when it has been determined in STEP SA23 that it is not measurement timing, the computation/control section 222 proceeds to STEP SA28 and performs a measured impedance (Zx) data smoothing process (e.g. a moving average process), i.e. $Z_x = (Z_{x-1} + Z_x)/2$, on the trunk impedance ($Ztm_x$) and the subcutaneous fat tissue layer impedances (ZFS1x, ZFS2x, ZFS3x). Then, in STEP SA29, the computation/control section 222 performs a trunk impedance measured data breathing change correction process. This correction process will be described later. Since the subcutaneous fat tissue layer impedances (ZFS1x, ZFS2x, ZFS3x) are hardly influenced by a change in breathing, they are not subjected to the correction process, unlike the trunk impedance.

Then, in STEP SA30, the computation/control section 222 performs a time-series stability confirmation process of measured impedance of each body part. This is carried out by determining whether each value after the trunk impedance measured data breathing change correction process in STEP SA29 has converged to a value within a predetermined change in a predetermined number of times. In STEP SA31, the computation/control section 222 determines whether the measured Ztmx, ZFS1x, ZFS2x and ZFS3x satisfy a stable condition. This determination is made such that a median breathing value is determined at the point when a median breathing value in each breathing cycle enters a stable range within a predetermined number of times. When it is determined in this STEP SA31 that the stable condition is satisfied, the computation/control section 222 proceeds to STEP SA32 and registers the impedance value of the determined median value as the impedance value of the trunk or the subcutaneous fat tissue layer impedance value and a final stable condition determined value as a measurement result value in the storage section 224. That is, the computation/control section 222 registers Ztmx, ZFS1x, ZFS2x and ZFS3x which satisfy the stable condition as Ztm, ZFS1, ZFS2 and ZFS3, respectively. Meanwhile, when it is determined in STEP SA31 that the stable condition is not satisfied, the computation/control section 222 returns to STEP SA23 and repeats the above processes.

Subsequent to STEP SA32, the computation/control section 222 performs an abnormal value determination process by drinking and eating and retention of urine in bladder in STEP SA33 and informs completion of measurements by means of an alarming buzzer 226 (refer to Fig. 78) in STEP SA34, thereby completing measurements.

The trunk impedance measured data breathing change correction process in STEP SA29 is the same as that described with respect to the subroutine flowchart of Fig. 65.

The abnormal value determination process by drinking and eating and retention of urine in bladder in STEP SA33 is the same as that described with respect to the subroutine flowchart of Fig. 66.

Example According to the Third Characteristic of the Invention

Next, an example according to the third characteristic of the truncal visceral/subcutaneous fat measuring method and apparatus of the present invention based on the above measurement principle of the present invention will be described.

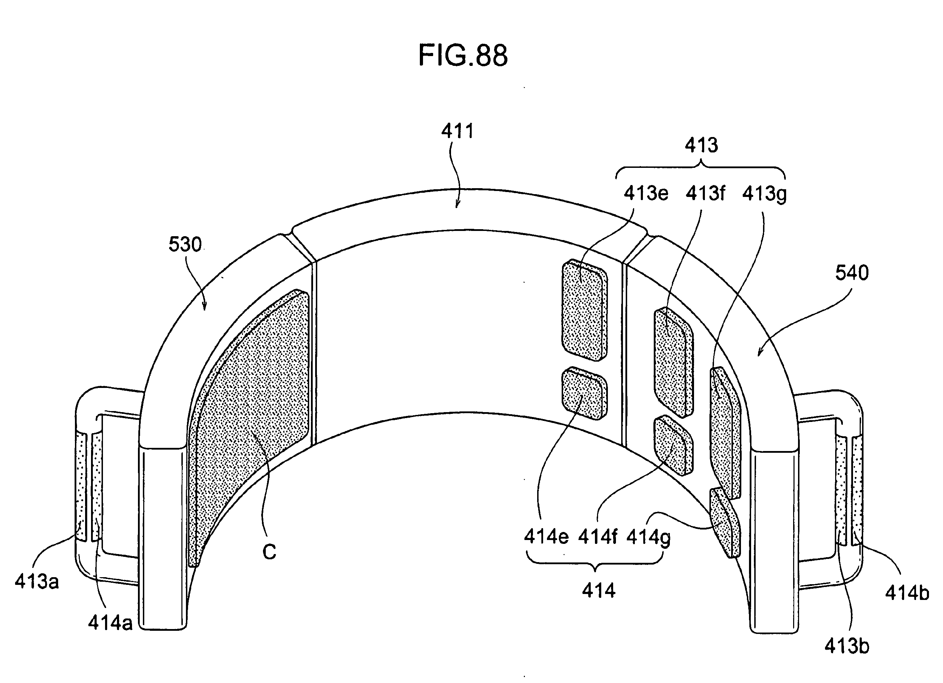

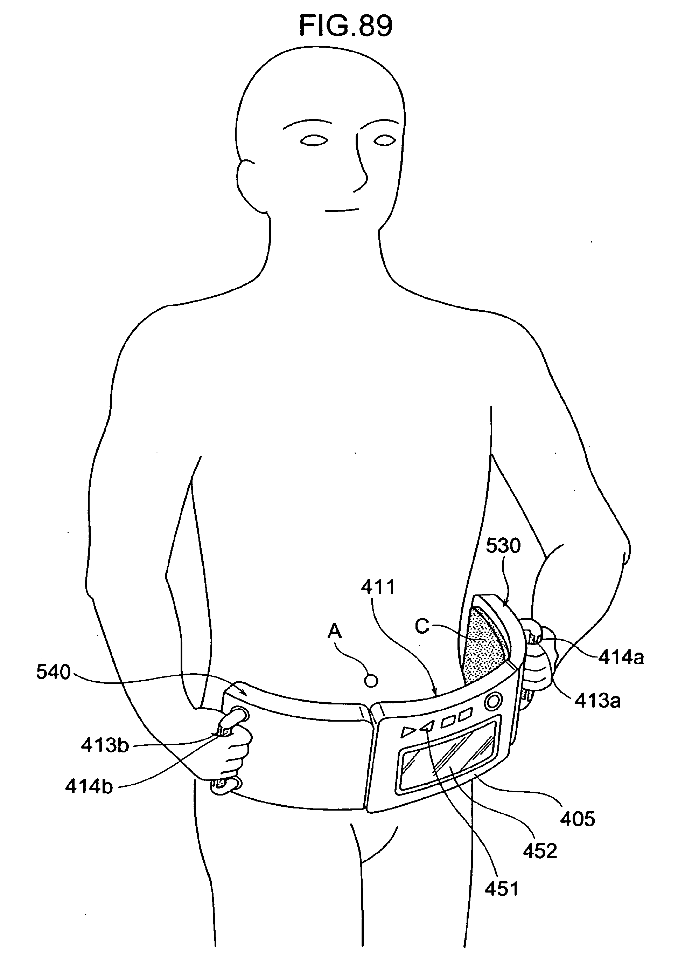

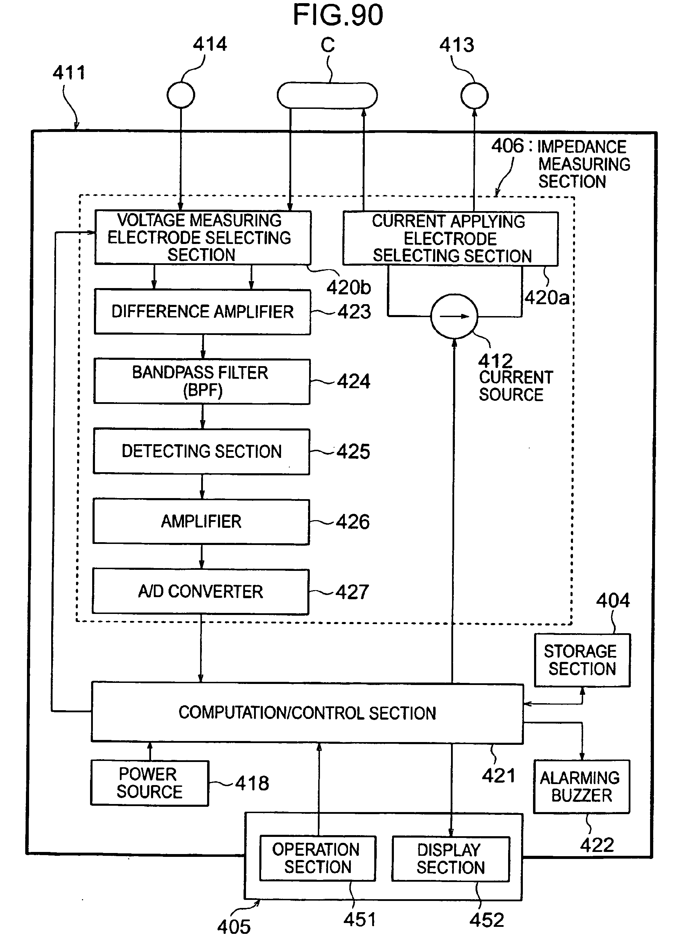

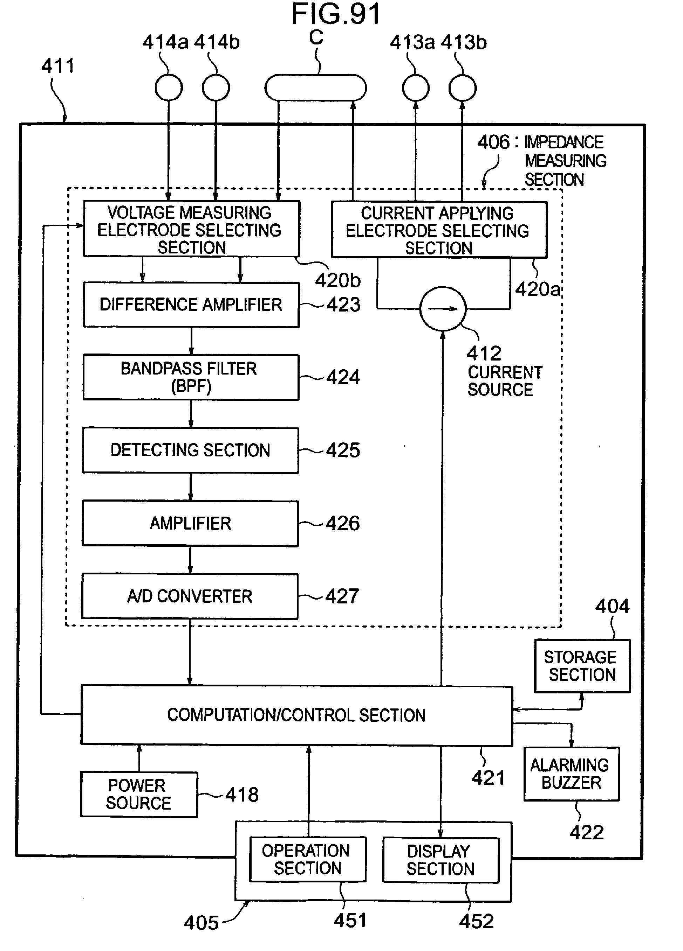

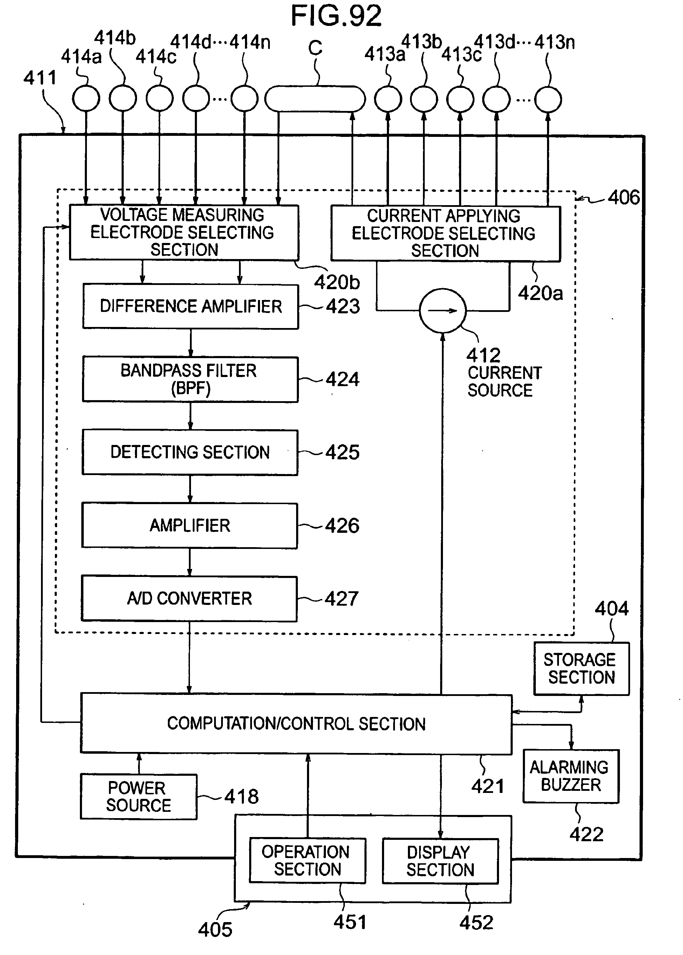

Fig. 88 is a schematic perspective view of the appearance of a first example of the truncal visceral/subcutaneous fat measuring apparatus according to the present invention. Fig. 89 is a schematic diagram for illustrating a use form of the truncal visceral/subcutaneous fat measuring apparatus of Fig. 88. Figs. 90 to 92 are block diagrams showing the main constitution of main unit 411 of Fig. 88 and electrode configurations in the present invention. As shown in Fig. 90, the present invention uses a three-electrode technique involving a shared electrode C, a current applying electrode 413 and a voltage measuring electrode 414. However, as will be described later, such electrode configurations as shown in Figs. 91 and 92 can also be used by providing a current applying electrode selecting section 420a and a voltage measuring electrode selecting section 420b.

In the electrode configuration of Fig. 91, two current applying electrodes 413a and 413b and two voltage measuring electrodes 414a and 414b are provided. As shown in Fig. 88, these electrodes are used as grip electrodes, for example. Meanwhile, in the electrode configuration of Fig. 92, a plurality of current applying electrodes 413a to 413n and a plurality of voltage measuring electrodes 414a to 414n are provided. These electrodes are used as grip electrodes and foot electrodes placed on the four extremities or electrodes placed on the trunk. According to a use form, an appropriate combination of electrodes are selected from these electrodes.

A truncal visceral/subcutaneous fat measuring apparatus 401 of the present invention comprises a main unit 411, a left-grip section 530 and a right-grip section 540. The grip sections 530 and 540 are connected to the main unit 411 via flexible joints so that they can make close contact with the abdomen of the trunk. To use the grip sections 530 and 540, a subject holds grips in both hands as shown in Fig. 89 and presses the grip sections against a body part to be measured, e.g. the abdomen. The left and right grips have grip electrodes. The left-hand grip electrodes comprise a current applying electrode 413a and a voltage measuring electrode 414a, and the right-hand grip electrodes comprise a current applying electrode 413b and a voltage measuring electrode 414b.

On the front surface of the main unit 411, an operation display panel 405 which has an operation section 451 and a display section 452 and an alarming buzzer 422 are provided. As is clear from Figs. 90 to 92, the main unit 411 incorporates, for example, a computation/control section 421, a power source 418, a storage section 404 and an impedance measuring section 406. As shown in Fig. 88, on the rear surfaces of the grip sections 530 and 540, i.e. the surfaces which are pressed against the abdomen, a shared electrode C, a plurality of current applying electrodes 413e, 413f and 413g for the abdomen of the trunk and a plurality of voltage measuring electrodes 414e, 414f and 414g for the abdomen of the trunk are provided. The current applying electrodes 413 (i.e. 413a to 413n) are electrodes for applying a current to a body part to be measured of a subject, and the voltage measuring electrodes 414 (i.e. 414a to 414n) are electrodes for measuring a potential difference in the body part to be measured of the subject.

In the example shown in Fig. 88, the shared electrode C is provided in the left-grip section 530. However, it may be provided in the right-grip section 540. Further, the current applying electrodes 413e, 413f and 413g and the voltage measuring electrodes 414e, 414f and 414g may be provided in the left-grip section 530 which is opposite to the side shown in Fig. 88, as long as they are provided on the side opposed to the shared electrode C. Further, some of the current applying electrodes and the voltage measuring electrodes which are generally provided in the left and right grip sections may be provided on the rear surface of the main unit, as shown in the drawing. In addition, when the main unit has no electrodes provided on the rear surface thereof, the main unit may be reversible. That is, the display panel 405 may be provided on the rear surface of the main unit other than the front surface thereof.

The operation section 451 can be used to input body specifying information including a body height and a body weight. The operation display panel 405 displays various results, advice information and the like through the display section 452. This operation display panel 405 may be formed as a touch-panel type liquid crystal display resulting from integration of the operation section 451 and the display section 452.

The computation/control section 421 performs various inputs and outputs, measurements, computations and the like, such as computations of trunk skeletal muscle tissue cross-sectional area, trunk skeletal muscle tissue layer impedance, visceral fat tissue impedance, visceral fat tissue volume, splanchnic organ tissue volume, splanchnic organ tissue impedance, subcutaneous fat tissue volume, trunk visceral fat/subcutaneous fat ratio and the like based on body weight specifying information (such as a body height or a body weight) input from the operation section 451, measured impedances and the above expressions, a process of removing the influence of change by breathing, a process of determining abnormality in splanchnic organ tissues, and the like.

The power source 418 supplies electric power to the sections in the electrical system of the present apparatus. The storage section 404 stores not only body specifying information such as a body height, a body weight, a trunk length and an abdominal circumferential length and the above expressions but also such appropriate messages for health guideline advice as will be described later.

The impedance measuring section 406 comprises a current source 412 for supplying a current to the current applying electrodes 413, a current applying electrode selecting section 420a for selecting the current applying electrodes 413, a voltage measuring electrode selecting section 420b for selecting the voltage measuring electrodes 414, a difference amplifier 423 for amplifying a potential difference measured by the voltage measuring electrodes 414, a bandpass filter (BPF) 424 for filtering, a detecting section 425, an amplifier 426, and an A/D converter 427.

The current applying electrodes 413 and the voltage measuring electrodes 414 may be implemented by metal-plating the surfaces of an SUS material and a resin material. In the electrodes of this type, the surfaces of the metal electrodes are coated with a water-retentive polymer film, so that the electrodes are used in measurement after sprayed or wetted with water. By being wetted with water, the electrodes can secure stability in electrical contact with the skin. Further, although not particularly shown, electrodes of stickable type can also be used. These secure stability in contact with the skin by attaching a replaceable adhesive pad to the base electrode surfaces of the electrodes. The electrodes of this type are commonly used in low-frequency therapy equipment or as electrodes for electrocardiograms, for example. They are classified into a disposable form which is removed and disposed after measurement and a form which is disposed and replaced only when the pad surface has become dirty and lost adhesiveness or water has evaporated and protected by a cover sheet until disposed. The electrode sections may adopt a detachable structure for long-time storage, hydration and cleaning of the electrode sections. As the detachable structure, a hook type which is often used for electrodes for electrocardiograms and a connector for a flexible substrate are conceived.

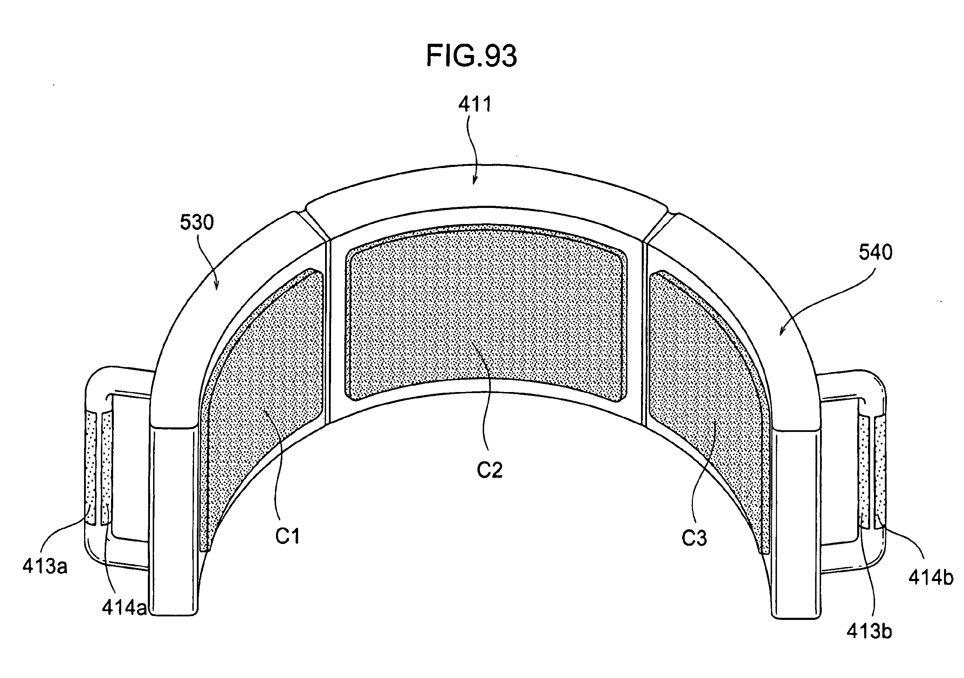

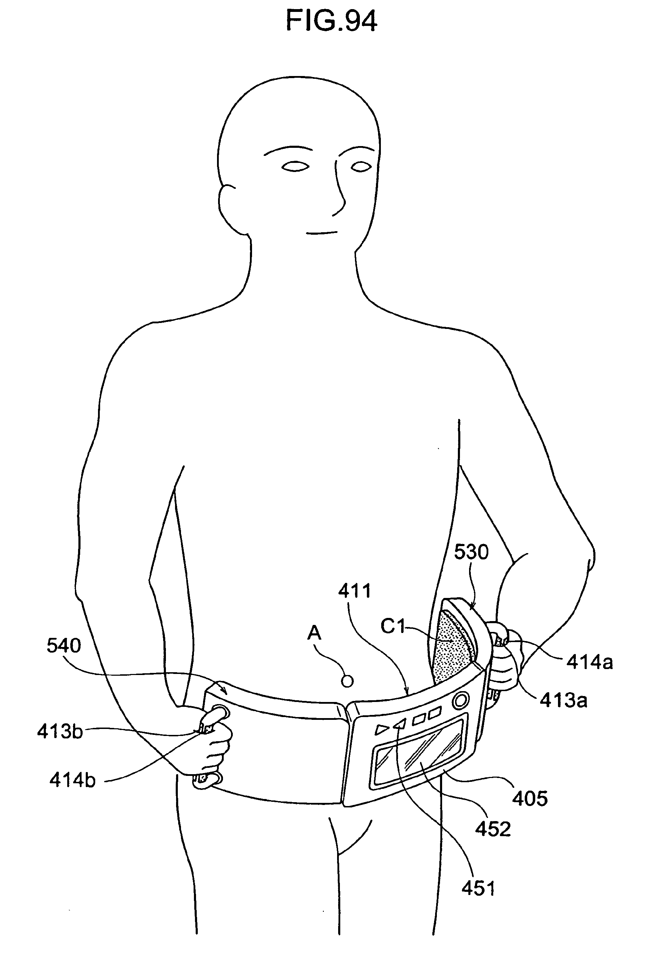

Fig. 93 is a schematic perspective view of the appearance of a second example of the truncal visceral/subcutaneous fat measuring apparatus according to the present invention. Fig. 94 is a schematic diagram for illustrating a use form of the apparatus of Fig. 93. The example of Fig. 93 has the same constitution as that of the example of Fig. 88 except that shared electrodes are provided on the main unit 411 and the left and right grip sections 530 and 540 in place of the electrodes for the abdomen of the trunk. That is, in the example of Fig. 93, the grip section 530, the main unit 411 and the grip section 540 are provided with shared electrodes C1, C2 and C3, respectively. Only any one of these shared electrodes C1, C2 and C3 may be used alone, or these electrodes may be used as one shared electrode by electrically short-circuiting these electrodes.

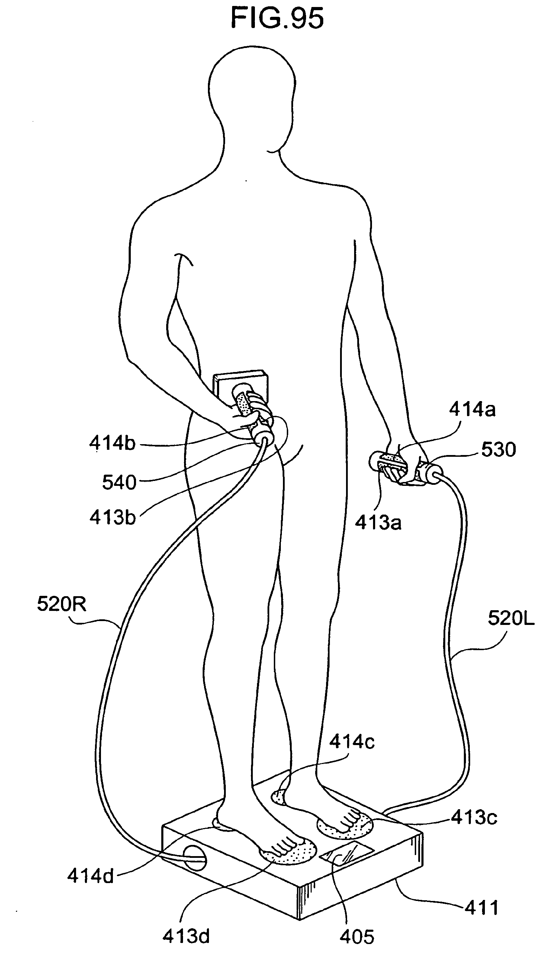

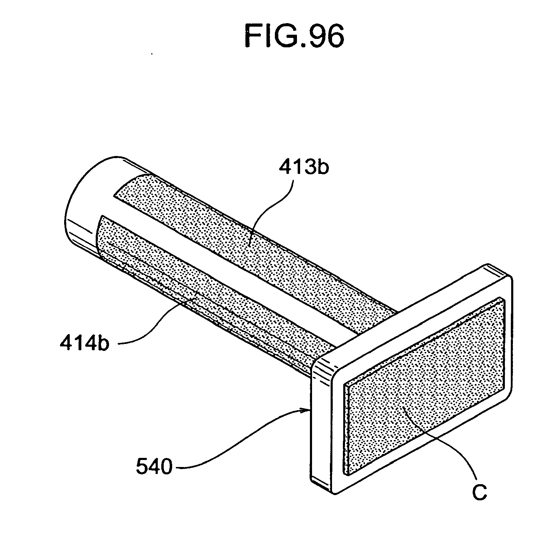

Fig. 95 is a schematic perspective view of the appearance of a third example of the truncal visceral/subcutaneous fat measuring apparatus according to the present invention. Fig. 96 is an enlarged view of a grip section 540 of the apparatus of Fig. 95. The example of Fig. 95 comprises a scale-shaped main unit 411 and grip sections 530 and 540 which can be pulled out of the main unit 411. The grip sections 530 and 540 are connected to the main unit 411 by electric cables 520L and 520R, respectively.

The grip section 530 comprises a current applying electrode 413a and a voltage measuring electrode 414a, and the grip section 540 comprises a current applying electrode 413b and a voltage measuring electrode 414b. Further, a shared electrode C is provided at one end of the grip section 540. As shown in Fig. 95, the shared electrode C is pressed against the abdomen at the time of measurement.

On the top surface of the main unit 411, foot electrodes for the lower extremity are provided. The foot electrodes comprise a current applying electrode 413c and a voltage measuring electrode 414c for the left foot and a current applying electrode 413d and a voltage measuring electrode 414d for the right foot. Further, an operation display panel 405 is also provided on the top surface of the main unit 411.

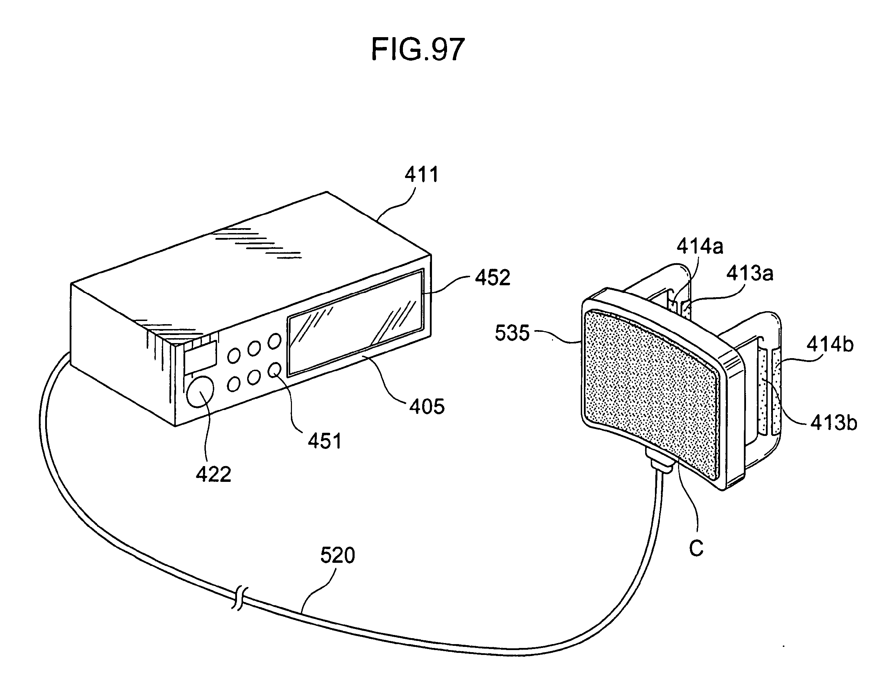

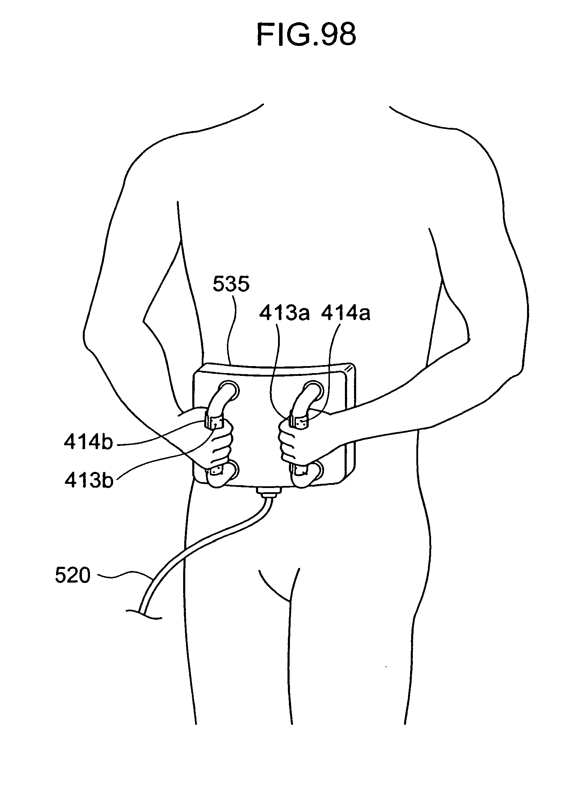

Fig. 97 is a schematic perspective view of the appearance of a fourth example of the truncal visceral/subcutaneous fat measuring apparatus according to the present invention. Fig. 98 is a schematic diagram for illustrating a use form of the apparatus of Fig. 97. The example of Fig. 97 comprises a main unit 411 and a grip section 535. The grip section 535 is equivalent to integration of the left and right grip sections in the first and second examples. The grip section 535 is connected to the main unit 411 by an electric cable 520.

A grip for holding the grip section 535 by the left hand has a current applying electrode 413a and a voltage measuring electrode 414a, and a grip for holding the grip section 535 by the right hand has a current applying electrode 413b and a voltage measuring electrode 414b. Further, a shared electrode C is provided on the contact surface of the grip section 535 which contacts the trunk. As shown in Fig. 98, the shared electrode C is pressed against the abdomen at the time of measurement.

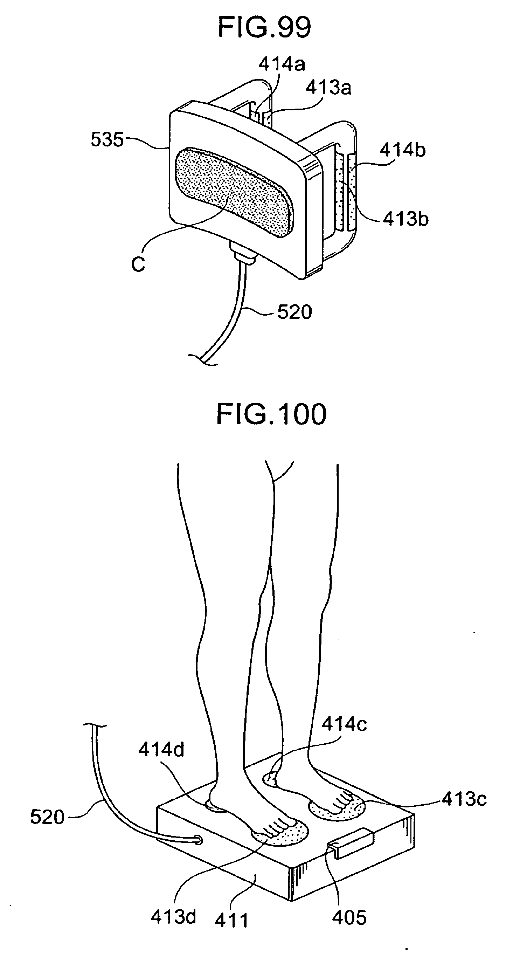

Figs. 99 and 100 are schematic perspective views of the appearance of a fifth example of the truncal visceral/subcutaneous fat measuring apparatus according to the present invention. This fifth example comprises a scale-shaped main unit 411 shown in Fig. 100 and a grip section 535 shown in Fig. 99. As in the case of the fourth example, the grip section 535 is equivalent to integration of the left and right grip sections. The grip section 535 is connected to the main unit 411 by an electric cable 520.

In the fifth example, as well as the fourth example, a grip for holding the grip section 535 by the left hand has a current applying electrode 413a and a voltage measuring electrode 414a, and a grip for holding the grip section 535 by the right hand has a current applying electrode 413b and a voltage measuring electrode 414b. Further, a shared electrode C is provided on the contact surface of the grip section 535 which contacts the trunk. As shown in the drawing, the shared electrode C is pressed against the abdomen at the time of measurement.

In the fifth example, foot electrodes for the lower extremity are provided on the top surface of the main unit 411. As in the case of the third example, the foot electrodes comprise a current applying electrode 413c and a voltage measuring electrode 414c for the left foot and a current applying electrode 413d and a voltage measuring electrode 414d for the right foot. Further, an operation display panel 45 is also provided on the top surface of the main unit 411.

In the above examples of the present invention, the current applying electrode and the voltage measuring electrode are provided on each of the left and right grips as grip electrodes. Alternatively, it is also possible that only the current applying electrodes are provided on either one of the grips and only the voltage measuring electrodes are provided on the other grip. Further, although the current applying electrode and the voltage measuring electrode are provided for each of the left and right feet as foot electrodes in the above examples of the present invention, it is also possible that only the current applying electrodes are provided for either one of the feet and only the voltage measuring electrodes are provided for the other foot.

The same descriptions as those given above with respect to Figs. 9 to 13 are also applied to the example according to the third characteristic of the present invention.

The present invention uses a three-electrode technique in which one current applying electrode and one voltage measuring electrode out of four electrodes in a four-electrode technique are implemented by one sharable electrode having a wide electrode area. This sharable electrode is referred to as a shared electrode C are described above. By widening the electrode area, the influence of contact area resistance with the skin and the influence of spreading resistance right underneath a current applying electrode can be alleviated or ignored.

According to one example of the present invention, limitations on the electrode area of the shared electrode C are as follows. That is, the electrode area is at least 36 cm$^2$ (at least 9 times the general electrode size 2.0 x 2.0 = 4.0 cm$^2$), the electrode width in the abdominal circumferential direction is at least about 1/10 of the abdominal circumference, and the electrode length in the trunk length direction is 3 cm to about 1/10 of the trunk length. In addition, unlike the shared electrode C, the current applying electrode 413 and the voltage measuring electrode 414 have a general electrode size of 2.0 x 2.0 = 4.0 cm$^2$.

According to one example of the present invention, the three-electrode technique according to the present invention is applied to an extremity lead system. In this case, only the shared electrode is placed on the trunk, and the other electrodes (the other current applying electrode and voltage measuring electrode) are placed on the extremities.

In this example, more useful effects are obtained by the following constitutions.

(1) Such an extremity lead system involves an electrode arrangement suited for measurement of visceral fat tissues of the abdomen of the trunk.

(2) The shared electrode is placed in a longitudinal shape in the navel circumferential direction.

(3) The shared electrode is placed in a longitudinal shape in the navel circumferential direction so as to cover one or both of the left and right joined tendons between the left and right rectus abdominis muscles and the left and right external abdominal oblique muscles on or near the navel circumferential.

(4) When the electrodes for the extremities are placed on the upper extremities, the current applying electrode is placed on either the left or right extremity, and the voltage measuring electrode is placed on the other extremity. The same applies to a case where the electrodes for the extremities are placed on the lower extremities.

(5) In this lead system, the shared electrode is placed on the abdomen of the trunk, and the other electrodes are placed on either the upper extremities or the lower extremities.

(6) In the case of combination with the upper extremities, the position of the shared electrode on the abdomen of the trunk is a position on the navel circumference or a position which is slightly shifted from the navel circumference position toward the lower extremities in the trunk length direction.

(7) In the case of combination with the lower extremities, the position of the shared electrode on the abdomen of the trunk is a position on the navel circumference or a position which is slightly shifted from the navel circumference position toward the upper extremities in the trunk length direction.

(8) The position of the navel is used to position the shared electrode since the position of the shared electrode is a position on the navel circumference or a position which is slightly higher or lower than the position on the navel circumference.

(9) In the case of combination with the upper extremities, the form of the apparatus may be a compo type (handy type) in which the shared electrode is placed on the surface which is pressed against the abdomen of the trunk, the operation display section is placed on a surface other than the above abdomen contacting surface, e.g. the front surface, the grip sections for pressing the apparatus against the abdomen are provided at the left and right sides of the apparatus and the electrodes to be placed on the extremities are provided on the left and right grip sections or a form in which components other than the electrodes are provided in a separate case and connected by an electric cable.

(10) In the case of combination with the lower extremities, the form of the apparatus may be a separate type in which the shared electrode is placed on the surface which is pressed against the abdomen of the trunk, the operation display section is placed on a surface other than the above abdomen contacting surface, e.g. the front surface, the grip section for pressing the apparatus against the abdomen is provided at one or both of the left and right sides of the apparatus and the left and right grip sections are electrically connected to foot electrodes by electric cables. A form in which the foot electrodes are integrated with the body weight measuring section is highly useful.

(11) The form of the apparatus may be such that the abdomen contacting electrode section is fixed by a belt in addition to being pressed against the abdomen

(12) The form of the apparatus comprises the present shared electrode for the abdomen in addition to foot electrodes and grip electrodes according to the conventional extremity lead system, thereby making possible measurement of the visceral fat tissues of the abdomen of the trunk by the present lead system.

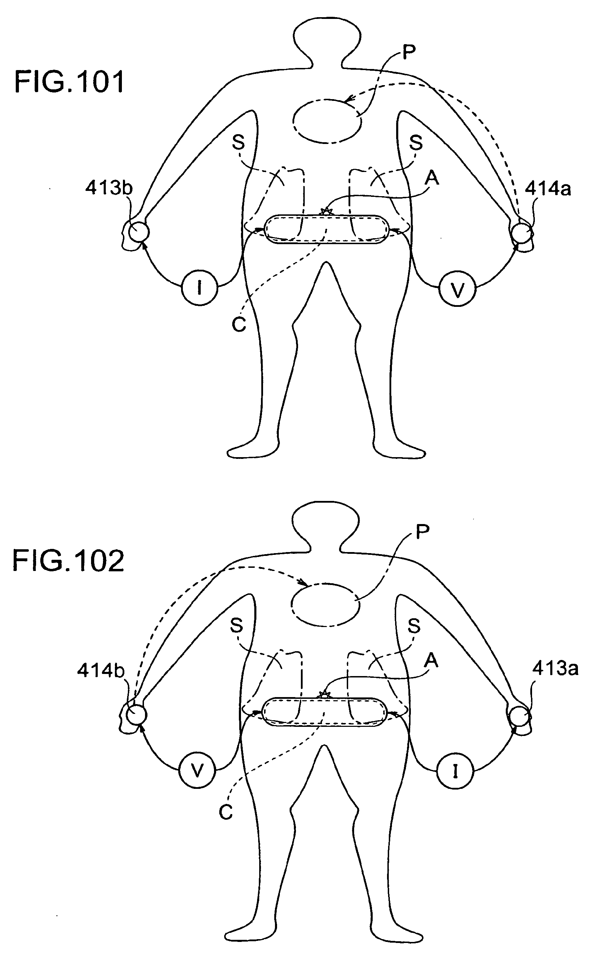

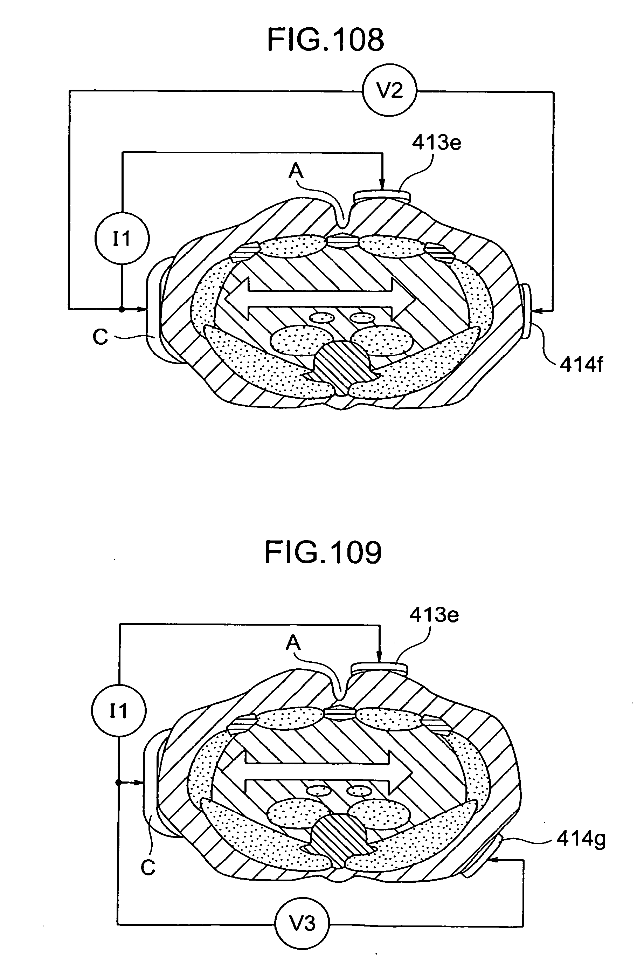

Figs. 101 to 111 illustrate electrode arrangement examples in the truncal visceral/subcutaneous fat measuring method of the present invention. In these electrode arrangement examples, a shared electrode is placed on the abdomen of the trunk, and a current applying electrode and a voltage measuring electrode are placed on other body parts such as extremities or the abdomen of the trunk. Particularly, Figs. 101 to 107 illustrate electrode arrangement examples in a extremity lead system, and a current applying electrode and a voltage measuring electrode are placed on the upper or lower extremities. Figs. 108 to 111 illustrate electrode arrangement examples on the abdomen of the trunk, and a current applying electrode and a voltage measuring electrode are placed on the trunk of the abdomen.

Fig. 101 illustrates an electrode arrangement example in combination with the upper extremities. In this electrode arrangement example, a right-hand current applying electrode 413b and a left-hand voltage measuring electrode 414a are used in combination with a shared electrode C placed on the abdomen of the trunk. The impedance of the trunk is obtained by measuring a potential difference V between the shared electrode C and the voltage measuring electrode 414a when a current I is applied between the shared electrode C and the current applying electrode 413b.

Fig. 102 also illustrates an electrode arrangement example in combination with the upper extremities. In this electrode arrangement example, a left-hand current applying electrode 413a and a right-hand voltage measuring electrode 414b are used in combination with a shared electrode C placed on the abdomen of the trunk. The impedance of the trunk is obtained by measuring a potential difference V between the shared electrode C and the voltage measuring electrode 414b when a current I is applied between the shared electrode C and the current applying electrode 413a.

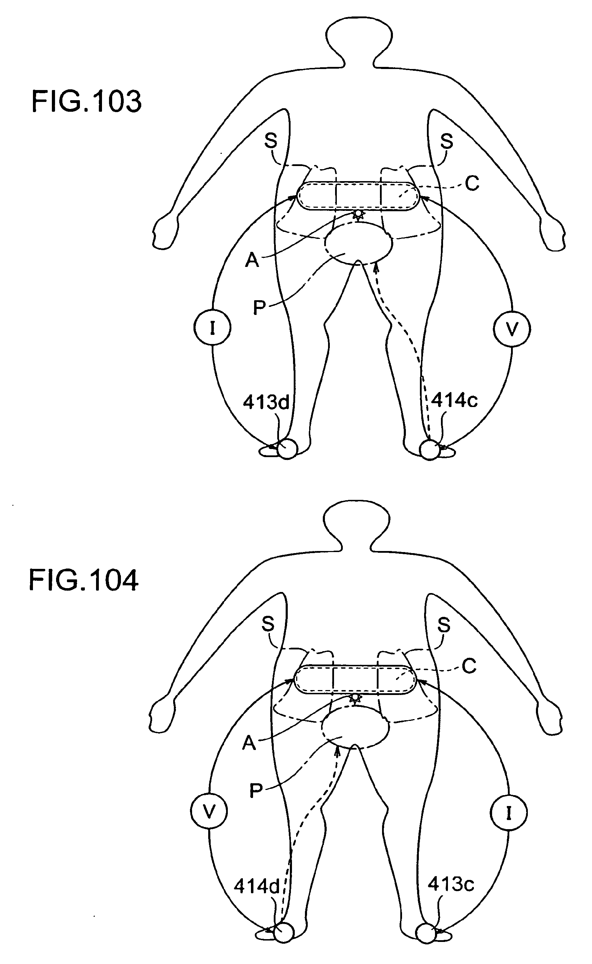

Fig. 103 illustrates an electrode arrangement example in combination with the lower extremities. In this electrode arrangement example, a right-foot current applying electrode 413d and a left-foot voltage measuring electrode 414c are used in combination with a shared electrode C placed on the abdomen of the trunk. The impedance of the trunk is obtained by measuring a potential difference V between the shared electrode C and the voltage measuring electrode 414c when a current I is applied between the shared electrode C and the current applying electrode 413d.

Fig. 104 also illustrates an electrode arrangement example in combination with the lower extremities. In this electrode arrangement example, a left-foot current applying electrode 413c and a right-foot voltage measuring electrode 414d are used in combination with a shared electrode C placed on the abdomen of the trunk. The impedance of the trunk is obtained by measuring a potential difference V between the shared electrode C and the voltage measuring electrode 414d when a current I is applied between the shared electrode C and the current applying electrode 413c.

As shown in Figs. 101 to 104, the shared electrode C is placed in a longitudinal shape in the navel circumferential direction. More specifically, the shared electrode is placed in a longitudinal shape in the navel circumferential direction so as to cover both of the joined tendons S between the left and right rectus abdominis muscles and the left and right external abdominal oblique muscles on or near the navel circumferential. That is, the shared electrode is placed so as to cover both of the left and right aponeuroses S with the navel interposed therebetween.

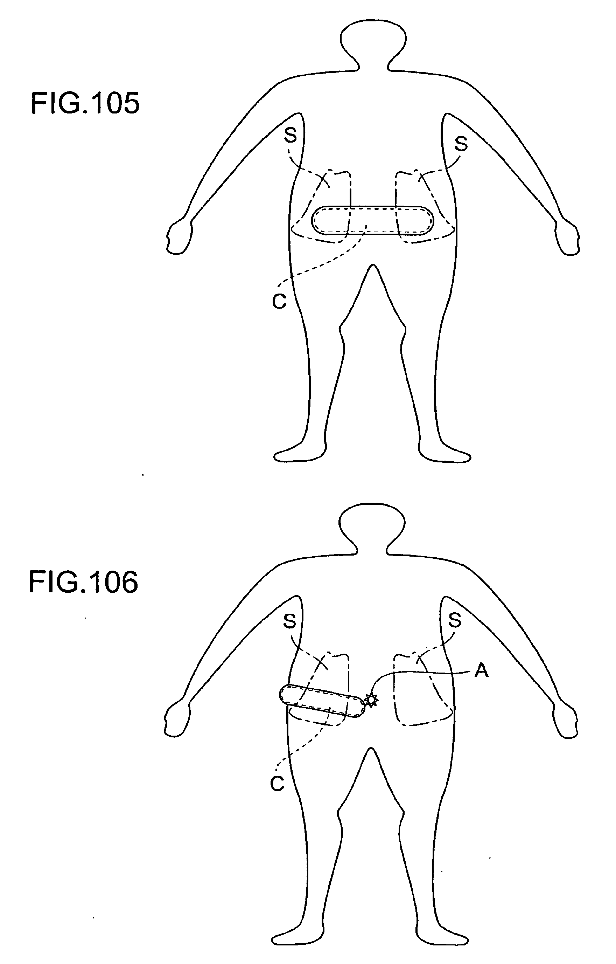

Alternatively, as shown in the electrode arrangement examples of Figs. 105 to 107, the shared electrode C may also be placed so as to cover either one of the left and right aponeuroses S.

As shown in Figs. 101 to 104, in the case of combination with the upper extremities, the shared electrode C on the abdomen of the trunk is placed at a position which is slightly shifted from the navel circumference position toward the lower extremities. Meanwhile, in the case of combination with the lower extremities, the shared electrode on the abdomen of the trunk is placed at a position which is slightly shifted from the navel circumference position toward the upper extremities. By placing the shared electrode at such positions, the information of body tissues between a virtual electrode position P on the trunk and the shared electrode C can be collected. Further, since the shared electrode C is placed on the navel circumference position or a higher or lower position in the vicinity of the navel circumference position, the position of the navel (the position of the navel A) is used to position the shared electrode. That is, the shared electrode C can be positioned easily by positioning the upper or lower edge of the shared electrode C at the position of the navel.

Figs. 108 to 111 are cross-sectional views of the abdomen of the trunk which illustrate electrode arrangement examples of combinations of the shared electrode and electrodes placed on the abdomen of the trunk in the case of cross-sectional energization in the abdominal circumferential portion at the navel height. In Figs. 108 to 111, current applying electrodes 413e and 413g are placed in a region adjacent to the navel and a region under the shoulder blade, and voltage measuring electrodes 414e, 414f and 414g are placed at remote sites where the influence of spreading resistance by these current applying electrodes can be avoided.

In the electrode arrangement example of Fig. 108, the impedance of the trunk for determining a visceral fat tissue volume is obtained by measuring a potential difference V2 between the shared electrode C and the voltage measuring electrode 414f when a current 4I1 is applied between the shared electrode C and the current applying electrode 413e.

Similarly, in the electrode arrangement example of Fig. 109, the impedance of the trunk for determining a visceral fat tissue volume is obtained by measuring a potential difference V3 between the shared electrode C and the voltage measuring electrode 414g when a current I1 is applied between the shared electrode C and the current applying electrode 413e.

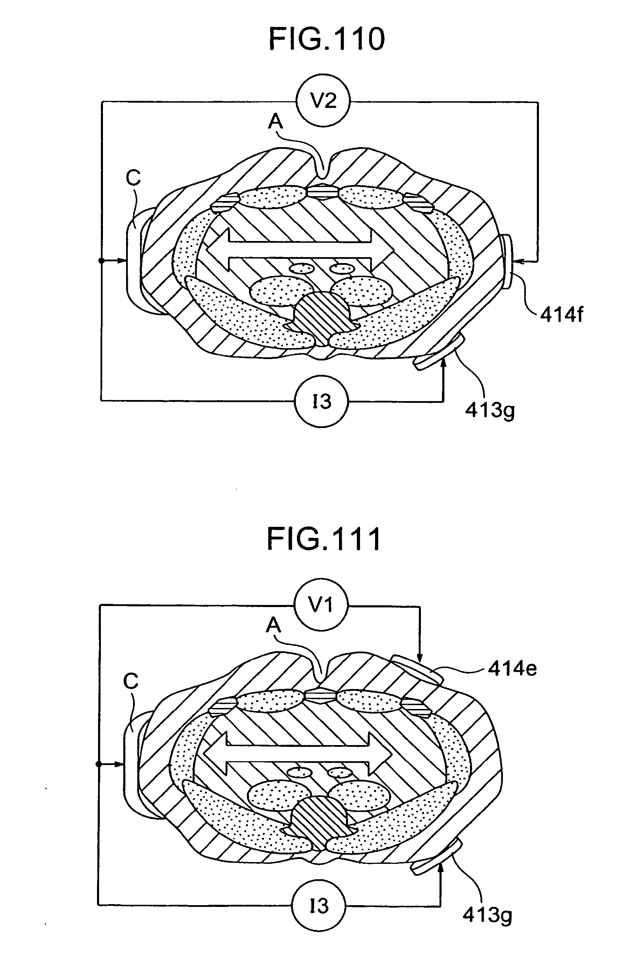

Similarly, in the electrode arrangement example of Fig. 110, the impedance of the trunk for determining a visceral fat tissue volume is obtained by measuring a potential difference V2 between the shared electrode C and the voltage measuring electrode 414f when a current I3 is applied between the shared electrode C and the current applying electrode 413g.

Similarly, in the electrode arrangement example of Fig. 111, the impedance of the trunk for determining a visceral fat tissue volume is obtained by measuring a potential difference V1 between the shared electrode C and the voltage measuring electrode 414e when a current I3 is applied between the shared electrode C and the current applying electrode 413g.

Next, the operations of the truncal visceral fat measuring apparatus in the example of the present invention shown in Figs. 88 to 111 will be described.

As for the operations of the truncal visceral fat measuring apparatus, the same processes as those described above with respect to the basic flowchart of Fig. 86 are performed.

The above subcutaneous fat tissue volume (AFS) estimation process in STEP SA9 is carried out by use of the numerical values stored in the storage section 404 and the above expression 22-1 or 22-2, as described above with respect to the subroutine flowchart of Fig. 73.

The above splanchnic organ tissue volume (AVM) and splanchnic organ tissue impedance (ZVM) estimation process in STEP SA10 is the same as that described with respect to the subroutine flowchart of Fig. 70.

The above visceral fat tissue impedance (ZFV) and visceral fat tissue volume (AFV) estimation process in STEP SA11 is the same as that described with respect to the subroutine flowchart of Fig. 71.

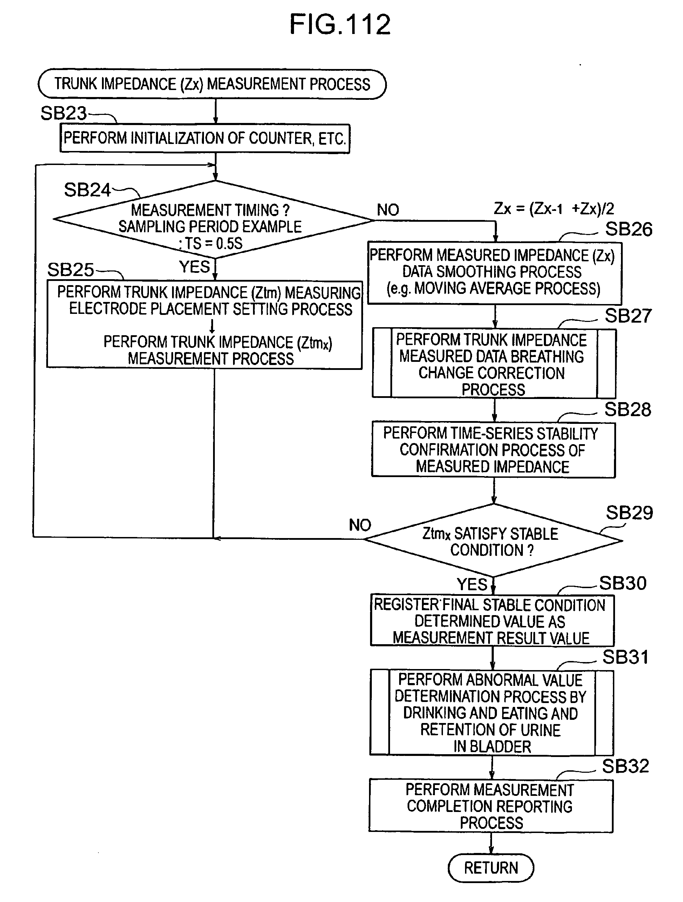

Next, the trunk impedance (Zx) measurement process in STEP SA6 will be described in detail with reference to the subroutine flowchart of Fig. 112 showing a first embodiment. In this first embodiment, "process of removing influence of change by breathing" and "process of determining abnormal value by drinking and eating and retention of water (e.g. urine) in bladder or the like" as described in (27) and (28) in the above 10. are conducted. Firstly, in STEP SB23, the computation/control section 421 initializes a counter and the like, e.g. the number of samples for measurement data of the impedance Ztm of the trunk.

Then, in STEP SB24, the computation/control section 421 determines whether it is measurement timing. When it has been determined that it is measurement timing, the computation/control section 421 performs a trunk impedance (Ztm) measuring electrode placement setting process and a trunk impedance ($Ztm_x$) measurement process in STEP SB25.

Meanwhile, when it has been determined in STEP SB24 that it is not measurement timing, the computation/control section 421 proceeds to STEP SB26 and performs a measured impedance (Zx) data smoothing process (e.g. a moving average process), i.e. $Z_x = (Z_{x-1} + Z_x)/2$, on the trunk impedance ($Ztm_x$). Then, in STEP SB27, the computation/control section 421 performs a trunk impedance measured data breathing change correction process.

Then, in STEP SB28, the computation/control section 421 performs a time-series stability confirmation process of measured impedance of each body part. This is carried out by determining whether each value after the trunk impedance measured data breathing change correction process in STEP SB27 has converged to a value within a predetermined change in a predetermined number of times.

In STEP SB29, the computation/control section 421 determines whether the measured $Ztm_x$ satisfies a stable condition. This determination is made such that a median breathing value is determined at the point when a median breathing value in each breathing cycle enters a stable range within a predetermined number of times. When it is determined in this STEP SB29 that the stable condition is satisfied, the computation/control section 421 proceeds to STEP SB30 and registers the impedance value of the determined median value as the impedance value of the trunk or the impedance of the subcutaneous fat tissue layer and a final stable condition determined value as a measurement result value in the storage section 404. That is, the computation/control section 421 registers $Ztm_x$ which satisfies the stable condition as Ztm. Meanwhile, when it is determined in STEP SB29 that the stable condition is not satisfied, the computation/control section 421 returns to STEP SB24 and repeats the above processes.

Subsequent to STEP SB30, the computation/control section 421 performs an abnormal value determination process by drinking and eating and retention of urine in bladder in STEP SB31 and informs completion of measurements by means of the alarming buzzer 422 (refer to Fig. 90) in STEP SB32, thereby completing measurements.

The trunk impedance measured data breathing change correction process in STEP SB27 is the same as that described above with respect to the subroutine flowchart of Fig. 65.

The abnormal value determination process by drinking and eating and retention of urine in bladder in STEP SB31 is the same as that described above with respect to the subroutine flowchart of Fig. 66.

By the above operations, the visceral fat tissue information of the trunk (abdomen) can be obtained, the process of removing the influence of change by breathing and the process of determining abnormality by drinking and eating and retention of water (e.g. urine) in bladder or the like can be performed, and advice information corresponding to the result of the abnormality determining process can be provided. Further, although trunk visceral fat tissue information is obtained as a body fat percentage in the above example, the present invention is not limited thereto. The present invention can obtain the information as a cross-sectional area, volume or weight by use of an appropriate conversion equation.

According to the present invention, while adhering to a conventional, simple measurement method, highly accurate screening information according to a required level can be exposed for the degree of accumulation of fat tissues adhering around splanchnic organ tissues.

According to the present invention, since the visceral fat tissues of the trunk can be measured with high accuracy by a small and simple apparatus, it can be used as an optimum apparatus for domestic use. Further, the present invention can also perform checking of the condition of the abdomen prior to measurement, i.e. early checking of abnormality in body fluid distribution in splanchnic organ tissues due to inflammation or illness and give appropriate health guideline advice according to the result of the checking. Thus, users can acquire a variety of information useful for proper exercise of daily diet by eating and exercise, retention of motivation therefor and sustainable self-management for maintenance and improvement of health in a simple manner, and the information is very useful.

According to the present invention, by limiting a body part where subcutaneous fat tissue thickness is measured and a measurement method, the subcutaneous fat tissue layer information of the trunk can be measured with high accuracy, and the visceral fat tissues of the trunk can be measured with high accuracy by using highly accurately measured subcutaneous fat tissue layer information for estimation of the visceral tissue information of the trunk.

**Claims**

1. A truncal visceral/subcutaneous fat measuring method which comprises the steps of:

   applying a current to a trunk by a pair of current applying electrodes (10d, 10e),
   measuring a potential difference (V) that has occurred in the trunk by a pair of voltage measuring electrodes (11c, 11e, 11f, 11g), and
   measuring an impedance of the trunk to acquire the visceral fat tissue information and/or subcutaneous fat tissue layer information of the trunk,
   wherein one of the current applying electrodes (10e) is placed on the trunk, the other current applying electrode (10d) is placed on a body part protruding from the trunk, the voltage measuring electrodes (11c, 11e, 11f, 11g) are placed at a remote position where the influence of spreading resistance right underneath the one current applying electrode (10e) is weak when acquiring the visceral fat tissue information of the trunk, and one of the voltage measuring electrodes (11f) is placed at a position where the influence of the spreading resistance right underneath the one current applying electrode (10e) is predominant when acquiring the subcutaneous fat tissue layer information of the trunk.

2. A truncal visceral/subcutaneous fat measuring method according to claim 1, wherein one of the current applying electrodes(10e) is placed on at least one body part where the subcutaneous fat tissue layer is thick and which is suited for estimating a subcutaneous fat tissue volume on the truncal abdominal circumference,
   the other current applying electrode(10d) is placed on a body part protruding from the trunk, one of the voltage measuring electrodes(11e, 11f) is placed in the vicinity of the one current applying electrode (10e) on the truncal abdominal circumference, and
   the other voltage measuring electrode (11c) is placed on a body part protruding from the trunk that is different from the body part on which the other current applying electrode (10d) is placed, to measure the impedance of the trunk so as to acquire the subcutaneous fat tissue layer information of the trunk.

3. A truncal visceral/subcutaneous fat measuring method according to claim 1, further comprising the steps of:

   measuring the bioelectrical impedance of a lower extremity, the bioelectrical impedance of an upper extremity, and the bioelectrical impedance of a trunk,
   determining the skeletal muscle tissue volume of the lower extremity based on the measured bioelectrical impedance of the lower extremity and body specifying information,
   determining the skeletal muscle tissue volume of the upper extremity based on the measured bioelectrical impedance of the supper extremity and the body specifying information,
   determining the skeletal muscle tissue volume of the trunk based on the determined skeletal muscle tissue volumes of the lower and upper extremities and the body specifying information,
   determining the impedance of the skeletal muscle tissue layer of the trunk based on the determined skeletal muscle tissue volume of the trunk and the body specifying information,
   determining the impedance of the subcutaneous fat tissue layer of the trunk by placing one of the current applying electrodes (10e) on at least one body part where the subcutaneous fat tissue layer is thick and which is suited for estimating a subcutaneous fat tissue volume on the truncal abdominal circumference, placing the other current applying electrode (10d) on a body part protruding from the trunk, placing one of the voltage measuring electrodes (11e) in the vicinity of the one current applying electrode on the truncal abdominal circumference, and placing the other voltage measuring electrode (11c) on a body part protruding from the trunk that is different from the body part on which the other current applying electrode (10d) is placed, so as to determine the impedance of the subcutaneous fat tissue layer of the trunk,
   determining the impedance of the splanchnic organ tissue of the trunk based on the body specifying information,
   determining the impedance of the visceral fat tissue of the trunk based on the determined bioelectrical impedance of the trunk, the determined impedance of the skeletal muscle tissue layer of the trunk, the determined impedance of the subcutaneous fat tissue layer of the trunk, and the determined impedance of the splanchnic organ tissue of the trunk, and

determining the visceral fat tissue volume of the trunk based on the determined impedance of the visceral fat tissue of the trunk and the body specifying information.

4. A truncal visceral/subcutaneous fat measuring method according to one of claims 1 to 3, wherein a shared electrode (C) serving as one of the current applying electrodes and one of the voltage measuring electrodes is placed on the trunk, the impedance of the trunk is measured by the shared electrode (C), the other current applying electrode and the other voltage measuring electrode, the shared electrode (C) has an electrode area wide enough so that the influence of contact area resistance with the skin and the influence of spreading resistance right underneath a current applying electrode can be alleviated.

5. A truncal visceral/subcutaneous fat measuring apparatus for performing a method according to one of claims 1 to 4, wherein the pair of current applying electrodes (10d, 10e) is configured to apply a current to the trunk and the pair of voltage measuring electrodes (11c, 11e, 11f, 11g) is configured to measure a potential difference (V) that has occurred in the trunk so as to acquire visceral fat tissue information and/or subcutaneous fat tissue layer information of the trunk by measuring the impedance of the trunk, and wherein a current applying electrode switching section (9) and a voltage measuring electrode switching section (12) is provided so as to switch the electrodes (10d, 10e, 11c, 11e, 11f, 11g) depending on their positions on the respective body parts.

6. A truncal visceral/subcutaneous fat measuring apparatus according to claim 5, further comprising:

lower-extremity bioelectrical impedance measuring means measuring the bioelectrical impedance of a lower extremity,

upper-extremity bioelectrical impedance measuring means measuring the bioelectrical impedance of an upper extremity,

trunk bioelectrical impedance measuring means measuring the bioelectrical impedance of the trunk,

trunk skeletal muscle tissue volume estimating means estimating the skeletal muscle tissue volume of the trunk,

trunk skeletal muscle tissue layer impedance estimating means estimating the impedance of the skeletal muscle tissue layer of the trunk based on the estimated skeletal muscle tissue volume of the trunk and body specifying information,

trunk subcutaneous fat tissue layer impedance estimating means estimating the impedance of the subcutaneous fat tissue layer of the trunk, the trunk subcutaneous fat tissue layer impedance estimating means comprising at least a pair of current applying electrodes that comprise one current applying electrode that is placed on at least one body part where the subcutaneous fat tissue layer is thick and which is suited for estimating a subcutaneous fat tissue volume on the truncal abdominal circumference and the other current applying electrode that is placed on a body part protruding from the trunk and at least a pair of voltage measuring electrodes that comprise one voltage measuring electrode that is placed in the vicinity of the one current applying electrode on the truncal abdominal circumference and the other voltage measuring electrode that is placed on a body part protruding from the trunk that is different from the body part on which the other current applying electrode is placed, and estimates the impedance of the subcutaneous fat tissue layer of the trunk,

trunk splanchnic organ tissue impedance estimating means estimating the impedance of the splanchnic organ tissue of the trunk based on the body specifying information,

trunk visceral fat tissue impedance estimating means estimating the impedance of the visceral fat tissue of the trunk based on the estimated bioelectrical impedance of the trunk, the estimated impedance of the skeletal muscle tissue layer of the trunk, the estimated impedance of the subcutaneous fat tissue layer of the trunk, and the estimated impedance of the splanchnic organ tissue of the trunk, and

trunk visceral fat tissue volume estimating means estimating the visceral fat tissue volume of the trunk based on the estimated impedance of the visceral fat tissue of the trunk and the body specifying information.

7. A truncal visceral/subcutaneous fat measuring apparatus according to claim 6, wherein the trunk skeletal muscle tissue volume estimating means estimates the skeletal muscle tissue volume of the trunk based on the measured bioelectrical impedances of the lower and upper extremities and the body specifying information.

8. A truncal visceral/subcutaneous fat measuring apparatus according to claim 6, further comprising:

lower-extremity skeletal muscle tissue volume estimating means estimating the skeletal muscle tissue volume of the lower extremity based on the measured bioelectrical impedance of the lower extremity and body specifying information, and

upper-extremity skeletal muscle tissue volume estimating means estimating the skeletal muscle tissue volume of the upper extremity based on the measured bioelectrical impedance of the upper extremity and the body specifying information,

wherein the trunk skeletal muscle tissue volume estimating means estimates the skeletal muscle tissue volume of the trunk based on the estimated skeletal muscle tissue volumes of the lower and upper extremities and the body specifying information.

**9.** A truncal visceral/subcutaneous fat measuring apparatus according to one of claims 5 to 8, wherein a shared electrode (C) serving as one of the current applying electrodes and one of the voltage measuring electrodes is placed on the trunk, the impedance of the trunk is measured by the shared electrode (C), the other current applying electrode and the other voltage measuring electrode, and the shared electrode (C) has an electrode area wide enough so that the influence of contact area resistance with the skin and the influence of spreading resistance right underneath a current applying electrode can be alleviated.

**Patentansprüche**

**1.** Körperorgan- / Unterhautfett- Messverfahren, das die Schritte aufweist, von:

Anlegen eines Stromes an einem Körper durch ein Paar von Stromanlegenden Elektroden (10d, 10e), Messen eines Potentialunterschiedes (V), der in dem Körper aufgetreten ist, durch ein Paar von Spannungsmesselektroden (11c, 11e, 11f, 11g), und

Messen einer Impedanz des Körpers, um die Organ- Fettgewebeinformation und / oder die Unterhautfettgewebeschicht- Information des Körpers zu erlangen,

wobei eine der Stromanlegenden Elektroden (10e) auf dem Körper platziert ist, die andere Stromanlegende Elektrode (10d) an einem Körperteil, der von dem Körper vorspringt, angeordnet ist ist, die Spannungsmesselektroden (11c, 11e, 11f, 11g) an einer entfernten Position angeordnet sind, wo der Einfluss des Ausbreitungswiderstandes direkt unterhalb der einen Stromanlegende Elektrode (10e) schwach ist, wenn die Organ- Fettgewebeinformation des Körpers erlangt wird, und eine der Spannungsmesselektroden (11f) an einer Position angeordnet wird, wo der Einfluss des Ausbreitungswiderstandes direkt unter der einen Stromanlegende Elektrode (10e) vorherrschend ist, wenn die Information zur Unterhautfettgewebeschicht des Körpers erlangt wird.

**2.** Körperorgan- / Unterhautfett- Messverfahren nach Anspruch 1, wobei einer der Stromanlegenden Elektroden (10e) an zumindest einem Körperteil platziert ist, wo die Unterhautfettgewebeschicht dick ist und die geeignet ist, um ein Unterhautfettgewebevolumen an dem abdominalen Umfang des Körpers abzuschätzen, die andere Stromanlegende Elektrode (10d) an einem Körperteil platziert ist, der von dem Körper vorspringt, eine der Spannungsmesselektroden (11e, 11f) in der Nähe der einen Stromanlegende Elektrode (10e) an dem abdominalen Umfang des Körpers platziert ist, und die andere Spannungsmesselektrode (11c) an einem Körperteil platziert ist, der von dem Körper vorspringt, der von dem Körperteil, an dem die andere Stromanlegende Elektrode (10d) platziert ist, verschieden ist, um die Impedanz des Körpers zu messen, um die Information zur Unterhautfettgewebeschicht des Körpers zu erlangen.

**3.** Körperorgan- / Unterhautfett- Messverfahren nach Anspruch 1, außerdem aufweisend die Schritte von:

Messen der bioelektrischen Impedanz einer unteren Extremität, der bioelektrischen Impedanz einer oberen Extremität und der bioelektrischen Impedanz des Körpers,

Bestimmen des skelettalen Muskelgewebevolumens der unteren Extremität auf der Grundlage der gemessenen bioelektrischen Impedanz der unteren Extremität und Körperspezifischer Information,

Bestimmen des skelettalen Muskelgewebevolumens der oberen Extremität auf der Grundlage der gemessenen bioelektrischen Impedanz der oberen Extremität und Körperspezifischer Information,

Bestimmen des skelettalen Muskelgewebevolumens des Körpers auf der Grundlage des bestimmten skelettalen Muskelgewebevolumens der unteren und oberen Extremitäten und von Körperspezifischen Informationen,

Bestimmen der Impedanz der skelettalen Muskelgewebeschicht des Körpers auf der Grundlage des bestimmten skelettalen Muskelgewebevolumens des Körpers und von Körperspezifischen Informationen,

Bestimmen der Impedanz der Unterhaut- Fettgewebeschicht des Körpers durch Platzieren von einer der Stromanlegenden Elektroden (10e) an zumindest einem Körperteil, wo die Unterhaut- Fettgewebeschicht dick ist und die zum Abschätzen eines Unterhaut- Fettgewebevolumens an dem abdominalen Umfang des Körpers geeignet ist, Platzieren der anderen Stromanlegende Elektrode (10d) an einem Körperteil, das von dem Körper vorspringt, Platzieren von einer der Spannungsmesselektroden (11e) in der Nähe der einen Stromanlegende Elektrode an dem abdominalen Umfang des Körpers und Platzieren der anderen Spannungsmesselektrode (11 c) an einem Körperteil, der von dem Körper vorspringt und von dem Körperteil, an dem die andere Stromanlegende Elektrode (10d) platziert ist, verschieden ist, um die Impedanz der Unterhaut- Fettgewebeschicht des Körpers zu bestimmen,

Bestimmen der Impedanz des Organgewebes des Körpers auf der Grundlage Körperspezifischer Informationen, Bestimmen der Impedanz des Körperorgan- Fettgewebes des Körpers auf der Grundlage der bestimmten bioelektrischen Impedanz des Körpers, der bestimmten Impedanz der skelettalen Muskelgewebeschicht des Körpers, der bestimmten Impedanz der Unterhaut- Fettgewebeschicht des Körpers und der bestimmten Impedanz des Organgewebes des Körpers, und

Bestimmen des Körperorgan- Fettgewebevolumens des Körpers auf der Grundlage der bestimmten Impedanz des Körperorgan- Fettgewebes des Körpers und der Körperspezifischen Informationen.

4. Körperorgan- / Unterhautfett- Messverfahren nach einem der Ansprüche 1 bis 3, wobei eine geteilte Elektrode (C), die als eine der Stromanlegende Elektroden und als eine der Spannungsmesselektroden dient, an dem Körper platziert ist, die Impedanz des Körpers durch die geteilte Elektrode (C), die andere Stromanlegende Elektrode und die andere Spannungsmesselektrode gemessen wird, wobei die geteilte Elektrode (C) eine genügend große Elektrodenfläche hat, so dass der Einfluss des Kontaktflächenwiderstandes bei der Haut und der Einfluss des Ausbreitungswiderstandes direkt unter einer Stromanlegende Elektrode gemildert werden kann.

5. Körperorgan- / Unterhautfett- Messvorrichtung zum Ausführen eines Verfahrens nach einem der Ansprüche 1 bis 4, wobei das Paar von Stromanlegenden Elektroden (10d, 10e) konfiguriert ist, einen Strom an den Körper anzulegen und das Paar von Spannungsmesselektroden (11c, 11e, 11f, 11g) konfiguriert ist, einen Potentialunterschied (V) zu messen, der in dem Körper aufgetreten ist, um die Körperorgan- Fettgewebeinformation und / oder die Information zur UnterhautFettgewebeschicht des Körpers durch Messen der Impedanz des Körpers zu erlangen, und wobei ein Stromanlegenden Elektroden- Schaltabschnitt (9) und ein Spannungsmesselektroden- Schaltabschnitt (12) vorgesehen ist, um die Elektroden (10d, 10e, 11c, 11e, 11f, 11g) in Abhängigkeit von ihren Positionen an den jeweiligen Körperteilen zu schalten.

6. Körperorgan- / Unterhautfett- Messvorrichtung nach Anspruch 5, außerdem aufweisend:

bioelektrische untere Extremitäten- Impedanz- Messeinrichtung, die die bioelektrische Impedanz einer unteren Extremität misst,

bioelektrische obere Extremitäten- Impedanz- Messeinrichtung, die die bioelektrische Impedanz einer oberen Extremität misst,

bioelektrische Rumpf- Impedanz- Messeinrichtung, die die bioelektrische Impedanz des Rumpfes misst,

Körper- skelettale Muskelgewebevolumen- Impedanz- Abschätzeinrichtung, die die Impedanz des skelettale Muskelgewebevolumen des Rumpfes abschätzt,

Körper- skelettale Muskelgewebeschicht- Impedanz- Abschätzeinrichtung, die die Impedanz der skelettalen Muskelgewebeschicht des Rumpfes auf der Grundlage des abgeschätzten skelettalen Muskelgewebevolumens des Körpers und Körperspezifischer Informationen abschätzt,

Rumpf- Unterhautfettgewebeschicht- Impedanz- Abschätzeinrichtung, die die Impedanz der Unterhautfettgewebeschicht des Rumpfes abschätzt,

wobei die Rumpf- Unterhautfettgewebeschicht- Impedanz- Abschätzeinrichtung aufweist zumindest ein Paar von Stromanlegenden Elektroden, die aufweisen eine Stromanlegende Elektrode, die an zumindest einem Körperteil platziert ist, wo die Unterhautfettgewebeschicht dick ist und die zum Abschätzen eines Fettgewebevolumens an dem abdominalen Umfang des Rumpfes geeignet ist und die andere Stromanlegende Elektrode, die an einem Körperteil platziert ist, der von dem Körper vorspringt und mit zumindest einem Paar von Spannungsmesselektroden, die eine Spannungsmesselektrode aufweisen, die in der Nähe der einen Stromanlegende Elektrode an dem abdominalen Umfang des Rumpfes platziert ist, und

die andere Spannungsmesselektrode, die an einem Körperteil platziert ist, der von dem Körper vorspringt, der von dem Körperteil verschieden ist, an dem die andere Stromanlegende Elektrode platziert ist, und die Impedanz der UnterhautFettgewebeschicht des Rumpfes abschätzt,

eine Rumpf- Organgewebe- Impedanz- Abschätzeinrichtung, die die Impedanz des Organgewebes des Rumpfes auf der Grundlage der Körperspezifischen Informationen abschätzt,

Körperorgan- Fettgewebe- Impedanz- Abschätzeinrichtung, die die Impedanz des Körpersorgan- Fettgewebes des Rumpfes auf der Grundlage der abgeschätzten bioelektrischen Impedanz des Rumpfes, der abgeschätzten Impedanz der skelettalen Muskelgewebeschicht des Rumpfes, der abgeschätzten Impedanz der Unterhaut- Fettgewebeschicht des Rumpfes und der abgeschätzten Impedanz des Organgewebes des Rumpfes abschätzt, und

Eine Rumpf- Körperorgan- Fettgewebevolumen- Abschätzeinrichtung, die das Körperorgan- Fettgewebevolumen des Rumpfes auf der Grundlage der abgeschätzten Impedanz des Körperorgan- Fettgewebes des Körpers und der Körperspezifischen Informationen abschätzt.

7. Körperorgan- / Unterhautfett- Messvorrichtung nach Anspruch 6, wobei die skelettale Rumpf- Muskelgewebevolumen- Abschätzeinrichtung das skelettale Muskelgewebevolumen des Rumpfes abschätzt, auf der Grundlage der gemessenen bioelektrischen Impedanzen der unteren und oberen Extremitäten und der Körperspezifischen Informationen.

8. Körperorgan- / Unterhautfett- Messvorrichtung nach Anspruch 6, außerdem aufweisend:

untere Extremitäten- skelettales Muskelgewebevolumen- Abschätzeinrichtung, die abschätzt das skelettale Muskelgewebevolumen der unteren Extremitäten auf der Grundlage der gemessenen bioelektrischen Impedanz der untere Extremität und
der Körperspezifischen Information abschätzt, und
obere Extremitäten- skelettales Muskelgewebevolumen- Abschätzeinrichtung, die das skelettale Muskelgewebevolumen der oberen Extremitäten auf der Grundlage der gemessenen bioelektrischen Impedanz der oberen Extremität und der Körperspezifischen Information abschätzt, wobei die skelettale Muskelgewebevolumen- Abschätzeinrichtung das skelettale Muskelgewebevolumen des Rumpfes auf der Grundlage des abgeschätzten skelettalen Muskelgewebevolumens der unteren und oberen Extremitäten und der Körperspezifischen Informationen abschätzt.

9. Körperorgan- / Unterhautfett- Messvorrichtung nach einem der Ansprüche 5 bis 8,
wobei eine geteilte Elektrode (C), die als eine der Stromanlegenden Elektroden und als eine der Spannungsmesselektroden dient, an dem Körper platziert ist,
wobei die Impedanz des Körpers durch die geteilte Elektrode (C), die andere Stromanlegende Elektrode und die andere Spannungsmesselektrode gemessen wird und wobei die geteilte Elektrode (C) eine genügend große Elektrodenfläche hat, so dass der Einfluss des Kontaktflächenwiderstandes mit der Haut und der Einfluss des Ausbreitungswiderstandes direkt unter einer Stromanlegende Elektrode gemildert werden kann.

**Revendications**

1. Procédé de mesure de graisse viscérale/sous-cutanée tronculaire qui comprend les étapes consistant à :

appliquer un courant à un tronc grâce à une paire d'électrodes d'application de courant (10d, 10e),
mesurer une différence de potentiel (V) qui est apparue dans le tronc grâce à une paire d'électrodes de mesure de tension (11c, 11e, 11f, 11g), et
mesurer une impédance du tronc pour obtenir les informations de tissu adipeux viscéral et/ou les informations de couche de tissu adipeux sous-cutané du tronc,

dans lequel l'une des électrodes d'application de courant (10e) est placée sur le tronc, l'autre électrode d'application de courant (10d) est placée sur une partie de corps dépassant du tronc, les électrodes de mesure de tension (11c, 11e, 11f, 11g) sont placées au niveau d'une position éloignée où l'influence de la résistance de propagation juste en dessous de l'une des électrodes d'application de courant (10e) est faible lors de l'obtention des informations de tissu adipeux viscéral du tronc, et l'une des électrodes de mesure de tension (11f) est placée au niveau d'une position où l'influence de la résistance de propagation juste en dessous de l'une des électrodes d'application de courant (10e) est prédominante lors de l'obtention des informations de couche de tissu adipeux sous-cutané du tronc.

2. Procédé de mesure de graisse viscérale/sous-cutanée tronculaire selon la revendication 1, dans lequel l'une des électrodes d'application de courant (10e) est placée sur au moins une partie de corps où la couche de tissu adipeux sous-cutané est épaisse et qui est faite pour évaluer un volume de tissu adipeux sous-cutané sur la circonférence abdominale tronculaire,
l'autre électrode d'application de courant (10d) est placée sur une partie de corps dépassant du tronc, l'une des électrodes de mesure de tension (11e, 11f) est placée à proximité de l'une des électrodes d'application de courant (10e) sur la circonférence abdominale tronculaire, et

l'autre électrode de mesure de tension (11c) est placée sur une partie de corps dépassant du tronc qui est différente de la partie de corps sur laquelle l'autre électrode d'application de courant (10d) est placée, pour mesurer l'impédance du tronc de manière à obtenir les informations de couche de tissu adipeux sous-cutané du tronc.

3. Procédé de mesure de graisse viscérale/sous-cutanée tronculaire selon la revendication 1, comprenant en outre les étapes consistant à :

mesurer l'impédance bioélectrique d'une extrémité inférieure, l'impédance bioélectrique d'une extrémité supérieure et l'impédance bioélectrique d'un tronc,

déterminer le volume de tissu musculaire squelettique de l'extrémité inférieure sur la base de l'impédance bioélectrique mesurée de l'extrémité inférieure et sur la base des informations relatives au corps,

déterminer le volume de tissu musculaire squelettique de l'extrémité supérieure sur la base de l'impédance bioélectrique mesurée de l'extrémité supérieure et sur la base des informations relatives au corps,

déterminer le volume de tissu musculaire squelettique du tronc sur la base des volumes de tissu musculaire squelettique déterminés des extrémités inférieure et supérieure ainsi que sur la base des informations relatives au corps,

déterminer l'impédance de la couche de tissu musculaire squelettique du tronc sur la base du volume de tissu musculaire squelettique déterminé du tronc et sur la base des informations relatives au corps,

déterminer l'impédance de la couche de tissu adipeux sous-cutané du tronc en plaçant l'une des électrodes d'application de courant (10e) sur au moins une partie de corps où la couche de tissu adipeux sous-cutané est épaisse et qui est faite pour évaluer un volume de tissu adipeux sous-cutané sur la circonférence abdominale tronculaire, en plaçant l'autre électrode d'application de courant (10d) sur une partie de corps dépassant du tronc, en plaçant l'une des électrodes de mesure de tension (11e) à proximité de l'une des électrodes d'application de courant sur la circonférence abdominale tronculaire, et en plaçant l'autre électrode de mesure de tension (11c) sur une partie de corps dépassant du tronc qui est différente de la partie de corps sur laquelle l'autre électrode d'application de courant (10d) est placée, de manière à déterminer l'impédance de la couche de tissu adipeux sous-cutané du tronc,

déterminer l'impédance du tissu d'organe splanchnique du tronc sur la base des informations relatives au corps,

déterminer l'impédance du tissu adipeux viscéral du tronc sur la base de l'impédance bioélectrique déterminée du tronc, sur la base de l'impédance déterminée de la couche de tissu musculaire squelettique du tronc, sur la base de l'impédance déterminée de la couche de tissu adipeux sous-cutané du tronc et sur la base de l'impédance déterminée du tissu d'organe splanchnique du tronc, et

déterminer le volume de tissu adipeux viscéral du tronc sur la base de l'impédance déterminée du tissu adipeux viscéral du tronc et sur la base des informations relatives au corps.

4. Procédé de mesure de graisse viscérale/sous-cutanée tronculaire selon l'une des revendications 1 à 3, dans lequel une électrode partagée (C) servant d'électrode parmi les électrodes d'application de courant et d'électrode parmi les électrodes de mesure de tension est placée sur le tronc, l'impédance du tronc est mesurée par l'électrode partagée (C), l'autre électrode d'application de courant et l'autre électrode de mesure de tension, l'électrode partagée (C) a une zone d'électrode assez large pour que l'influence de la résistance de zone de contact avec la peau et l'influence de la résistance de propagation juste en dessous d'une électrode d'application de courant puissent être réduites.

5. Appareil de mesure de graisse viscérale/sous-cutanée tronculaire destiné à effectuer un procédé selon l'une des revendications 1 à 4, dans lequel la paire d'électrodes d'application de courant (10d, 10e) est configurée pour appliquer un courant au tronc et la paire d'électrodes de mesure de tension (11c, 11e, 11f, 11g) est configurée pour mesurer une différence de potentiel (V) qui est apparue dans le tronc de manière à obtenir des informations de tissu adipeux viscéral et/ou des informations de couche de tissu adipeux sous-cutané du tronc en mesurant l'impédance du tronc, et dans lequel une section de commutation d'électrode d'application de courant (9) et une section de commutation d'électrode de mesure de tension (12) sont fournies de manière à commuter les électrodes (10d, 10e, 11c, 11e, 11f, 11g) suivant leurs positions sur les parties de corps respectives.

6. Appareil de mesure de graisse viscérale/sous-cutanée tronculaire selon la revendication 5, comprenant en outre :

un moyen de mesure d'impédance bioélectrique d'extrémité inférieure mesurant l'impédance bioélectrique d'une extrémité inférieure,

un moyen de mesure d'impédance bioélectrique d'extrémité supérieure mesurant l'impédance bioélectrique d'une extrémité supérieure,

un moyen de mesure d'impédance bioélectrique de tronc mesurant l'impédance bioélectrique du tronc,

un moyen d'évaluation de volume de tissu musculaire squelettique de tronc évaluant le volume de tissu musculaire squelettique du tronc,

un moyen d'évaluation d'impédance de couche de tissu musculaire squelettique de tronc évaluant l'impédance de la couche de tissu musculaire squelettique du tronc sur la base du volume de tissu musculaire squelettique évalué du tronc et sur la base des informations relatives au corps,

un moyen d'évaluation d'impédance de couche de tissu adipeux sous-cutané de tronc évaluant l'impédance de la couche de tissu adipeux sous-cutané du tronc, le moyen d'évaluation d'impédance de couche de tissu adipeux sous-cutané de tronc comprenant au moins une paire d'électrodes d'application de courant, qui comprennent une électrode d'application de courant, qui est placée sur au moins une partie de corps où la couche de tissu adipeux sous-cutané est épaisse et qui est faite pour évaluer un volume de tissu adipeux sous-cutané sur la circonférence abdominale tronculaire, et l'autre électrode d'application de courant, qui est placée sur une partie de corps dépassant du tronc, ainsi qu'au moins une paire d'électrodes de mesure de tension, qui comprennent une électrode de mesure de tension placée à proximité de l'une des électrodes d'application de courant sur la circonférence abdominale tronculaire, et l'autre électrode de mesure de tension placée sur une partie de corps dépassant du tronc qui est différente de la partie de corps sur laquelle l'autre électrode d'application de courant est placée, et évalue l'impédance de la couche de tissu adipeux sous-cutané du tronc,

un moyen d'évaluation d'impédance de tissu d'organe splanchnique de tronc évaluant l'impédance du tissu d'organe splanchnique du tronc sur la base des informations relatives au corps,

un moyen d'évaluation d'impédance de tissu adipeux viscéral de tronc évaluant l'impédance du tissu adipeux viscéral du tronc sur la base de l'impédance bioélectrique évaluée du tronc, sur la base de l'impédance évaluée de la couche de tissu musculaire squelettique du tronc, sur la base de l'impédance évaluée de la couche de tissu adipeux sous-cutané du tronc et sur la base de l'impédance évaluée du tissu d'organe splanchnique du tronc, et

un moyen d'évaluation de volume de tissu adipeux viscéral de tronc évaluant le volume de tissu adipeux viscéral du tronc sur la base de l'impédance évaluée du tissu adipeux viscéral du tronc et sur la base des informations relatives au corps.

7.  Appareil de mesure de graisse viscérale/sous-cutanée tronculaire selon la revendication 6, dans lequel le moyen d'évaluation de volume de tissu musculaire squelettique de tronc évalue le volume de tissu musculaire squelettique du tronc sur la base des impédances bioélectriques mesurées des extrémités inférieure et supérieure ainsi que sur la base des informations relatives au corps.

8.  Appareil de mesure de graisse viscérale/sous-cutanée tronculaire selon la revendication 6, comprenant en outre :

un moyen d'évaluation de volume de tissu musculaire squelettique d'extrémité inférieure évaluant le volume de tissu musculaire squelettique de l'extrémité inférieure sur la base de l'impédance bioélectrique mesurée de l'extrémité inférieure ainsi que sur la base des informations relatives au corps, et

un moyen d'évaluation de volume de tissu musculaire squelettique d'extrémité supérieure évaluant le volume de tissu musculaire squelettique de l'extrémité supérieure sur la base de l'impédance bioélectrique mesurée de l'extrémité supérieure ainsi que sur la base des informations relatives au corps,

dans lequel le moyen d'évaluation de volume de tissu musculaire squelettique de tronc évalue le volume de tissu musculaire squelettique du tronc sur la base des volumes de tissu musculaire squelettique évalués des extrémités inférieure et supérieure ainsi que sur la base des informations relatives au corps.

9.  Appareil de mesure de graisse viscérale/sous-cutanée tronculaire selon l'une des revendications 5 à 8, dans lequel une électrode partagée (C) servant d'électrode parmi les électrodes d'application de courant et d'électrode parmi les électrodes de mesure de tension est placée sur le tronc, l'impédance du tronc est mesurée par l'électrode partagée (C), l'autre électrode d'application de courant et l'autre électrode de mesure de tension, et l'électrode partagée (C) a une zone d'électrode assez large pour que l'influence de la résistance de zone de contact avec la peau et l'influence de la résistance de propagation juste en dessous d'une électrode d'application de courant puissent être réduites.

# FIG.1

# FIG.2

**3** BODY PART IMPEDANCE MEASURING SECTION

**10**:CURRENT APPLYING ELECTRODES    **11**:VOLTAGE MEASURING ELECTRODES

10a 10b 10c 10n    11a 11b 11c 11n

**2**

BODY WEIGHT MEASURING SECTION

CURRENT APPLYING ELECTRODE SWITCHING SECTION    **9**

VOLTAGE MEASURING ELECTRODE SWITCHING SECTION    **12**

VOLTAGE MEASURING SECTION    **13**

**8**:CURRENT SUPPLY SECTION

**1**

POWER SUPPLY SECTION

COMPUTATION/CONTROL SECTION

STORAGE SECTION

**4**

**7**

**6** PRINT SECTION

**5** DISPLAY/INPUT SECTION

INPUT SECTION **5a**    DISPLAY SECTION **5b**

BUZZER ALARMING SECTION **15**

# FIG.3

# FIG.4

# FIG.5A

# FIG.5B

# FIG.5C

# FIG.5D

# FIG.6

VISCERAL
FAT TISSUE: FV

SPLANCHNIC
ORGAN TISSUE: VM

SKELETAL MUSCLE
TISSUE LAYER: MM

SUBCUTANEOUS
FAT TISSUE LAYER: FS

# FIG.7

# FIG.8

# FIG.9

# FIG.10

# FIG.11

## FIG.12

## FIG.13

# FIG.14

## FIG.15

## FIG.16

## FIG.17

## FIG.18

## FIG.19

## FIG.20

## FIG.21

## FIG.22

## FIG.23

## FIG.24

## FIG.25

## FIG.26

FIG.27

EP 1 741 385 B1

## FIG.28

## FIG.29

# FIG.30

EP 1 741 385 B1

# FIG.31

EP 1 741 385 B1

## FIG.32

## FIG.33

# FIG.34

EP 1 741 385 B1

# FIG.35

EP 1 741 385 B1

# FIG.36

# FIG.37

## FIG.38

## FIG.39

# FIG.40

# FIG.41

# FIG.42

SKELETAL
MUSCLE
TISSUE LAYER
IMPEDANCE
ZMM

UPPER-EXTREMITY
IMPEDANCE
Zh

SKELETAL
MUSCLE
TISSUE LAYER
IMPEDANCE
ZMM

SPLANCHNIC
ORGAN
TISSUE
IMPEDANCE
ZVM

VISCERAL
FAT TISSUE
IMPEDANCE
ZFV

Ztm

IMPEDANCE ZMM1
OF SKELETAL
MUSCLE TISSUE
LAYER WHERE
RECTUS ABDOMINIS
MUSCLE IS DOMINANT

APONEUROSIS
ZAM1

IMPEDANCE ZMM2 OF
SKELETAL MUSCLE
TISSUE LAYER IN BACK
MUSCLE OR OBLIQUE
ABDOMINAL MUSCLE

APONEUROSIS
ZAM2

UPPER-EXTREMITY
IMPEDANCE
Zh

SPLANCHNIC
ORGAN
TISSUE
IMPEDANCE
ZVM

VISCERAL
FAT TISSUE
IMPEDANCE
ZFV

Ztm

LOWER-EXTREMITY
IMPEDANCE
Zf

LOWER-EXTREMITY
IMPEDANCE
Zf

107

EP 1 741 385 B1

# FIG.43

I=I11+I21

UPPER-EXTREMITY IMPEDANCE Zh

I ↓

I21 ←    → I11

SKELETAL MUSCLE TISSUE LAYER IMPEDANCE ZMM

IMPEDANCE ZMM1 OF SKELETAL MUSCLE TISSUE LAYER WHERE RECTUS ABDOMINIS MUSCLE IS DOMINANT

APONEUROSIS ZAM1

IMPEDANCE ZMM2 OF SKELETAL MUSCLE TISSUE LAYER IN BACK MUSCLE OR OBLIQUE ABDOMINAL MUSCLE

APONEUROSIS ZAM2

SPLANCHNIC ORGAN TISSUE IMPEDANCE ZVM

VISCERAL FAT TISSUE IMPEDANCE ZFV

LOWER-EXTREMITY IMPEDANCE Zf

UPPER-EXTREMITY IMPEDANCE Zh

I ↓

I21 ←    → I11

SKELETAL MUSCLE TISSUE LAYER IMPEDANCE ZMM

IMPEDANCE ZMM1 OF SKELETAL MUSCLE TISSUE LAYER WHERE RECTUS ABDOMINIS MUSCLE IS DOMINANT

SPLANCHNIC ORGAN TISSUE IMPEDANCE ZVM

VISCERAL FAT TISSUE IMPEDANCE ZFV

LOWER-EXTREMITY IMPEDANCE Zf

EP 1 741 385 B1

# FIG.44

EP 1 741 385 B1

I=I12+I22

UPPER-EXTREMITY IMPEDANCE Zh

I↓

I22 ← → I12

Ⓘ

SKELETAL MUSCLE TISSUE LAYER IMPEDANCE ZMM

APONEUROSIS ZAM1

SPLANCHNIC ORGAN TISSUE IMPEDANCE ZVM

IMPEDANCE ZMM1 OF SKELETAL MUSCLE TISSUE LAYER WHERE RECTUS ABDOMINIS MUSCLE IS DOMINANT

IMPEDANCE ZMM2 OF SKELETAL MUSCLE TISSUE LAYER IN BACK MUSCLE OR OBLIQUE ABDOMINAL MUSCLE

Ⓥ

APONEUROSIS ZAM2

VISCERAL FAT TISSUE IMPEDANCE ZFV

LOWER-EXTREMITY IMPEDANCE Zf

---

UPPER-EXTREMITY IMPEDANCE Zh

I↓

I22 ← → I12

Ⓘ

SKELETAL MUSCLE TISSUE LAYER IMPEDANCE ZMM

APONEUROSIS ZAM1

SPLANCHNIC ORGAN TISSUE IMPEDANCE ZVM

IMPEDANCE ZMM1 OF SKELETAL MUSCLE TISSUE LAYER WHERE RECTUS ABDOMINIS MUSCLE IS DOMINANT

IMPEDANCE ZMM2 OF SKELETAL MUSCLE TISSUE LAYER IN BACK MUSCLE OR OBLIQUE ABDOMINAL MUSCLE

Ⓥ

APONEUROSIS ZAM2

VISCERAL FAT TISSUE IMPEDANCE ZFV

$I = I13 + I23$

**UPPER-EXTREMITY IMPEDANCE Zh**

**SKELETAL MUSCLE TISSUE LAYER IMPEDANCE ZMM**

**APONEUROSIS ZAM1**

**SPLANCHNIC ORGAN TISSUE IMPEDANCE ZVM**

**IMPEDANCE ZMM1 OF SKELETAL MUSCLE TISSUE LAYER WHERE RECTUS ABDOMINIS MUSCLE IS DOMINANT**

**IMPEDANCE ZMM2 OF SKELETAL MUSCLE TISSUE LAYER IN BACK MUSCLE OR OBLIQUE ABDOMINAL MUSCLE**

I   V

**VISCERAL FAT TISSUE IMPEDANCE ZFV**

**APONEUROSIS ZAM2**

I23   I13

**LOWER-EXTREMITY IMPEDANCE Zf**

I

---

**SKELETAL MUSCLE TISSUE LAYER IMPEDANCE ZMM**

**APONEUROSIS ZAM1**

**SPLANCHNIC ORGAN TISSUE IMPEDANCE ZVM**

I

**IMPEDANCE ZMM1 OF SKELETAL MUSCLE TISSUE LAYER WHERE RECTUS ABDOMINIS MUSCLE IS DOMINANT**

**IMPEDANCE ZMM2 OF SKELETAL MUSCLE TISSUE LAYER IN BACK MUSCLE OR OBLIQUE ABDOMINAL MUSCLE**

V

**VISCERAL FAT TISSUE IMPEDANCE ZFV**

**APONEUROSIS ZAM2**

I23   I13

**LOWER-EXTREMITY IMPEDANCE Zf**

I

EP 1 741 385 B1

# FIG.46A

# FIG.46B

# FIG.46C

## FIG.47A

## FIG.47B

## FIG.47C

## FIG.48A

10f

I7

V7

11e

11f

10e

## FIG.48B

10f

11f

I8

V8

10e

11e

## FIG.48C

10f

I7

V9"

V9

V9'

V9'"

11e

11d

11f

11c

10e

# FIG.49A

# FIG.49B

# FIG.50A

# FIG.50B

# FIG.51A

# FIG.51B

# FIG.51C

# FIG.51D

# FIG.51E

## FIG.52

## FIG.53

# FIG.54A

# FIG.54B

# FIG.54C

FIG.55

FIG.56

FIG.57

# FIG.58

# FIG.59

## FIG.60

S1 — ( START )

S2 — INPUT BODY SPECIFYING INFORMATION

S3 — INPUT MORPHOMETRICAL VALUES SUCH AS EXTREMITY LENGTH, TRUNK LENGTH AND ABDOMINAL CIRCUMFERENTIAL LENGTH ?

→ YES → S4 — INPUT MORPHOMETRICAL VALUE INFORMATION

↓ NO

S5 — PERFORM MORPHOMETRICAL INFORMATION ESTIMATION PROCESS

S6 — PERFORM EXTREMITY AND TRUNK IMPEDANCE (Zx) MEASUREMENT PROCESS

S7 — PERFORM BODY COMPOSITION INFORMATION (SUCH AS BODY FAT PERCENTAGE) COMPUTATION PROCESS

S8 — PERFORM EXTREMITY SKELETAL MUSCLE TISSUE VOLUME ESTIMATION PROCESS

S9 — PERFORM MID-TRUNK SKELETAL MUSCLE TISSUE VOLUME (MMtm) ESTIMATION PROCESS

S10 — PERFORM MID-TRUNK SKELETAL MUSCLE TISSUE LAYER IMPEDANCE (ZMM) ESTIMATION PROCESS

S11 — PERFORM SUBCUTANEOUS FAT TISSUE VOLUME (FS) AND SUBCUTANEOUS FAT TISSUE LAYER IMPEDANCE (ZFS) ESTIMATION PROCESS

S12 — PERFORM SPLANCHNIC ORGAN TISSUE VOLUME (VM) AND SPLANCHNIC ORGAN TISSUE IMPEDANCE (ZVM) ESTIMATION PROCESS

S13 — PERFORM VISCERAL FAT TISSUE IMPEDANCE (ZFV) AND VISCERAL FAT TISSUE VOLUME (FV) ESTIMATION PROCESS

S14 — PERFORM VISCERAL FAT/SUBCUTANEOUS FAT RATIO (V/S) COMPUTATION PROCESS

S15 — PERFORM VISCERAL FAT PERCENTAGE (%VFat) COMPUTATION PROCESS

S16 — DISPLAY VISCERAL FAT TISSUE INFORMATION (FV, %VFat), BODY COMPOSITION INFORMATION (%Fat, MMI, MMu, MMtm), AND ADVICE GUIDANCE

( END ) — S17

120

**FIG.61**

```
┌──────────────────────────────────────────────────────────┐
( SUBCUTANEOUS FAT TISSUE VOLUME (FS) AND SUBCUTANEOUS      )
( FAT TISSUE LAYER IMPEDANCE (ZFS) ESTIMATION PROCESS       )
└──────────────────────────────────────────────────────────┘
```

S18 → PERFORM SUBCUTANEOUS FAT TISSUE VOLUME (FS) ESTIMATION PROCESS

S19 → PERFORM SUBCUTANEOUS FAT TISSUE LAYER IMPEDANCE (ZFS) ESTIMATION PROCESS

RETURN

**FIG.62**

SPLANCHNIC ORGAN TISSUE VOLUME (VM) AND SPLANCHNIC ORGAN TISSUE IMPEDANCE (ZVM) ESTIMATION PROCESS

S20 → PERFORM SPLANCHNIC ORGAN TISSUE VOLUME (VM) ESTIMATION PROCESS

S21 → PERFORM SPLANCHNIC ORGAN TISSUE IMPEDANCE (ZVM) ESTIMATION PROCESS

RETURN

**FIG.63**

VISCERAL FAT TISSUE IMPEDANCE (ZFV) AND VISCERAL FAT TISSUE VOLUME (FV) ESTIMATION PROCESS

S22 → PERFORM VISCERAL FAT TISSUE IMPEDANCE (ZFV) ESTIMATION PROCESS

S23 → PERFORM VISCERAL FAT TISSUE VOLUME (FV) ESTIMATION PROCESS

RETURN

# FIG.64

```
┌─────────────────────────────────────────────────┐
│ EXTREMITY AND TRUNK IMPEDANCE (Zx)                │
│ MEASUREMENT PROCESS (1)                           │
└─────────────────────────────────────────────────┘
                        │
S24 ──┤ INITIALIZATION    F ← "0" │
                        │
S25 ──◇ MEASUREMENT TIMING ?  SAMPLING PERIOD EXAMPLE : Ts = 0.5S ◇── NO ──→ Zx = (Zx-1 +Zx)/2  S32
                        │ YES                                                  │
S26 ──┌──────────────────────────────────┐          ┌──────────────────────────────┐
      │ PERFORM MID-TRUNK IMPEDANCE (Ztm) │          │ PERFORM MEASURED              │
      │ MEASURING ELECTRODE               │          │ IMPEDANCE (Zx) DATA           │
      │ PLACEMENT SETTING PROCESS         │          │ SMOOTHING PROCESS             │
      │                                   │          │ (e.g. MOVING AVERAGE PROCESS) │
      │ PERFORM MID-TRUNK IMPEDANCE (Ztmx)│          └──────────────────────────────┘ S33
      │ MEASUREMENT PROCESS               │                       │
      └──────────────────────────────────┘          ┌──────────────────────────────┐
                        │                            │ PERFORM MID-TRUNK             │
S27 ──◇ F="0"?                                       │ IMPEDANCE MEASURED DATA       │
       LAST MEASURED UPPER ──── NO ──────────┐       │ BREATHING CHANGE              │
       EXTREMITY REGION ?                    │       │ CORRECTION PROCESS            │
                        │ YES                │       └──────────────────────────────┘
                        │                    │                    (A)
S30 ──┌──────────────────────────┐    ┌──────────────────────────┐ S28
      │ PERFORM LOWER EXTREMITY   │    │ PERFORM UPPER EXTREMITY   │
      │ REGION IMPEDANCE (Zl)     │    │ REGION IMPEDANCE (Zu)     │
      │ MEASURING ELECTRODE       │    │ MEASURING ELECTRODE       │
      │ PLACEMENT SETTING PROCESS │    │ PLACEMENT SETTING PROCESS │
      │                           │    │                           │
      │ PERFORM LOWER EXTREMITY   │    │ PERFORM UPPER EXTREMITY    │
      │ REGION IMPEDANCE (Zlx)    │    │ REGION IMPEDANCE (Zux)     │
      │ MEASUREMENT PROCESS       │    │ MEASUREMENT PROCESS        │ S29
      └──────────────────────────┘    └──────────────────────────┘
S31 ──┤ F ← "1" │               S29 ──┤ F ← "0" │
                        │                    │
                       (A)
                        │
S34 ──┌──────────────────────────────────┐
      │ PERFORM TIME-SERIES STABILITY     │
      │ CONFIRMATION PROCESS OF MEASURED  │
      │ IMPEDANCE OF EACH BODY PART       │
      └──────────────────────────────────┘
                        │
S35 ──◇ Zlx, Zux and Ztmx SATISFY STABLE CONDITION ? ◇── YES ──→
       NO                                                        S36
                                              ┌──────────────────────────────┐
                                              │ REGISTER FINAL STABLE          │
                                              │ CONDITION DETERMINED VALUE     │
                                              │ AS MEASUREMENT RESULT VALUE    │ S37
                                              └──────────────────────────────┘
                                              ┌──────────────────────────────┐
                                              │ PERFORM ABNORMAL VALUE         │
                                              │ DETERMINATION PROCESS BY       │
                                              │ DRINKING AND EATING AND        │
                                              │ RETENTION OF                   │
                                              │ URINE IN BLADDER               │
                                              └──────────────────────────────┘
                                                       │
                                                   ( RETURN )
```

EXTREMITY AND TRUNK IMPEDANCE (Zx) MEASUREMENT PROCESS (1)

S24 — INITIALIZATION    F ← "0"

S25 — MEASUREMENT TIMING ?  SAMPLING PERIOD EXAMPLE : Ts = 0.5S — NO

$Zx = (Zx-1 + Zx)/2$   S32

S26 — PERFORM MID-TRUNK IMPEDANCE (Ztm) MEASURING ELECTRODE PLACEMENT SETTING PROCESS → PERFORM MID-TRUNK IMPEDANCE (Ztmx) MEASUREMENT PROCESS — YES

S32 — PERFORM MEASURED IMPEDANCE (Zx) DATA SMOOTHING PROCESS (e.g. MOVING AVERAGE PROCESS)

S33 — PERFORM MID-TRUNK IMPEDANCE MEASURED DATA BREATHING CHANGE CORRECTION PROCESS

S27 — F="0"? LAST MEASURED UPPER EXTREMITY REGION ? — NO

S30 — PERFORM LOWER EXTREMITY REGION IMPEDANCE (Zl) MEASURING ELECTRODE PLACEMENT SETTING PROCESS → PERFORM LOWER EXTREMITY REGION IMPEDANCE (Zlx) MEASUREMENT PROCESS

S28 — PERFORM UPPER EXTREMITY REGION IMPEDANCE (Zu) MEASURING ELECTRODE PLACEMENT SETTING PROCESS → PERFORM UPPER EXTREMITY REGION IMPEDANCE (Zux) MEASUREMENT PROCESS

S31 — F ← "1"

S29 — F ← "0"

S34 — PERFORM TIME-SERIES STABILITY CONFIRMATION PROCESS OF MEASURED IMPEDANCE OF EACH BODY PART

S35 — Zlx, Zux and Ztmx SATISFY STABLE CONDITION ? — NO / YES

S36 — REGISTER FINAL STABLE CONDITION DETERMINED VALUE AS MEASUREMENT RESULT VALUE

S37 — PERFORM ABNORMAL VALUE DETERMINATION PROCESS BY DRINKING AND EATING AND RETENTION OF URINE IN BLADDER

RETURN

# FIG.65

```
    ┌──────────────────────────────────────┐
    │  MID-TRUNK IMPEDANCE MEASURED         │
    │  DATA BREATHING CHANGE CORRECTION     │
    │  PROCESS                              │
    └──────────────────────────────────────┘
                        │
    S38 ─┐  ┌────────────────────────────┐
         │  │ PERFORM STRANGE CLIMAX      │
         └─ │ DETECTION PROCESS           │
            │ FROM TIME-SERIES DATA       │
            └────────────────────────────┘
                        │
    S39 ─┐           ◇ STRANGE CLIMAX ?
      NO └────────◇
                        │ YES
    S40 ─┐      ◇ MAXIMUM VALUE ?  ── NO ──┐
                        │ YES                │
                                            │
    S42 ─┐ ┌─────────────────────┐   ┌──────────────────────┐ ─ S41
         │ │ PERFORM MAXIMUM VALUE│   │ PERFORM MINIMUM VALUE │
         └─│ DETERMINED DATA      │   │ DETERMINED DATA       │
           │ MOVING AVERAGING     │   │ MOVING AVERAGING      │
           │ PROCESS              │   │ PROCESS               │
           └─────────────────────┘   └──────────────────────┘
                        │
    S43 ─┐   ◇ MAXIMUM VALUE AND MINIMUM
         │     VALUE DATA FOR ONE BREATHING ── NO ──┐
         └───  CYCLE SECURED ?                       │
                        │ YES                        │
    S44 ─┐ ┌──────────────────────────────┐         │
         │ │ PERFORM BREATHING CHANGE      │         │
         └─│ MEDIAN VALUE COMPUTATION      │         │
           │ PROCESS (AVERAGE VALUE        │         │
           │ COMPUTATION OF MAXIMUM VALUE  │         │
           │ AND MINIMUM VALUE DATA)       │         │
           └──────────────────────────────┘         │
                        │                            │
                    ┌──────────┐
                    │  RETURN  │
                    └──────────┘
```

# FIG.66

ABNORMAL VALUE DETERMINATION
PROCESS BY DRINKING AND EATING AND
RETENTION OF URINE IN BLADDER

S45 — CHECK WHETHER MID-TRUNK
IMPEDANCE (Ztm) IS WITHIN
NORMAL ALLOWABLE RANGE

S46 — WITHIN NORMAL
ALLOWABLE RANGE ?

NO

S47

YES

S48 — PERFORM PROCESS OF REPORTING
MESSAGE THAT CONDITION OF
MIDDLE PORTION (ABDOMEN) OF
TRUNK IS NORMAL AND
BUZZER ALARMING PROCESS

PERFORM PROCESS OF REPORTING
MESSAGE THAT CONDITION OF
MIDDLE PORTION (ABDOMEN) OF
TRUNK IS ABNORMAL AND
BUZZER ALARMING PROCESS

RETURN

## FIG.67

EXTREMITY AND TRUNK IMPEDANCE (Zx)
MEASUREMENT PROCESS (2)

S49 — MEASUREMENT OF TRUNK

MEASUREMENT TIMING ?
SAMPLING PERIOD EXAMPLE: Ts = 0.5S — NO

YES

S50 — PERFORM MID-TRUNK IMPEDANCE (Ztmrr) MEASURING ELECTRODE PLACEMENT SETTING PROCESS: PASS CURRENT BETWEEN RIGHT ARM AND RIGHT LEG
↓
PERFORM MID-TRUNK IMPEDANCE (Ztmrrx) MEASUREMENT PROCESS

S51 — PERFORM MID-TRUNK IMPEDANCE (Ztmll) MEASURING ELECTRODE PLACEMENT SETTING PROCESS: PASS CURRENT BETWEEN LEFT ARM AND LEFT LEG
↓
PERFORM MID-TRUNK IMPEDANCE (Ztmllx) MEASUREMENT PROCESS

S52 — PERFORM MID-TRUNK IMPEDANCE (Ztmrl) MEASURING ELECTRODE PLACEMENT SETTING PROCESS: PASS CURRENT BETWEEN RIGHT ARM AND LEFT LEG
↓
PERFORM MID-TRUNK IMPEDANCE (Ztmrlx) MEASUREMENT PROCESS

S53 — PERFORM MID-TRUNK IMPEDANCE (Ztmlr) MEASURING ELECTRODE PLACEMENT SETTING PROCESS: PASS CURRENT BETWEEN LEFT ARM AND RIGHT LEG
↓
PERFORM MID-TRUNK IMPEDANCE (Ztmlrx) MEASUREMENT PROCESS

S54 — PERFORM MEASURED IMPEDANCE (Zx) DATA SMOOTHING PROCESS (e.g. MOVING AVERAGE PROCESS)

S55 — PERFORM MID-TRUNK IMPEDANCE MEASURED DATA BREATHING CHANGE CORRECTION PROCESS FOR EACH LEAD SYSTEM

S56 — PERFORM TIME-SERIES STABILITY CONFIRMATION PROCESS OF MEASURED IMPEDANCE FOR EACH LEAD SYSTEM

S57 — Ztmx SATISFY STABLE CONDITION FOR EACH LEAD SYSTEM ? — NO

YES

S58 — PERFORM PROCESS OF DETERMINING ABNORMALITY IN TRUNK ABDOMINAL SPLANCHNIC ORGAN TISSUE AND THE LIKE

S59 — REGISTER FINAL STABLE CONDITION DETERMINED VALUE AS MEASUREMENT RESULT VALUE

S63 — PERFORM MEASURED IMPEDANCE (Zx) DATA SMOOTHING PROCESS (e.g. MOVING AVERAGE PROCESS)

S64 — PERFORM TIME-SERIES STABILITY CONFIRMATION PROCESS OF MEASURED IMPEDANCE OF EACH BODY PART

S65 — Zlx and Zux SATISFY STABLE CONDITION ? — NO

YES

S66 — REGISTER FINAL STABLE CONDITION DETERMINED VALUE AS MEASUREMENT RESULT VALUE

MEASUREMENT OF EXTREMITIES

S60 — MEASUREMENT TIMING ? SAMPLING PERIOD EXAMPLE : Ts = 0.5S — NO

YES

S61 — PERFORM LOWER EXTREMITY REGION IMPEDANCE (Zl) MEASURING ELECTRODE PLACEMENT SETTING PROCESS
↓
PERFORM LOWER EXTREMITY REGION IMPEDANCE (Zlx) MEASUREMENT PROCESS

S62 — PERFORM UPPER EXTREMITY REGION IMPEDANCE (Zu) MEASURING ELECTRODE PLACEMENT SETTING PROCESS
↓
PERFORM UPPER EXTREMITY REGION IMPEDANCE (Zux) MEASUREMENT PROCESS

RETURN

# FIG.68

```
( PROCESS OF DETERMINING ABNORMALITY IN TRUNK
  ABDOMINAL SPLANCHNIC ORGAN TISSUE AND THE LIKE )
```

S67 → CHECK WHETHER RELATIONSHIP BETWEEN MID-TRUNK IMPEDANCES (Ztm) BY EACH LEAD SYSTEM SATISFY NORMAL BALANCE CONDITION

S68 → SATISFY NORMAL CONDITION ? — YES / NO

S80 → PERFORM PROCESS OF REPORTING MESSAGE THAT CONDITION OF MIDDLE PORTION (ABDOMEN) OF TRUNK IS NORMAL AND BUZZER ALARMING PROCESS

$Ztmlr \fallingdotseq Ztmll$ and $Ztmrr \fallingdotseq Ztmrl$ — S69

S70 → SATISFY NORMAL CONDITION ? — YES / NO

S71 → $Ztmrl < Ztmrr$

S72 → SATISFY NORMAL CONDITION ? — YES / NO

S78 → INFORM MEASUREMENT RESULT DISPLAY AND THE LIKE OF "ABNORMALITY IN CONDITION OF UPPER RIGHT REGION OF TRUNK"

S77 → INFORM MEASUREMENT RESULT DISPLAY AND THE LIKE OF "ABNORMALITY IN CONDITION OF LOWER LEFT REGION OF TRUNK"

S73 → $Ztmrl > Ztmrr$

S74 → SATISFY NORMAL CONDITION ? — YES / NO

S76 → INFORM MEASUREMENT RESULT DISPLAY AND THE LIKE OF "ABNORMALITY IN CONDITION OF LOWER RIGHT REGION OF TRUNK"

S75 → INFORM MEASUREMENT RESULT DISPLAY AND THE LIKE OF "ABNORMALITY IN CONDITION OF UPPER LEFT REGION OF TRUNK"

S79 → PERFORM PROCESS OF REPORTING MESSAGE THAT CONDITION OF MIDDLE PORTION (ABDOMEN) OF TRUNK IS ABNORMAL AND BUZZER ALARMING PROCESS

( RETURN )

# FIG.69

S81 — ( START )

S82 → INPUT BODY SPECIFYING INFORMATION
(SUCH AS BODY HEIGHT H,
BODY WEIGHT W, SEX, AGE)

S83 → INPUT
MORPHOMETRICAL VALUES
SUCH AS TRUNK LENGTH AND ABDOMINAL
CIRCUMFERENTIAL
LENGTH ?

— YES → S84 → INPUT MORPHOMETRICAL
VALUE INFORMATION

NO

S85 → PERFORM MORPHOMETRICAL
INFORMATION ESTIMATION PROCESS

S86 → PERFORM TRUNK IMPEDANCE (Zx)
MEASUREMENT PROCESS

S87 → PERFORM TRUNK SKELETAL MUSCLE
TISSUE CROSS-SECTIONAL AREA
(AMM) ESTIMATION PROCESS

S88 → PERFORM TRUNK SKELETAL MUSCLE
TISSUE LAYER IMPEDANCE (ZMM)
ESTIMATION PROCESS

S89 → PERFORM SUBCUTANEOUS
FAT TISSUE VOLUME (AFS)
ESTIMATION PROCESS

S90 → PERFORM SPLANCHNIC ORGAN
TISSUE VOLUME (AVM) AND
SPLANCHNIC ORGAN TISSUE
IMPEDANCE (ZVM)
ESTIMATION PROCESS

S91 → PERFORM VISCERAL FAT TISSUE
IMPEDANCE (ZFV) AND VISCERAL
FAT TISSUE VOLUME (AFV)
ESTIMATION PROCESS

S92 → PERFORM VISCERAL FAT/
SUBCUTANEOUS FAT RATIO (V/S)
COMPUTATION PROCESS

S93 → PERFORM BODY MASS INDEX (BMI)
COMPUTATION PROCESS

PERFORM TRUNK BODY FAT
PERCENTAGE (%Fatt)
COMPUTATION PROCESS — S94

PERFORM VISCERAL FAT
PERCENTAGE (%VFat)
COMPUTATION PROCESS — S95

DISPLAY VISCERAL FAT
INFORMATION (AFV, %VFat),
BODY COMPOSITION
INFORMATION
(%Fatt, AMM, AFS, AVM),
BMI, AND ADVICE GUIDANCE — S96

( END ) — S97

127

# FIG.70

```
┌──────────────────────────────────────────┐
│   SPLANCHNIC ORGAN TISSUE VOLUME (AVM)     │
│      AND SPLANCHNIC ORGAN TISSUE           │
│  IMPEDANCE (ZVM) ESTIMATION PROCESS        │
└──────────────────────────────────────────┘
                     │
                     ▼
S98 ┌──────────────────────────────────────┐
    │  PERFORM SPLANCHNIC ORGAN TISSUE       │
    │   VOLUME (AVM) ESTIMATION PROCESS      │
    └──────────────────────────────────────┘
                     │
                     ▼
S99 ┌──────────────────────────────────────┐
    │  PERFORM SPLANCHNIC ORGAN TISSUE       │
    │  IMPEDANCE (ZVM) ESTIMATION PROCESS    │
    └──────────────────────────────────────┘
                     │
                     ▼
               (  RETURN  )
```

# FIG.71

```
┌──────────────────────────────────────────┐
│   VISCERAL FAT TISSUE IMPEDANCE (ZFV)      │
│  AND VISCERAL FAT TISSUE VOLUME (AFV)      │
│          ESTIMATION PROCESS                │
└──────────────────────────────────────────┘
                     │
                     ▼
S100 ┌──────────────────────────────────────┐
     │    PERFORM VISCERAL FAT TISSUE         │
     │  IMPEDANCE (ZFV) ESTIMATION PROCESS    │
     └──────────────────────────────────────┘
                     │
                     ▼
S101 ┌──────────────────────────────────────┐
     │    PERFORM VISCERAL FAT TISSUE         │
     │   VOLUME (AFV) ESTIMATION PROCESS      │
     └──────────────────────────────────────┘
                     │
                     ▼
               (  RETURN  )
```

# FIG.72

```
( TRUNK IMPEDANCE (Zx) MEASUREMENT PROCESS )
                         │
S102 ─┐                  ▼
     ┌──────────────────────────────────────────┐
     │ PERFORM INITIALIZATION OF COUNTER, ETC.   │
     └──────────────────────────────────────────┘
                         │
  S103 ─┐                ▼
      ┌────────────────────────────┐   NO           Zx = (Zx-1 +Zx)/2
     ◇  MEASUREMENT                  ◇──────────────┐        ─┐ S106
      ◇ TIMING ? SAMPLING PERIOD    ◇               ▼
       ◇     EXAMPLE               ◇     ┌────────────────────────────┐
        └──────────────────────────┘     │ PERFORM MEASURED IMPEDANCE │
S104 ─┐         YES │                     │ (Zx) DATA SMOOTHING PROCESS│
     ┌──────────────▼───────────────┐     │ (e.g. MOVING AVERAGE PROCESS)│
     │ PERFORM TRUNK IMPEDANCE (Ztm) │     └────────────────────────────┘
     │ MEASURING ELECTRODE PLACEMENT │                  │       ─┐ S107
     │ SETTING PROCESS               │                  ▼
     │          ↓                    │     ┌────────────────────────────┐
     │ PERFORM TRUNK IMPEDANCE (Ztmx)│     │ PERFORM TRUNK IMPEDANCE    │
     │ MEASUREMENT PROCESS           │     │ MEASURED DATA BREATHING    │
S105 ─┐                              │     │ CHANGE CORRECTION PROCESS  │
     ┌──────────────────────────────┐     └────────────────────────────┘
     │ PERFORM SUBCUTANEOUS FAT TISSUE│                 │       ─┐ S108
     │ LAYER IMPEDANCE (ZFS) MEASURING│                 ▼
     │ ELECTRODE PLACEMENT SETTING    │     ┌────────────────────────────┐
     │ PROCESS                        │     │ PERFORM TIME-SERIES STABILITY│
     │           ↓                    │     │ CONFIRMATION PROCESS OF    │
     │ PERFORM SUBCUTANEOUS FAT TISSUE│     │ MEASURED IMPEDANCE         │
     │ LAYER IMPEDANCE (ZFSx)         │     └────────────────────────────┘
     │ MEASUREMENT PROCESS            │                 │
     └────────────────────────────────┘                ▼        ─┐ S109
              │            NO            ◇ Ztmx and ZFSx SATISFY ◇
              └───────────────────────── ◇ STABLE CONDITION ?    ◇
                                          └───────────────────────┘
                                                YES │    ─┐ S110
                                          ┌────────▼───────────────┐
                                          │ REGISTER FINAL STABLE   │
                                          │ CONDITION DETERMINED    │
                                          │ VALUE AS MEASUREMENT    │
                                          │ RESULT VALUE            │
                                          └─────────────────────────┘
                                                    │     ─┐ S111
                                          ┌─────────▼────────────────┐
                                          │ PERFORM ABNORMAL VALUE    │
                                          │ DETERMINATION PROCESS BY  │
                                          │ DRINKING AND EATING AND   │
                                          │ RETENTION OF URINE IN     │
                                          │ BLADDER                   │
                                          └───────────────────────────┘
                                                    │     ─┐ S112
                                          ┌─────────▼────────────────┐
                                          │ PERFORM MEASUREMENT       │
                                          │ COMPLETION REPORTING      │
                                          │ PROCESS                   │
                                          └───────────────────────────┘
                                                    │
                                                    ▼
                                              ( RETURN )
```

# FIG.73

```
┌─────────────────────────────────────────────┐
│ SUBCUTANEOUS FAT TISSUE VOLUME (AFS)          │
│          ESTIMATION PROCESS                    │
└─────────────────────────────────────────────┘
                      │
                      ▼
           ┌──────────────────────────┐
S113───────│ PERFORM SUBCUTANEOUS FAT │
           │    TISSUE VOLUME (AFS)    │
           │    ESTIMATION PROCESS     │
           └──────────────────────────┘
                      │
                      ▼
                ( RETURN )
```

# FIG.74

# FIG.75

FIG.76

# FIG.77

# FIG.78

FIG.79

FIG.80

FIG.81

FIG.82

FIG.83

FIG.84

# FIG.85

EP 1 741 385 B1

## FIG.86

SA1 — ( START )

SA2 — INPUT BODY SPECIFYING INFORMATION
(SUCH AS BODY HEIGHT H,
BODY WEIGHT W, SEX, AGE)

SA3 — INPUT
MORPHOMETRICAL VALUES
SUCH AS TRUNK LENGTH AND ABDOMINAL
CIRCUMFERENTIAL
LENGTH ?

YES → SA4 — INPUT MORPHOMETRICAL
VALUE INFORMATION

NO

SA5 — PERFORM MORPHOMETRICAL
INFORMATION ESTIMATION PROCESS

SA6 — PERFORM TRUNK IMPEDANCE (Zx)
MEASUREMENT PROCESS

SA7 — PERFORM TRUNK SKELETAL
MUSCLE TISSUE CROSS-SECTIONAL
AREA (AMM) ESTIMATION PROCESS

SA8 — PERFORM TRUNK SKELETAL MUSCLE
TISSUE LAYER IMPEDANCE (ZMM)
ESTIMATION PROCESS

SA9 — PERFORM SUBCUTANEOUS FAT TISSUE
VOLUME (AFS) ESTIMATION PROCESS

SA10 — PERFORM SPLANCHNIC ORGAN
TISSUE VOLUME (AVM) AND
SPLANCHNIC ORGAN TISSUE
IMPEDANCE (ZVM)
ESTIMATION PROCESS

SA11 — PERFORM VISCERAL FAT TISSUE
IMPEDANCE (ZFV) AND VISCERAL FAT
TISSUE VOLUME (AFV)
ESTIMATION PROCESS

SA12 — PERFORM VISCERAL FAT/SUBCUTANEOUS
FAT RATIO (V/S) COMPUTATION PROCESS

SA13 — PERFORM BODY MASS INDEX (BMI)
COMPUTATION PROCESS

PERFORM TRUNK BODY FAT
PERCENTAGE (%Fatt)
COMPUTATION PROCESS — SA14

PERFORM VISCERAL FAT
PERCENTAGE (%VFat)
COMPUTATION PROCESS — SA15

DISPLAY VISCERAL
FAT TISSUE
INFORMATION (AFV, %VFat),
BODY COMPOSITION
INFORMATION
(%Fatt, AMM, AFS, AVM), BMI,
AND ADVICE GUIDANCE — SA16

( END ) — SA17

# FIG.87

```
( TRUNK IMPEDANCE (Zx) MEASUREMENT PROCESS )
                         │
SA22 ⌐                   ▼
┌─────────────────────────────────────────────┐
│    PERFORM INITIALIZATION OF COUNTER, ETC.    │
└─────────────────────────────────────────────┘
                         │
SA23 ⌐                   ▼
        ◇─────────────────────────◇   NO         Zx = (Zx-1 + Zx)/2
       ╱   MEASUREMENT TIMING ?     ╲ ──────────┐          ⌐SA28
       ╲   SAMPLING PERIOD EXAMPLE: ╱           ▼
        ◇       Ts = 0.5s          ◇   ┌──────────────────────────┐
SA24 ⌐          │ YES                  │ PERFORM MEASURED IMPEDANCE (Zx) │
       ▼                               │      DATA SMOOTHING PROCESS     │
┌──────────────────────────┐          │  (e.g. MOVING AVERAGE PROCESS)  │
│  PERFORM TRUNK IMPEDANCE (Ztm) │     └──────────────────────────┘
│  MEASURING ELECTRODE PLACEMENT │              │         ⌐SA29
│       SETTING PROCESS          │              ▼
│           ↓                    │     ┌──────────────────────────┐
│  PERFORM TRUNK IMPEDANCE (Ztmx)│     │   PERFORM TRUNK IMPEDANCE      │
│     MEASUREMENT PROCESS        │     │  MEASURED DATA BREATHING       │
└──────────────────────────┘          │ CHANGE CORRECTION PROCESS      │
SA25 ⌐          │                      └──────────────────────────┘
       ▼                                        │         ⌐SA30
┌──────────────────────────┐                    ▼
│  PERFORM SUBCUTANEOUS FAT      │     ┌──────────────────────────┐
│  TISSUE LAYER IMPEDANCE (ZFS1) │     │  PERFORM TIME-SERIES STABILITY │
│ MEASURING ELECTRODE PLACEMENT  │     │  CONFIRMATION PROCESS OF       │
│ SETTING PROCESS (SIDE OF NAVEL)│     │    MEASURED IMPEDANCE          │
│           ↓                    │     └──────────────────────────┘
│  PERFORM SUBCUTANEOUS FAT      │                │
│ TISSUE LAYER IMPEDANCE (ZFS1x) │  NO    ◇────────────────◇  SA31
│     MEASUREMENT PROCESS        │ ◄──── ╱  Ztmx, ZFS1x, ZFS2x, ╲
└──────────────────────────┘     │      ╲  ZFS3x SATISFY STABLE  ╱
SA26 ⌐          │                 │       ◇   CONDITION ?       ◇
       ▼                          │           │ YES          SA32
┌──────────────────────────┐     │           ▼
│  PERFORM SUBCUTANEOUS FAT      │ │  ┌──────────────────────────┐
│  TISSUE LAYER IMPEDANCE (ZFS2) │ │  │   REGISTER FINAL STABLE        │
│ MEASURING ELECTRODE PLACEMENT  │ │  │ CONDITION DETERMINED VALUE     │
│ SETTING PROCESS (APONEUROSIS)  │ │  │ AS MEASUREMENT RESULT VALUE    │
│           ↓                    │ │  └──────────────────────────┘
│  PERFORM SUBCUTANEOUS FAT      │ │           │         ⌐SA33
│ TISSUE LAYER IMPEDANCE (ZFS2x) │ │           ▼
│     MEASUREMENT PROCESS        │ │  ┌──────────────────────────┐
└──────────────────────────┘     │  │  PERFORM ABNORMAL VALUE        │
SA27 ⌐          │                 │  │  DETERMINATION PROCESS         │
       ▼                          │  │ BY DRINKING AND EATING AND     │
┌──────────────────────────┐     │  │    RETENTION OF URINE          │
│  PERFORM SUBCUTANEOUS FAT      │ │  │      IN BLADDER                │
│  TISSUE LAYER IMPEDANCE (ZFS3) │ │  └──────────────────────────┘
│ MEASURING ELECTRODE PLACEMENT  │ │           │         ⌐SA34
│SETTING PROCESS (SIDE APONEUROSIS)│ │          ▼
│           ↓                    │ │  ┌──────────────────────────┐
│  PERFORM SUBCUTANEOUS FAT      │ │  │    PERFORM MEASUREMENT         │
│ TISSUE LAYER IMPEDANCE (ZFS3x) │ │  │ COMPLETION REPORTING PROCESS   │
│     MEASUREMENT PROCESS        │ │  └──────────────────────────┘
└──────────────────────────┘     │           │
       │                          │           ▼
       └──────────────────────────┘     ( RETURN )
```

# FIG.88

EP 1 741 385 B1

# FIG.89

# FIG.90

# FIG.91

414a 414b    C    413a 413b

411

406 : IMPEDANCE MEASURING SECTION

| VOLTAGE MEASURING ELECTRODE SELECTING SECTION | CURRENT APPLYING ELECTRODE SELECTING SECTION |

420b    420a

DIFFERENCE AMPLIFIER ～423

BANDPASS FILTER (BPF) ～424

412 CURRENT SOURCE

DETECTING SECTION ～425

AMPLIFIER ～426

A/D CONVERTER ～427

404

STORAGE SECTION

COMPUTATION/CONTROL SECTION

421

ALARMING BUZZER

POWER SOURCE ～418

422

| OPERATION SECTION | DISPLAY SECTION |

405    451    452

# FIG.92

414b 414d···414n 413b
414a 414c C 413a 413c 413d···413n

411

406

VOLTAGE MEASURING
ELECTRODE SELECTING
SECTION

CURRENT APPLYING
ELECTRODE SELECTING
SECTION

420b

420a

DIFFERENCE AMPLIFIER ~423

412
CURRENT
SOURCE

BANDPASS FILTER
(BPF) ~424

DETECTING SECTION ~425

AMPLIFIER ~426

A/D CONVERTER ~427

404

STORAGE
SECTION

COMPUTATION/CONTROL SECTION

POWER
SOURCE ~418

421

ALARMING
BUZZER

OPERATION
SECTION

DISPLAY
SECTION

422

405 451 452

# FIG.93

EP 1 741 385 B1

# FIG.94

# FIG.95

# FIG.96

413b

414b

540

C

# FIG.97

# FIG.98

# FIG.99

# FIG.100

FIG.101

FIG.102

FIG.103

FIG.104

FIG.105

FIG.106

# FIG.107

# FIG.108

# FIG.109

# FIG.110

# FIG.111

# FIG.112

```
    ( TRUNK IMPEDANCE (Zx) MEASUREMENT PROCESS )
SB23─┐                      │
     │ PERFORM INITIALIZATION OF COUNTER, ETC. │
```

SB24 — MEASUREMENT TIMING ? SAMPLING PERIOD EXAMPLE : TS = 0.5S  — NO — $Zx = (Zx_{-1} + Zx)/2$

SB26 — PERFORM MEASURED IMPEDANCE (Zx) DATA SMOOTHING PROCESS (e.g. MOVING AVERAGE PROCESS)

SB25 — YES — PERFORM TRUNK IMPEDANCE (Ztm) MEASURING ELECTRODE PLACEMENT SETTING PROCESS → PERFORM TRUNK IMPEDANCE (Ztmx) MEASUREMENT PROCESS

SB27 — PERFORM TRUNK IMPEDANCE MEASURED DATA BREATHING CHANGE CORRECTION PROCESS

SB28 — PERFORM TIME-SERIES STABILITY CONFIRMATION PROCESS OF MEASURED IMPEDANCE

SB29 — Ztmx SATISFY STABLE CONDITION ? — NO

SB30 — YES — REGISTER FINAL STABLE CONDITION DETERMINED VALUE AS MEASUREMENT RESULT VALUE

SB31 — PERFORM ABNORMAL VALUE DETERMINATION PROCESS BY DRINKING AND EATING AND RETENTION OF URINE IN BLADDER

SB32 — PERFORM MEASUREMENT COMPLETION REPORTING PROCESS

( RETURN )

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3396677 B
- JP 3396674 B
- WO 0224069 A1